# FASCICULE DE BREVET EUROPEEN

(11) **EP 2 470 514 B1**
(45) Date de publication et mention de la délivrance du brevet: **12.07.2017**
(21) Numéro de dépôt: 10763200.2
(22) Date de dépôt: 26.08.2010
(51) Int. Cl.: C07D 261/08, C07D 231/12, C07D 237/10, C07D 237/12, C07D 239/26, C07D 277/30, C07D 277/66, C07D 285/06, C07D 333/24, C07D 207/327, A61K 31/165, A61K 31/33, A61K 31/42, C12Q 1/37

(54) **PSEUDO-DIPEPTIDES COMME INHIBITEURS DE MMP**
PSEUDO-DIPEPTIDE ALS MMP HEMMER
PSEUDO-DIPEPTIDES AS MMP INHIBITORS

(30) Priorité: 26.08.2009 FR 0904061
(43) Date de publication de la demande: 04.07.2012
(73) Titulaire: Commissariat à l'Énergie Atomique et aux Énergies Alternatives, 75015 Paris (FR)
(72) Inventeur: DEVEL, Laurent, F-92240 Malakoff (FR); BEAU, Fabrice, F-91300 Massy (FR); CZARNY, Bertrand, F-92240 Malakoff (FR); DIVE, Vincent, F-91120 Palaiseau (FR)
(74) Mandataire: Gevers & Orès
(86) Numéro de dépôt international: PCT/FR2010/000581
(87) Numéro de publication internationale: WO 2011/023864

(56) Documents cités:
- WO-A1-2006/090235
- US-A1- 2003 129 672
- LAURENT DEVEL ET AL.: "Development of selective inhibitors and substrate of matrix metalloproteinase-12", JOURNAL OF BIOLOGICAL CHEMISTRY., vol. 281, no. 16, 21 avril 2006 (2006-04-21), pages 11152-11160, XP002581157, USTHE AMERICAN SOCIETY OF BIOLOGICAL CHEMISTS, INC., BALTIMORE, MD. ISSN: 0021-9258 cité dans la demande
- S. M. DIXON ET AL.: "Slow-binding human serine racemase inhibitors from high-throughput screening of combinatorial libraries", JOURNAL OF MEDICINAL CHEMISTRY., vol. 49, no. 8, 21 mars 2006 (2006-03-21), pages 2388-2397, XP002581158, USAMERICAN CHEMICAL SOCIETY. WASHINGTON. ISSN: 0022-2623
- ANNE-SOPHIE DABERT-GAY ET AL.: "Molecular determinants of matrix metalloproteinase-12 covalent modification by a photoaffinity prob", JOURNAL OF BIOLOGICAL CHEMISTRY, vol. 283, no. 45, 7 novembre 2008 (2008-11-07), pages 31058-31067, XP002615480, U.S.A. ISSN: 0021-9258
- ANDREAS FAUST ET AL.: "Synthesis and evaluation of a novel hydroxamate based fluorescent photoprobe for imaging of matrix metalloproteinases", BIOCONJUGATE CHEMISTRY., vol. 20, 17 avril 2009 (2009-04-17), pages 904-912, XP002615481, ACS, WASHINGTON, DC. ISSN: 1043-1802 cité dans la demande

### SEQUENCE LISTING

<110> COMMISSARIAT A L'ENERGIE ATOMIQUE ET AUX ENERGIES ALTERNATIVES DIVE, Vincent BEAU, Fabrice CZARNY, Bertrand DEVEL, Laurent
<120> Inhibiteurs de MMP
<130> F263-350PCT
<160> 2
<170> PatentIn version 3.5
<210> 1
   <211> 8
   <212> PRT
   <213> artificial sequence
<220>
   <223> séquence consensus du marqueur TAG
<220>
   <221> MISC_FEATURE
   <222> (1)..(1)
   <223> x quand il est présent comprend au moins deux amino-acides naturels ou non naturels
<220>
   <221> MISC_FEATURE
   <222> (2)..(2)
   <223> X consiste en n'importe quel amino acide naturel ou non naturel comprenant un groupement OH sur sa chaîne latérale
<220>
   <221> MISC_FEATURE
   <222> (3)..(3)
   <223> X consiste en n'importe quel amino-acide à l'exception de la cystéine
<220>
   <221> MISC_FEATURE
   <222> (4)..(4)
   <223> X consiste en un amino-acide choisi parmi : arginine, glycine, lysine
<220>
   <221> MISC_FEATURE
   <222> (5)..(5)
   <223> X consiste en au moins un amino-acide choisi parmi : alanine, glycine, lysine, arginine
<220>
   <221> MISC_FEATURE
   <222> (6)..(6)
   <223> X consiste en n'importe quel amino-acide à l'exception de la cystéine
<220>
   <221> MISC_FEATURE
   <222> (7)..(7)
   <223> X quand il est présent comprend la séquence peptidique RRMQYNRR dans laquelle au moins un des résidus est remplacé par un amino-acide naturel ou non naturel dans lequel la chaîne latérale présente dans le résidu initial qu'il remplace est absente
<220>
   <221> MISC_FEATURE
   <222> (8)..(8)
   <223> X quand il est présent comprend au moins deux amino-acides naturels ou non naturels
<400> 1
   Xaa Xaa Xaa Xaa Xaa Xaa Xaa Xaa
   1 5
<210> 2
   <211> 8.
   <212> PRT
   <213> artificial sequence
<220>
   <223> fragment de la séquence consensus du marqueur TAG
<400> 2
   Arg Arg Met Gln Tyr Asn Arg Arg
   1 5

## Description

L'invention concerne des dérivés pseudo-dipeptidiques et leurs utilisations en particulier en tant qu'inhibiteurs de métallo protéinases appartenant à la famille des zinc-métallo protéinases, les métallo protéinases de la matrice extra cellulaire ou MMP, ainsi que des dérivés pseudo-peptidiques marqués et leurs utilisations en tant qu'agents de contraste pour la détection des MMP sous forme active.

Elle concerne également une composition pharmaceutique comprenant ces dérivés.

Chez les humains, les métallo protéinases de la matrice extra cellulaire ou MMP représentent une famille de 23 membres. Tous ces membres sont très proches d'un point de vue structural et sont collectivement capables d'hydrolyser l'ensemble des composants protéiques de la matrice extra cellulaire (Brinckerhoff et al, 2002 Nat Rev Mol Cell Biol (1)).

Ainsi, cette famille de protéinases a été impliquée dans tous les procédés nécessitant un remodelage des tissus et des mouvements cellulaires associés (Page-McCaw et al, 2007 Nat Rev Mol Cell Biol (2)), qui sont les caractéristiques communes observées dans de nombreuses maladies humaines telles que le cancer.

Cependant, dans les dix dernières années, le spectre des protéines qui peuvent être hydrolysées par les MMP s'est largement étendu.

En fait, il apparaît maintenant que ces protéinases peuvent également hydrolyser des protéines qui n'appartiennent pas à la matrice extra cellulaire comme les chémokines ou les cytokines mais aussi certains récepteurs et facteurs de croissance pour n'en mentionner que quelques-uns (Egeblad et al. 2002 Nat Rev cancer (3)).

Ce large spectre d'activités a amené à considérer les MMP comme des cibles thérapeutiques dans une vaste gamme de pathologies humaines, (Fingleton et al., 2007 Curr Pharm Des (4), et Hu et al. 2007 Nat Drug Dis (5)).

Dans le passé, les inhibiteurs des MMP ont été principalement évalués dans le traitement des maladies cancéreuses (Overall et al., 2002 Nat Rev Cancer (6)).

Cependant, ces essais cliniques ont été décevants, principalement parce que les inhibiteurs sélectionnés pour cette application étaient non sélectifs vis-à-vis des MMP, c'est-à-dire qu'ils pouvaient bloquer toutes les MMP avec la même efficacité.

Or, à ce jour, les applications thérapeutiques pour les inhibiteurs des MMP sont principalement centrées sur des composés présentant un profil de sélectivité élevé, c'est-à-dire des inhibiteurs capables de bloquer seulement quelques MMP ou encore mieux une seule MMP.

Ces inhibiteurs sont appelés inhibiteurs de MMP hautement sélectifs.

En particulier, des inhibiteurs puissants et sélectifs de la MMP-12 ont été recherchés car cette MMP est considérée comme impliquée dans de nombreuses maladies inflammatoires, en particulier la maladie pulmonaire obstructive chronique (COPD).

On retrouve également la MMP-12 impliquée dans des pathologies humaines comme l'arthrite, l'arthrite rhumatoïde, l'athérosclérose et les ruptures d'anévrisme.

De plus, une augmentation de l'expression de la MMP-12 dans plusieurs cancers humains a également été reportée, suggérant une application thérapeutique possible pour les inhibiteurs de la MMP-12 dans certains cancers.

La MMP-12 est également appelée "macrophage élastase".

Des composés présentant un profil de sélectivité relativement bon en faveur de la MMP-12 ont été décrits, en particulier dans la demande internationale WO 2008/057254.

La structure chimique de ces composés est caractérisée par la présence d'un groupe alkylcarboxylate dont la fonction est d'interagir avec l'atome de zinc présent dans le site actif de toutes les MMP.

Un des composés décrits a la structure suivante :

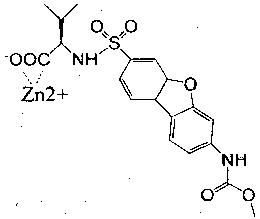

Devel et al. ont reporté le premier exemple d'un inhibiteur très puissant et très sélectif de la MMP-12, dans J. Biol. Chem. 2006 (7).

Ce composé a la formule suivante :

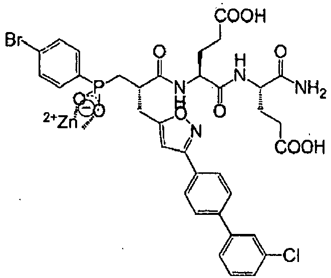

Ce composé, appelé ci-après RXP470, a une valeur de constante d'inhibition Ki de 0,4 nM pour la MMP-12 humaine et est de deux à trois ordres de grandeur moins puissant envers les MMP 1, 2, 3, 7, 8, 9, 11, 13 et 14.

A nouveau, la structure chimique de cet inhibiteur est caractérisée par la présence d'un groupe, ici phosphoryle, dont la fonction est d'interagir avec l'atome de zinc des sites actifs des MMP.

Cependant, la présence du groupe phosphoryle chargé négativement (PO₂⁻) dans ce type d'inhibiteurs limite leur passage de la barrière intestinale et empêche donc leur administration orale.

Dabert-Gay et al. ont également décrit des inhibiteurs de métallo protéinases de la matrice extracellulaire (MMP) porteur d'un groupe phosphoryle (Journal of Biology Chemistry, Vol. 283, No. 45, pp. 31058-31067).

On a alors recherché des inhibiteurs de MMP, et en particulier de la MMP-12, qui n'incorporent pas dans leurs structures de groupes chimiques capables d'interagir avec l'atome de zinc du site actif des MMP.

Les résultats les plus encourageants ont été obtenus pour la MMP-13 avec des composés qui ont la formule suivante :

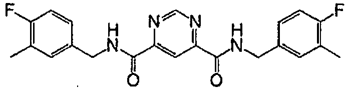

Cette nouvelle famille d'inhibiteurs exploite la capacité de ces composés à induire lors de leur liaison au site actif de la MMP-13 un changement conformationnel au niveau de la cavité profonde S_{1'}, située dans le site actif de la MMP-13.

Cependant, comme discuté par les auteurs (Engel et al. 2005 Chem Biol (8)), seule la cavité S_{1'} de la MMP-13 possède cette aptitude à changer de conformation suite à la liaison de certains inhibiteurs, une propriété expliquant la très grande sélectivité de ces inhibiteurs pour la MMP-13, n'interagissant que faiblement avec la MMP-12.

Ainsi, il existe dans l'art antérieur un besoin pour des inhibiteurs de MMP, et en particulier de la MMP-12, qui ne comportent pas de groupe liant le zinc.

Or, on a découvert que de façon surprenante, des composés dérivés du RXP470, mais n'incorporant pas de groupe phosphoryle substitué, ont une activité d'inhibition vis-à-vis des MMP et en particulier vis-à-vis de la MMP-12.

De plus, après modification et optimisation de leurs structures chimiques, certains de ces composés sont des inhibiteurs puissants et sélectifs de la MMP-12.

Ainsi, l'invention propose des composés de formule (1) suivante : 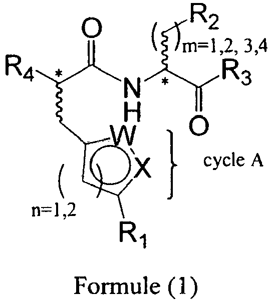 dans laquelle :
- n vaut 1 ou 2,
- lorsque n = 1 : W et X, indépendamment l'un de l'autre, sont O, N ou C,
- lorsque n = 2, W et X sont C,
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène, 3a,7a-dihydrobenzo[*d*]thiazole, 3-aminophényle, 3-hydroxyphényle, 3-nitrophényle, 3-carboxyphényle, 3-chlorophényle, 3,5-dichlorophényle, 3-méthoxyphényle, 3-hydroxyméthylphényle, ou un cycle thiophène substitué en positions, indépendamment l'une de l'autre, 2 et/ou 3 et/ou 4, par un groupe choisi parmi un groupe méthyle, phényle, 3a,7a-dihydrobenzo[*d*]thiazole ou un atome d'hydrogène.
- m est un entier compris entre 1 et 4 inclus, et
- lorsque m = 1, R₂ est un groupe acide carboxylique, ou 4-hydroxyphényle, ou *1H*-imidazole ou hydroxyle ou isopropyle ou méthyle,
- lorsque m = 2, R₂ est groupe acide carboxylique ou carboxamide,
- lorsque m = 3, R₂ est un groupe acide carboxylique,
- lorsque m = 4, R₂ est un groupe amino,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1) via une fonction amino, et
- R₄ est H ou un groupe carboxyméthyle -CH₂COOH, et de préférence R₄ est H,
et les diastéréoisomères et énantiomères de ceux-ci.

Dans un premier mode de réalisation, les composés de l'invention sont caractérisés en ce que dans la formule (1) W est O, X est N, et n = 1, formant un cycle A qui est un cycle isoxazole et en ce qu'ils ont la formule (1-A) suivante : 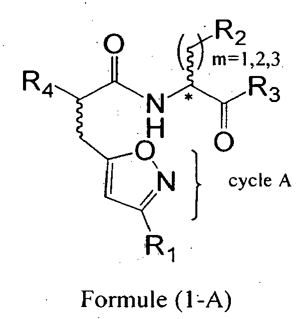 dans laquelle :
- R₁ est un groupe phényle, biphényle ou 3'-chlorobiphényle,
- m est un entier compris entre 1 et 3 inclus,
- R₂ est un groupe acide carboxylique lorsque m vaut 1 ou 3, ou lorsque m vaut 2, un groupe acide carboxylique ou un groupe carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-A) via une fonction amino, et
- R₄ est H ou un groupe carboxyméthyle -CH₂COOH,
et les diastéréoisomères et énantiomères de ceux-ci.

Dans ce premier mode de réalisation, les composés de l'invention sont de préférence choisis parmi les composés de formules (3) à (23) suivantes :

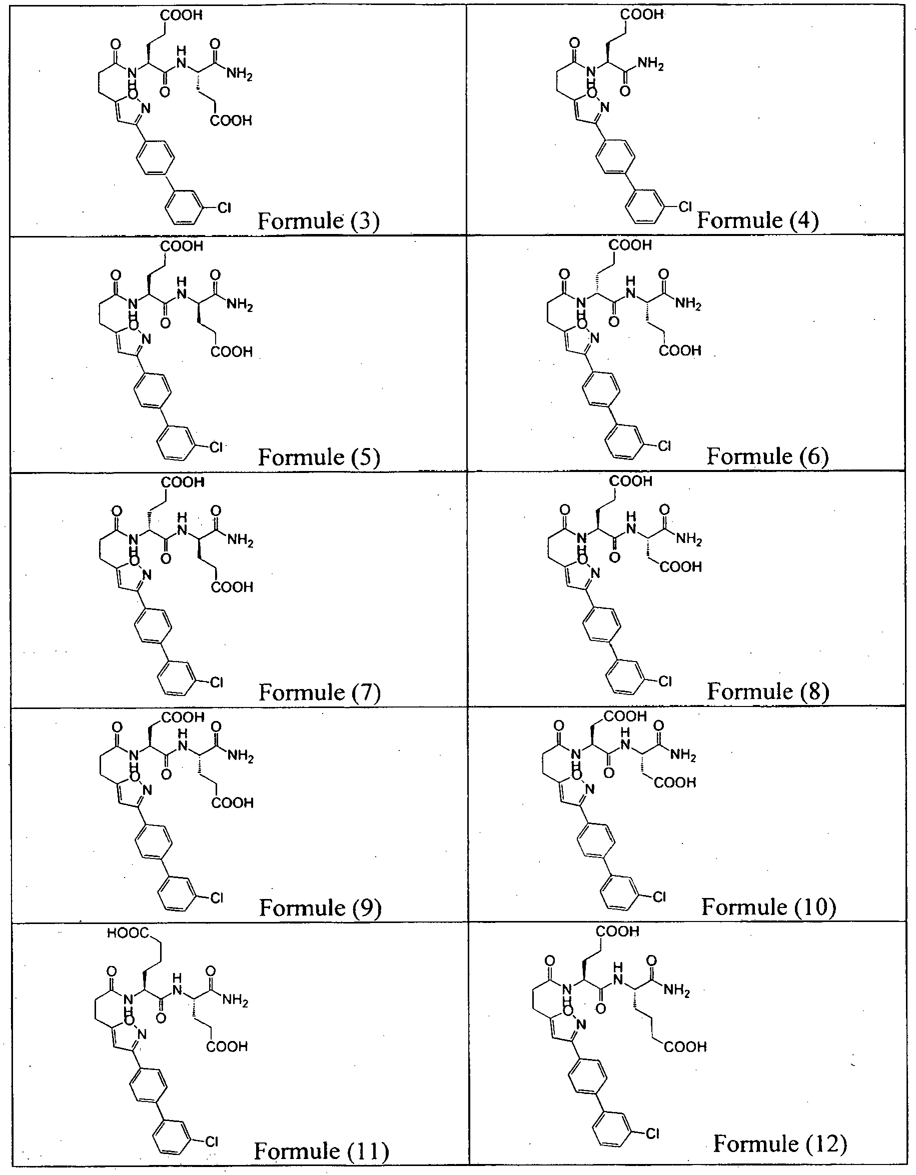

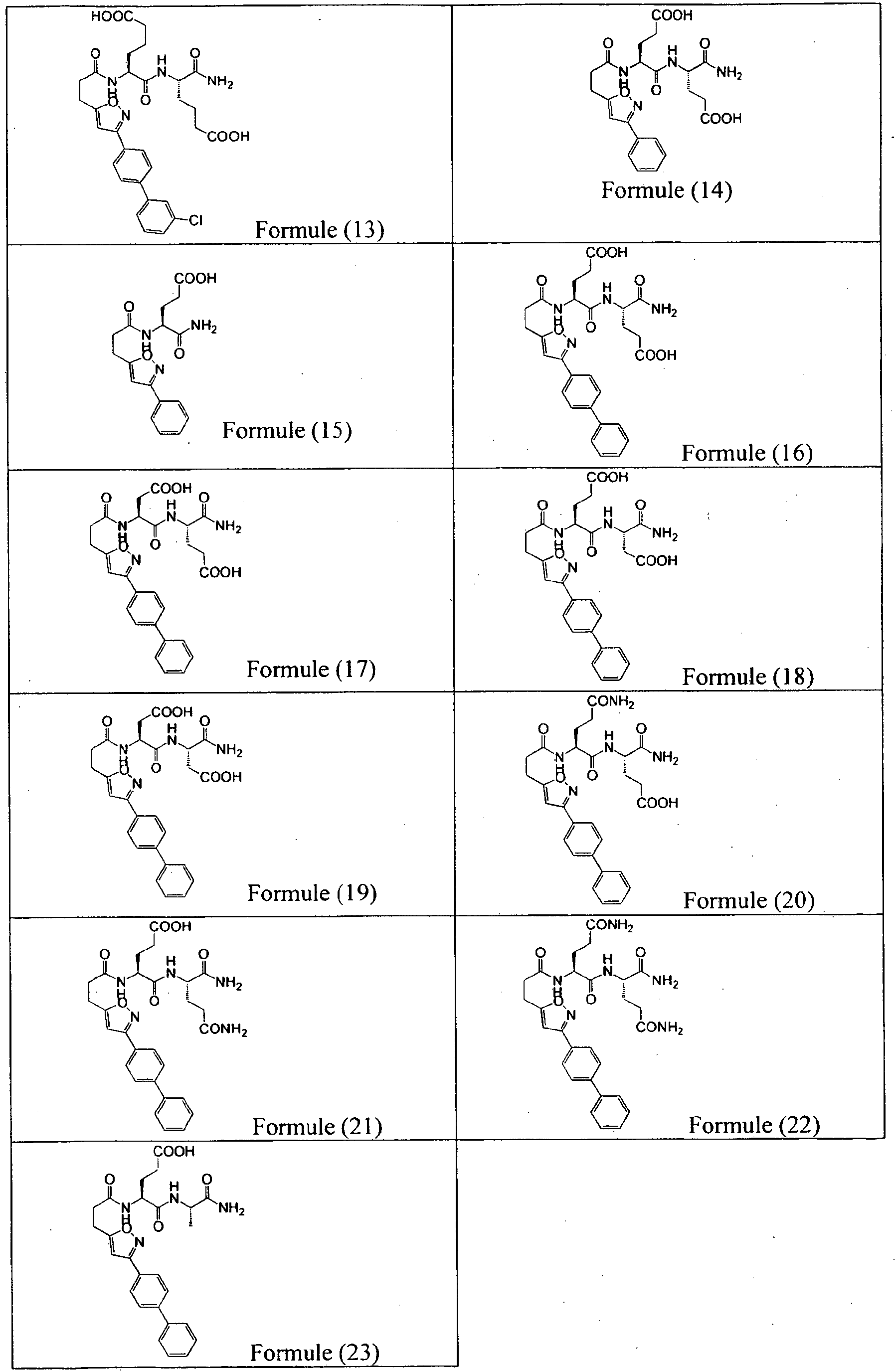

Dans un second mode de réalisation, les composés de l'invention sont caractérisés en ce que dans la formule (1) :
- n = 1,
- W est N,
- X est O,
- R₁ est un groupe phényle, biphényle ou 3'-chlorobiphényle,
- m = 1, 2 ou 3
- lorsque m = 1 ou 3, R₂ est un groupe acide carboxylique, et lorsque m vaut 2, R₂ est un groupe acide carboxylique ou carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1) via une fonction amino, et
- R₄ est H ou un groupe carboxyméthyle -CH₂COOH, et en ce qu'ils ont la formule (1-B) suivante : 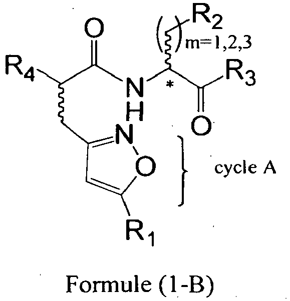
et les diastéréoisomères et énantiomères de ceux-ci.

Dans ce second mode de réalisation, le composé préféré de l'invention a la formule (25) suivante :

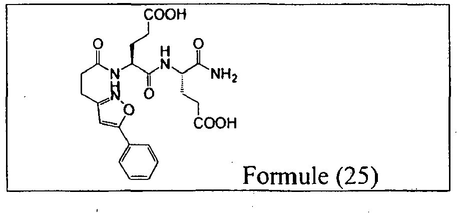

Dans un troisième mode de réalisation, les composés de l'invention sont caractérisés en ce que dans la formule (1), W et X sont C et n = 2, formant ainsi un cycle A qui est un cycle benzène et en ce qu'ils ont la formule (1-C) suivante : 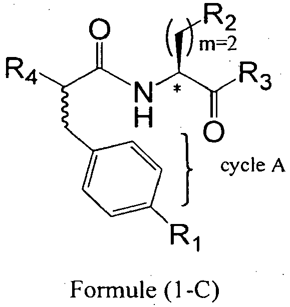 dans laquelle :
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène, et 3a,7a-dihydrobenzo[*d*]thiazole,
- m = 2, et
- R₂ est un groupe acide carboxylique, et
- R₃ est choisi parmi un groupe amino; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-C) via une fonction amino, et
- R₄ est H ou un groupe carboxyméthyle -CH₂COOH,
et les diastéréoisomères de ceux-ci.

Dans ces composés, le carbone asymétrique est de configuration (S).

Dans ce troisième mode de réalisation, les composés de l'invention sont de préférence choisis parmi les composés de formules (28) à (39) suivantes :

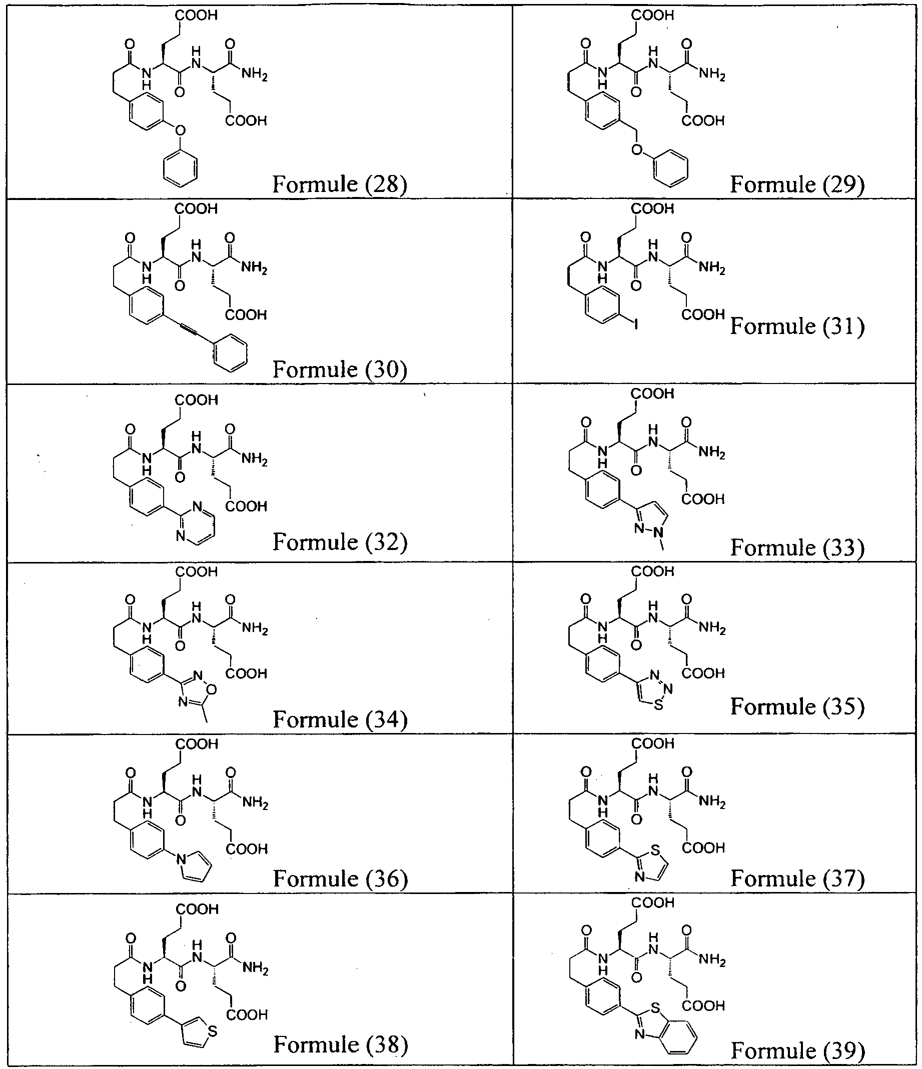

Dans un quatrième mode de réalisation, les composés de l'invention sont caractérisés en ce que dans la formule (1), W et X sont C et n = 2, formant ainsi un cycle A qui est un cycle benzène et en ce qu'ils ont la formule (1-D) suivante : 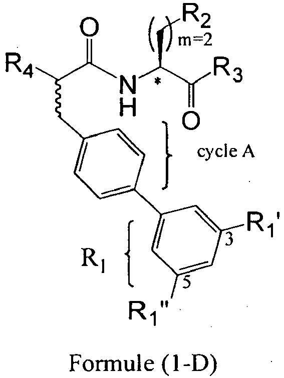 dans laquelle :
- R₁ est :
- soit un groupe phényle non substitué (R_{1'} = H et R_{1"} = H)
- soit un groupe phényle monosubstitué en position 3 par un groupe amino (R_{1'} = NH₂, R_{1"} = H) ou par un groupe hydroxyle (R_{1'} = OH, R_{1"} = H) ou par un groupe nitro (R_{1'} = NO₂, R_{1"} = H) ou par un groupe carboxyle (R_{1'} = COOH, R_{1"} = H) ou par un atome de chlore (R_{1'} = Cl, R_{1"} = H) ou par un groupe méthoxy (R_{1'} = OMe, R_{1"} = H) ou par un groupe hydroxyméthyle (R_{1'} = CH₂OH, R_{1"} = H),
- soit un groupe phényle di-substitué en positions 3 et 5 par un atome de chlore (R_{1'} = Cl et R_{1"} = Cl),
- m = 2,
- R₂ est un groupe acide carboxylique, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-D) via une fonction amino, et
- R₄ est H ou un groupe carboxyméthyle -CH₂COOH,
et les diastéréoisomères de ceux-ci.

Dans ce quatrième mode de réalisation, les composés de l'invention sont de préférence choisis parmi les composés de formules (40) et (42) à (60) suivantes :

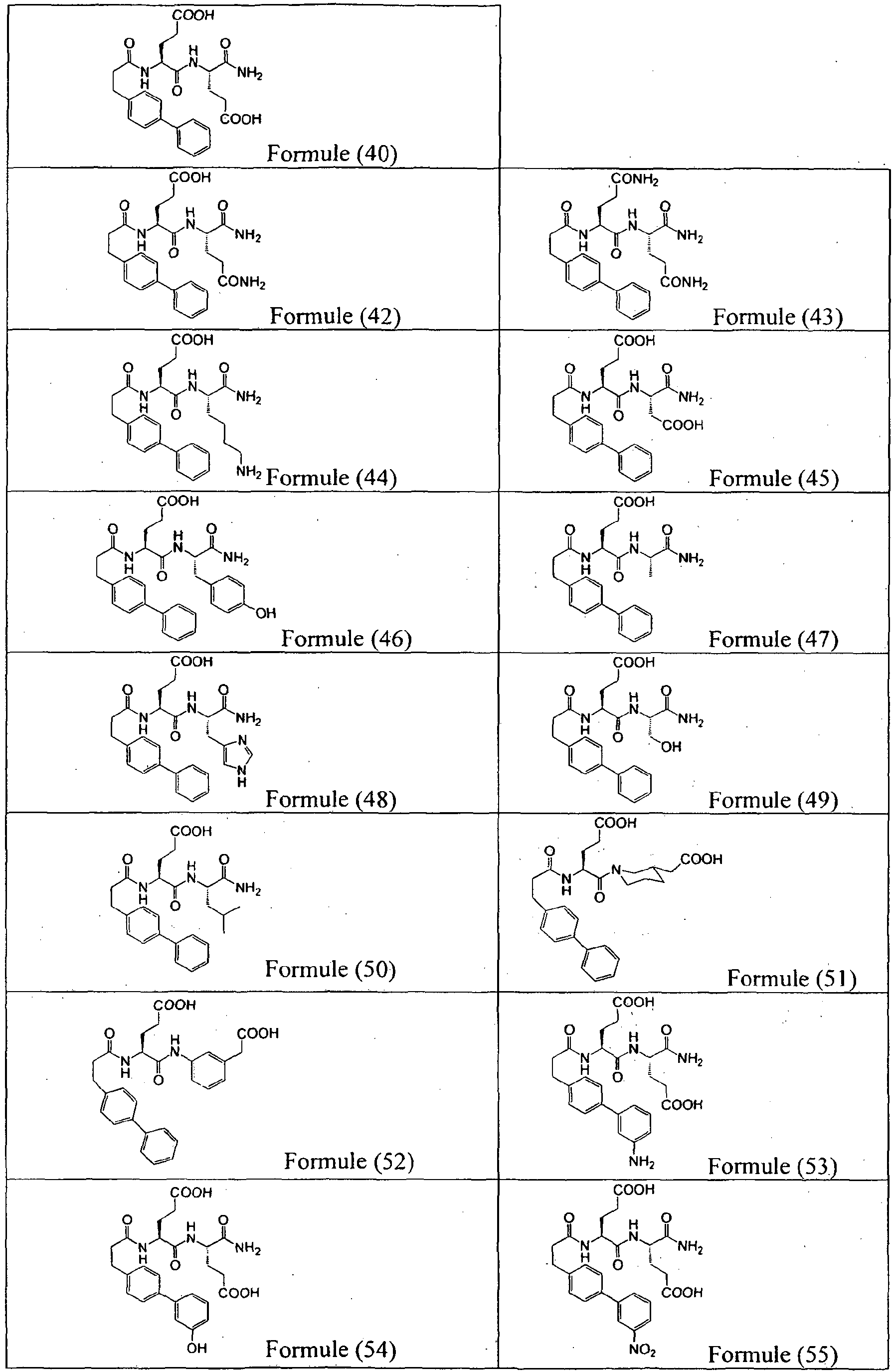

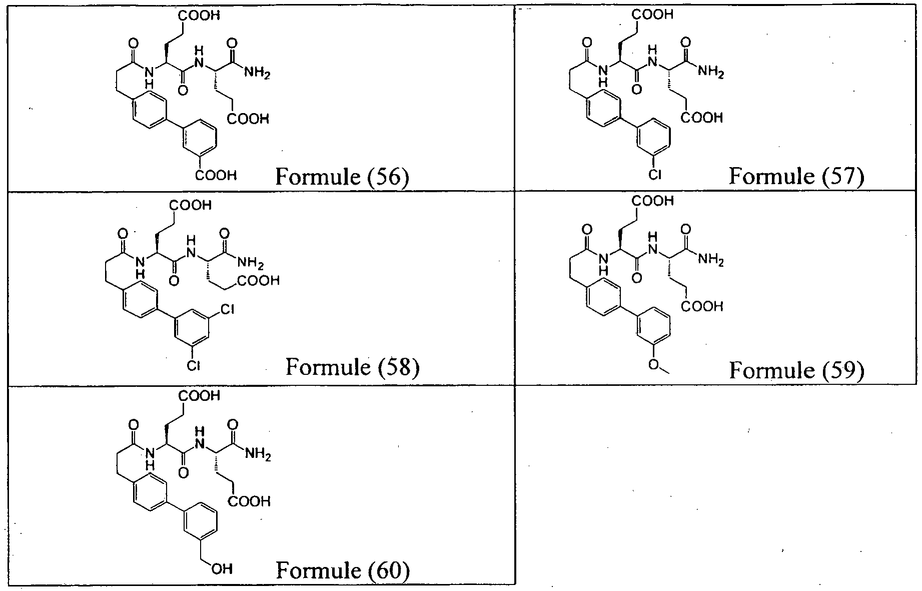

Dans un cinquième mode de réalisation, les composés de l'invention sont caractérisés en ce que dans la formule (1), W et X sont C, n = 2 et R₁ est un groupe biphényle et en ce qu'ils répondent à la formule (1-E) suivante : 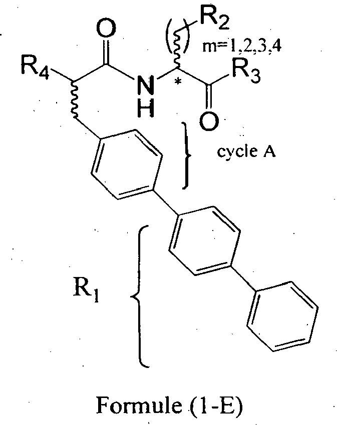 dans laquelle :
- m = 1, 2, 3 ou 4,
- lorsque m = 1, R₂ est un groupe acide carboxylique, 4-hydroxyphényle ou *1H*-imidazole ou hydroxyle ou un isopropyle ou méthyle,
- lorsque m = 2, R₂ est groupe acide carboxylique ou carboxamides,
- lorsque m = 3, R₂ est groupe acide carboxylique,
- lorsque m = 4, R₂ est un groupe amino,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-E) via une fonction amino, et
- R₄ est H ou un groupe carboxyméthyle -CH₂COOH,
et les diastéréoisomères et énantiomères de ceux-ci.

Dans ce cinquième mode de réalisation, les composés préférés de l'invention sont choisis parmi les composés de formules (61) à (79) suivantes :

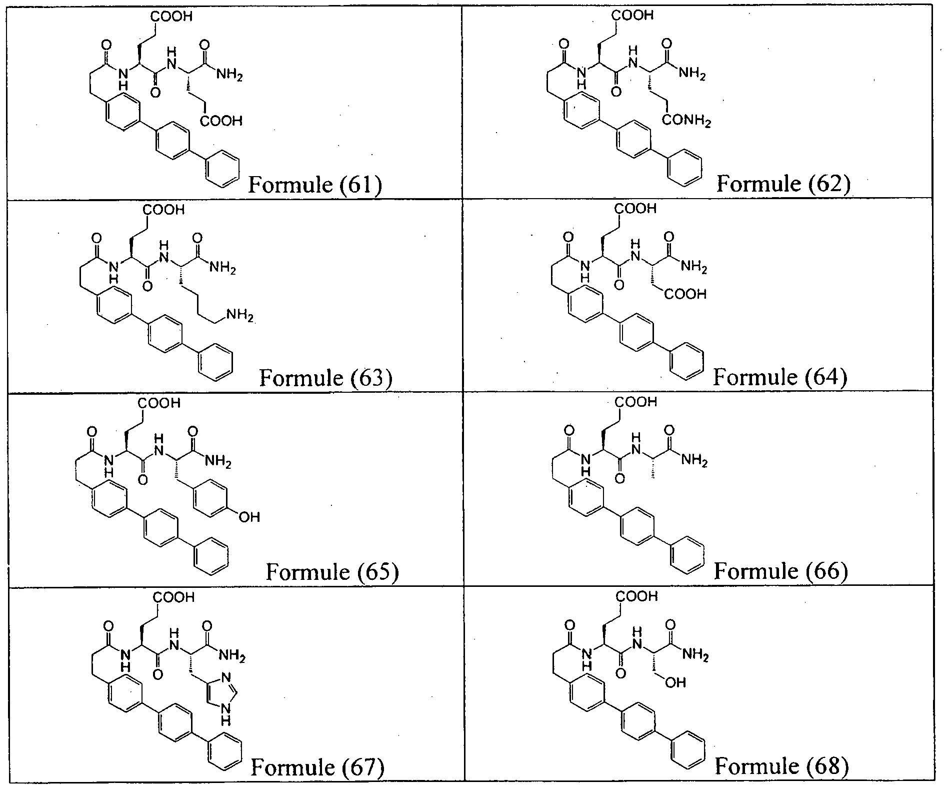

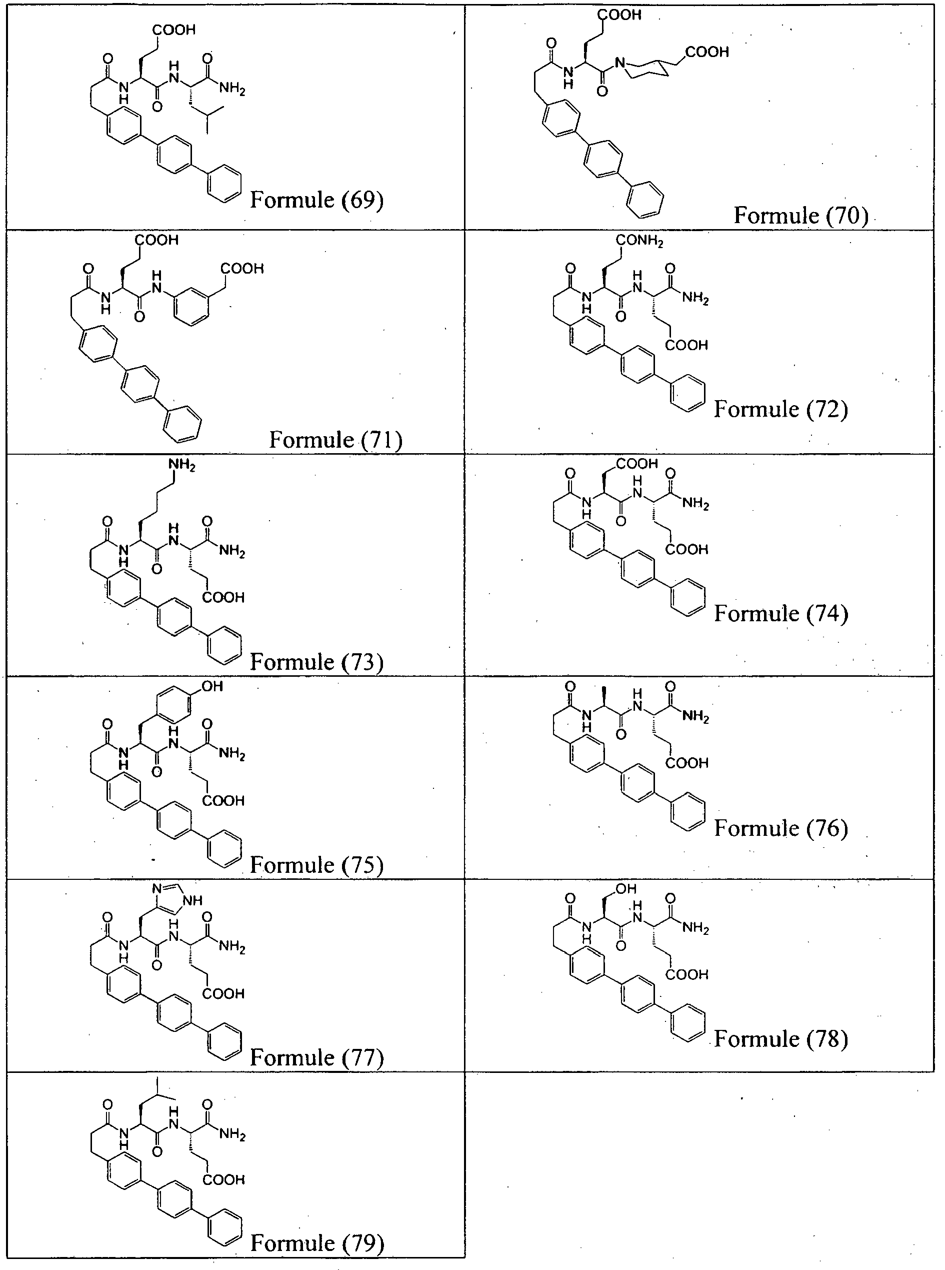

Dans un sixième mode de réalisation, les composés de l'invention sont caractérisés en ce que dans la formule (1), W et X sont C et n = 2, formant un cycle A qui est un cycle benzène, et R₁ est un cycle thiophène substitué par un groupe R_{1'''} et en ce qu'ils ont la formule (1-F) suivante : 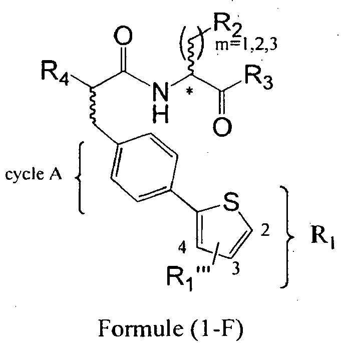 dans laquelle :
- R₁ est :
- soit un cycle thiophène non substitué (R_{1'''} = H),
- soit un cycle thiophène monosubstitué en position 2 par un groupe choisi parmi un groupe méthyle (R_{1'''} = CH₃), phényle (R_{1'''} = Ph), 3a,7a-dihydrobenzo[*d*]thiazole,
- soit un cycle thiophène monosubstitué en position 3 par un groupe choisi parmi un groupe méthyle (R_{1'''} = CH₃) ou phényle (R_{1'''} = Ph),
- soit un cycle thiophène monosubstitué en position 4 par un groupe méthyle (R_{1'''} = CH₃).
- m = 1,2 ou 3, et
- R₂ est un groupe acide carboxylique ou imidazole lorsque m = 1, ou un groupe acide carboxylique ou carboxamide lorsque m = 2, ou un groupe acide carboxylique lorsque m = 3,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F) via une fonction amino, et
- R₄ est H ou un groupe carboxyméthyle -CH₂COOH,
et les diastéréoisomères et énantiomères de ceux-ci.

Dans ce sixième mode de réalisation, les composés de l'invention et sont de préférence choisis parmi les composés de formules (80) à (107) suivantes :

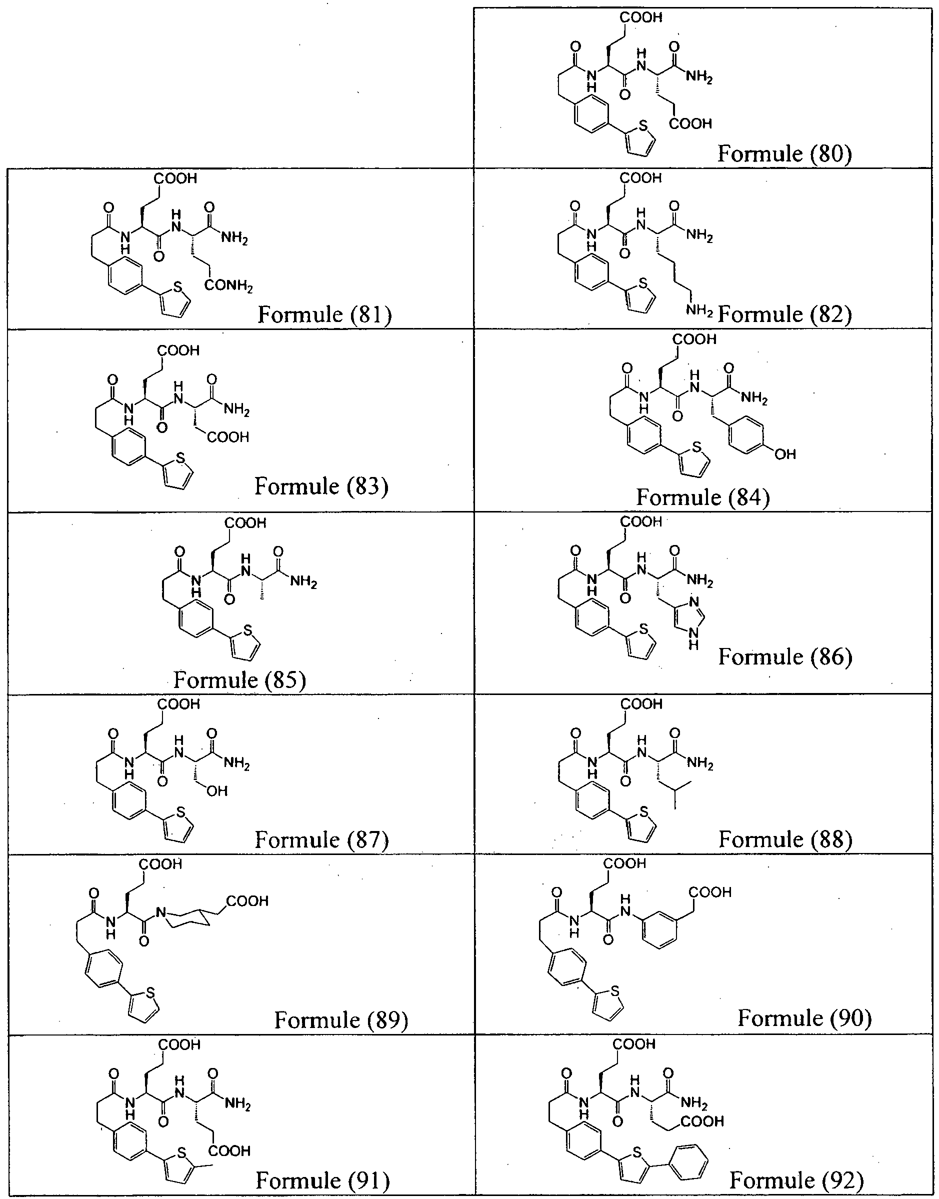

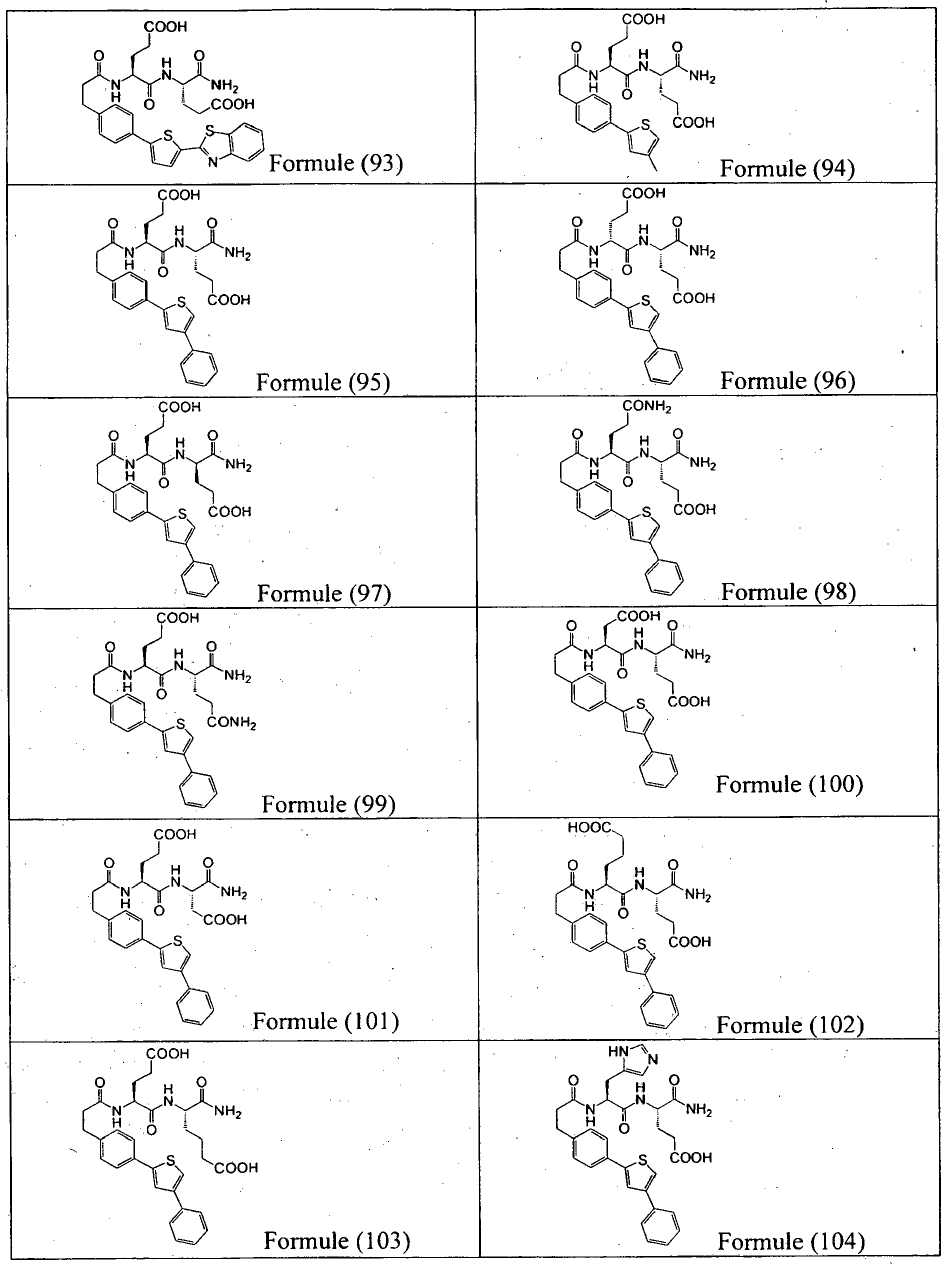

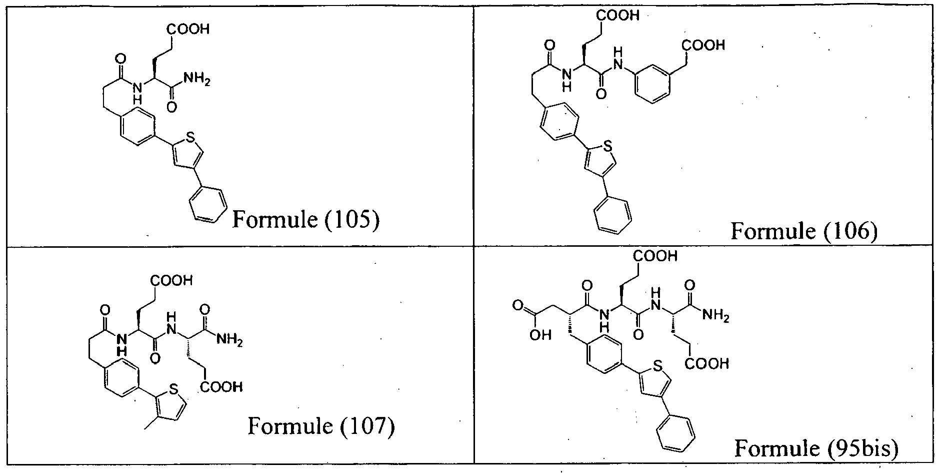

Dans ce sixième mode de réalisation, les composés plus préférés de l'invention sont choisis parmi les composés de formule (1-F) dans laquelle le cycle R₁ est un cycle thiophène monosubstitué soit en position 2 par groupe méthyle ou phényle, soit en position 3 par un groupe phényle.

Ces composés ont la formule (1-F1) suivante : 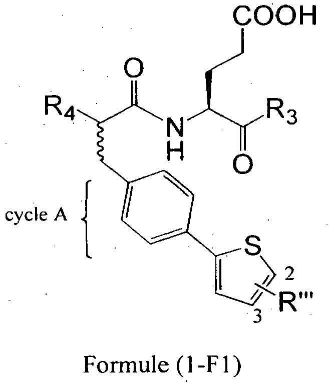 dans laquelle :
- R_{1'''} est soit en position 2 soit en position 3 du cycle thiophène et est choisi parmi un groupe méthyle (R_{1'''} = CH₃) ou phényle (R_{1'''} = Ph), et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F1) via une fonction amino, et
- R₄ est H ou un groupe carboxyméthyle -CH₂COOH,
et les diastéréoisomères de ceux-ci.

Les composés préférés de formule (1-F1) sont les composés de formules (91), (92), (95), (97), (99), (101), (103), (105), (106) et (95bis) suivantes :

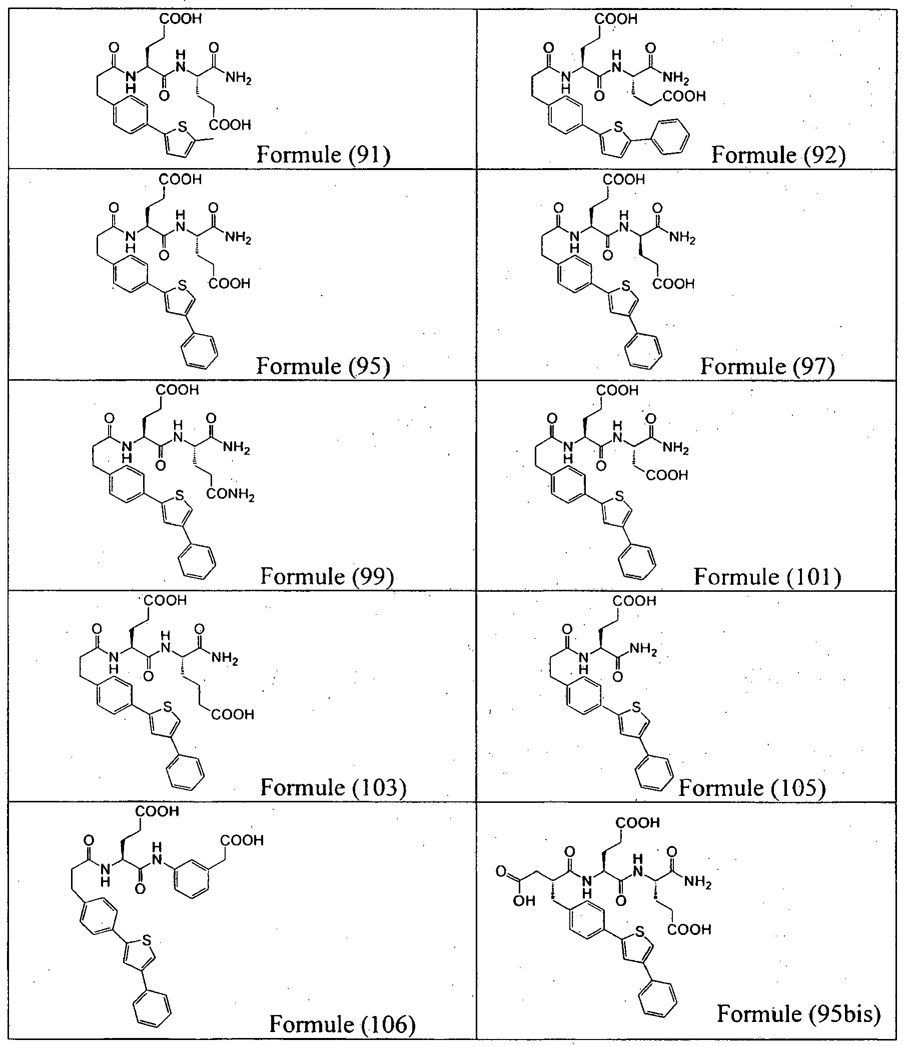

Mais les composés les plus préférés de l'invention sont les composés de formule (1-F2) dans laquelle R₁ est un cycle thiophène monosubstitué en position 3 par un cycle phényle, et qui répondent à la formule (1-F2) suivante : 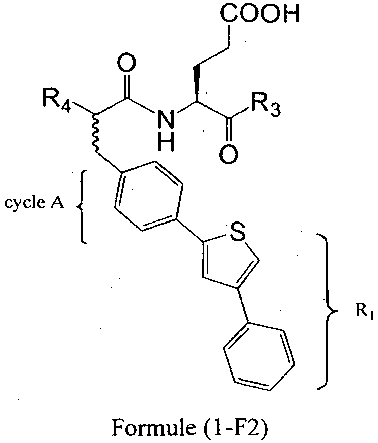 dans laquelle :
- R₃ est choisi parmi un groupe amino ; un groupe carboxyméthyle pipéridine ; un groupe carboxyméthyle 3-aminophényle ; un résidu glutamate de configuration L ou D, homoglutamate, aspartaté, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F2) via une fonction amino, et
- R₄ est H ou un groupe carboxyméthyle -CH₂COOH,
et en particulier les composés de formules (95), (97), (99), (101), (103), (105), (106) et (95bis) suivantes :

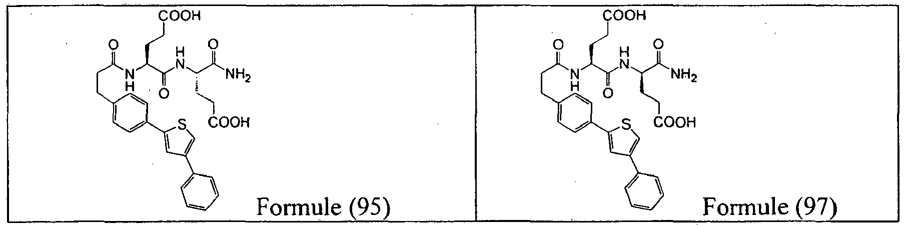

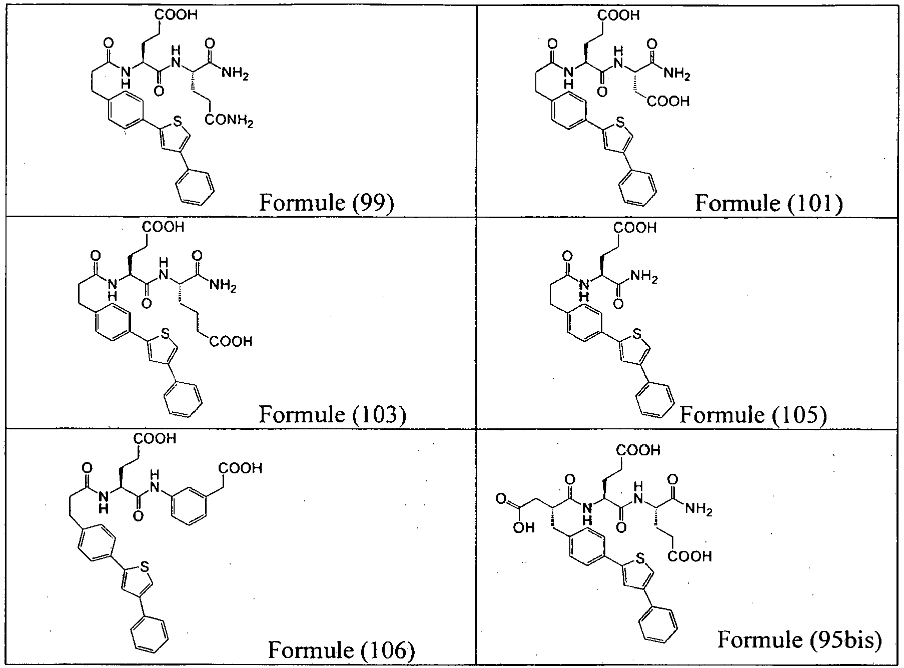

et les diastéréosimères de ceux-ci.

L'invention propose également les composés de l'invention et leurs énantiomères et diastéreoisomères pour une utilisation à titre de médicament.

L'invention propose encore les composés de l'invention et leurs énantiomères et diastéreoisomères pour une utilisation à titre d'inhibiteurs des métallo-protéinases de la matrice extracellulaire.

Plus particulièrement, l'invention propose les composés de formule (91), (92), (95), (97), (99), (101), (103), (105), (106) et (95bis) pour une utilisation à titre d'inhibiteurs de la métallo-protéinase de la matrice extracellulaire-12 (MMP-12), et plus particulièrement les composés (95), (97), (99), (101), (103), (105), (106) et (95bis).

L'invention propose de plus une composition pharmaceutique comprenant au moins un des composés de l'invention ou un de leurs énantiomères ou diastéreoisomères, et un excipient pharmaceutiquement acceptable.

L'invention propose enfin les composés de l'invention et leurs énantiomères et diastéreoisomères pour une utilisation à titre de médicament pour le traitement du cancer, des maladies inflammatoires, de la maladie pulmonaire obstructive chronique (COPD), de l'arthrite, de l'arthrite rhumatoïde, de l'athérosclérose et de la rupture d'anévrisme.

L'invention sera mieux comprise, et d'autres caractéristiques et avantages de celle-ci apparaîtront plus clairement, à la lecture de la description qui suit.

Les composés de l'invention ont la formule générale (1) suivante : 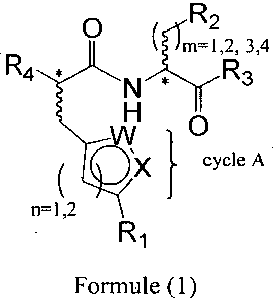 dans laquelle :
- n vaut 1 ou 2,
- lorsque n = 1 : W et X, indépendamment l'un de l'autre, sont O, N ou C,
- lorsque n = 2 : W et X sont C,
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène, 3a,7a-dihydrobenzo[*d*]thiazole, 3-aminophényle, 3-hydroxyphényle, 3-nitrophényle, 3-carboxyphényle, 3-chlorophényle, 3,5-dichlorophényle, 3-méthoxyphényle, 3-hydroxyméthylphényle, ou un cycle thiophène substitué en positions, indépendamment l'une de l'autre, 2 et/ou 3 et/ou 4, par un groupe choisi parmi un groupe méthyle, phényle, 3a,7a-dihydrobenzo[*d*]thiazole ou un atome d'hydrogène,
- m est un entier compris entre 1 et 4 inclus, et
- lorsque m = 1, R₂ est un groupe acide carboxylique, ou 4-hydroxyphényle, ou *1H*-imidazole ou hydroxyle ou isopropyle ou méthyle,
- lorsque m = 2, R₂ est groupe acide carboxylique ou carboxamide,
- lorsque m = 3, R₂ est un groupe acide carboxylique,
- lorsque m = 4, R₂ est un groupe amino,
- R₃ est choisi parmi un groupe amino; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1) via une fonction amino, et
- R₄ est H ou un groupe carboxyméthyle -CH₂COOH, et de préférence R₄ est H.

Plus précisément lorsque R₂ est un groupe acide carboxylique, c'est-à-dire un groupe - COOH, ce qui est possible lorsque m = 1, 2 ou 3, le groupe R₂ peut être en configuration (S) ou (R) lorsque m vaut 2.

De la même façon, lorsque R₃ est un résidu glutamate, ce résidu peut être en configuration L ou D.

Lorsque le groupement R₄ est un groupe carboxyméthyle -CH₂COOH, le carbone asymétrique (C*) porteur du groupement R₄ peut être en configuration (S) ou (R), et de préférence en configuration (S).

Ainsi, les diastéréoisomères et énantiomères des composés de formule (1) ci-dessus sont également un objet de l'invention.

Selon la nature du cycle A, dans la formule (1) différentes familles sont définies.

Dans la première famille, le cycle A est un cycle isoxazole, c'est-à-dire que W est O, X est N, et n = 1 et m vaut 1, 2 ou 3.

Les composés appartenant à cette première famille sont les composés de formule (1-A) suivante : 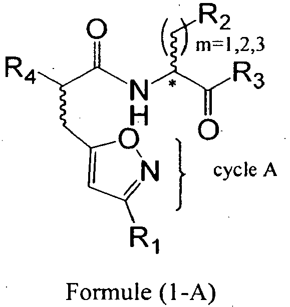 dans laquelle :
- R₁ est un groupe phényle, biphényle ou 3'-chlorobiphényle,
- m est un entier compris entre 1 et 3 inclus,
- R₂ est un groupe acide carboxylique lorsque m vaut 1 ou 3, ou lorsque m vaut 2, un groupe acide carboxylique ou un groupe carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-A) via une fonction amino, et
- R₄ est H ou un groupe carboxyméthyle -CH₂COOH,
et les diastéréoisomères et énantiomères de ceux-ci.

On note que dans cette première famille :
- lorsque m = 1, 2 ou 3 et que R₂ est un groupe COOH (acide carboxylique), R₂ forme avec le groupe NH et le groupe C(=O)-R₃ auxquels il est lié, un résidu, respectivement, aspartate, glutamate, et homoglutamate,
- lorsque m = 2 et que R₂ est un groupe carboxamide, R₂ forme avec le groupe NH et le groupe C(=O)-R₃ auxquels il est lié, un résidu glutamine,
- lorsque m = 1 et que R₂ est un groupe 4-hydroxyphényle, ou *1H-*imidazole ou hydroxyle ou isopropyle ou méthyle, R₂ forme avec le groupe NH et le groupe C(=O)-R₃ auxquels il est lié, respectivement, un résidu tyrosine, histidine, sérine, leucine, ou alanine,
- lorsque m = 4 et R₂ est un groupe amino, R₂ forme avec le groupe NH et le groupe C(=O)-R₃ auxquels il est lié un résidu lysine.

Les composés préférés de cette première famille sont les composés de formules (3) à (23) suivantes :

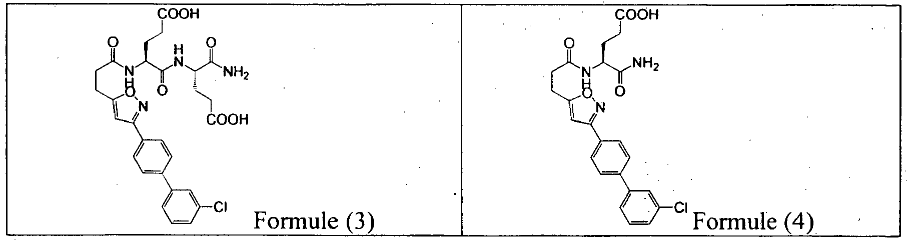

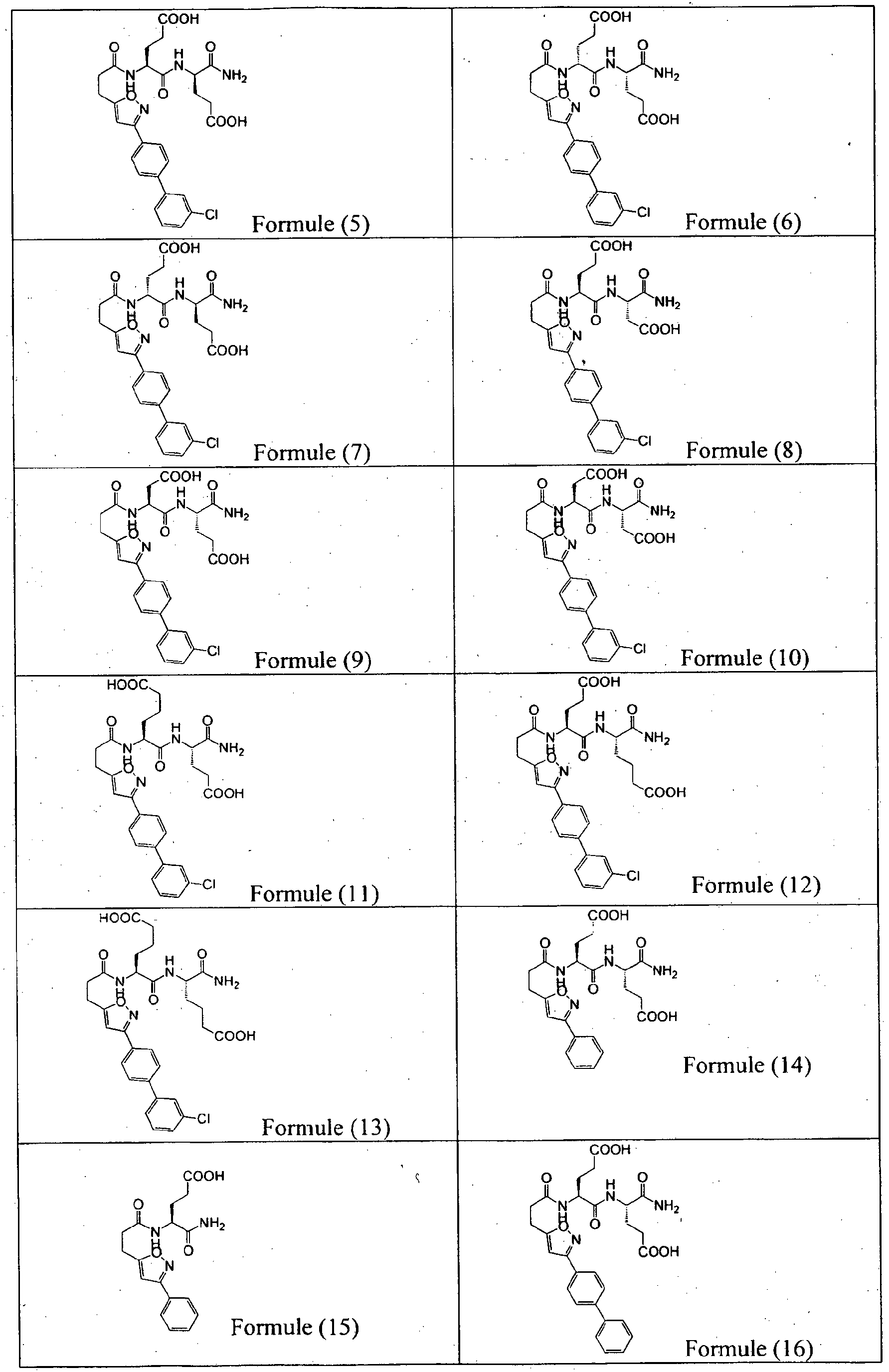

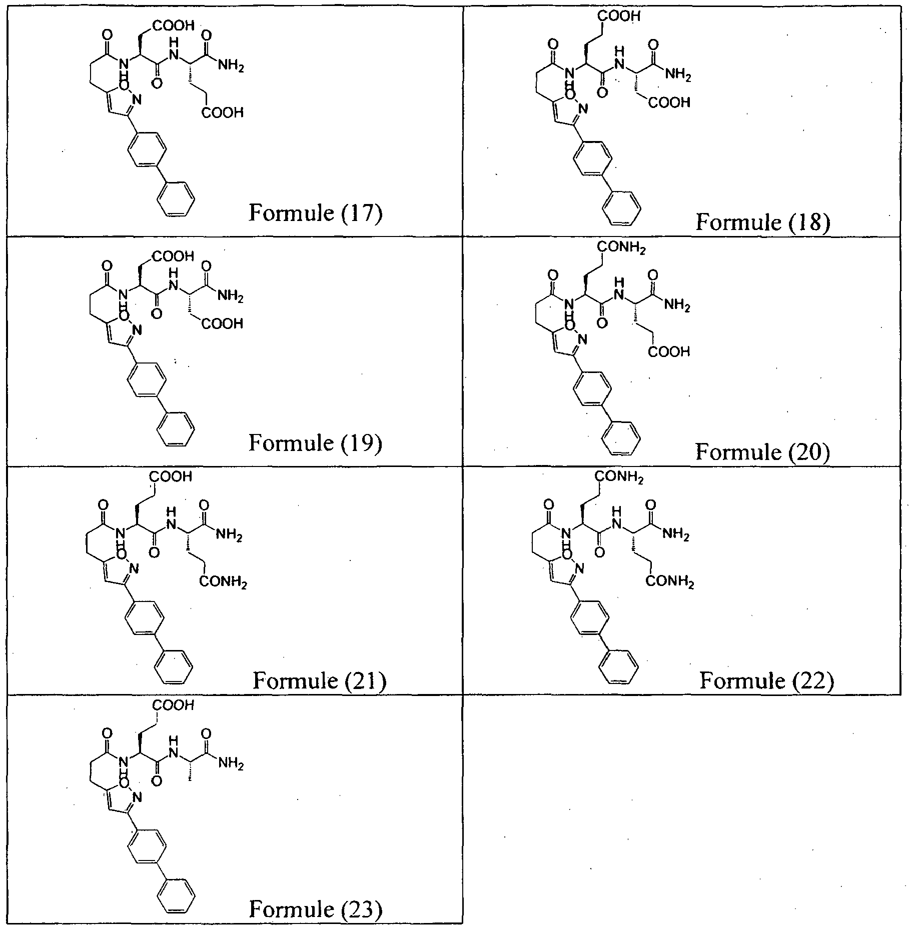

La constante d'inhibition Ki de ces composés a été déterminée selon le protocole décrit par Devel et al, 2006, J. Biol. Chem (7).

Les valeurs de Ki obtenues sont reportées au tableau 1.

On rappellera que plus le Ki d'un composé est faible, plus son potentiel d'inhibition vis-à-vis de la cible choisie est élevé.

Le composé (3) de cette première sous-famille correspond au RXP470 ayant subi une élimination du groupe phosphinique substitué (R-PO₂-CH₂).

En comparant le Ki du composé (3) et celui du composé RXP470 (qui est également reporté au tableau I) on constate que la sélectivité du composé (3) vis-à-vis des différentes MMP est plus faible que celle du composé RXP470, le composé (3) est en effet un inhibiteur assez puissant des MMP 2, 3, 10,12 et 13.

On constate également que le potentiel d'inhibition du composé (3) reste assez élevé vis-à-vis de la MMP-12. Ce composé (3) appartient donc à une nouvelle famille de composés qui, après optimisation de leur structure chimique, permettrait d'accéder à des inhibiteurs sélectifs de la MMP-12.

Ainsi, de façon surprenante, en éliminant la partie phosphinique dans le composé RXP470 et en faisant varier la nature des substituants R₁, R₂ et R₃, ainsi que leurs différentes configuration L ou D ou (S) ou (R), on obtient des inhibiteurs de la MMP-12.

Même plus, certains composés de cette série ont des puissances d'inhibition comparables envers trois MMP, les MMP-10, MMP-12 et MMP-13, faisant de ces inhibiteurs des principes actifs susceptibles d'être utilisés pour la fabrication d'un médicament pour traiter les pathologies dans lesquelles ces MMP sont surexprimées.

La seconde famille des composés de l'invention est celle dans laquelle le cycle A est un hétérocycle isoxazole avec W est N, X est O et n est égal à 1.

Ces composés ont la formule (1-B) suivante : 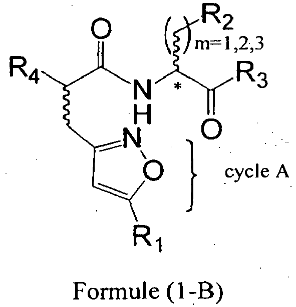 dans laquelle :
- n = 1,
- W est N,
- X est O,
- R₁ est un groupe phényle, biphényle ou 3'-chlorobiphényle
- m est un entier compris entre 1 et 3 inclus,
- lorsque m vaut 1 ou 3, R₂ est un groupe acide carboxylique, et lorsque m vaut 2, R₂ est un groupe acide carboxylique ou un groupe carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-B) via une fonction amino, et
- R₄ est H ou un groupe carboxyméthyle -CH₂COOH.

Les diastéréoisomères et énantiomères des composés de cette seconde famille font également partie de l'invention.

Le composé préféré de cette seconde famille des composés de l'invention est le composé de formule (25) suivante :

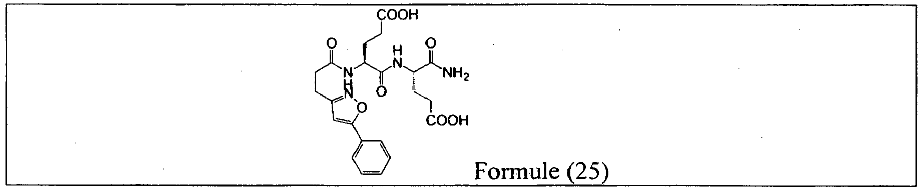

On a également synthétisé les composés de formules (24), (26) et (27) suivantes :

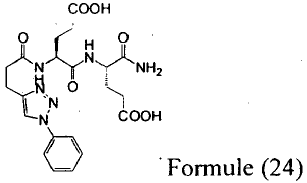

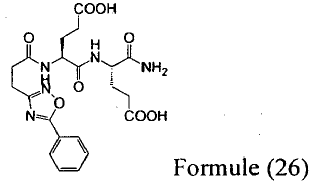

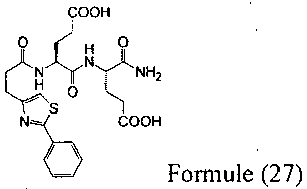

Dans ces composés, la nature des hétéroatomes et leur nombre dans le cycle A, qui est comme dans le composé de formule (25) un cycle à 5 atomes substitué en position 3 par un groupe phényle, ont été variés.

Les Ki de ces composés ont été déterminés et sont reportés au tableau I.

On voit à partir du tableau I que les composés de formules (24), (26) et (27) n'ont pas d'activité d'inhibition alors que les composés de la première famille et celui de formule (25) sont des inhibiteurs des MMP.

Cela montre que la nature du cycle A joue un rôle dans la puissance d'inhibition envers les MMP.

On a alors synthétisé d'autres composés dans lesquels le cycle A est un cycle benzène.

Ainsi, dans la troisième famille des composés de formule (1), le cycle A est un cycle benzène, c'est-à-dire que W et X sont C et n = 2.

De plus, dans ces composés, m = 2.

Ces composés ont la formule générale (1-C) suivante : 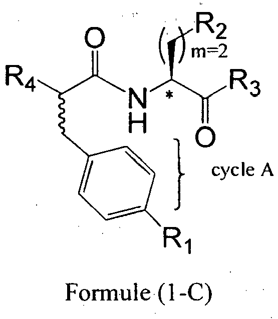 dans laquelle :
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène, 3a,7a-dihydrobenzo[*d*]thiazole,
- m = 2,
- R₂ est un groupe acide carboxylique, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartàte, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-C) via une fonction amino, et
- R₄ est H ou un groupe carboxyméthyle -CH₂COOH.

Les diastéréoisomères de ces composés font également partie de l'invention.

Les composés préférés de cette troisième famille ont les formules (28) à (39) suivantes :

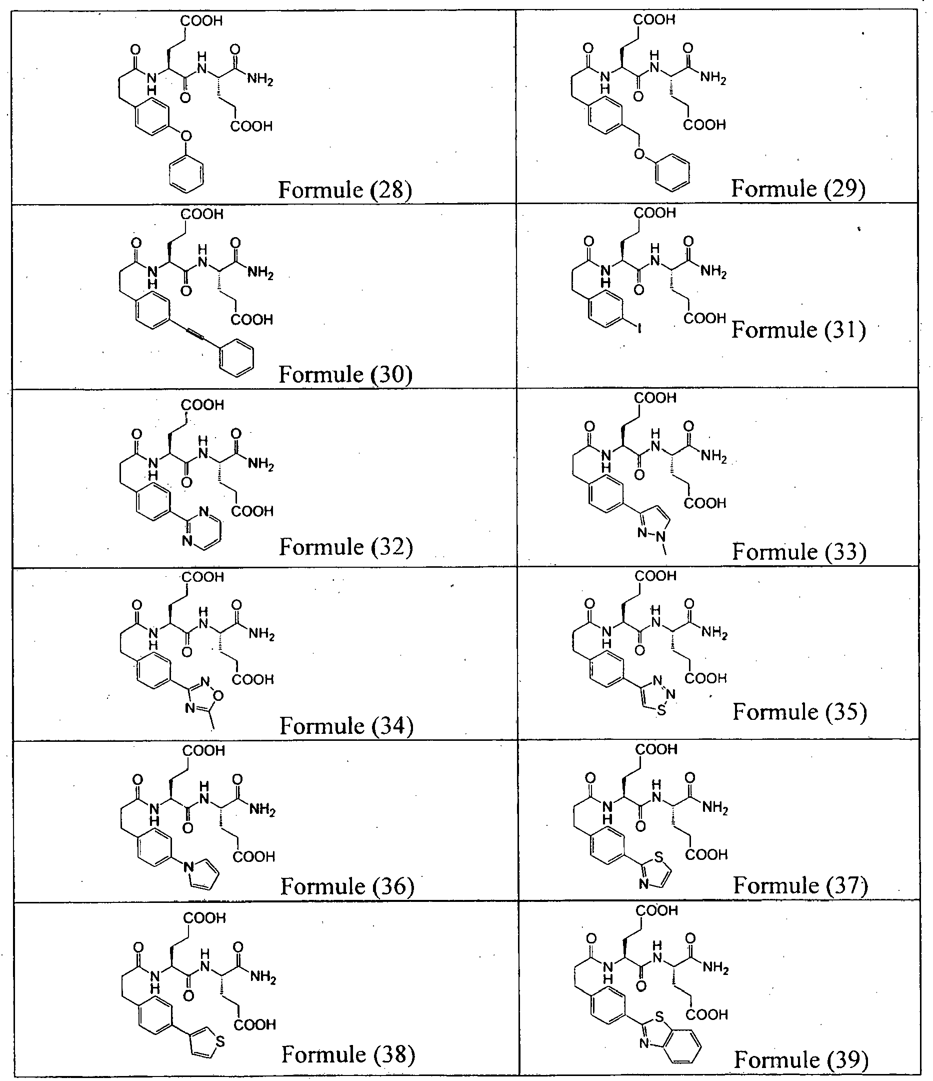

Les Ki de ces composés ont été déterminés et sont reportés au tableau I.

À partir des valeurs de Ki de ces composés, on constate que, dans certains cas, les constantes d'inhibition sont améliorées lorsque le cycle A est un cycle phényle.

On a alors synthétisé des composés dans lesquels le cycle A est un cycle phényle, éventuellement lui-même substitué.

La quatrième sous-famille des composés de formule (1) de l'invention est caractérisée par la présence d'un cycle A qui est un cycle benzène, c'est-à-dire que dans le cycle A, W et X sont C et n = 2 et par le fait que le groupe R₁ est un groupe phényle, éventuellement lui-même substitué.

Ces composés ont la formule générale (1-D) suivante : 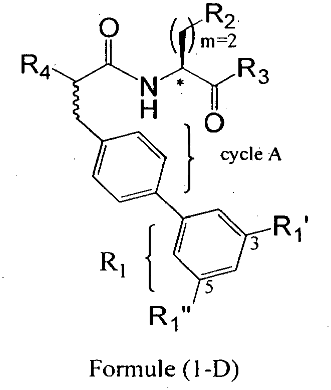 dans laquelle :
- R₁ est:
- soit un groupe phényle non substitué (R_{1'} = H et R_{1"} = H)
- soit un groupe phényle monosubstitué en position 3 par un groupe amino (R_{1'} = NH₂, R_{1"} = H) ou par un groupe hydroxyle (R_{1'} = OH, R_{1"} = H) ou par un groupe nitro (R_{1'} = NO₂, R_{1"} = H) ou par un groupe carboxyle (R_{1'} = COOH, R_{1"} = H) ou par un atome de chlore (R_{1'} = Cl, R_{1"} = H) ou par un groupe méthoxy (R_{1'} = OMe, R_{1"} = H) ou par un groupe hydroxyméthyle (R_{1'} = CH₂OH, R_{1"} = H),
- soit un groupe phényle di-substitué en positions 3 et 5 par un atome de chlore (R_{1'} = Cl et R_{1"} = Cl),
- m=2,
- R₂ est un groupe acide carboxylique, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-D) via une fonction amino, et
- R₄ est H ou un groupe carboxyméthyle -CH₂COOH.

Les diastéréoisomères de ces composés font également partie de l'invention.

Dans ces composés, le carbone asymétrique (C*) porteur du groupement -CH₂-CH₂-R₂ est de configuration (S).

Les composés préférés de cette quatrième famille ont les formules (40) et (42) à (60) suivantes :

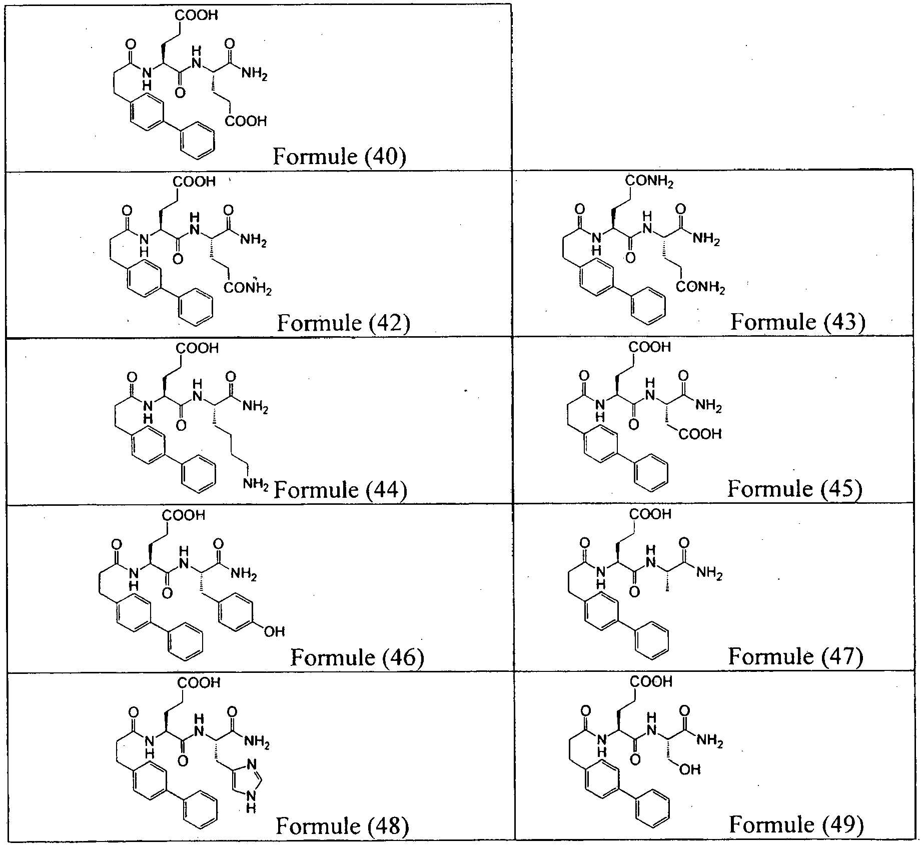

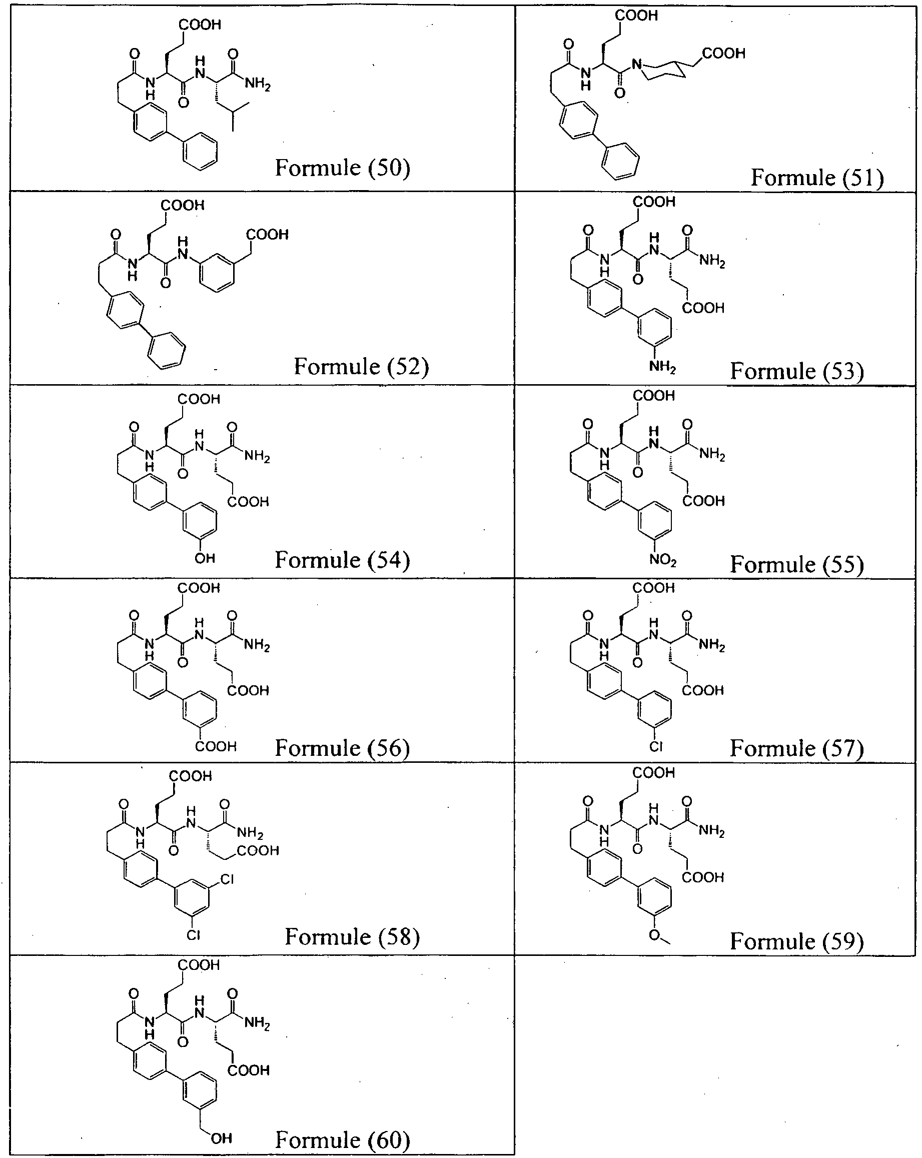

Les Ki de ces composés ont été mesurés et sont reportés au tableau I.

On voit à partir du tableau I que ces composés présentent une sélectivité améliorée vis-à-vis de la MMP-12 ou vis-à-vis des MMP-2 et 12 ou vis-à-vis des MMP-3 et 12.

Ces composés peuvent donc avantageusement être utilisés à titre de médicament pour le traitement des maladies dans lesquelles ces MMP sont surexprimées.

La cinquième famille de composés de l'invention est la famille des composés dans lesquels le cycle A est un cycle phényle substitué par un groupe bi-phényle, les substituants R₂, R₃ et R₄ étant variables.

Ces composés ont la formule (1-E) suivante : 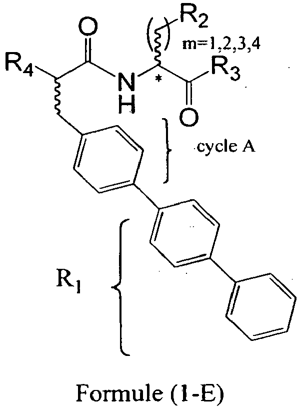 dans laquelle :
- m vaut 1, 2, 3 ou 4,
- lorsque m = 4, R₂ est un groupe amino,
- lorsque m = 1 ou 2 ou 3, R₂ est un groupe acide carboxylique,
- lorsque m = 2, R₂ est un groupe carboxamide,
- lorsque m = 1, R₂ est un groupe 4-hydroxyphényle ou un *1H*-imidazole ou hydroxyle ou isopropyle ou méthyle,
- R₃ est choisi parmi un groupe glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, ou un groupe carboxyméthyle pipéridine, un groupe carboxyméthyle 3-aminophényle ou un groupe amino, ledit groupement R₃ étant lié au groupement carbonyle de la formule (1-E) via une fonction amino, et
- R₄ est H ou un groupe carboxyméthyle -CH₂COOH.

Les diastéréoisomères et énantiomères de ces composés font également partie de l'invention.

Les composés préférés de cette famille sont les composés de formules (61) à (79) suivantes :

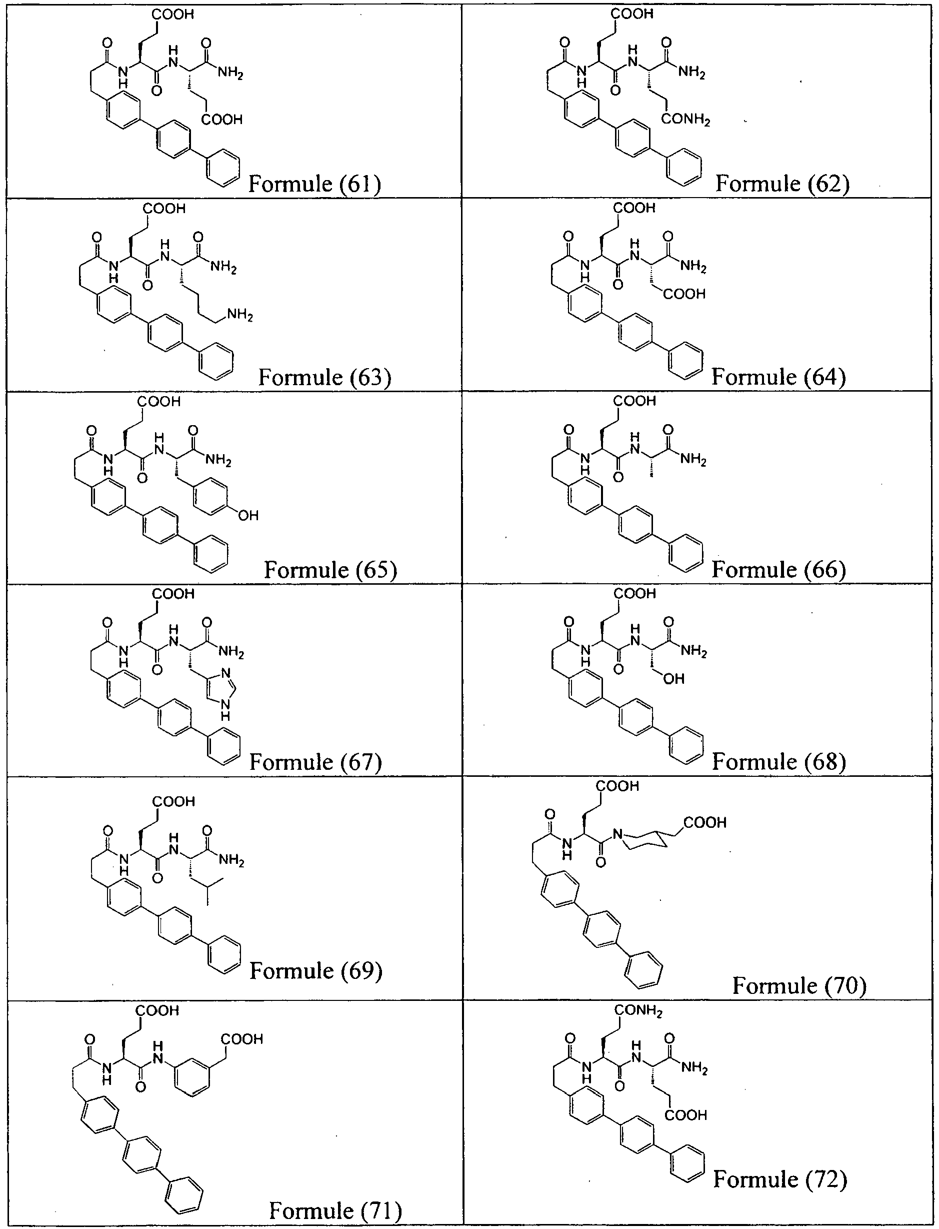

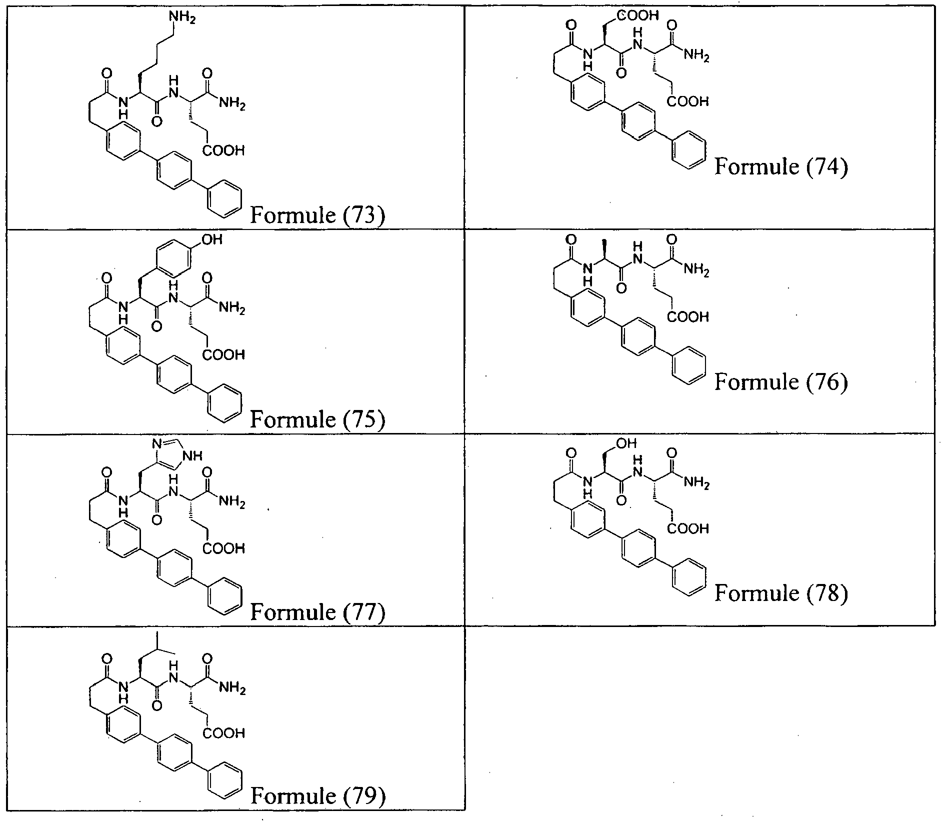

Comme l'indiquent les valeurs de Ki de ces composés, reportées au tableau I, ces composés se comportent essentiellement comme des inhibiteurs puissants de la MMP-12 et de la MMP-13. Cette série de composés a donc des applications en tant que principe actif pour la fabrication d'un médicament pour traiter des pathologies dans lesquelles les MMP-12 et MMP-13 sont surexprimées.

Cependant, en examinant les valeurs de Ki des composés appartenant à la troisième famille (1C), mais également celles des composés appartenant à la quatrième famille (1D) et à la cinquième famille (1E) de l'invention, on constate que la puissance et la sélectivité des composés vis-à-vis de la MMP-12 sont améliorées non seulement lorsque le cycle A est un cycle phényle, mais également lorsque celui-ci est substitué par un hétérocycle thiophène.

On a alors synthétisé des composés de formule (1) dans lesquels le cycle A est un cycle benzène, c'est-à-dire que W et X sont C et n = 2, et dans laquelle R₁ est un cycle thiophène non substitué ou substitué.

Ces composés ont la formule (1-F) suivante : 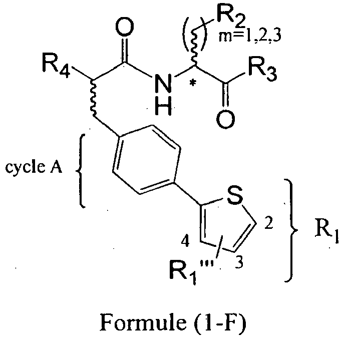 dans laquelle :
- R₁ est :
- soit un cycle thiophène non substitué (R_{1'''} = H),
- soit un cycle thiophène monosubstitué en position 2 par un groupe choisi parmi un groupe méthyle (R_{1'''} = CH₃), phényle (R_{1'''} = Ph) ou 3a,7a-dihydrobenzo[*d*]thiazole,
- soit un cycle thiophène monosubstitué en position 3 par un groupe choisi parmi un groupe méthyle (R_{1'''} = CH₃) ou phényle (R_{1'''} = Ph),
- soit un cycle thiophène monosubstitué en position 4 par un groupe méthyle (R_{1'''} = CH₃).
- m = 1,2 ou 3, et
- R₂ est un groupe acide carboxylique ou imidazole lorsque m = 1, ou un groupe acide carboxylique ou carboxamide lorsque m = 2, ou un groupe acide carboxylique lorsque m = 3, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F) via une fonction amino, et
- R₄ est H ou un groupe carboxyméthyle -CH₂COOH.

Les diastéréoisomères et énantiomères de ces composés font également partie de l'invention.

Les composés préférés de cette famille sont les composés de formules (80) à (107) suivantes :

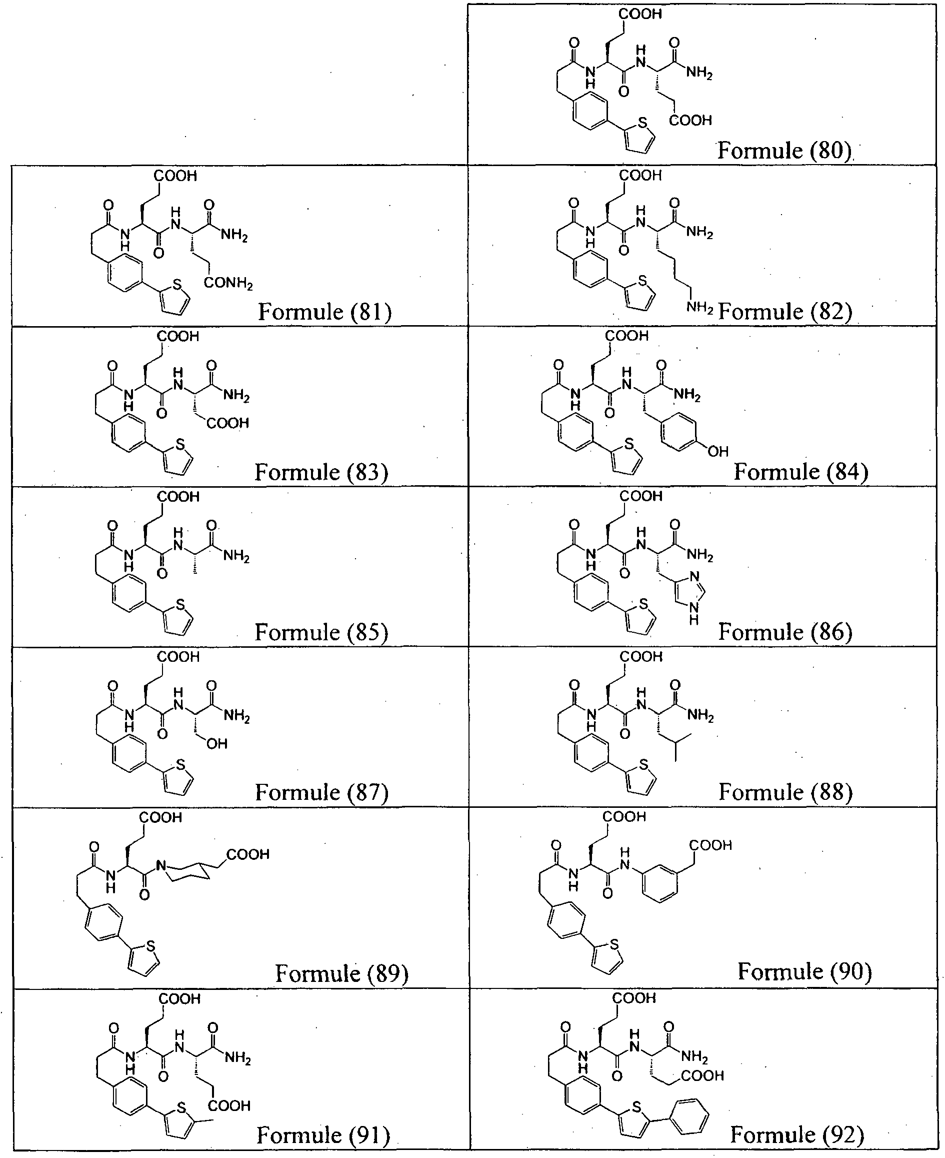

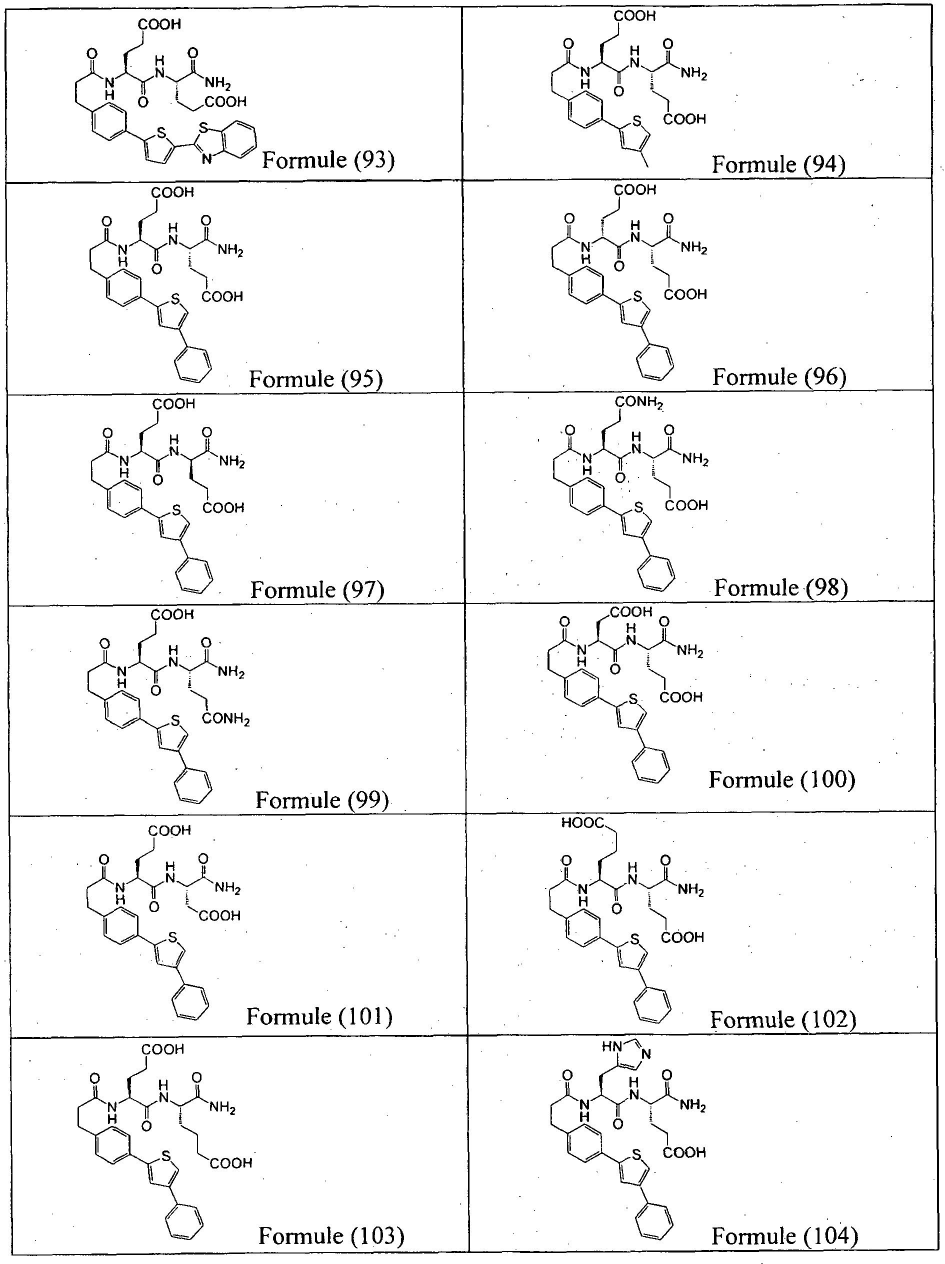

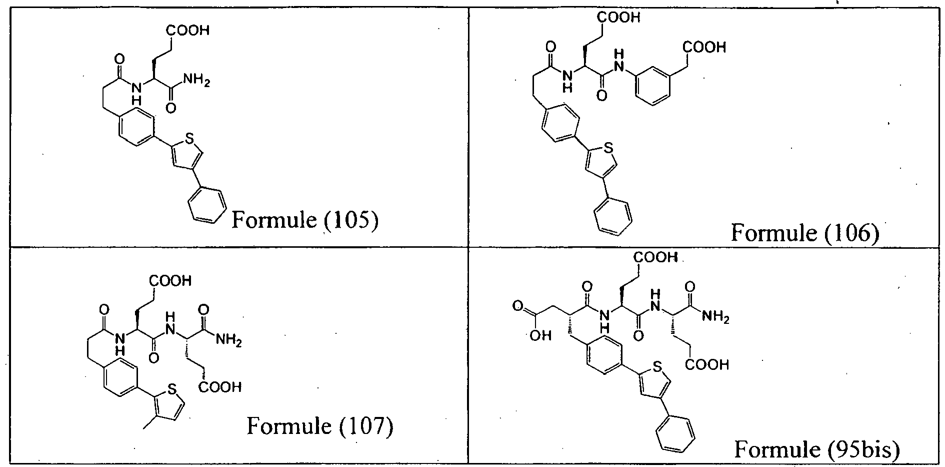

Les constantes d'inhibition de ces composés ont été déterminées et sont reportées au tableau I.

On voit à partir du tableau I que ces composés présentent une puissance d'inhibition élevée envers la MMP-12 et la MMP-8.

Ainsi, ces composés peuvent être utilisés avantageusement pour la fabrication de médicaments pour le traitement des pathologies dans lesquelles les MMP-12 et MMP-8 sont surexprimées.

Mais on constate surtout, à partir du tableau I que les composés ayant la formule (1-F) dans laquelle le cycle thiophène est substitué soit en position 2 soit en position 3 par un groupe méthyle (R_{1'''} = CH₃) ou phényle (R_{1'''} = Ph), sont des inhibiteurs parmi les plus puissants et les plus sélectifs de la MMP-12.

Dès lors les composés les plus préférés de l'invention sont les composés de formule (1-F1) suivante : 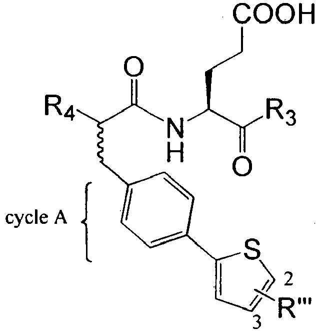 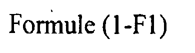 dans laquelle :
- R_{1'''} est soit en position 2 soit en position 3 du cycle thiophène et est choisi parmi un groupe méthyle (R_{1'''}=CH₃) ou phényle (R_{1'''}=Ph), et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F1) via une fonction amino, et
- R₄ est H ou un groupe carboxyméthyle -CH₂COOH.

Les diastéréoisomères de ces composés sont également un objet de l'invention.

Parmi les composés de formule (1-F1), les composés de formule de formules (91), (92), (95), (97), (99), (101), (103), (105), (106) et (95bis) suivantes sont préférés:

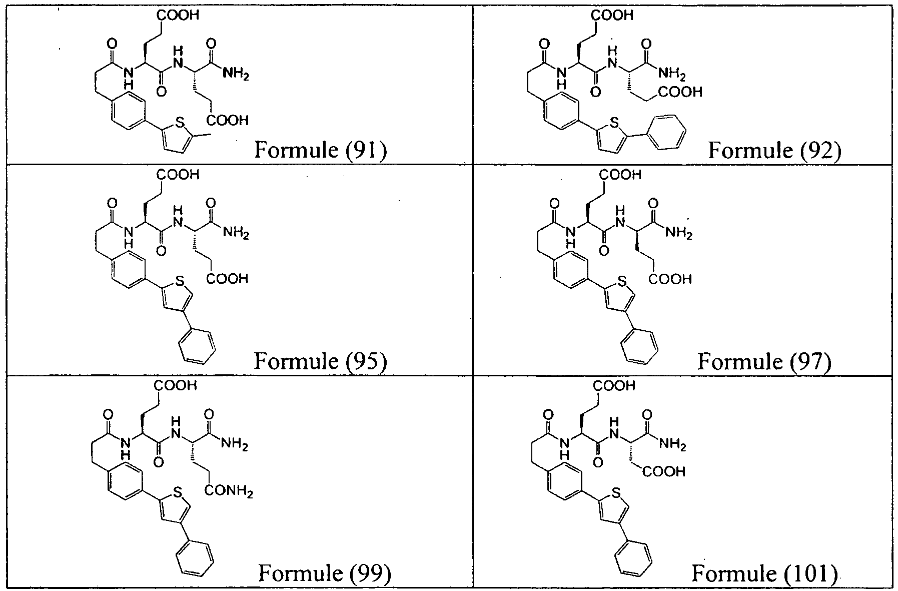

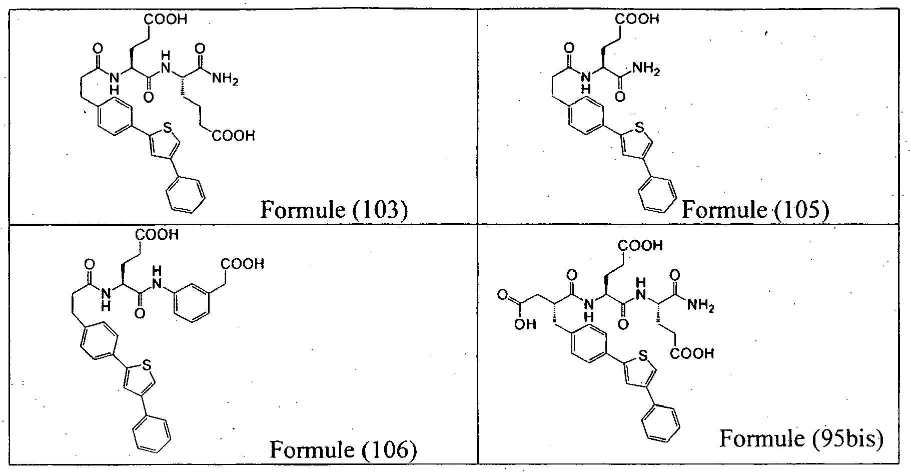

Mais, parmi ces composés de formule (1-F1), on voit à partir du tableau I que les composés ayant la formule (1-F1) dans laquelle R_{1'''} figure en position 3 du cycle thiophène et est un groupe phényle (R_{1'''} = Ph), sont les inhibiteurs les plus puissants et les plus sélectifs vis-à-vis de la MMP-12.

Ces composés ont la formule (1-F2) suivante : 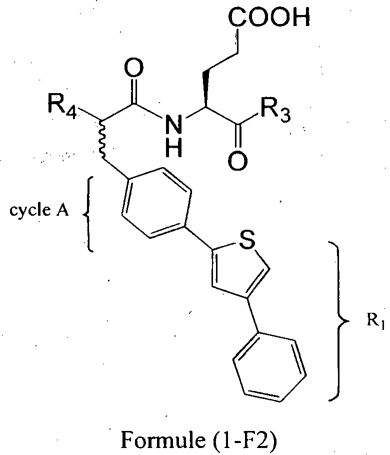 dans laquelle :
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F2) via une fonction amino, et
- R₄ est H ou un groupe carboxyméthyle -CH₂COOH.

Ainsi, les composés tout particulièrement préférés de l'invention de formule 1-F2 sont les composés de formules (95), (97), (99), (101), (103), (105), (106) et (95bis) suivantes :

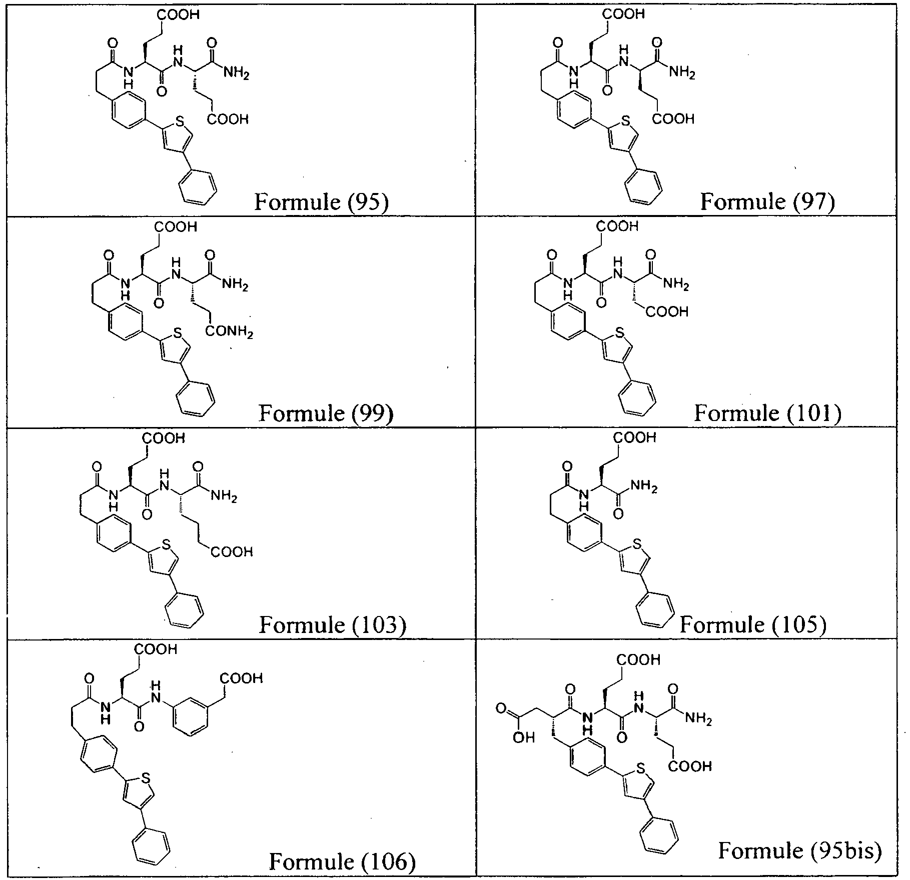

**Tableau I**

| Ki(nM) | | | | | | | | |
|---|---|---|---|---|---|---|---|---|
| | MMP-2h | MMP-3h | MMP-8h | MMP-9h | MMP-10h | MMP-12h | MMP-13h | MMP-14h |
| **RXP470** | 72 | 58 | 77 | 850 | 8,3 | 0,2 | 13 | 80 |
| **3** | 76 | 62 | 181 | 565 | 47 | 8,3 | 40 | >1000 |
| **4** | 90 | 118 | 119 | 120 | 122 | 11,8 | 46 | >1000 |
| **5** | 112 | 240 | 552 | >1000 | 44 | 8,9 | 53 | >1000 |
| **6** | 403 | >1000 | >5000 | >5000 | 489 | 401 | 235 | >10000 |
| **7** | 538 | 897 | >1000 | >5000 | 111 | 268 | 114 | >10000 |
| **8** | 112 | 243 | 556 | >1000 | 35 | 11 | 30 | >1000 |
| **9** | 91 | 381 | 899 | >1000 | 520 | 64 | 35 | >5000 |
| **10** | 86 | 85 | 649 | >1000 | 93 | 59 | 18 | >1000 |
| **11** | >1000 | 302 | >1000 | >10000 | 556 | 254 | 191 | >10000 |
| **12** | 150 | 129 | 452 | >1000 | 51 | 8,2 | 52 | >5000 |
| **13** | >1000 | 575 | 638 | 605 | >5000 | 348 | 91 | 553 |
| **14** | 756 | >10000 | 112 | >1000 | >1000 | 119 | >1000 | >1000 |
| **15** | 762 | >10000 | 114 | >1000 | >5000 | 398 | >1000 | >1000 |
| | | | | | | | | |
| **16** | 83 | 78 | 383 | >1000 | 114 | 3,4 | 60 | >1000 |
| **17** | 65 | 699 | >1000 | >1000 | >1000 | 53 | 36 | >5000 |
| **18** | 93 | 440 | 832 | >1000 | 196 | 8 | 30 | >1000 |
| **19** | 52 | 750 | >1000 | >1000 | 527 | 54 | 24 | >1000 |
| **20** | 834 | >1000 | >1000 | >1000 | >5000 | 412 | 234 | >10000 |
| **21** | 39,3 | 105 | 261 | >1000 | 256 | 5,4 | 49 | >1000 |
| **22** | >1000 | >10000 | >1000 | >1000 | >10000 | 830 | 436 | >10000 |
| **23** | 78 | 460 | 351 | >1000 | 276 | 1.1,2 | 80 | >1000 |
| **24** | >5000 | >1000 | >1000 | >1000 | >1000 | >1000 | >5000 | >5000 |
| **25** | >1000 | >5000 | 332 | >5000 | >1000 | 160 | 550 | >1000 |
| **26** | >5000 | >5000 | >5000 | >1000 | >5000 | >1000 | >1000 | >5000 |
| **27** | >1000 | >1000 | >1000 | >5000 | >5000 | >5000 | 900 | >5000 |
| **28** | >1000 | >5000 | >1000 | >10000 | >1000 | 856 | 309 | >1000 |
| **29** | >1000 | >10000 | >1000 | >1000 | >1000 | 469 | 117 | >5000 |
| **30** | >1000 | >1000 | 818 | 235 | >1000 | 154 | 598 | >1000 |
| **31** | >1000 | >5000 | 839 | >1000 | >1000 | 155 | >1000 | >1000 |
| **32** | >5000 | >1000 | >1000 | >1000 | >5000 | 969 | 641 | >1000 |
| **33** | >1000 | >10000 | >1000 | >1000 | >10000 | 797 | 518 | >1000 |
| **34** | >5000 | >1000 | >1000 | >5000 | >1000 | 887 | >1000 | >1000 |
| **35** | 442 | 281 | 231 | 199 | 679 | 158 | 212 | 438 |
| **36** | >1000 | >1000 | 373 | >1000 | >1000 | 59 | >1000 | >5000 |
| **37** | 657 | >5000 | >1000 | >1000 | >1000 | 369 | 240 | >1000 |
| **38** | 225 | 337 | 52 | 141 | 446 | 12 | 532 | 410 |
| **39** | 151 | 88 | 146 | 618 | 99 | 2,2 | 134 | 547 |
| **40** | 445 | >5000 | 226 | >1000 | >1000 | 18,6 | 689 | >5000 |
| **42** | 448 | >5000 | 285 | >1000 | >1000 | 48 | 884 | >1000 |
| **43** | 319 | >1000 | 657 | >1000 | >5000 | 306 | 259 | >5000 |
| **44** | >1000 | >10000 | 414 | >5000 | >5000 | 119 | >1000 | >5000 |
| **45** | 896 | >5000 | 233 | >1000 | >1000 | 37 | >1000 | >5000 |
| **46** | 632 | >10000 | 270 | >1000 | >1000 | 52 | >1000 | >5000 |
| **47** | >1000 | >10000 | 311 | >1000 | >1000 | 58 | >1000 | >5000 |
| **48** | 847 | >10000 | 339 | >5000 | >1000 | 75 | >1000 | >10000 |
| **49** | 790 | >5000 | 204 | >1000 | >1000 | 41 | >1000 | >1000 |
| **50** | >1000 | >50000 | 364 | >5000 | >5000 | 76 | >1000 | >5000 |
| **51** | >1000 | >10000 | 929 | >1000 | >10000 | 259 | >1000 | >5000 |
| **52** | 546 | >5000 | 63 | >1000 | 433 | 12,7 | >1000 | >1000 |
| **53** | >1000 | >1000 | >1000 | >1000 | >1000 | 190 | >1000 | >1000 |
| **54** | >10000 | >10000 | >1000 | >1000 | >1000 | 176 | >10000 | >10000 |
| **55** | >1000 | 878 | >1000 | 685 | >1000 | 73 | 436 | >5000 |
| **56** | >5000 | >10000 | >1000 | >1000 | >10000 | 91 | >5000 | >10000 |
| **57** | >1000 | >10000 | >1000 | >1000 | >5000 | 57 | >5000 | >10000 |
| **58** | >1000 | >1000 | >5000 | 391 | >1000 | 56 | >1000 | >1000 |
| **59** | >5000 | >10000 | >1000 | >5000 | >1000 | 35 | >5000 | >10000 |
| **60** | >5000 | >10000 | 809 | >5000 | >5000 | 52 | >5000 | >10000 |
| **61** | 53 | 74 | 132 | >1000 | 76 | 1,63 | 20 | >1000 |
| **62** | 45 | 244 | 452 | >1000 | 102 | 5,2 | 20 | >1000 |
| **63** | 110 | >1000 | 621 | >1000 | 447 | 14,3 | 55 | >1000 |
| **64** | 55 | 168 | 287 | >1000 | 35 | 3,3 | 23 | >5000 |
| **65** | 73 | 620 | 793 | >1000 | 119 | 6,6 | 33 | >1000 |
| **66** | 49 | 238 | 532 | >10000 | 130 | 2,2 | 16,4 | >10000 |
| **67** | 68 | 337 | 526 | >1000 | 168 | 10,2 | 36 | >1000 |
| **68** | 31 | 108 | 227 | >1000 | 71 | 3,5 | 16,6 | >1000 |
| **69** | 332 | 258 | 567 | >1000 | 382 | 7,8 | 81 | >5000 |
| **70** | 12 | 231 | 105 | >5000 | 421 | 4,7 | 17 | >1000 |
| **71** | 18,1 | 34 | 99 | 645 | 7,7 | 1,05 | 10,2 | 696 |
| **72** | 386 | 64 | >1000 | >5000 | >1000 | 134 | 180 | >5000 |
| **73** | 541 | 300 | >1000 | >10000 | >1000 | 254 | 97 | >10000 |
| **74** | 39 | 16 | 865 | >1000 | 430 | 10,5 | 13,5 | >1000 |
| **75** | >1000 | 252 | >5000 | >10000 | >1000 | 122 | 232 | >10000 |
| **76** | 854 | 142 | >1000 | >5000 | >10000 | 251 | 427 | >5000 |
| **77** | >1000 | >1000 | >1000 | >50000 | >5000 | 234 | 377 | >10000 |
| **78** | 729 | 116 | >1000 | >5000 | >5000 | 206 | 367 | >1000 |
| **79** | >1000 | 203 | >1000 | >100000 | >1000 | >1000 | 443 | >10000 |
| **80** | 142 | >1000 | 40 | >1000 | 373 | 8,6 | 321 | >1000 |
| **81** | 204 | >10000 | 48 | >1000 | 651 | 18,6 | 801 | >1000 |
| **82** | 234 | >10000 | 73 | >1000 | >1000 | 44 | >1000 | >1000 |
| **83** | 334 | >10000 | 49 | >1000 | 435 | 14,2 | >1000 | >1000 |
| **84** | 140 | >5000 | 41 | 859 | 514 | 20,9 | 584 | >1000 |
| **85** | 348 | >10000 | 62 | >5000 | 835 | 25,6 | >1000 | >1000 |
| **86** | 371 | >10000 | 63 | >1000 | >1000 | 34 | >1000 | >1000 |
| **87** | 147 | >10000 | 47 | >1000 | 742 | 16,4 | >1000 | >1000 |
| **88** | 298 | >10000 | 73 | >1000 | >1000 | 32 | >1000 | >1000 |
| **89** | 484 | >100000 | 94 | >1000 | >5000 | 124 | >1000 | >1000 |
| **90** | 116 | >1000 | 18 | 284 | 143 | 8 | 366 | 430 |
| **91** | 97 | >1000 | 10,3 | 242 | 353 | 1,84 | 564 | >1000 |
| **92** | 279 | 108 | 381 | 874 | 156 | 2,58 | 200 | >1000 |
| **93** | >1000 | >1000 | 307 | >1000 | 38,8 | 17 | 33 | >5000 |
| **94** | 868 | >1000 | 233 | >1000 | >1000 | 22 | >1000 | >10000 |
| **95** | >1000 | >1000 | 410 | >10000 | 872 | 1,92 | 684 | >1000 |
| **96** | >1000 | >10000 | >10000 | >1000 | >1000 | 144 | >1000 | >10000 |
| **97** | >1000 | >5000 | 694 | >5000 | 693 | 3,7 | 714 | >1000 |
| **98** | >5000 | >10000 | >10000 | >5000 | >10000 | 317 | >1000 | >10000 |
| **99** | >1000 | >10000 | >1000 | >5000 | >1000 | 5,4 | 522 | >1000 |
| **100** | 862 | >1000 | 559 | >1000 | >1000 | 15,1 | 559 | >1000 |
| **101** | >1000 | >5000 | 379 | >5000 | 571 | 2,56 | 933 | >1000 |
| **102** | >1000 | >10000 | >5000 | >5000 | >5000 | 56 | 635 | >10000 |
| **103** | >1000 | >5000 | 656 | >5000 | 845 | 2,9 | 563 | >1000 |
| **104** | >5000 | >10000 | >1000 | >5000 | >5000 | 40 | >1000 | >5000 |
| **105** | 377 | >1000 | 203 | >1000 | >1000 | 3,65 | 603 | >1000 |
| **106** | 339 | 791 | 675 | 396 | 318 | 4,3 | 132 | >1000 |
| **107** | >1000 | >10000 | 766 | >10000 | >1000 | 84 | >1000 | >5000 |

Dans ce tableau I ainsi que dans le tableau Il suivant, « h » correspond à humaine. L'ensemble des composés a donc été évalué sur des MMP humaines.

Par ailleurs les pseudo peptides de formules (3), (14), (40), (61), (80), (95), (97), (99), (101), (103), (105), (106) et (95bis) ont également été évalués sur deux autres MMP, la MMP-1h et la MMP-7h.

Les résultats obtenus sont présentés dans le tableau II suivant, et dans la tableau IV ci-après :

**Tableau II**

| Ki (nM) | | | | | | | | | | |
|---|---|---|---|---|---|---|---|---|---|---|
| | MMP-1h | MMP-2h | MMP-3h | MMP-7h | MMP-8h | MMP-9h | MMP-10h | MMP-12h | MMP-13h | MMP-14h |
| **3** | >10000 | 76 | 62 | >1000 | 181 | 565 | 47 | 8,3 | 40 | >1000 |
| **14** | >10000 | 756 | >10000 | >10000 | 112 | >1000 | >1000 | 119 | >1000 | >1000 |
| **40** | >100000 | 445 | >5000 | >10000 | 226 | >1000 | >1000 | 18,6 | 689 | >5000 |
| **61** | >5000 | 53 | 74 | 502 | 132 | >1000 | 76 | 1,63 | 20 | >1000 |
| **80** | >100000 | 142 | >1000 | >1000 | 40 | >1000 | 373 | 8,6 | 321 | >1000 |
| **95** | >10000 | >1000 | >1000 | >1000 | 410 | >10000 | 872 | 1,92 | 684 | >1000 |
| **97** | >100000 | >1000 | >5000 | >10000 | 694 | >5000 | 693 | 3,7 | 714 | >1000 |
| **99** | >10000 | >1000 | >10000 | >10000 | >1000 | >5000 | >1000 | 5,4 | 522 | >1000 |
| **101** | >100000 | >1000 | >5000 | >100000 | 379 | >5000 | 571 | 2,56 | 933 | >1000 |
| **103** | >10000 | >1000 | >5000 | >5000 | 656 | >5000 | 845 | 2,9 | 563 | >1000 |
| **105** | >10000 | 377 | >1000 | >10000 | 203 | >1000 | >1000 | 3,65 | 603 | >1000 |
| **106** | >1000 | 339 | 791 | >1000 | 675 | 396 | 318 | 4,3 | 132 | >1000 |

Les résultats reportés au tableau II, ainsi que dans le tableau IV, confirment d'une part que les composés de l'invention sont des inhibiteurs puissants des MMP et en particulier de la MMP-12, avec des Ki de l'ordre du nanomolaire, et d'autre part, que les composés de formules (95), (97), (99), (101), (103), (105) et (95bis) sont des composés hautement sélectifs de MMP-12, avec un facteur de sélectivité F > 100 avec F = Ki MMP-x/Ki MMP-12.

Ainsi, les composés de l'invention peuvent être utilisés à titre de médicament, ou d'inhibiteurs de MMP ou encore pour la fabrication d'un médicament pour traiter les désordres dans lesquels une ou plusieurs MMP sont surexprimées.

Plus particulièrement, les composés de l'invention de formules (61) à (79) peuvent être utilisés pour la fabrication d'un médicament pour le traitement de pathologies dans lesquelles MMP-12 et MMP-13 sont surexprimées et les composés de formules (80) à (90) peuvent être utilisés pour la fabrication d'un médicament pour le traitement de pathologies dans lesquelles MMP-12 et MMP-8 sont surexprimées.

Mais encore, les composés de formules (95), (97), (99), (101), (103), (105), (106) et (95bis) peuvent être utilisés avantageusement en tant qu'inhibiteurs de MMP-12 et utilisés pour la fabrication d'un médicament pour le traitement des désordres dans lesquels MMP-12 est surexprimée, en particulier pour le traitement du cancer, les maladies inflammatoires telle que la maladie pulmonaire obstructive chronique (COPD), l'arthrite, l'arthrite rhumatoïde, l'athérosclérose et les ruptures d'anévrisme.

Un autre objet de l'invention concerne des composés de formule (2) suivante, résultants du marquage des composés de formule (1) tels que définis précédemment par un marqueur. Les composés de formule (2) répondent à la formule suivante : 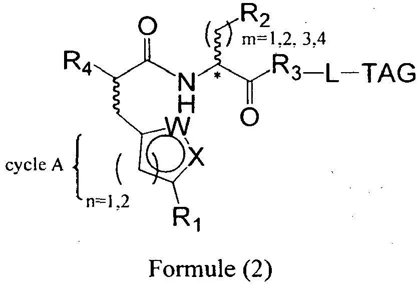 dans laquelle n, m, W, X, R₁, R₂, R₃ et R₄ ont la même définition que ci-dessus, et :
- L est un bras espaceur choisi parmi les chaînes alkyles en C₁-C₁₂, et les éthers de glycol dans lesquels la chaîne carbonée comporte de 2 à 12 atomes de carbone, et
- TAG est un marqueur,
le groupement R₃ étant lié au bras espaceur L via une fonction carboxamide -C(=O)NH₂ terminale.

On entend par marqueur toute entité susceptible d'être détectée par des moyens appropriés, les marqueurs utilisés dans le cadre de l'invention correspondant typiquement aux marqueurs utilisés par l'homme de l'art dans le domaine de la biologie pour marquer des molécules d'intérêt biologiques, notamment dans le cadre de la réalisation de diagnostic.

La propriété physique détectable des marqueurs de l'invention peut être une réactivité spécifique vis-à-vis d'une source électromagnétique telle qu'un champ magnétique, comme par exemple par imagerie par résonance magnétique, ou vis-à-vis d'un rayonnement lumineux pouvant être focalisé, comme par exemple par imagerie par fluorescence avec les fluorophores, ou encore vis-à-vis d'un rayonnement nucléaire, comme par exemple à l'aide d'isotopes.

Les fluorophores utilisés dans le cadre de l'invention peuvent être des composés fluorescents aromatiques dont les transitions π-π sont caractérisées par des coefficients d'absorption molaires et des rendements quantiques de fluorescence élevées, lesdits fluorophores pouvant être choisis parmi la rhodamine, la fluorescéïne, la pyronine, la coumarine, la benzophénone, l'anthrone, la fluorénone, la pyridine, la quinoléine, l'acridine, le naphtalène, l'anthracène, la naphtacène, la pentacène, le xanthène et leurs dérivés.

Différentes familles de marqueurs et différentes techniques de détection associées connues de l'homme de l'art sont décrites dans l'ouvrage Anti-Cancer Agents in Medicinal Chemistry, 2008, 8, 497-522. Plus spécifiquement, il est possible de se référer aux fluorophores cités dans Cytometry Part A 69A : 863-871 (2006) et aux nanoparticules mentionnées dans le document Anal. Bioanal. Chem., 384 : 620-630 (2006).

Selon un mode de réalisation encore plus préféré, le marqueur TAG peut être choisi parmi :
- les fluorophores tels que définis ci-dessus, ces derniers pouvant répondre à l'une des formules suivantes : 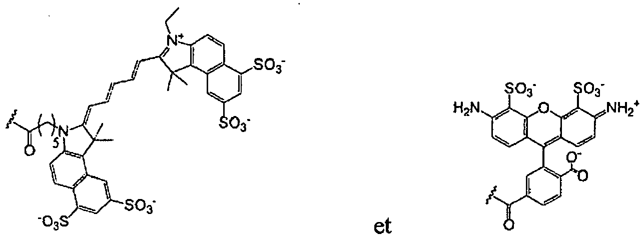
- les composés porteurs d'un isotope fluore 18 (¹⁸F), tel que : 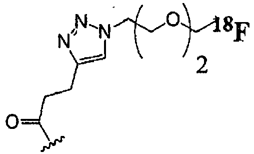
- les agents chélatants porteurs d'un isotope technétium 99 (99mTc), lesdits agents chélatants pouvant éventuellement comprendre de 2 à 6 atomes d'azote, et de préférence 4 atomes d'azote, et éventuellement de 1 à 6 fonctions carboxylate, et de préférence 3 fonctions carboxylate, tel que : 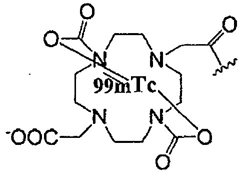
- les agents chélatants porteurs d'un atome de gadolinium Gd(III), lesdits agents chélatants pouvant éventuellement comprendre de 2 à 6 atomes d'azote, et de préférence 4 atomes d'azote, et éventuellement de 1 à 6 fonctions carboxylate, et de préférence 3 fonctions carboxylate, tel que : 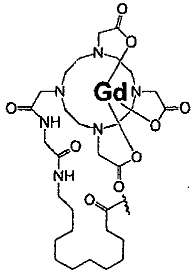
- les marqueurs peptidiques tels que ceux définis dans la Demande Internationale WO 2010/076654, sélectionnés parmi les séquences suivantes :
   a) X₁X₁X₂X₃X₄X₅X_{b}X_{c} (SEQ ID NO : 1),
      dans laquelle :
      - Xₐ, X_{b} et X_{c} peuvent être présents ou absents,
      - Xₐ ou X_{c} quand ils sont présents comprennent au moins deux aminoacides naturels ou non naturels,
      - X_{b} quand il est présent comprend la séquence peptidique RRMQYNRR (SEQ ID NO : 2) dans laquelle au moins un des résidus est remplacé par un amino-acide naturel ou non naturel dans lequel la chaîne latérale présente dans le résidu initial qu'il remplace est absent,
      - X₁ consiste en n'importe quel amino-acide naturel ou non naturel comprenant un groupement OH sur sa chaîne latérale,
      - X₂ consiste en n'importe quel amino-acide à l'exception de la Cystéine,
      - X₃ consiste en un amino-acide choisi parmi : Arginine, Glycine, Lysine,
      - X₄ consiste en au moins un amino-acide choisi parmi : Alanine, Glycine, Lysine, Arginine,
      - X₅ consiste en n'importe quel amino-acide à l'exception de la Cystéine ;
   b) la version retro-inversée d'un marqueur peptidique tel que defini selon le groupe a).

La nature du bras espaceur L séparant le marqueur TAG de la partie inhibitrice interagissant avec le site actif des MMP dépend de la fonctionnalisation initiale du support solide utilisé. Selon un mode de réalisation préféré de l'invention, le bras espaceur L des composés de formule (2) est une chaîne alkyle en C₁-C₂ ou une chaîne polyéthoxylées -(CH₂-CH₂-O)ₙ- dans laquelle n est compris entre 1 et 6.

Ainsi, les composés de formule (2) peuvent être utilisés comme agents de contraste pour la détection des MMP, et plus particulièrement pour la détection de la MMP-12. Les composés de formule (2) peuvent notamment être utilisés pour l'imagerie non invasive de la plaque d'athérome (F. A. Jaffer et al., Arterioscler Thromb Vasc. Biol. 2009, (10)).

Selon la nature du marqueur TAG, différentes techniques d'imagerie peuvent être envisagées parmi le PET (*Positron Emission Tomography*), MRI (*Magnetic Resonance Imaging*) ou NIRF (*Near-Infrared Fluorescence imaging*).

Afin de mieux faire comprendre l'invention, on va en décrire maintenant plusieurs modes de réalisation.

Les composés de l'invention ont été synthétisés comme décrit ci- après.

### Matériels et méthodes :

Tous les réactifs et solvants commercialement disponibles ont été utilisés tels que reçus, sans purification additionnelle.

Les Synphase lanternes^{®} (polyamide, lanterne série D, Rink amide protégé par le groupement Fmoc, 8 µmol/lanterne ou polyamide, lanterne série D, hydroxyméthylphénoxy, 8 µmol/lanterne), sont commercialisées par Mimotopes (Australie).

Les acides aminés naturels protégés par le groupement Fmoc proviennent de chez Novabiochem.

L'homoglutamate protégé par le groupement Fmoc est commercialisé par Bachem.

Les Fmoc-3-aminophénylacétique et (S)-Fmoc-(3-carboxyméthyle)-pipéridine sont commercialisés par la société NeoMPS.

Le 6-chloro-1-hydroxybenzotriazole (ClHOBt) est commercialisé par la société Molekula.

La diisopropylcarbodiimide (DIC), l'acide trifluoroacétique (TFA) et le triisopropylsilane (TIS) sont commercialisés par la société Aldrich.

La N,N-diméthylformamide anhydre (DMF) est commercialisé par la société Fluka.

Les expériences aux micro-ondes ont été effectuées sur un appareil de type Discover (CEM µWave) dans des tubes de réaction, scellés, de 10 mL ou en utilisant le mode à récipient ouvert avec le kit SPS.

Les plaques de chromatographie sur couche mince (CCM) étaient des feuilles d'aluminium de couches minces revêtues d'un gel de silice 60F₂₅₄ commercialisées par la société Merck.

Les blocs maloniques précurseurs ont été purifiés par chromatographie flash sur gel de silice Si 60, 40-43µm.

Les spectres de RMN ¹H ont été enregistrés sur un instrument Bruker à 250 MHz.

Les déplacements chimiques sont reportés en ppm avec le solvant comme étalon interne (CDCl₃ : 7,26 ppm ; MeOH d₄=3,31 ppm ; DMSO d₆ = 2,50ppm).

Les données sont reportées comme suit : déplacement chimique, multiplicité (s = singulet, d = doublet, t = triplet, q = quartet, br = large, m = multiplet), intégration et constantes de couplage (Hz).

Les spectres de RMN ¹³C ont été enregistrés sur des instruments de RMN à 125 MHz avec un découplage complet des protons.

Les déplacements chimiques sont reportés en ppm avec le solvant comme étalon interne (CDCl₃ : 77,16 ppm ; MeOH d₄ = 49,00ppm ; DMSO d₆ = 39,52ppm).

Les mesures de densité optique (DO) ont été effectuées avec un spectrophotomètre Beckman DU640B.

Les spectres de masse en mode d'ionisation « electrospray » (ESMS) ont été enregistrés sur une plateforme de spectrométrie de masse ESI-QTRAP (Applied Biosystems-MDS Sciex, Université Pierre et Marie Curie (UPMC), Paris, France).

Les spectres de masse à haute résolution (HRMS) ont été enregistrés en utilisant un spectromètre de masse MALDI-TOF 4800 (Applied Biosystems, Foster City, USA) en mode réflectron positif dans l'intervalle m/z de 100-700.

Chaque spectre était le résultat de 1000 à 2000 coups (20 positions différentes à l'intérieur de chaque tâche et 50 coups par sous-spectre) et une calibration interne a été effectuée en utilisant un m/z de la matrice 4-HCCA (acide Cyano-4-hydroxycinnamique)

Les séparations analytiques et préparatives RP-HPLC ont été effectuées, respectivement, sur un appareil de séparation de marque Thermo et un appareil de marque Gilson en utilisant soit une colonne analytique de type Ascentis express (100 x 4,6mm, 10µ, 100Å) soit une colonne semi-préparative de type Kromasil AIT C18 (250 x 20 mm, 10µ, 100Å) avec respectivement des débits de 1,8 et 3 mL.min⁻¹.

La détection a été effectuée à 230 nm.

Un système de solvant consistant de (A) 0,1% TFA dans 90% eau-10% acétonitrile et (B) 0,09% TFA dans 90% acétonitrile-10% eau a été utilisé. Les temps de rétention (t_{R}) obtenus en mode analytique (colonne Ascentis Express) sont reportés en minutes.

La composition en acide aminé de chaque pseudopeptide a été réalisée en conditions standards : chaque échantillon est évaporé sous vide et hydrolysé en tube scellé sous vapeur d'acide chlorhydrique 6 N en présence d'un cristal de phénol pendant 17 h à 110°C à l'aide du système « PicoTag » (Waters Associates, Milford, MA). L'hydrolysat est ensuite dissout dans 100 µL d'eau MilliQ et 90 µL de cette solution (contenant au minimum 200 pmol de chaque acide aminé) sont analysés et quantifiés par dérivatisation à la ninhydrine sur un appareil de type « aminoTac JLC-500/V amino acids analyzer » (JEOL, Japan). Une calibration standard en présence d'une solution en acides aminés dont la concentration est connue est effectuée avant chaque analyse.

Les composés (3) à (107) ont été synthétisés selon le Schéma 1 général suivant :

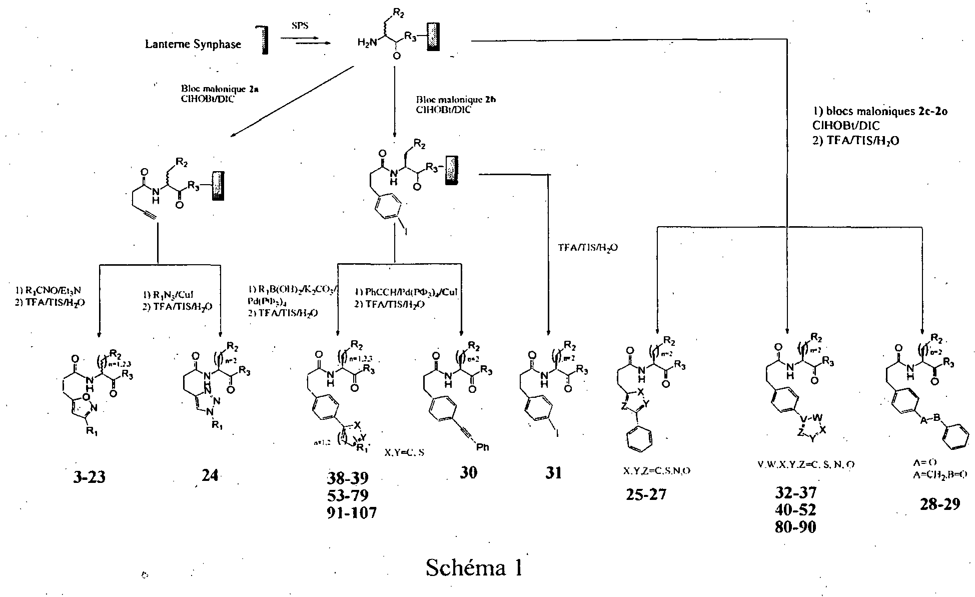

### Synthèse des blocs maloniques précurseurs :

Les blocs maloniques précurseurs sont synthétisés selon le Schéma 2 suivant :

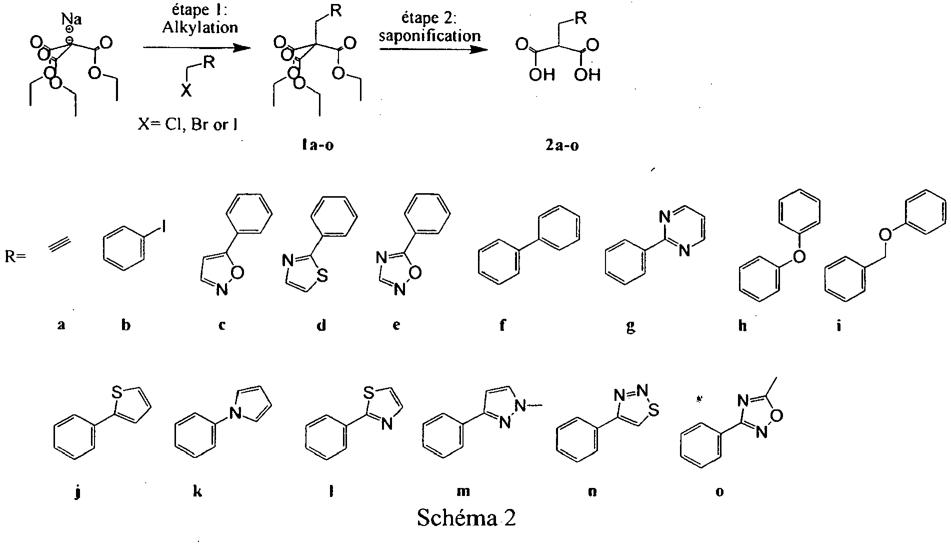

### Etape 1: étape d'alkylation.

Dans un réacteur à micro-ondes de 10 mL le triéthyl ester de méthane sodé (3,9 mmol, 1eq), un dérivé de type halogénure d'alkyle (4,3 mmol, 1,1eq) et du DMF anhydre (5 mL) ont été mélangés et agités à 100°C sous irradiations micro-ondes (300W) pendant 5 minutes.

La fin de la réaction a été contrôlée par chromatographie sur couche mince (CCM) avec un mélange d'éluant (Cyclohexane CHX/Acétate d'éthyle EtOAc: 9/1).

Le mélange réactionnel a ensuite été évaporé sous pression réduite et la solution brute a été mise en suspension dans l'acétate d'éthyle EtOAc/eau H₂O (1/1 : 10 mL/10 mL).

La phase aqueuse a été extraite avec de l'acétate d'éthyle EtOAc (2 x 10mL).

Les phases organiques ont été réunies puis lavées avec une solution saturée en chlorure de sodium NaCl (20 mL) et enfin séchées sur sulfate de magnésium (MgSO₄) anhydre.

Le solvant a ensuite été concentré sous vide et le produit brut a été purifié par chromatographie flash (CHX/EtOAc) pour donner les triesters **1a-o.**

### Triéthyl but-3-yne-1,1,1-tricarboxylate 1a

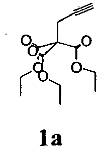

Préparé à partir de bromure de propargyle (Fluka 81831, 80% dans le toluène) selon le protocole général d'alkylation pour donner le composé titre sous la forme d'une huile jaune clair (rendement 88%).

RMN ¹H (CDCl₃) : δ 1,29 (t, 9H, *J* = 7Hz) ; 2,05 (t, 1H, *J* = 2,75Hz) ; 3,01 (d, 2H, *J* = 2,75Hz) ; 4,29 (q, 6H*, J* = 7Hz).

RMN ¹³C (CDCl₃) : δ 14,00; 23,41 ; 62,70; 64,68; 70,88 ; 78,87 ; 165,90.

### Triéthyl 2-(4-iodophényl)éthane-1,1,1-tricarboxylate 1b

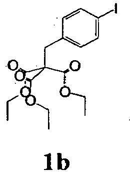

Préparé à partir de bromure de 4-iodobenzyle (Aldrich 515604) selon le protocole général d'alkylation pour donner le composé titre sous la forme d'une huile jaune clair (rendement 94%).

RMN ¹H (CDCl₃) : δ 1,21 (t, 9H, *J* = 6,75Hz) ; 3,44 (s, 2H) ; 4,19 (q, 6H, *J* = 6,75Hz) ; 7,05 (d, 2H, *J=* 8Hz) ; 7,56 (d, 2H, *J* = 8Hz).

### Triéthyl 2-(5-phénylisoxazol-3-yl)éthane-1,1,1-tricarboxylate 1c

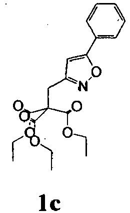

Préparé à partir de 3-chlorométhyl-5-phényl isoxazole (Maybridge CC30524) selon le protocole générale d'alkylation pour donner le composé titre sous la forme d'une huile jaune clair (rendement 73%).

RMN ¹H (CDCl₃) : δ 1,27 (t, 9H, *J* = 7,25Hz) ; 3,59 (s, 2H) ; 4,28 (q, 6H, *J =* 7,25Hz) ; 6,53 (s, 1 H) ; 7,44 (m, 3H) ; 7,74 (m, 2H).

### Triéthyl 2-2-phénylthiazol-4yl)éthane-1,1,1-tricarboxylate 1d

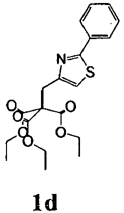

Préparé à partir de 4-(chlorométhyl)-2-phényl-1,3-thiazole (Maybridge CC18324) selon le protocole général d'alkylation pour donner le composé titre sous la forme d'une huile jaune clair (rendement 58%).

RMN ¹H (CDCl₃) : δ 1,25 (t, 9H, *J* = 7,25Hz) ; 3,70 (s, 2H) ; 4,24 (q, 6H, *J* = 7,25Hz) ; 7,11 (s, 1H) ; 7,43 (m, 3H) ; 7,9 (m, 2H).

RMN ¹³C (CDCl3) : δ 13,95 ; 14,03 ; 34,49 ; 62,31 ; 62,54 ; 65,73 ; 116,16 ; 126,51 ; 128,94; 129,91 ; 133,75 ; 152,20 ; 164,07; 166,75; 166,63.

[M+H]⁺ = 406.1, [M+Na]⁺ = 428,1.

### Triéthyl 2-(5-phényl-1,2,4-oxadiazol-3-yl)éthane-1,1,1-tricarboxylate 1e

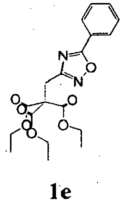

Préparé à partir de 3-chlorométhyl-5-phényl-1,2,4-oxadiazole (Maybridge, SEW02030) selon le protocole générale d'alkylation pour donner le composé titre sous la forme d'une huile jaune (rendement 55%).

RMN ¹H (CDCl₃) : δ 1,21 (t, 9H,J= 7,25Hz) ; 3,72 (s, 2H) ; 4,29 (q, 6H, *J* = 7,25Hz) ; 7,5 (m, 3H) ; 8,02 (d, 2H, *J* = 8Hz).

RMN ¹³C (CDCl₃) : δ 13,94; 14,05 ; 29,59; 62,56 ; 62,75; 64,35 ; 124,33 ; 128,14 ; 129,13 ; 132,74 ; 164,08 ; 166,06 ; 167,77 ; 175,15.

[M+H]⁺ = 391,3 ; [M+Na]⁺ = 413,2.

### Triéthyl 2-(biphényl-4-yl)éthane-1,1,1-tricarboxylate 1f

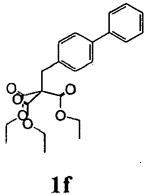

Préparé à partir de 4-(bromométhyl)-4-biphényle 96% (Acros 368950050) selon le protocole général d'alkylation pour donner le composé titre sous la forme d'une huile jaune pâle (rendement 91 %).

RMN ¹H (CDCl₃) : δ 1,22 (t, 9H, *J* = 7,25Hz) ; 3,56 (s, 2H) ; 4,28 (q, 6H, *J* = 7,25Hz) ; 7,29-7,58 (m, 9H).

RMN ¹³C (CDCl₃) : δ 13,99; 27,05 ; 38,46; 62,32 ; 66,89 ; 126,80 ; 127,15 ; 127,31 ; 128,86 ; 131,10 ; 134,80 ; 140,00 ; 141,00 ; 166,66.

### Triéthyl 2-(4-(pyrimidin-2-yl)phényl)éthane-1,1,1-tricarboxylate 1g

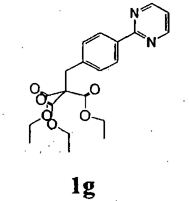

Préparé à partir de 2-[4-(chlorométhyl)phényl]pyrimidine (Maybridge, CC56224) selon le protocole général d'alkylation pour donner le composé titre sous la forme d'une huile jaune pâle (rendement ≥ 95%).

RMN ¹H (CDCl₃) : δ 1,21 (t, 9H, *J* = 7,25Hz) ; 3,59 (s, 2H) ; 4,19 (q, 6H, *J* = 7,25Hz) ; 7,17 (s, 1H, *J* = 4,75Hz) ; 7,40 (d, 2H, *J* = 8,25Hz) ; 8,32 (d, 2H, *J* = 8,25Hz) ; 8,78 (d, 2H, *J* = 4,75Hz).

### Triéthyl 2-(4-phénoxyphényl)éthane-1,1,1-tricarboxylate 1h

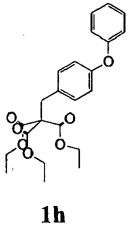

Préparé à partir de 1-(bromométhyl)-4-phénoxybenzène (Maybridge CC53708) selon le protocole générale d'alkylation pour donner le composé titre sous la forme d'une huile jaune pâle (rendement = 36%).

RMN ¹H (CDCl₃) : δ 1,23 (t, 9H, *J* = 7,25Hz) ; 3,49 (s, 2H) ; 4,20 (q, 6H, *J* = 7,25Hz) ; 6,88 (d, 2H, *J* = 8,5Hz) ; 6,97 (d, 2H, *J =* 8,5Hz) ; 7,08 (t, 1H, *J* = 7,25Hz) ; 7,29 (m, 4H).

RMN ¹³C (CDCl₃) : δ 13,99 ; 38,07 ; 62,27 ; 66,89 ; 118,36 ; 119,01 ; 123,34 ; 129,83 ; 130,40 ; 132,09 ; 156,44 ; 157,25 ; 166,62.

### Triéthyl 2-(4-(phénoxyméthyl)phényl)éthane-1,1,1-tricarboxylate 1i

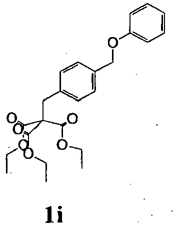

Préparé à partir de 1-(bromométhyl)-4-(phénoxyméthyl) benzène (Maybridge CC63708) selon le protocole général d'alkylation pour donner le composé titre sous la forme d'une huile jaune clair (rendement ≥ 95%).

RMN ¹H (CDCl₃) : δ 1,21 (t, 9H, *J* = 5,75Hz) ; 3,51 (s, 2H) ; 4,18 (q, 6H, *J* = 5,75Hz) ; 5,00 (s, 2H) ; 6,93 (m, 3H) ; 7,26 (m, 6H).

### Triéthyl 2-(4-(thiophèn-2-yl)phényl)éthane-1,1,1-tricarboxylate 1j

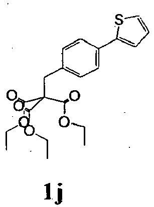

Préparé à partir de 2-[4-(bromométhyl)phényl]thiophène (Maybridge, CC12008) selon le protocole général d'alkylation pour donner le composé titre sous la forme d'une huile jaune (rendement ≥ 95%).

RMN ¹H (CDCl₃) : δ 1,22 (t, 9H, *J* = 7Hz) ; 3,51 (s, 2H) ; 4,31 (q, 6H, *J* = 7Hz) ; 7,05 (m, 1 H) ; 7,27 (m, 4H) ; 7,50 (d, 2H, *J* = 8,25Hz).

### Triéthyl 2-(4-(1H-pyrrol-1-yl)phényl)éthane-1,1,1-tricarboxylate 1k

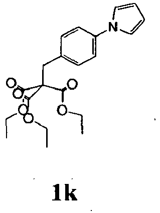

Préparé à partir de 1-[4-(bromométhyl)phényl]-1H-pyrrole (Maybridge, CC25508) selon le protocole général d'alkylation pour donner le composé titre sous la forme d'une huile jaune (rendement ≥ 95%).

RMN ¹H (CDCl₃) : δ 1,21 (t, 9H, *J=* 7,25Hz) ; 3,52 (s, 2H) ; 4,20 (q, 6H, *J* = 7,25Hz) ; 6,31 (m, 2H) ; 7,04 (m, 2H) ; 7,29 (m, 4H).

### Triéthyl 2-(4-(thiazol-2-yl)phényl)éthane-1,1,1-tricarboxylate 1l

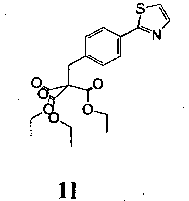

Préparé à partir de 2-[4-(chlorométhyl)phényl]-1,3-thiazole (Maybridge, CC40224) selon le protocole général d'alkylation pour donner le composé titre sous la forme d'une huile jaune (rendement ≥ 95%).

RMN ¹H (CDCl₃) : δ 1,20 (t, 9H, *J=* 7,25Hz) ; 3,54 (s, 2H) ; 4,19 (q, 6H, *J* = 7,25Hz) ; 7,33 (m, 4H) ; 7,83 (m, 2H).

### Triéthyl 2-(4-(1-méthyl-1H-pyrazol-3-yl)phényl)éthane-1,1,1-tricarboxylate 1m

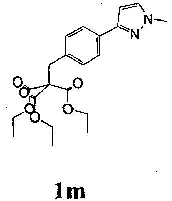

Préparé à partir de 3-[4-(chlorométhyl)phényl]-1-méthyl-1*H*-pyrazole (Maybridge, CC23824) selon le général protocole d'alkylation pour donner le composé titre sous la forme d'une huile jaune (rendement ≥ 95%).

RMN ¹H (CDCl₃) : δ 1,20 (t, 9H, *J* = 7,25Hz) ; 3,51 (s, 2H) ; 3,93 (s, 3H) ; 4,19 (q, 6H, *J* = 7,25Hz) ; 6,49 (d, 1H, *J* = 2,25Hz) ; 7,28 (d, 2H, *J=* 8Hz) ; 7,65 (d, 2H, *J* = 8Hz) ; 7,99 (s, 1H).

### Triéthyl 2-(4-(1,2,3-thiadiazol-4-yl)phényl)éthane-1,1,1-tricarboxylate 1n

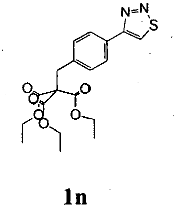

Préparé à partir de 4-[4-(bromométhyl)phényl]-1,2,3-thiadiazole (Maybridge, CC16408) selon le protocole général d'alkylation pour donner le composé titre sous la forme d'une huile jaune (rendement ≥ 95%).

RMN ¹H (CDCl₃) : δ 1,22 (t, 9H, *J =* 7Hz) ; 3,58 (s, 2H) ; 4,21 (q, 6H, *J* = 7Hz) ; 7,42 (d, 2H, *J* = 8,25Hz) ; 7,92 (d, 2H, *J* = 8,25Hz) ; 8,61 (s, 1H).

### Triéthyl 2-(4-(5-méthyl-1,2,4-oxadiazol-3-yl)phényl)éthane-1,1,1-tricarboxylate 1o

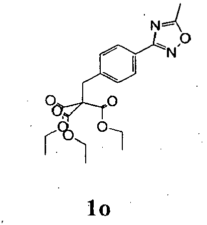

Préparé à partir de 3-[4-(bromométhyl)phényl]-5-méthyl-1,2,4-oxadiazole (Maybridge, CC34808) selon le protocole général d'alkylation pour donner le composé titre sous la forme d'une huile jaune (rendement ≥ 95%).

RMN ¹H (CDCl₃) : δ 1,22 (t, 9H*, J* = 7Hz) ; 2,64 (s, 3H) ; 3,58 (s, 2H) ; 4,20 (q, 6H, *J* = 7Hz); 7,39 (d, 2H, *J* = 8,25Hz) ; 7,93 (d, 2H, *J* = 8,25Hz).

### Etape 2 : étape de saponification.

Les triesters **1a-o** (3,93 mmol) ont été solubilisés dans l'éthanol absolu (10 mL) et de l'hydroxyde de potassium (23,58 mmol, 6 eq) a été ajouté.

Le mélange en solution a été agité à température ambiante pendant 1h puis évaporé sous pression réduite.

Le produit brut a été repris dans l'acide chlorhydrique HCl 1M/EtOAc (1/1 : 10 mL/10 mL).

La phase aqueuse a été saturée avec du NaCl et extraite avec EtOAc (2 x 10mL).

Les phases organiques ont été regroupées, lavées avec une solution saturée en NaCl (20 mL) et séchées sur MgSO₄ anhydre.

Après évaporation, le solide brut a été trituré dans DCM (1 mL) puis filtré pour donner les dérivés maloniques **2a-o.**

### Acide 2-(prop-2-ynyl malonique 2a

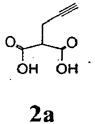

Préparé à partir du triester **la** selon le protocole de saponification pour donner le composé titre sous la forme d'un solide blanc (rendement 86 %).

RMN ¹H (MeOH d₄) : δ 2,33 (t, 1H, *J* = 2,75Hz) ; 2,69 (dt, 2H*, J* = *2,75Hz, J=* 5,25Hz) ; 3,51 (t, 1H, *J* = 5,25Hz).

### Acide 2-(4-iodobenzyl) malonique 2b

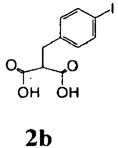

Préparé à partir du triester **1b** selon le protocole de saponification pour donner le composé titre sous la forme d'un solide blanc (rendement 78%).

RMN ¹H (MeOH d₄) : δ 3,10 (d, 2H, *J* = 7,75Hz) ; 3,61 (t, 1H, *J* = 7,75Hz). 7,04 (d, *2H, J=* 8Hz) ; 7,61 (d, 2H, *J* = 8Hz).

RMN ¹³C (MeOH d₄) : δ 35,20 ; 54,72 ; 92,45 ; 132,11 ; 138,60 ; 139,58 ; 172,33.

Masse haute résolution *m*/*z* pour C₁₀H₉INaO₄ (M+Na⁺)⁺, calculée 342,9443 ; mesurée 342,9430.

### Acide 2-((5-phénylisoxazol-3-yl)méthyl) malonique 2c

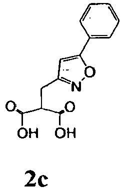

Préparé à partir du triester **le** selon le protocole de saponification pour donner le composé titre sous la forme d'un solide blanc (rendement 53%).

RMN ¹H (MeOH d₄) : 3,26 (d, 2H, *J* = 7,5 Hz) ; 3,86 (t, 1H, *J* = 7,5 Hz) ; 6,71 (s, 1H); 7,49 (m, 3H); 7,81 (m, 2H).

RMN ¹³C (MeOH d₄) : δ 26,51 ; 51,66 ; 100,83 ; 126,73; 128,58 ; 130,19 ; 131,44 ; 163,40; 171,28 ; 171,97.

Masse haute résolution *m*/*z* pour C₁₃H₁₂NO₅ (M+H⁺)⁺, calculée 262,0715 ; mesurée 262,0714.

### Acide 2-((2-phénylthiazol-4-yl)méthyl) malonique 2d

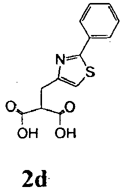

Préparé à partir du triester **1d** selon le protocole de saponification pour donner le composé titre sous la forme d'un solide blanc (rendement 35%).

RMN ¹H (MeOH d₄) : δ 3,37 (d, 2H, *J* = 7,75 Hz) ; 3,93 (t, 1H, *J* = 7,75 Hz) ; 7,32 (s, 1H) ; 7,49 (m, 3H) ; 7,94 (m, 2H).

RMN ¹³C (MeOH d₄) : δ 31,17 ; 52,76 ; 116,79 ; 127,47 ; 127,62 ; 130,20 ; 131,51 ; 131,66 ; 134,03 ; 154,62 ; 172,25.

Masse haute résolution *m*/*z* pour C₁₃H₁₂NO₄S (M+H⁺)⁺, calculée 278,0496 ; mesurée 278,0488.

### Acide 2-((5-phényl-1,2,4-oxadiazol-3-yl)méthyl) malonique 2e

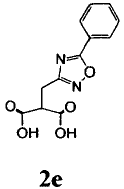

Préparé à partir du triester **le** selon le protocole de saponification pour donner le composé titre sous la forme d'un solide blanc (rendement 30 %).

RMN ¹H (MeOH d₄) : δ 3,35 (d, 2H, *J* = 7,5Hz) ; 3,99 (t, 1H, *J* = 7,5Hz). 7,55-7,66 (m, 3H) ; 8,12 (m, 2H).

RMN ¹³C (MeOH d₄) : δ 26,54 ; 125,21 ; 128,99 ; 129,03 ; 130,41 ; 134,19; 170,21 ; 171,62 ; 177,05.

Masse haute résolution *m*/*z* pour C₁₂H₁₁N₂O₅ (M+H⁺)⁺, calculée 263,0668 ; mesurée 263,0661.

### Acide 2-(biphényl-4-ylméthyl) malonique 2f

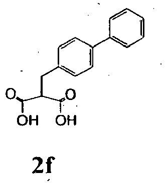

Préparé à partir du triester 1**f** selon le protocole de saponification pour donner le composé titre sous la forme d'un solide blanc (rendement 66%).

RMN ¹H (MeOH d₄) : δ 3,20 (d, 2H, *J* = 8Hz) ; 3,67 (t, 1H, *J* = 8Hz) ; 7,30-7,59 (m, 9H).

RMN ¹³C (MeOH d₄) : δ 35,50 ; 55,02 ; 127,85 ; 128,02; 128,20 ; 129,82 ; 130,38 ; 138,87 ; 140,81 ; 142,16 ; 172,50.

Masse haute résolution *m*/*z* pour C₁₆H₁₄NaO₄ (M+Na⁺)⁺, calculée 293,0790 ; mesurée 293,0797.

### Acide 2-(4-(pyrimidin-2-yl)benzyl) malonique 2g

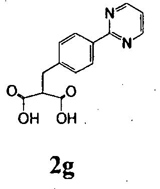

Préparé à partir du triester **1g** selon le protocole de saponification pour donner le composé titre sous la forme d'un solide blanc (rendement 59%).

RMN ¹H (MeOH d₄) : δ 3,22 (d, 2H, *J* = 7,75Hz) ; 3,69 (t, 1H, *J* = 7,75Hz) ; 7,33 (m, 3H) ; 8,28 (d, 2H, *J =* 8,25Hz) ; 8,79 (d, *2H, J=* 4,75Hz).

RMN ¹³C (MeOH d₄) : δ 35,60; 54,70 ; 120,62 ; 129,30 ; 130,22 ; 137,09 ; 142,97 ; 158,68 ; 165,60 ; 172,40.

Masse haute résolution *m*/*z* pour C₁₄H₁₃N₂O₄ (M+H⁺)⁺, calculée 273,0875 ; mesurée 273,0881.

### Acide 2-(4-phenoxybenzyl) malonique 2h

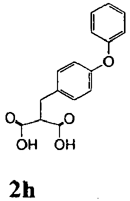

Préparé à partir du triester **1h** selon le protocole de saponification pour donner le composé titre sous la forme d'un solide blanc (rendement 78%).

RMN ¹H (MeOH d₄) : δ 3,14 (d, 2H*, J* = 7,75Hz) ; 3,62 (t, 1H, *J* = 7,75Hz) ; 6,74 (d, 2H, *J* = 8,5Hz) ; 6,95 (d, 2H*, J* = 8Hz) ; 7,08 (t, 1H, *J* = 7,25Hz) ; 7,23 (d, 2H, *J* = 8,5Hz) ; 7,33 (m, 2H).

RMN ¹³C (MeOH d₄) : δ 35,05; 55,01 ; 119,65 ; 119,70 ; 124,23 ; 130,82 ; 131,32 ; 134,37 ; 134,70 ; 157,32 ; 158,80 ; 172,47.

Masse haute résolution *m*/*z* pour C₁₆H₁₄NaO₅ (M+Na⁺)⁺, calculée 309,0739 ; mesurée 309,0726.

### Acide 2-(4-(phénoxyméthyl)benzyl) malonique 2i

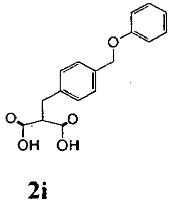

Préparé à partir du triester **1i** selon le protocole de saponification pour donner le composé titre sous la forme d'un solide blanc (rendement 73%).

RMN ¹H (MeOH d₄) : δ 3,16 (m, 2H) ; 3,63 (t, 1H, *J* = 8Hz) ; 5,03 (s 3H) ; 6,93 (m, 3H); 7,20-7,37 (m, 6H).

RMN ¹³C (MeOH d4): δ 35,48 ; 70,60 ; 115,84 ; 121,87 ; 128,77 ; 129,99 ; 130,44 ; 137,06 ; 139,39 ; 160,18 ; 172,51.

Masse haute résolution *m*/*z* pour C₁₇H₁₆NaO₅ (M+Na⁺)⁺, calculée 323,0895 ; mesurée 323,0903.

### Acide 2-(4-(thiophèn-2-yl)benzyl) malonique 2j

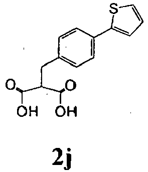

Préparé à partir du triester **1j** selon le protocole de saponification pour donner le composé titre sous la forme d'un solide blanc (rendement 68%).

RMN ¹H (MeOH d₄) : δ 3,16 (d, 2H, *J* = 7,75Hz) ; 3,65 (t, 1H, *J* = 7,75Hz) ; 7,06 (m, 1H) ; 7,26 (d, 2H, *J=* 8,25Hz) ; 7,33 (m, 2H) ; 7,54 (d, 2H, *J=* 8,25Hz).

RMN ¹³C (MeOH d₄) : 34,06 ; 53,47 ; 122,55 ; 124,14 ; 125,34 ; 127,61 ; 129,06 ; 129,09 ; 132,76 ; 137,70 ; 143,80 ; 170,97.

Masse haute résolution *m*/*z* pour C₁₄H₁₃O₄S (M+H⁺)⁺, calculée 277,0535 ; mesurée 277,0538.

### Acide 2-(4-(1H-pyrrol-1-yl)benzyl) malonique 2k

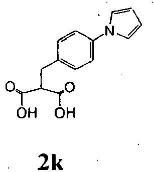

Préparé à partir du triester **1k** selon le protocole de saponification pour donner le composé titre sous la forme d'un solide blanc (rendement 84%).

RMN ¹H (MeOH d₄) : δ 3,17 (d, 2H, *J* = 8Hz) ; 3,65 (t, 1H, *J* = 8Hz) ; 6,25 (m, 2H) ; 7,14 (m, 2H) ; 7,35 (m, 4H).

RMN ¹³C (MeOH d₄) : 33,76 ; 53,56 ; 109,80 ; 109,84 ; 118,51 ; 119,53 ; 119,55 ; 129,70 ; 129,74 ; 135,53 ; 139,32 ; 170,94.

Masse haute résolution *m*/*z* pour C₁₄H₁₄NO₄ (M+H⁺)⁺, calculée 260,0923 ; mesurée 260,0908.

### Acide 2-(4-(thiazol-2-yl)benzyl) malonique 2l

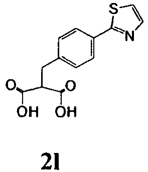

Préparé à partir du triester **1l** selon le protocole de saponification pour donner le composé titre sous la forme d'un solide blanc (rendement 84%).

RMN ¹H (MeOH d₄) : δ 3,19 (d, 2H, *J* = 7,5Hz) ; 3,68 (t, 1H, *J* = 7,5Hz) ; 7,35 (d, *2H, J=* 8,25Hz) ; 7,54 (d, 1H, *J* = 3,25Hz) ; 7,82 (m, 3H).

RMN ¹³C (MeOH d₄) : 34,17 ; 53,25 ; 119,20 ; 126,33 ; 129,35 ; 131,46 ; 141,08 ; 142,81 ; 168,66 ; 170,83.

Masse haute résolution *m*/*z* pour C₁₃H₁₂NO₄S (M+H⁺)⁺, calculée 278,0487 ; mesurée 278,0483.

### Acide 2-(4-(1-méthyl-1H-pyrazol-3-yl)benzyl) malonique 2m

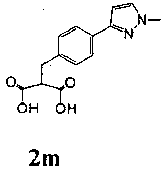

Préparé à partir du triester **1m** selon le protocole de saponification pour donner le composé titre sous la forme d'un solide blanc (rendement 94%).

RMN ¹H (MeOH d₄) : δ 3,16 (d, 2H, *J* = 7,75Hz) ; 3,64 (t, 1H, *J* = 7,75Hz) ; 3,88 (s, 3H) ; 6,55 (d, 1H, *J* = 2,25Hz) ; 7,26 (d, 2H, *J* = 8Hz) ; 7,54 (d, 1H, *J* = 2,25Hz) ; 7,65 (d, 2H, *J* = 8Hz).

RMN ¹³C (MeOH d₄) : δ 34,17 ; 37,37 ; 53,53 ; 102,41 ; 125,26 ; 128,77 ; 128,80 ; 131,51 ; 132,07 ; 137,93 ; 151,37 ; 171,02.

Masse haute résolution *m*/*z* pour C₁₄H₁₅N₂O₄ (M+H⁺)⁺, calculée 275,1032 ; mesurée 275,1020.

### Acide 2-(4-(1,2,3-thiadiazol-4-yl)benzyl) malonique 2n

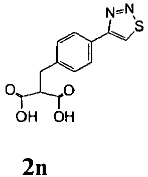

Préparé à partir du triester **1n** selon le protocole de saponification pour donner le composé titre sous la forme d'un solide blanc (rendement 70%).

RMN ¹H (MeOH d₄) : δ 3,23 (d, 2H, *J* = 7,75Hz) ; 3,71 (t, 1H, *J* = 7,75Hz) ; 7,40 (d, 2H, *J=* 8,25Hz) ; 7,99 (d, 2H, *J* = 8,25Hz) ; 9,14 (s, 1H).

RMN ¹³C (MeOH d₄) : δ 34,18; 53,39; 126,99 ; 129,23 ; 129,33 ; 131,04 ; 139,85 ; 162,43 ; 170,92.

Masse haute résolution *m*/*z* pour C₁₂H₁₁N₂O₄S (M+H⁺)⁺, calculée 279,0440 ; mesurée 279,0434.

### Acide 2-(4-(5-méthyl-1,2,4-oxadiazol-3-yl)benzyl) malonique 2o

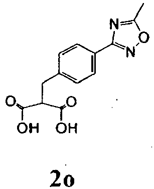

Préparé à partir du triester **1o** selon le protocole de saponification pour donner le composé titre sous la forme d'un solide blanc (rendement 89%).

RMN ¹H (MeOH d₄) : δ 2,64 (s, 3H) ; 3,22 (d, 2H, *J* = 7,75Hz) ; 3,69 (t, 1H, *J* = 7,75Hz) ; 7,40 (d, 2H, *J* = 8Hz) ; 7,94 (d, *2H, J=* 8,25Hz).

RMN ¹³C (MeOH d₄) : δ 10,62 ; 34,26 ; 53,22 ; 124,92 ; 126,90 ; 129,16 ; 142,07 ; 167,82 ; 170,79; 177,28.

Masse haute résolution *m*/*z* pour C₁₃H₁₃N₂O₅ (M+H⁺)⁺, calculée 277,0824 ; mesurée 277,0831.

### Synthèse des pseudo-peptides 25-27, 28-29, 31, 32-37, 40-52 et 80-90.

Synthèse des pseudo-peptides 25-27, 28-29, 31, 32-37, 40-50 et 80-88, sur lanterne Synphase possédant un lien de type « Rink amide » : Une stratégie Fmoc standard a été utilisée pour construire la séquence peptidique. Les lanternes sont préalablement gonflées dans le DCM pendant 15 minutes. Le groupement protecteur Fmoc

est déprotégé sous irradiations micro-ondes (3 x 3min, 60°C, 25W) en présence de pipéridine à 20% dans le DMF (Diméthyle formamide). Après lavage des lanternes (DMF/2 x 5 min puis DCM/2 x 5 min) et pré-activation des acides aminés à température ambiante pendant 5 minutes (10 eq de Fmoc-AA-OH, 10 eq de Cl-HOBt et 10 eq de DIC dans le DMF anhydre), les lanternes sont immergées dans la solution de couplage et la réaction est effectuée sous irradiation micro-ondes (10 min, 60°C, 25W). Ce couplage est réalisé une deuxième fois. Ce cycle de déprotection du groupement Fmoc et d'incorporation d'un acide aminé est répété une deuxième fois pour la synthèse des pseudo-dipeptedides. Finalement les blocs maloniques précurseurs **(2b-2o)** sont incorporés de la façon suivante : pré-activation du bloc malonique en présence de DIC (5 eq) et de Cl-HOBt (5 eq) dans le DMF anhydre pendant 5 minutes à température ambiante puis immersion des lanternes dans la solution de couplage. La réaction est ensuite réalisée sous irradiation micro-ondes (10 min, 60°C, 25W). Enfin les lanternes sont lavées (DMF/2 x 5min puis DCM/2x5min).

Synthèse des pseudo-peptides 51-52 et 89-90 sur Lanterne Synphase incorporant un lien de type « hydroxyméthylphénoxy » : les acides aminés non naturels Fmoc-3-aminophénylacétique (10 eq) ou (S)-Fmoc-(3-carboxyméthyl)-pipéridine (10eq) sont préalablement activés en présence de DIC (5eq) dans une solution de DCM anhydre/DMF anhydre (9/1) pendant 10 minutes à température ambiante. Les lanternes, parallèlement gonflées dans le DCM, sont ensuite immergées dans la solution de couplage. Du DMAP (0,5 eq) est ajouté et le mélange réactionnel est doucement agité pendant une heure à température ambiante. Les lanternes sont ensuite lavées (DMF/2 x 5 min puis DCM/2 x 5 min) et les acides aminés naturels ainsi que les blocs maloniques précurseurs sont incorporés comme décrit au-dessus.

### Réaction de cyclo-addition 1,3 dipolaire et accès aux pseudo-peptides 3-23 et 24.

Après construction de la séquence peptidique et incorporation du bloc malonique **2a** comme décrit précédemment, une réaction de cyclo-addition 1,3 dipolaire est réalisée sur support solide.

Accès au pseudo-peptide 3-23 : le motif isoxazole est généré selon la méthode mise au point au laboratoire et décrite par Makaritis A. *et al* (Makaritis A. et al 2003 Chem. Eur. J. (9)). L'oxime précurseur (10 eq) est dissout dans du DCM anhydre et deux gouttes de pyridine sont ajoutées. NCS (10 eq) est ensuite ajouté à température ambiante et après 10 min d'agitation le mélange réactionnel est chauffé pendant une heure à 45°C. Après refroidissement, les lanternes sont immergées dans le mélange réactionnel et de la triéthylamine est ajoutée (20 eq). Après 12 heures d'agitation douce à température ambiante, cette opération est à nouveau répétée avec un mélange réactionnel fraîchement préparé. Les lanternes sont enfin lavées (DMF/2 x 5 min et DCM/2 x 5 min).

Accès au pseudo-peptide 24 : les lanternes sont immergées dans un mélange réactionnel contenant de l'azoture de phényle (10 eq), une solution d'iodure de cuivre (I) dans du THF (2 eq théorique à partir d'une solution dont la concentration est estimée à 0,18 M) et de la triéthylamine (50 eq). La réaction de cyclo-addition est alors effectuée sous irradiation micro-ondes en tube scellé (80°C, 10 min, 300W). Les lanternes sont enfin lavées (DMF/2 x 5 min et DCM/2 x 5 min).

### Réaction de Suziki ou réaction de Sonogashira sur support solide, accès aux pseudo-peptides 38-39, 53-79, 91-107 et 30.

Après construction de la séquence peptidique et incorporation du bloc malonique **2b** comme décrit précédemment, une réaction de couplage au palladium sur support solide est réalisée comme suit.

Réaction de Suzuki : Les lanternes sont immergées dans un mélange réactionnel contenant un précurseur de type acide boronique ou ester pinacolique (10 eq, 0,2 M dans du DMF préalablement dégazé), du carbonate de potassium (10 eq, 0,16M dans l'eau MilliQ) et du Pd(PPh₃)₄ (1 eq, 0,08 M dans du DMF préalablement dégazé). La réaction de couplage est alors effectuée sous irradiation micro-ondes en tube scellé (80°C, 5 min, 300W). Les lanternes sont enfin lavées (DMF/2 x 5 min et DCM/2 x 5 min).

Réaction de Sonogashira : Les lanternes sont immergées dans un mélange réactionnel contenant du phénylacétylène (10 eq, 0,2 M dans du DMF préalablement dégazé), du Pd (PPh₃)₄ (1 eq, 0,08 M dans du DMF préalablement dégazé) et de l'iodure de cuivre (1 eq) dans une solution de DMF/DIEA (1/1). La réaction de couplage est alors effectuée sous irradiation micro ondes en tube scellé (80°C, 30 min, 300 W). Les lanternes sont enfin lavées (DMF/2 x 5 min et DCM/2 x 5 min).

### Clivage du support solide, purification, caractérisation, conditionnement et stockage des pseudo-peptides.

Chaque pseudo-peptide synthétisé comme décrit ci-dessus est ensuite clivé de son support comme suit. La lanterne est immergée dans une solution de clivage (TFA/TIS/H₂O : 95/2,5/2,5). Après 1 heure d'agitation à température ambiante, la lanterne est transférée dans une nouvelle solution de clivage (TFA/DCM : 1/1) et agitée pendant trente minutes à température ambiante. Les deux solutions de clivage sont ensuite regroupées, évaporées sous pression réduite et le brut réactionnel est repris dans une solution A/B : 1/1 avec A : 0,1% de TFA dans 90% d'eau MilliQ/10% d'acétonitrile et B : 0,09% de TFA dans 90% d'acétonitrile/10% d'eau MilliQ. Chaque pseudo-peptide est ensuite purifié en HPLC phase inverse sur une colonne semi-préparative de type AIT C18 Kromasil (250 x 20 mm, débit = 3mL.min⁻¹, Détection UV à 230 nm) en utilisant un gradient linéaire comme suit : de 0 à 40 min : de 0 à 100% de B, avec A : 0,1% de TFA dans 90% d'eau MilliQ/10% d'acétonitrile, et B : 0,09% de TFA dans 90% d'acétonitrile/10% d'eau MilliQ. Après lyophilisation, chaque pseudo peptide est repris dans une solution d'éthanol absolu/eau MilliQ : 1/1. La solution est neutralisée (pH = 7-8) à l'aide d'une solution de NaHCO₃ à 1M. La concentration de chaque solution est déterminée par analyse de la composition en acides aminés. Toutes les solutions contenant les pseudo-peptides sont stockées au réfrigérateur à +4°C. Les données analytiques pour chaque pseudo-peptide sont résumées dans le tableau III ci-après.

**Tableau III**

| | Formule | Nom | Données analytiques |
|---|---|---|---|
| **3** | 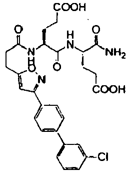 | Acide (*S*)-5-amino-4-((*S*)-4-carboxy-2-(3-(3-(3'-chlorobiphényl-4-yl)isoxazol-5-yl)propanamido)butanamido)-5-oxopentanoïque | Ascentis Express : |
| | | | t_{R}=5,40min |
| | | | ε₂₇₂=31915 M⁻¹.cm⁻¹ RMN ¹H (DMSO d₆) : δ 1,75 (m, 2H) ; 1,89 (m, 2H) ; 2,23 (m, 4H) ; 2,62 (m, 2H) ; 3,03 (m, 2H) ; 4,21 (m, 2H) ; 6,86 (s, 1H) ; 7,11 (s, 1H) ; 7,32 (s, 1H) ; 7,51 (m, 2H) ; 7,72 (d, 1H, *J*=7,25Hz) ; 7,84 (m, 3H) ; 7,94 (d, 2H, *J*=8,25Hz) ; 8,00 (d, 1H, *J*=7,75Hz) ; 8,28 (d, 1H, *J*=7,25Hz) ; |
| | | | Masse haute résolution *m*/*z* pour C₂₈H₃₀ClN₄O₈ (M+H⁺)⁺ : calculée 585,1752 ; mesurée 585,1733. |
| **4** | 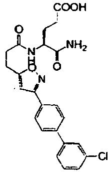 | Acide (*S*)-5-amino-4-(3-(3-(3'-chlorobiphényl-4-yl)isoxazol-5-yl)propanamido)-5-oxopentanoïque | Ascentis Express: |
| | | | t_{R}=5,69min |
| | | | ε₂₇₂=18230 M⁻¹.cm⁻¹ |
| | | | Masse haute résolution *m*/*z* pour C₂₃H₂₃ClN₃O₅ (M+H⁺)⁺ : calculée 456,1326 ; mesurée 456,1330. |
| **5** | 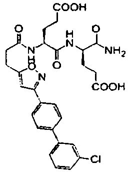 | Acide (*R*)-5-amino-4-((*S*)-4-carboxy-2-(3-(3-(3'-chlorobiphényl-4-yl)isoxazol-5-yl)propanamido)butanamido)-5-oxopentanoïque | Ascentis Express: |
| | | | t_{R}=5,46min |
| | | | ε₂₇₂=29100 M⁻¹.cm⁻¹ |
| | | | Masse haute résolution *m*/*z* pour C₂₈H₃₀ClN₄O₈ (M+H⁺)⁺ : calculée 585,1752 ; mesurée 585,1746. |
| **6** | 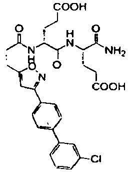 | Acide (*S*)-5-amino-4-((*R*)-4-carboxy-2-(3-(3-(3'-chlorobiphényl-4-yl)isoxazol-5-yl)propanamido)butanamido)-5-oxopentanoïque | Ascentis Express : |
| | | | t_{R}=5,47min |
| | | | ε₂₇₂=29240 M⁻¹.cm⁻¹ |
| | | | Masse haute résolution *m*/*z* pour C₂₈H₃₀ClN₄O₈ (M+H⁺)⁺ : calculée 585,1752 ; mesurée 585,1746. |
| **7** | 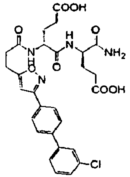 | Acide (*R*)-5-amino-4-((*R*)-4-carboxy-2-(3-(3-(3'-chlorobiphényl-4-yl)isoxazol-5-yl)propanamido)butanamido)-5-oxopentanoïque | Ascentis Express : |
| | | | t_{R}=5,49min |
| | | | ε₂₇₂=29834 M⁻¹.cm⁻¹ |
| | | | Masse haute résolution *m*/*z* pour C₂₈H₃₀ClN₄O₈ (M+H⁺)⁺ : calculée 585,1752 ; mesurée 585,1741. |
| **8** | 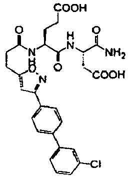 | Acide (*S*)-5-((*S*)-1-amino-3-carboxy-1-oxopropan-2-ylamino)-4-(3-(3-(3'-chlorobiphény)-4-yl)isoxazol-5-yl)propanamido)-5-oxopentanoïque | Ascentis Express : |
| | | | t_{R}=5,47min |
| | | | ε₂₇₂=32941 M⁻¹.cm⁻¹ |
| | | | Masse haute résolution *m*/*z* pour C₂₇H₂₈ClN₄O₈ (M+H⁺)⁺ : calculée 571,1595 ; mesurée 571,1594. |
| **9** | 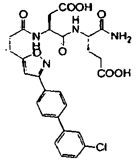 | Acide (*S*)-5-amino-4-((*S*)-3-carboxy-2-(3-(3-(3'-chlorobiphényl-4-yl)isoxazol-5-yl)propanamido)propanamido)-5-oxopentanoïque | Ascentis Express : |
| | | | t_{R}=5,49min |
| | | | ε₂₇₂=30769 M⁻¹.cm⁻¹ |
| | | | Masse haute résolution *m*/*z* pour C₂₇H₂₈ClN₄O₈ (M+H⁺)⁺ : calculée 571,1555 ; mesurée 571,1598. |
| **10** | 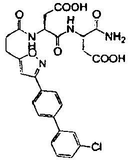 | Acide (*S*)-4-amino-3-((*S*)-3-carboxy-2-(3-(3-(3'-chlorobiphényl-4-yl)isoxazol-5-yl)propanamido)propanamido)-4-oxobutanoïque | Ascentis Express : |
| | | | t_{R}=5,51min |
| | | | ε₂₇₂=31507 M⁻¹.cm⁻¹ |
| | | | Masse haute résolution *m*/*z* pour C₂₆H₂₆ClN₄O₈ (M+H⁺)⁺ : calculée 557,1438 ; mesurée 557,1453. |
| **11** | 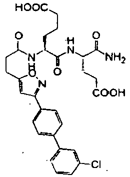 | Acide (*S*)-6-((*S*)-1-amino-4-carboxy-1-oxobutan-2-ylamino)-5-(3-(3-(3'-chlorobiphényl-4-yl)isoxazol-5-yl)propanamido)-6-oxohexanoïque | Ascentis Express : |
| | | | t_{R}=5,53min |
| | | | ε₂₇₂=30827 M⁻¹.cm⁻¹ |
| | | | Masse haute résolution *m*/*z* pour C₂₉H₃₂ClN₄O₈₈ (M+H⁺)⁺ : calculée 599,1909 ; mesurée 599,1897. |
| **12** | 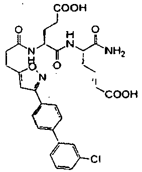 | Acide (*S*)-6-amino-5-(*S*)-4-carboxy-2-(3-(3-(3'-chlorobiphényl-4-yl)isoxazol-5-yl)propanamido)butanamido)-6-oxohexanoïque | Ascentis Express : |
| | | | t_{R}=5,51min |
| | | | ε₂₇₂=31655 M⁻¹.cm⁻¹ |
| | | | Masse haute résolution *m*/*z* pour C₂₉H₃₂ClN₄O₈₈ (M+H⁺)⁺ : calculée 599,1909 ; mesurée 599,1905. |
| **13** | 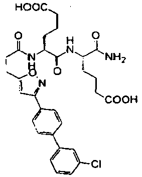 | Acide (*S*)-6-amino-5-((*S*)-5-carboxy-2-(3-(3-(3'-chlorobiphényl-4-yl)isoxazol-5-yl)propanamido)pentanamido)-6-oxohexanoïque | Ascentis Express : |
| | | | t_{R}=5,57min |
| | | | ε₂₇₂=31915 M⁻¹.cm⁻¹ |
| | | | Masse haute résolution *m*/*z* pour C₃₀H₃₄ClN₄O₈ (M+H⁺)⁺ : calculée 613,2065 ; mesurée 613,2077. |
| **14** | 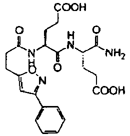 | Acide (*S*)-5-amino-4-((*S*)-4-carboxy-2-(3-(3-phénylisoxazol-5-yl)propanamido)butanamido)-5-oxopentanoïque | Ascentis Express : |
| | | | t_{R}=2,89min |
| | | | ε₂₄₁=11950 M⁻¹.cm⁻¹ |
| | | | Masse haute résolution *m*/*z* pour C₂₂H₂₇N₄O₈₈ (M+H⁺)⁺ : calculée 475,1828 ; mesurée 475,1823. |
| **15** | 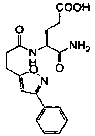 | Acide (*S*)-5-amino-5-oxo-4-(3-(3-phénylisoxazol-5-yl)propanamido)pentanoïque | Ascentis Express : |
| | | | t_{R}=3,26min |
| | | | ε₂₄₁=5996 M⁻¹.cm⁻¹ |
| | | | Masse haute résolution *m*/*z* pour C₁₇H₂₀N₃O₅ (M+H⁺)⁺ : calculée 346,1403 ; mesurée 346,1395. |
| **16** | 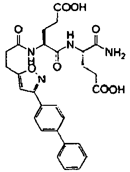 | Acide (*S*)-5-amino-4-((*S*)-2-(3-(3-(biphényl-4-yl)isoxazol-5-yl)propanamido)-4-carboxybutanamido)-5-oxopentanoïque | Ascentis Express : |
| | | | t_{R}=4,82min |
| | | | ε₂₇₃=35600 M⁻¹.cm⁻¹ |
| | | | Masse haute résolution *m*/*z* pour C₂₈H₃₁N₄O₈ (M+H⁺)⁺ : calculée 551,2142 ; mesurée 551,2135. |
| **17** | 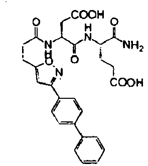 | Acide (*S*)-5-amino-4-((*S*)-2-(3-(3-(biphényl-4-yl)isoxazol-5-yl)propanamido)-3-carboxypropanamido)-5-oxopentanoïque | Ascentis Express : |
| | | | t_{R}=4,84min |
| | | | ε₂₇₃=28375 M⁻¹.cm⁻¹ |
| | | | Masse haute résolution *m*/*z* pour C₂₇H₂₉N₄O₈ (M+H⁺)⁺ : calculée 537,1985 ; mesurée 537,1996. |
| **18** | 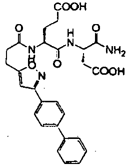 | Acide (*S*)-5-((*S*)-1-amino-3-carboxy-1-oxopropan-2-ylamino)-4-(3-(3-(biphényl-4-yl)isoxazol-5-yl)propanamido)-5-oxopentanoïque | Ascentis Express : |
| | | | t_{R}=4,85min |
| | | | ε₂₇₃=25370 M⁻¹.cm⁻¹ |
| | | | Masse haute résolution *m*/*z* pour C₂₇H₂₉N₄O₈ (M+H⁺)⁺ : calculée 537,1985 ; mesurée 537,1995. |
| **19** | 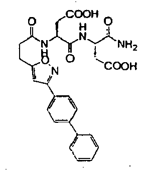 | Acide (*S*)-4-amino-3-((*S*)-2-(3-(3-(biphényl-4-yl)isoxazol-5-yl)propanamido)-3-carboxypropanamido)-4-oxobutanoïque | Ascentis Express : |
| | | | t_{R}=4,85min |
| | | | ε₂₇₃=28120 M⁻¹.cm⁻¹ |
| | | | Masse haute résolution *m*/*z* pour C₂₆H₂₇N₄O₈ (M+H⁺)⁺ : calculée 523,1828; mesurée 523,1810. |
| **20** | 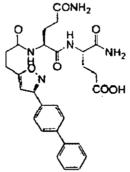 | Acide (*S*)-5-amino-4-((*S*)-5-amino-2-(3-(3-(biphényl-4-yl)isoxazol-5-yl)propanamido)-5-oxopentanamido)-5-oxopentanoïque | Ascentis Express : |
| | | | t_{R}=4,75min |
| | | | ε₂₇₃=35825 M⁻¹.cm⁻¹ |
| | | | Masse haute résolution *m*/*z* pour C₂₈H₃₂N₅O₇ (M+H⁺)⁺ : calculée 550,2302, mesurée 550,2319. |
| **21** | 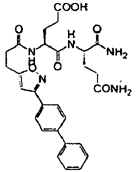 | Acide (*S*)-4-(3-(3-(biphényl-4-yl)isoxazol-5-y))propanamido)-5-((*S*)-1,5-diamino-1,5-dioxopentan-2-ylamino)-5-oxopentanoïque | Ascentis Express : |
| | | | t_{R}=4,77min |
| | | | ε₂₇₃=34482 M⁻¹.cm⁻¹ |
| | | | Masse haute résolution *m*/*z* pour C₂₈H₃₂N₅O₇ (M+H⁺)⁺ : calculée 550,2302; mesurée 550,2299. |
| **22** | 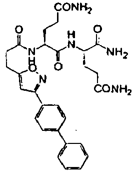 | (*S*)-2-(3-(3-(biphényl-4-yl)isoxazol-5-yl)propanamido)-*N*¹-((*S*)-1,5-diamino-1,5-dioxopentan-2-yl)pentanediamide | Ascentis Express : |
| | | | t_{R}=4,39min |
| | | | ε₂₇₃=23214 M⁻¹.cm⁻¹ |
| | | | Masse haute résolution *m*/*z* pour C₂₈H₃₃N₆O₆ (M+H⁺)⁺ : calculée 549,2462; mesurée 549,2454. |
| **23** | 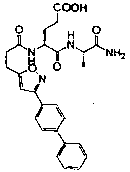 | Acide (*S*)-5-((*S*)-1-amino-1-oxopropan-2-ylamino)-4-(3-(3-(biphényl-4-yl)isoxazol-5-yl)propanamido)-5-oxopentanoïque | Ascentis Express : |
| | | | t_{R}=5,00min |
| | | | ε₂₇₃=34078 M⁻¹.cm⁻¹ |
| | | | Masse haute résolution *m*/*z* pour C₂₆H₂₉N₄O₆ (M+H⁺)⁺ : calculée 493,2087 ; mesurée 493,2093. |
| **24** | 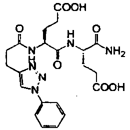 | Acide (*S*)-5-amino-4-((*S*)-4-carboxy-2-(3-(1-phényl-1*H*-1,2,3-triazol-4-yl)propanamido)butanamido)-5-oxopentanoïque | Ascentis Express : |
| | | | t_{R}=2,23min |
| | | | ε₂₄₈=4827 M⁻¹.cm⁻¹ |
| | | | Masse haute résolution *m*/*z* pour C₂₁H₂₇N₆O₇ (M+H⁺)⁺ : calculée 475,1941; mesurée 475,1953. |
| **25** | 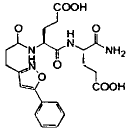 | Acide (*S*)-5-amino-4-((*S*)-4-carboxy-2-(3-(5-phénylisoxazol-3-yl)propanamido)butanamido)-5-oxopentanoïque | Ascentis Express : |
| | | | t_{R}=3,27min |
| | | | ε₂₆₃=23750 M⁻¹.cm⁻¹ |
| | | | Masse haute résolution *m*/*z* pour C₂₂H₂₇N₄O₈ (M+H⁺)⁺ : calculée 475,1829 ; mesurée 475,1843. |
| **26** | 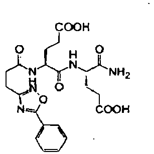 | Acide (*S*)-5-amino-4-((*S*)-4-carboxy-2-(3-(5-phényl-1,2,4-oxadiazol-3-yl)propanamido)butanamido)-5-oxopentanoïque | Ascentis Express : |
| | | | t_{R}=3,32min |
| | | | ε₂₅₃=17391 M⁻¹.cm⁻¹ |
| | | | Masse haute résolution *m*/*z* pour C₂₁H₂₆N₅O₈ (M+H⁺)⁺ : calculée 476,1782 ; mesurée 476,1794. |
| **27** | 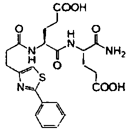 | Acide (*S*)-5-amino-4-((*S*)-4-carboxy-2-(3-(2-phénylthiazol-4-yl)propanamido)butanamido)-5-oxopentanoïque | Ascentis Express : |
| | | | t_{R}=3,23min |
| | | | ε₂₉₄=13992 M⁻¹.cm⁻¹ |
| | | | Masse haute résolution *m*/*z* pour C₂₂H₂₇N₄O₇S (M+H⁺)⁺ : calculée 491,1601 ; mesurée 491,1613. |
| **28** | 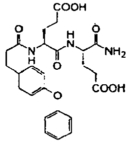 | Acide (*S*)-5-amino-4-((*S*)-4-carboxy-2-(3-(4-phénoxyphényl)propanamido)butanamido)-5-oxopentanoïque | Ascentis Express : |
| | | | t_{R}=4,73min |
| | | | ε₂₇₂=1593 M⁻¹.cm⁻¹ |
| | | | Masse haute résolution *m*/*z* pour C₂₅H₃₀N₃O₈ (M+H⁺)⁺ : calculée 500,2033 ; mesurée 500,2024. |
| **29** | 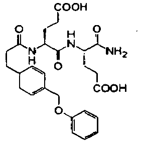 | Acide (*S*)-5-amino-4-((*S*)-4-carboxy-2-(3-(4-(phénoxyméthyl)phényl)propanamido)butanamido)-5-oxopentanoïque | Ascentis Express : |
| | | | t_{R}=3,87min |
| | | | ε₂₇₄=3584 M⁻¹.cm⁻¹ |
| | | | Masse haute résolution *m*/*z* pour C₂₆H₃₁N₃NaO₈ (M+Na⁺)⁺ : calculée 536,2009 ; mesurée 536,2000. |
| **30** | 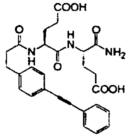 | Acide (*S*)-5-amino-4-((*S*)-4-carboxy-2-(3-(4-(phényléthynyl)phényl)propanamido)butanamido)-5-oxopentanoïque | Ascentis Express : |
| | | | t_{R}=4,93min |
| | | | ε₂₈₄=42452 M⁻¹.cm⁻¹ |
| | | | ε₃₀₂=37736 M⁻¹.cm⁻¹ |
| | | | Masse haute résolution *m*/*z* pour C₂₇H₃₀N₃O₇ (M+H⁺)⁺ : calculée 508,2084 ; mesurée 508,2076. |
| **31** | 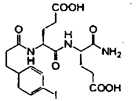 | Acide (*S*)-5-amino-4-((*S*)-4-carboxy-2-(3-(4-iodophényl)propanamido)butanamido)-5-oxopentanoïque | Ascentis Express : |
| | | | t_{R}=3,69min |
| | | | ε₂₅₉=370 M⁻¹.cm⁻¹ |
| | | | Masse haute résolution *m*/*z* pour C₁₉H₂₅IN₃O₇ (M+H⁺)⁺ : calculée 534,0737 ; mesurée 534,0734. |
| **32** | 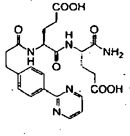 | Acide (*S*)-5-amino-4-((*S*)-4-carboxy-2-(3-(4-(pyrimidin-2-yl)phényl)propanamido)butanamido)-5-oxopentanoïque | Ascentis Express : |
| | | | t_{R}=2,40min |
| | | | ε₂₆₆=20275 M⁻¹.cm⁻¹ |
| | | | Masse haute résolution *m*/*z* pour C₂₃H₂₈N₅O₇ (M+H⁺)⁺ : calculée 486,1989 ; mesurée 486,1982. |
| **33** | 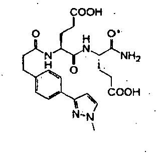 | Acide (*S*)-5-amino-4-((*S*)-4-carboxy-2-(3-(4-(1-méthyl-1*H*-pyrazol-3-yl)phényl)propanamido)butanamido)-5-oxopentanoïque | Ascentis Express : |
| | | | t_{R}=2,60min |
| | | | ε₂₅₇=24934 M⁻¹.cm⁻¹ |
| | | | Masse haute résolution *m*/*z* pour C₂₃H₃₀N₅O₇ (M+M⁺)⁺ : calculée 488,2145 ; mesurée 488,2143. |
| **34** | 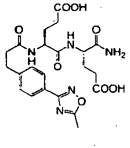 | Acide (*S*)-5-amino-4-((*S*)-4-carboxy-2-(3-(4-(5-méthyl-1,2,4-oxadiazol-3-yl)phényl)propanamido)butanamido)-5-oxopentanoïque | Ascentis Express : |
| | | | t_{R}=2,77min |
| | | | ε₂₄₆=15544 M⁻¹.cm⁻¹ |
| | | | Masse haute résolution *m*/*z* pour C₂₂H₂₈N₅O₈ (M+H⁺)⁺ : calculée 490,1938 ; mesurée 490,1922. |
| **35** | 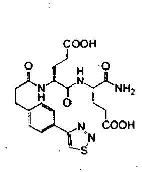 | Acide (*S*)-4-(3-(4-(1,2,3-thiadiazol-4-yl)phényl)propanamido)-5-((*S*)-1-amino-4-carboxy-1-oxobutan-2-ylamino)-5-oxopentanoïque | Ascentis Express : |
| | | | t_{R}=3,27min |
| | | | ε₂₄₅=10729 M⁻¹.cm⁻¹ |
| | | | Masse haute résolution *m*/*z* pour C₂₁H₂₆N₅O₇S (M+H⁺)⁺ : calculée 492,1553 ; mesurée 492,1551. |
| **36** | 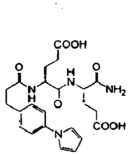 | Acide (*S*)-4-(3-(4-(1*H*-pyrrol-1-yl)phényl)propanamido)-5-((*S*)-1-amino-4-carboxy-1-oxobutan-2-ylamino)-5-oxopentanoïque | Ascentis Express : |
| | | | t_{R}=3,69min |
| | | | ε₂₅₃=14677 M⁻¹.cm⁻¹ |
| | | | Masse haute résolution *m*/*z* pour C₂₃H₂₈N₄NaO₇ (M+Na⁺)⁺ : calculée 495,1856 ; mesurée 495,1860. |
| **37** | 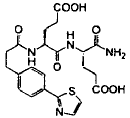 | Acide (*S*)-5-amino-4-((*S*)-4-carboxy-2-(3-(4-(thiazol-2-yl)phényl)propanamido)butanamido)-5-oxopentanoïque | Ascentis Express : |
| | | | t_{R}=2,69min |
| | | | ε₂₈₈=27972 M⁻¹. cm⁻¹ |
| | | | Masse haute résolution *m*/*z* pour C₂₂H₂7N₄O₇S (M+H⁺)⁺ : calculée 491,1601 ; mesurée 491,1585. |
| **38** | 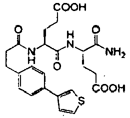 | Acide (*S*)-5-amino-4-((*S*)-4-carboxy-2-(3-(4-(thiophèn-3-yl)phényl)propanamido)butanamido)-5-oxopentanoïque | Ascentis Express : |
| | | | t_{R}=3,97min |
| | | | ε₂₆₂=14495 M⁻¹. cm⁻¹ |
| | | | Masse haute résolution *m*/*z* pour C₂₃H₂₈N₃O₇S (M+H⁺)⁺ : calculée 490,1648 ; mesurée 490,1636. |
| **39** | 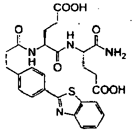 | Acide (*S*)-5-amino-4-((*S*)-2-(3-(4-(benzo[*d*]thiazol-2-yl)phényl)propanamido)-4-carboxybutanamido)-5-oxopentanoïque | Ascentis Express : |
| | | | t_{R}=5,15min |
| | | | ε₂₇₇=28729 M⁻¹. cm⁻¹ |
| | | | ESI *m*/*z* (M+H⁺)⁺=540.1 |
| **40** | 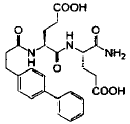 | Acide (*S*)-5-amino-4-((*S*)-2-(3-(biphényl-4-yl)propanamido)-4-carboxybutanamido)-5-oxopentanoïque | Ascentis Express : |
| | | | t_{R}=4,26min |
| | | | ε₂₅₃=22540 M⁻¹. cm⁻¹ |
| | | | RMN ¹H (DMSO d₆): δ 1,71 (m, 2H); 1,87 (m, 2H) ; 2,22 (m, 4H) ; 2,51 (m, 2H) ; 2,85 (m, 2H) ; 4,21 (m, 2H) ; 7,12 (s, 1H) ; 7,33 (m, 4H) ; 7,45 (t, 2H, *J*=7,25Hz) 7,56 (d, 2H, *J*=7,25Hz) ; 7,63 (d, 2H, *J*=7,25Hz) ; 7,96 (d, 1H, *J*=7,75Hz) ; 8,13 (d, 1H, *J*=7,5Hz). |
| | | | Masse haute résolution *m*/*z* pour C₂₅H₃₀N₃O₇ (M+H⁺)⁺ : calculée 484,2084 ; mesurée 484,2084. |
| **42** | 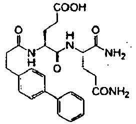 | Acide (*S*)-4-(3-(biphényl-4-yl)propanamido)-5-((*S*)-1,5-diamino-1,5-dioxopentan-2-ylamino)-5-oxopentanoïque | Ascentis Express : |
| | | | t_{R}=4,10min |
| | | | ε₂₅₃=25357M⁻¹.cm⁻¹ |
| | | | Masse haute résolution *m*/*z* pour C₂₅H₃₁N₄O₆ (M+H⁺)⁺ : calculée. 483,2244 ; mesurée 483,2246. |
| **43** | 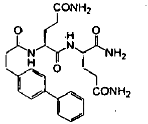 | (*S*)-2-(3-(biphényl-4-yl)propanamido)-*N*¹-((*S*)-1,5-diamino-1,5-dioxopentan-2-y))pentanediamide | Ascentis Express : |
| | | | t_{R}=3,83min |
| | | | ε₂₅₃=45205M⁻¹.cm⁻¹ |
| | | | Masse haute résolution *m*/*z* pour C₂₅H₃₂N₅O₅ (M+H⁺)⁺ : calculée 482,2403 ; mesurée 482,2397. |
| **44** | 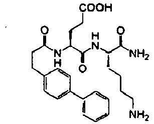 | Acide (*S*)-4-(3-(biphényl-4-yl)propanamido)-5-((*S*)-1,6-diamino-1-oxohexan-2-ylamino)-5-oxopentanoïque | Ascentis Express : |
| | | | t_{R}=3,69min |
| | | | ε₂₅₃=22540M⁻¹.cm⁻¹ |
| | | | Masse haute résolution *m*/*z* pour C₂₆H₃₅N₄O₅ (M+H⁺)⁺ : calculée 483,2607 ; mesurée 483,2598 |
| **45** | 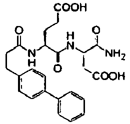 | Acide (*S*)*-*5*-*((*S*)-1-amino-3-carboxy-1-oxopropan-2-ylam ino)-4-(3-(biphényl-4-yl)propanamido)-5-oxopentanoïque | Ascentis Express : |
| | | | t_{R}=4,07min |
| | | | ε₂₅₃=22540M⁻¹.cm⁻¹ |
| | | | Masse haute résolution *m*/*z* pour C₂₄H₂₈N₃O₇ (M+H⁺)⁺ : calculée 470,1927 ; mesurée 470,1928 |
| **46** | 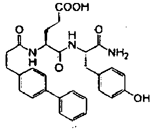 | Acide (*S*)-5-((*S*)-1-amino-3-(4-hydroxyphényl)-1-oxopropan-2-ylamino)-4-(3-(biphényl-4-yl)propanamido)-5-oxopentanoïque | Ascentis Express : |
| | | | t_{R}=4,58min |
| | | | ε₂₅₃=22540M⁻¹.cm⁻¹ |
| | | | Masse haute résolution *m*/*z* pour C₂₉H₃₂N₃O₆ (M+H⁺)⁺ : calculée 518,2281 ; mesurée 518,2281 |
| **47** | 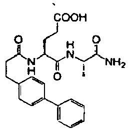 | Acide (*S*)-5-((*S*)-1-amino-1-oxopropan-2-ylamino)-4-(3-(biphényl-4-yl)propanamido)-5-oxopentanoïque | Ascentis Express : |
| | | | t_{R}=4,39min |
| | | | ε₂₅₃=22540M⁻¹.cm⁻¹ |
| | | | Masse haute résolution *m*/*z* pour C₂₃H₂₈N₃O₅ (M+H⁺)⁺ : calculée 426,2028 ; mesurée 426,2025 |
| **48** | 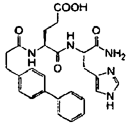 | Acide (*S*)-5-((*S*)-1-amino-3-(1*H*-imidazol-4-yl)-1-oxopropan-2-ylamino)-4-(3-(biphényl-4-yl)propanamido)-5-oxopentanoïque | Ascentis Express : |
| | | | t_{R}=3,64min |
| | | | ε₃₅₃₌22540M⁻¹.cm⁻¹ |
| | | | Masse haute résolution *m*/*z* pour C₂₆H₃₀N₅O₅ (M+H⁺)⁺ : calculée 492,2247 ; mesurée 492,2259 |
| **49** | 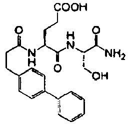 | Acide (*S*)-5-((*S*)-1-amino-3-hydroxy-1-oxopropan-2-ylamino)-4-(3-(biphényl-4-yl)propanamido)-5-oxopentanoïque | Ascentis Express : |
| | | | t_{R}=4,02min |
| | | | ε₂₅₃=22540M⁻¹.cm⁻¹ |
| | | | Masse haute résolution *mlz* pour C₂₃H₂₈N₃O₆ (M+H⁺)⁺ : calculée 442,1978 ; mesurée 442,1975 |
| **50** | 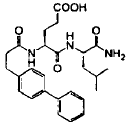 | Acide (*S*)-5-((*S*)-1-amino-4-méthyl-1-oxopentan-2-ylamino)-4-(3-(biphényl-4-yl)propanamido)-5-oxopentanoïque | Ascentis Express : |
| | | | t_{R}=5,75min |
| | | | ε₂₅₃=22540M⁻¹.cm⁻¹ |
| | | | Masse haute résolution *m*/*z* pour C₂₆H₃₄N₃O₅ (M+H⁺)⁺ : calculée 468,2498 ; mesurée 468,2491 |
| **51** | 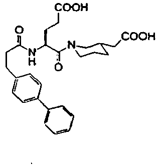 | Acide (*S*)-4-(3-(biphényl-4-yl)propanamido)-5-((*S*)-3-(carboxyméthyl)piperidin-1-yl)-5-oxopentanoïque | Ascentis Express : |
| | | | t_{R}=5,16min |
| | | | ε₂₅₃=35271M⁻¹.cm⁻¹ |
| | | | Masse haute résolution *m*/*z* pour C₂₇H₃₃N₂O₆ (M+H⁺)⁺ : calculée 481,2339 ; mesurée 481,2338 |
| **52** | 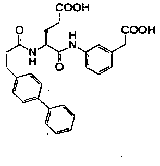 | Acide (*S*)-4-(3-(biphényl-4-yl)propanamido)-5-(3-(carboxyméthyl)phénylamino)=5-oxopentanoïque | Ascentis Express : |
| | | | t_{R}=5,51min |
| | | | ε₂₅₀=18309M⁻¹.cm⁻¹ |
| | | | Masse haute résolution *m*/*z* pour C₂₈H₂₈N₂NaO₆ (M+Na⁺)⁺ : calculée 511,1845 ; mesurée 511,1855 |
| **53** | 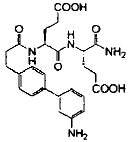 | Acide (*S*)-5-amino-4-((*S*)-2-(3-(3'-aminobiphényl-4-yl)propanamido)-4-carboxybutanamido)-5-oxopentanoïque | Ascentis Express : |
| | | | t_{R}=1,93min |
| | | | ε₂₆₀=9580M⁻¹.cm⁻¹ |
| | | | Masse haute résolution *m*/*z* pour C₂₅H₃₀N₄NaO₇ (M+Na⁺)⁺ : calculée 521,2012 ; mesurée 521,2019 |
| **54** | 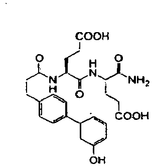 | Acide (*S*)-5-amino-4-((*S*)-4-carboxy-2-(3-(3'-hydroxybiphényl-4-yl)propanamido)butanamido)-5-oxopentanoïque | Ascentis Express : |
| | | | t_{R}=3,24min |
| | | | ε₂₅₄=13970M⁻¹.cm⁻¹ |
| | | | Masse haute résolution *m*/*z* pour C₂₅H₃₀N₃O₈ (M+H⁺)⁺ : calculée 500,2033 ; mesurée 500,2031 |
| **55** | 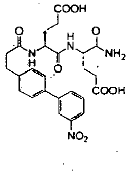 | Acide (*S*)-5-amino-4-((*S*)-4-carboxy-2-(3-(3'-nitrobiphényl-4-yl)propanamido)butanamido)-5-oxopentanoïque | Ascentis Express : |
| | | | t_{R}=4,20min |
| | | | ε₂₅₄=20158M⁻¹.cm⁻¹ |
| | | | Masse haute résolution *m*/*z* pour C₂₅H₂₉N₄O₉ (M+H⁺)⁺ : calculée 529,1935 ; mesurée 529,1954 |
| **56** | 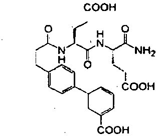 | Acide 4'-(3-((*S*)-1-((*S*)-1-amino-4-carboxy-1-oxobutan-2-ylamino)-4-carboxy-1-oxobutan-2-ylamino)-3-oxopropyl)biphényl-3-carboxylique | Ascentis Express : |
| | | | t_{R}=3,29min |
| | | | ε₂₅₇=6082M⁻¹.cm⁻¹ |
| | | | Masse haute résolution *m*/*z* pour C₂₆H₃₀N₃O₉ (M+H⁺)⁺ : calculée 528,1982 ; mesurée 528,1991 |
| **57** | 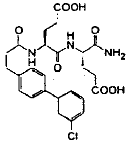 | Acide (*S*)-5-amino-4-((*S*)-4-carboxy-2-(3-(3'-chlorobiphényl-4-yl)propanamido)butanamido)-5-oxopentanoïque | Ascentis Express : |
| | | | t_{R}=4,84min |
| | | | ε₂₆₀=18195M⁻¹.cm⁻¹ |
| | | | Masse haute résolution *m*/*z* pour C₂₅H₂₉ClN₃O₇ (M+Na⁺)⁺ : calculée 540,1513 ; mesurée 540,1482 |
| **58** | 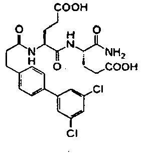 | Acide (*S*)-5-amino-4-((*S*)-4-carboxy-2-(3-(3',5'-dichlorobiphényl-4-yl)propanamido)butanamido)-5-oxopentanoïque | Ascentis Express : |
| | | | t_{R}=5,48min |
| | | | ε₂₆₀=21808M⁻¹.cm⁻¹ |
| | | | Masse haute résolution *mlz* pour C₂₅H₂₈Cl₂N₃O₇ (M+H⁺)⁺ : calculée 552,1304 ; mesurée 552,1320 |
| **59** | 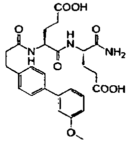 | Acide (*S*)-5-amino-4-((*S*)-4-carboxy-2-(3-(3'-methoxybiphényl-4-y))propanamido)butanamido)-5-oxopentanoïque | Ascentis Express : |
| | | | t_{R}=4,37min |
| | | | ε₂₅₄=12000M⁻¹.cm⁻¹ |
| | | | Masse haute résolution *m*/*z* pour C₂₆H₃₂N₃O₈ (M+H⁺)⁺ : calculée 514,2189 ; mesurée 514,2177 |
| **60** | 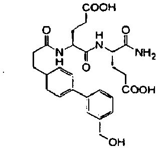 | Acide (*S*)-5-amino-4-((*S*)-4-carboxy-2-(3-(3'-(hydroxyméthyl)biphényl-4-yl)propanamido)butanamido)-5-oxopentanoïque | Ascentis Express : |
| | | | t_{R}=3,21min |
| | | | ε₂₅₄=21560M⁻¹.cm⁻¹ |
| | | | Masse haute résolution *m*/*z* pour C₂₆H₃₂N₃O₈ (M+H⁺)⁺ : calculée 514,2189 ; mesurée 514,2164 |
| **61** | 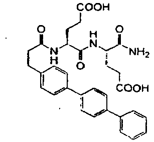 | Acide (*S*)-5-amino-4-((*S*)-4-carboxy-2-(3-(4'-phénylbiphényl-4-yl)propanamido)butanamido)-5-oxopentanoïque | Ascentis Express : |
| | | | t_{R}=5,70min |
| | | | ε₂₈₀=44827M⁻¹.cm⁻¹ |
| | | | RMN ¹H (DMSO d₆): δ 1,74 (m, 2H) ; 1,90 (m, 2H) ; 2,21 (m, 4H) ; 2,51 (m, 2H) ; 2,83 (m, 2H) ; 4,19 (m, 2H) ; 7,11 (s, 1H) ; 7,31 (m, 3H) ; 7,38 (d, 1H, *J*=7,25Hz) ; 7,48 (t, 2H, *J*=7,25Hz) ; 7,64 (d, 2H, *J*=8Hz) ; 7,72 (m, 6H) ; 7,95 (d, 1H, *J*=7,75Hz) ; 8,14 (d, 1H, *J*=7,5Hz). |
| | | | Masse haute résolution *m*/*z* pour C₃₁H₃₃N₃NaO₇ (M+Na⁺)⁺ : calculée 582,2216 ; mesurée 582,2210 |
| **62** | 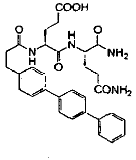 | Acide (*S*)-5-((*S*)-1,5-diamino-1,5-dioxopentan-2-ylamino)-4-(3-(4'-phénylbiphényl-4-yl)propanamido)-5-oxopentanoïque | Ascentis Express : |
| | | | t_{R}=5,40min |
| | | | ε₂₈₀=44827M⁻¹.cm⁻¹ |
| | | | Masse haute résolution *m*/*z* pour C₃₁H₃₄N₄NaO₆ (M+Na⁺)⁺ : calculée 581,2376 ; mesurée 581,2388 |
| **63** | 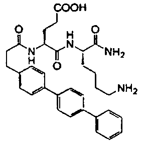 | Acide (*S*)-5-((*S*)-1,6-diamino-1-oxohexan-2-ylamino)-4-(3-(4'-phénylbiphényl-4-yl)propanamido)-5-oxopentanoïque | Ascentis Express : |
| | | | t_{R}=5,17min |
| | | | ε₂₅₄=44827M⁻¹.cm⁻¹ |
| | | | Masse haute résolution m/z pour C₃₂H₃₉N₄O₅ (M+H⁺)⁺ : calculée |
| | | | 559,2920 ; mesurée 559,2921 |
| **64** | 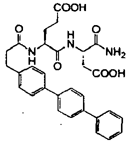 | Acide (*S*)-5-((*S*)-1-amino-3-carboxy-1-oxopropan-2-ylamino)-4-(3-(4'-phénylbiphényl-4-yl)propanamido)-5-oxopentanoïque | Ascentis Express : |
| | | | t_{R}=5,72min |
| | | | ε₂₈₀=44827M⁻¹.cm⁻¹ |
| | | | Masse haute résolution *m*/*z* pour C₃₀H₃₁N₃NaO₇ (M+Na⁺)⁺ : calculée 568,2060 ; mesurée 568,2058 |
| **65** | 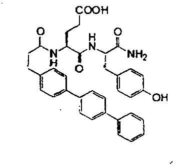 | Acide (*S*)-5-((*S*)-1-amino-3-(4-hydroxyphényl)-1-oxopropan-2-ylamino)-4-(3-(4'-phénylbiphényl-4-yl)propanamido)-5-oxopéntanoïque | Ascentis Express : |
| | | | t_{R}=6,07min |
| | | | ε₂₈₀=44827M⁻¹.cm⁻¹ |
| | | | Masse haute résolution *m*/*z* pour C₃₅H₃₅N₃NaO₆ (M+Na⁺)⁺ : calculée 616,2423 ; mesurée 616,2426 |
| **66** | 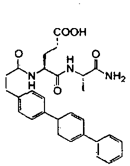 | Acide (*S*)*-*5*-*((*S*)-1-amino-1-oxopropan-2-ylamino)-4-(3-(4'-phénylbiphényl-4-yl)propanamido)-5-oxopentanoïque | Ascentis Express : |
| | | | t_{R}=5,99min |
| | | | ε₂₈₀=44827M⁻¹.cm⁻¹ |
| | | | Masse haute résolution *m*/*z* pour C₂₉H₃₁N₃NaO₅ (M+Na⁺)⁺ : calculée 524,2161 ; mesurée 524,2156 |
| **67** | 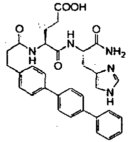 | Acide (*S*)-5-((*S*)-1-amino-3-(1*H*-imidazol-4-yl)-1-oxopropan-2-ylamino)-4-(3-(4'-phénylbiphényl-4-yl)propanamido)-5-oxopentanoïque | Ascentis Express : |
| | | | t_{R}=5,21min |
| | | | ε₂₅₄=44827M⁻¹.cm⁻¹ |
| | | | Masse haute résolution *m*/*z* pour C₃₂H₃₄N₅O₅ (M+H⁺)⁺ : calculée 568,2560 ; mesurée 568,2579 |
| **68** | 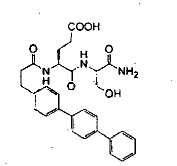 | Acide (*S*)-5-((*S*)-1-amino-3-hydroxy-1-oxopropan-2-ylamino)-4-(3-(4'-phénylbiphényl-4-yl)propanamido)-5-oxopentanoïque | Ascentis Express : |
| | | | t_{R}=5,61min |
| | | | ε₂₈₀=44827M⁻¹.cm⁻¹ |
| | | | Masse haute résolution *m*/*z* pour C₂₉H₃₁N₃NaO₆ (M+Na⁺)⁺ : calculée 540,2111 ; mesurée 540,2100 |
| **69** | 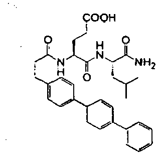 | Acide (*S*)-5-(*S*)-1-amino-4-méthyl-1-oxopentan-2-ylamino)-4-(3-(4'-phénylbiphényl-4-yl)propanamido)-5-oxopentanoïque | Ascentis Express : |
| | | | t_{R}=6,69min |
| | | | ε₂₈₀=44827M⁻¹.cm⁻¹ |
| | | | Masse haute résolution *m*/*z* pour C₃₂H₃₇N₃NaO₅ (M+Na⁺)⁺ : calculée 566,2631 ; mesurée 566,2610 |
| **70** | 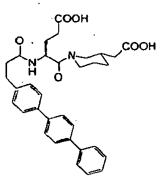 | Acide (*S*)-5-((*S*)-3-(carboxyméthyl)piperidin-1-yl)-4-(3-(4'-phénylbiphényl-4-yl)propanamido)-5-oxopentanoïque | Ascentis Express : |
| | | | t_{R}=6,63min |
| | | | ε₂₈₀=56364M⁻¹.cm⁻¹ |
| | | | Masse haute résolution *m*/*z* pour C₃₃H₃₆N₂NaO₆ (M+Na⁺)⁺ : calculée 579,2471 ; mesurée 579,2453 |
| **71** | 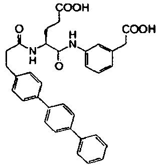 | Acide (*S*)-5-(3-(carboxyméthyl)phénylamino)-4-(3-(4'-phénylbiphényl-4-yl)propanamido)-5-oxopentanoïque | Ascentis Express : |
| | | | t_{R}=6,85min |
| | | | ε₂₈₀=53398M⁻¹.cm⁻¹ |
| | | | Masse haute résolution *m*/*z* pour C₃₃H₃₆N₂NaO₆ (M+Na⁺)⁺ : calculée 587,2158 ; mesurée 587,2138 |
| **72** | 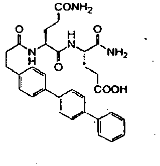 | Acide (*S*)-5-amino-4-((*S*)-5-amino-2-(3-(4'-phénylbiphényl-4-yl)propanamido)-5-oxopentanamido)-5-oxopentanoïque | Ascentis Express : |
| | | | t_{R}=5,51min |
| | | | ε₂₈₀=44827M⁻¹.cm⁻¹ |
| | | | Masse haute résolution *m*/*z* pour C₃₁H₃₄N₄NaO₆ (M+Na⁺)⁺ : calculée 581,2376 ; mesurée 581,2386 |
| **73** | 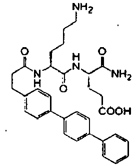 | Acide (*S*)-5-amino-4-((*S*)-6-amino-2-(3-(4'-phénylbiphényl-4-yl)propanamido)hexanamido)-5-oxopentanoïque | Ascentis Express : |
| | | | t_{R}=5,14min |
| | | | ε₂₅₄=44827M⁻¹.cm⁻¹ |
| | | | Masse haute résolution *m*/*z* pour C₃₂H₃₉N₄O₅ (M+H⁺)⁺ : calculée 559,2920 ; mesurée 559,2944 |
| **74** | 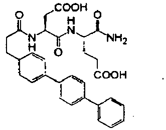 | Acide (*S*)-5-amino-4-((*S*)-3-carboxy-2-(3-(4'-phénylbiphényl-4-yl)propanamido)propanamido)-5-oxopentanoïque | Ascentis Express : |
| | | | t_{R}=5,73min |
| | | | ε₂₈₀=44827M⁻¹.cm⁻¹ |
| | | | Masse haute résolution *m*/*z* pour C₃₀H₃₁N₃NaO₇ (M+Na⁺)⁺ : calculée 568,2060 ; mesurée 568,2068 |
| **75** | 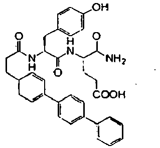 | Acide (*S*)-5-amino-4-((*S*)-3-(4-hydroxyphényl)-2-(3-(4'-phénylbiphényl-4-yl)propanamido)propanamido)-5-oxopentanoïque | Ascentis Express : |
| | | | t_{R}=6,17min |
| | | | ε₂₈₀=44827M⁻¹.cm⁻¹ |
| | | | Masse haute résolution *m*/*z* pour C₃₅H₃₅N₃NaO₆ (M+Na⁺)⁺ : calculée 616,2423 ; mesurée 616,2435 |
| **76** | 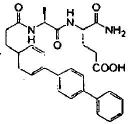 | Acide (*S*)-5-amino-4-((*S*)-2-(3-(4'-phénylbiphényl-4-yl)propanamido)propanamido)-5-oxopentanoïque | Ascentis Express : |
| | | | t_{R}=5,96min |
| | | | ε₂₈₀=44827M⁻¹.cm⁻¹ |
| | | | Masse haute résolution *m*/*z* pour C₂₉H₃₁N₃NaO₅ (M+Na⁺)⁺ : calculée 524,2161 ; mesurée 524,2178 |
| **77** | 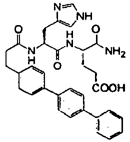 | Acide (*S*)-4-((*S*)-3-(1*H*-imidazol-4-yl)-2-(3-(4'-phénylbiphényl-4-yl)propanamido)propanamido)-5-amino-5-oxopentanoïque | Ascentis Express : |
| | | | t_{R}=5,25min |
| | | | ε₂₅₄=44827M⁻¹.cm⁻¹ |
| | | | Masse haute résolution *m*/*z* pour C₃₂H₃₄N₅O₅ (M+H⁺)⁺ : calculée 568,2560 ; mesurée 568,2554 |
| **78** | 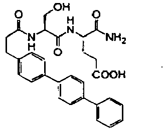 | Acide (*S*)-5-amino-4-((*S*)-3-hydroxy-2-(3-(4'-phénylbiphényl-4-yl)propanamido)propanamido)-5-oxopentanoïque | Ascentis Express : |
| | | | t_{R}=5,81min |
| | | | ε₂₈₀=44827M⁻¹.cm⁻¹ |
| | | | Masse haute résolution *m*/*z* pour C₂₉H₃₁N₃NaO₆ (M+Na⁺)⁺ : calculée 540,2111 ; mesurée 540,2080 |
| **79** | 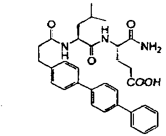 | Acide (*S*)-5-amino-4-((*S*)-4-méthyl-2-(3-(4'-phénylbiphényl-4-yl)propanamido)pentanamido)-5-oxopentanoïque | Ascentis Express : |
| | | | t_{R}=6,74min |
| | | | ε₂₈₀=44827M⁻¹.cm⁻¹ |
| | | | Masse haute résolution *m*/*z* pour C₃₂H₃₇N₃NaO₅ (M+Na⁺)⁺ : calculée 566,2631 ; mesurée 566,2617 |
| **80** | 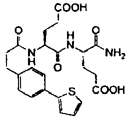 | Acide (*S*)-5-amino-4-((*S*)-4-carboxy-2-(3-(4-(thiophèn-2-yl)phényl)propanamido)butanamido)-5-oxopentanoïque | Ascentis Express : |
| | | | t_{R}=4,10min |
| | | | ε₂₈₅=22000M⁻¹.cm⁻¹ |
| | | | RMN ¹H (DMSO d₆) : δ 1,75 (m, 2H) ; 1,91 (m, 2H) ; 2,22 (m, 4H) ; 2,51 (m, 2H) ; 2,82 (m, 2H) ; 4,20 (m, 2H) ; 7,11 (m, 2H) ; 7,25 (d, 2H, *J*=8Hz) ; 7,32 (s, 1H) ; 7,46 (d, 1H, *J*=3,5Hz); 7,51 (d, 1H, *J*=5,25Hz) ; 7,56 (d, 2H, *J*=8Hz) ; 7,95 (d, 1 H, *J*=8Hz) ; 8,14 (d, 1H, *J*=7,5Hz). |
| | | | Masse haute résolution *m*/*z* pour C₂₃H₂₇N₃NaO₇S (M+Na⁺)⁺ : calculée 512,1467 ; mesurée 512,1458. |
| **81** | 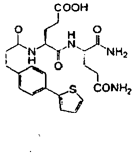 | Acide (*S*)-5-((*S*)-1,5-diamino-1,5-dioxopentan-2-ylamino)-5-oxo-4-(3-(4-(thiophèn-2-yl)phényl)propanamido)pentanoïque | Ascentis Express : |
| | | | t_{R}=3,85min |
| | | | ε₂₈₅=20000M⁻¹.cm⁻¹ |
| | | | Masse haute résolution *m*/*z* pour C₂₃H₂₉N₄O₆S (M+H⁺)⁺ : calculée 489,1808 ; mesurée 489,1795 |
| **82** | 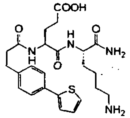 | Acide (*S*)-5-((*S*)-1,6-diamino-1-oxohexan-2-ylamino)-5-oxo-4-(3-(4-(thiophèn-2-yl)phényl)propanamido)pentanoïque | Ascentis Express : |
| | | | t_{R}=4,10min |
| | | | ε₂₈₅=18487M⁻¹.cm⁻¹ |
| | | | Masse haute résolution *m*/*z* pour C₂₄H₃₃N₄O₅S (M+H⁺)⁺ : calculée 489,2172 ; mesurée 489,2190 |
| **83** | 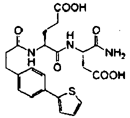 | Acide (*S*)-5-((*S*)-1-amino-3-carboxy-1-oxopropan-2-ylamino)-5-oxo-4-(3-(4-(thiophèn-2-yl)phényl)propanamido)pentanoïque | Ascentis Express : |
| | | | t_{R}=4,55min |
| | | | ε₂₈₅=18156M⁻¹.cm⁻¹ |
| | | | Masse haute résolution *m*/*z* pour C₂₂H₂₅N₃NaO₇S (M+Na⁺)⁺ : calculée 498,1311 ; mesurée 498,1314 |
| **84** | 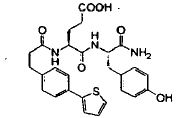 | Acide (*S*)-5-((*S*)-1-amino-3-(4-hydroxyphényl)-1-oxopropan-2-ylamino)-5-oxo-4-(3-(4-(thiophèn-2-yl)phényl)propanamido)pentanoïque | Ascentis Express : |
| | | | t_{R}=4,52min |
| | | | ε₂₈₅= 22000M⁻¹.cm⁻¹ |
| | | | Masse haute résolution *m*/*z* pour C₂₇H₂₉N₃NaO₆S (M+Na⁺)⁺ : calculée 546,1674 ; mesurée 546,1661 |
| **85** | 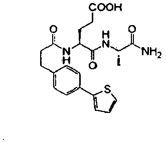 | Acide (*S*)-5-((*S*)-1-amino-1-oxopropan-2-ylamino)-5-oxo-4-(3-(4-(thiophèn-2-yl)phényl)propanamido)pentanoïque | Ascentis Express : |
| | | | t_{R}=4,18min |
| | | | ε₂₈₅=18627M⁻¹.cm⁻¹ |
| | | | Masse haute résolution *m*/*z* pour C₂₁H₂₆N₃O₅S (M+H⁺)⁺ : calculée 432,1593 ; mesurée 432,1580 |
| **86** | 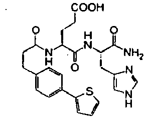 | Acide (*S*)-5-((*S*)-1-amino-3-(1*H*-imidazol-4-yl)-1-oxopropan-2-ylamino)-5-oxo-4-(3-(4-(thiophèn-2-yl)phényl)propanamido)pentanoïque | Ascentis Express : |
| | | | t_{R}=3,69min |
| | | | ε₂₈₅=19433M⁻¹.cm⁻¹ |
| | | | Masse haute résolution *m*/*z* pour C₂₄H₂₈N₅O₅S (M+H⁺)⁺ : calculée 498,1811 ; mesurée 498,1807 |
| **87** | 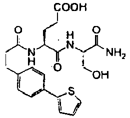 | Acide (*S*)-5-((*S*)-1-amino-3-hydroxy-1-oxopropan-2-ylamino)-5-oxo-4-(3-(4-(thiophèn-2-yl)phényl)propanamido)pentanoïque | Ascentis Express : |
| | | | t_{R}=3,94min |
| | | | ε₂₈₅=15886M⁻¹.cm⁻¹ |
| | | | Masse haute résolution *m*/*z* pour C₂₁H₂₆N₁O₆S (M+H⁺)⁺ : calculée 448,1542 ; mesurée 448,1542 |
| **88** | 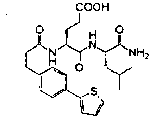 | Acide (*S*)-5-((*S*)-1-amino-4-méthyl-1-oxopentan-2-ylamino)-5-oxo-4-(3-(4-(thiophèn-2-yl)phényl)propanamido)pentanoïque | Ascentis Express : |
| | | | t_{R}=5,13min |
| | | | ε₂₈₅= 20833M⁻¹.cm⁻¹ |
| | | | Masse haute résolution *m*/*z* pour C₂₄H₃₁N₃NaO₅S (M+Na⁺)⁺ : calculée 496,1882 ; mesurée 496,1878 |
| **89** | 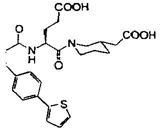 | Acide (*S*)-5-((*S*)-3-(carboxyméthyl)piperidin-1-yl)-5-oxo-4-(3-(4-(thiophèn-2-yl)phényl)propanamido)pentanoïque | Ascentis Express : |
| | | | t_{R}=4,97min |
| | | | ε₂₈₈=28274M⁻¹.cm⁻¹ |
| | | | Masse haute résolution *m*/*z* pour C₂₅H₃₁N₂O₆S (M+H⁺)⁺ : calculée 487,1903 ; mesurée 487,1894 |
| **90** | 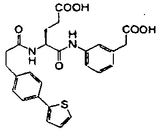 | Acide (*S*)-5-(3-(carboxyméthyl)phénylamino)-5-oxo-4-(3-(4-(thiophèn-2-yl)phényl)propanamido)pentanoïque | Ascentis Express : |
| | | | t_{R}=5,30min |
| | | | ε₂₄₆= 16666M⁻¹.cm⁻¹ |
| | | | ε₂₈₂= 25000M⁻¹.cm⁻¹ |
| | | | Masse haute résolution *m*/*z* pour C₂₆H₂₆N₂NaO₆S (M+Na⁺)⁺ : calculée 517,1409 ; mesurée 517,1390 |
| **91** | 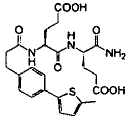 | Acide (*S*)-5-amino-4-((*S*)-4-carboxy-2-(3-(4-(5-méthylthiophèn-2-yl)phényl)propanamido)butanamido)-5-oxopentanoïque | Ascentis Express : |
| | | | t_{R}⁼₄,61min |
| | | | ε₂₉₂= 21893M⁻¹.cm⁻¹ |
| | | | Masse haute résolution *m*/*z* pour C₂₄H₂₉N₃NaO₇S (M+Na⁺)⁺: calculée 526,1624 ; mesurée 526,1620 |
| **92** | 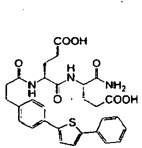 | Acide (*S*)-5-amino-4-((*S*)-4-carboxy-2-(3-(4-(5-phénylthiophèn-2-yl)phényl)propanamido)butanamido)-5-oxopentanoïque | Ascentis Express : |
| | | | t_{R}=5,72min |
| | | | ε₃₃₈= 30000M⁻¹.cm⁻¹ |
| | | | RMN ¹H (DMSO d₆): δ 1,76 (m, 2H) ; 1,91 (m, 2H) ; 2,22 (m, 4H) ; 2,51 (m, 2H) ; 2,82 (m, 2H) ; 4,21 (m, 2H) ; 7,11 (s, 1H) ; 7,30 (m, 4H) ; 7,44 (t, 2H, J=7,25Hz) ; 7,49 (d, 1H, *J*=4Hz) ; 7,54 (d, 1H, *J*=3,75Hz) ; 7,60 (d, 2H, *J*=8Hz) ; 7,70 (d, 2H, *J*=7,5Hz) ; 7,96 (d, 1H, *J*=7,75Hz) ; 8,16 (d, 1H, *J*=7,25Hz). |
| | | | Masse haute résolution *m*/*z* pour C₂₉H₃₁N₃NaO₇S (M+Na⁺)⁺ : calculée 588,1780 ; mesurée 588,1776 |
| **93** | 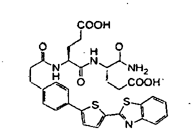 | Acide (*S*)-5-amino-4-((*S*)-2-(3-(4-(5-(benzo[*d*]thiazol-2-yl)thiophèn-2-yl)phényl)propanamido)-4-carboxybutanamido)-5-oxopentanoïque | Ascentis Express : |
| | | | t_{R}=4,97min |
| | | | ε₃₂₀=46154M⁻¹.cm⁻¹ |
| | | | Masse haute résolution *m*/*z* pour C₃₀H₃₁N₄O₇S₂ (M+H⁺)⁺ : calculée 623,1634 ; mesurée 623,1616 |
| **94** | 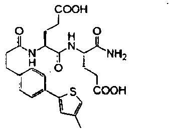 | Acide (*S*)-5-amino-4-((*S*)-4-carboxy-2-(3-(4-(4-méthylthiophèn-2-yl)phényl)propanamido)butanamido)-5-oxopentanoïque | Ascentis Express : |
| | | | t_{R}=4,58min |
| | | | ε₂₉₀=16369M⁻¹.cm⁻¹ |
| | | | Masse haute résolution *m*/*z* pour C₂₄H₃₀N₃O₇S (M+H⁺)⁺ : calculée 504,1804 ; mesurée 504,1782 |
| **95** | 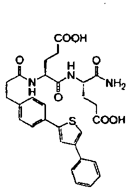 | Acide (*S*)-5-amino-4-((*S*)-4-carboxy-2-(3-(4-(4-phénylthiophèn-2-yl)phényl)propanamido)butanamido)-5-oxopentanoïque | Ascentis Express : |
| | | | t_{R}=5,61min |
| | | | ε₂₅₉=34583M⁻¹.cm⁻¹ |
| | | | RMN ¹H (DMSO d₆): δ 1,76 (m, 2H) ; 1,91 (m, 2H) ; 2,22 (m, 4H) ; 2,51 (m, 2H) ; 2,84 (m, 2H) ; 4,21 (m, 2H) 7,11 (s, 1H) ; 7,30 (m, 4H), 7,44 (t, 2H, *J*=7Hz) ; 7,65 (d, 2H, *J*=8,25Hz) ; 7,79 (d, 2H, *J*=7,25Hz) ; 7,85 (s, 1H) ; 7,96 (m, 2H) ; 8,16 (d, 1H, *J*=7,5Hz). |
| | | | Masse haute résolution *m*/*z* pour C₂₉H₃₂N₃O₇S (M+H⁺)⁺ : calculée 566,1961 ; mesurée 566,1953 |
| **96** | 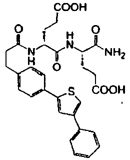 | Acide (*S*)-5-amino-4-((*R*)-4-carboxy-2-(3-(4-(4-phénylthiophèn-2-yl)phényl)propanamido)butanamido)-5-oxopentanoïque | Ascentis Express : |
| | | | t_{R}=5,64min |
| | | | ε₂₅₉= 36111M⁻¹.cm⁻¹ |
| | | | Masse haute résolution *m*/*z* pour C₂₉H₃₁N₃NaO₇S (M+Na⁺)⁺ : calculée 588,1780 ; mesurée 588,1770 |
| **97** | 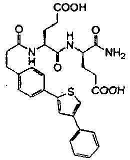 | Acide (*R*)-5-amino-4-((*S*)-4-carboxy-2-(3-(4-(4-phénylthioph'en-2-yl)phényl)propanamido)butanamido)-5-oxopentanoïque | Ascentis Express : |
| | | | t_{R}=5,63min |
| | | | ε₂₅₉= 31176M⁻¹.cm⁻¹ |
| | | | Masse haute résolution *m*/*z* pour C₂₉H₃₁N₃NaO₇S (M+Na⁺)⁺ : calculée 588,1780 ; mesurée 588,1802 |
| **98** | 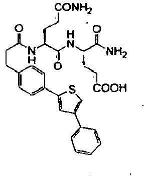 | Acide (*S*)-5-amino-4-((*S*)-5-amino-5-oxo-2-(3-(4-(4-phénylthiophèn-2-yl)phényl)propanamido)pentanamido)-5-oxopentanoïque | Ascentis Express : |
| | | | t_{R}=5,35min |
| | | | ε₂₅₉= 32857M⁻¹.cm⁻¹ |
| | | | Masse haute résolution *m*/*z* pour C₂₉H₃₂N₄NaO₆S (M+Na⁺)⁺ : calculée 587,1940 ; mesurée 587,1938 |
| **99** | 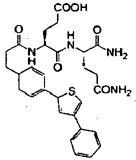 | Acide (*S*)-5-((*S*)-1,5-diamino-1,5-dioxopentan-2-ylamino)-5-oxo-4-(3-(4-(4-phénylthiophèn-2-yl)phényl)propanamido)pentanoïque | Ascentis Express : |
| | | | t_{R}=5,38min |
| | | | ε₂₅₉= 28729M⁻¹.cm⁻¹ |
| | | | Masse haute résolution *m*/*z* pour C₂₉H₃₂N₄NaO₆S (M+Na⁺)⁺ : calculée 587,1940 ; mesurée 587,1964 |
| **100** | 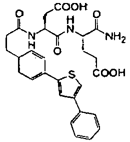 | Acide (*S*)-5-amino-4-((*S*)-3-carboxy-2-(3-(4-(4-phénylthiophèn-2-yl)phényl)propanamido)propanamido)-5-oxopentanoïque | Ascentis Express : |
| | | | t_{R}=5,64min |
| | | | ε₂₅₉= 35652M⁻¹.cm⁻¹ |
| | | | Masse haute résolution *m*/*z* pour C₂₈H₂₉N₃NaO₇S (M+Na⁺)⁺ : calculée 574,1624 ; mesurée 574,1617 |
| **101** | 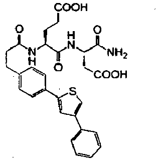 | Acide (*S*)-5-((*S*)-1-amino-3-carboxy-1-oxopropan-2-ylamino)-5-oxo-4-(3-(4-(4-phénylthiophèn-2-yl)phényl)propanamido)pentanoïque | Ascentis Express : |
| | | | t_{R}=5,63min |
| | | | ε₂₅₉= 30797M⁻¹.cm⁻¹ |
| | | | Masse haute résolution *m*/*z* pour C₂₈H₂₉N₃NaO₇S (M+Na⁺)⁺ : calculée 574,1624 ; mesurée 574,1645 |
| **102** | 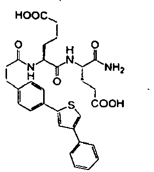 | Acide (*S*)-6-((*S*)-1-amino-4-carboxy-1-oxobutan-2-ylamino)-6-oxo-5-(3-(4-(4-phénylthiophèn-2-yl)phényl)propanamido)hexanoïque | Ascentis Express : |
| | | | t_{R}=5,69min |
| | | | ε₂₅₉= 45833M⁻¹.cm⁻¹ |
| | | | Masse haute résolution *m*/*z* pour C₃₀H₃₃N₃NaO₇S (M+Na⁺)⁺ : calculée 602,1937 ; mesurée 602,1938 |
| **103** | 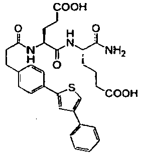 | Acide (*S*)-6-amino-5-((*S*)-4-carboxy-2-(3-(4-(4-phénylthiophèn-2-yl)phényl)propanamido)butanamido)-6-oxohexanoïque | Ascentis Express : |
| | | | t_{R}=5,67min |
| | | | ε₂₅₉= 32482M⁻¹.cm⁻¹ |
| | | | Masse haute résolution *m*/*z* pour C₃₀H₃₃N₃NaO₇S (M+Na⁺)⁺ : calculée 602,1937 ; mesurée 602,1938 |
| **104** | 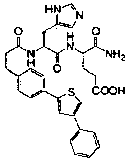 | Acide (*S*)-4-((*S*)-3-(1*H*-imidazol-5-yl)-2-(3-(4-(4-phénylthiophèn-2-yl)phényl)propanamido)propanamido)-5-amino-5-oxopentanoïque | Ascentis Express : |
| | | | t_{R}=5,17min |
| | | | ε₂₅₉=32335M⁻¹.cm⁻¹ |
| | | | Masse haute résolution *m*/*z* pour C₃₀H₃₂N₅O₅S (M+H⁺)⁺ : calculée 574,2124 ; mesurée 574,2108 |
| **105** | 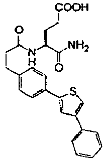 | Acide (*S*)-5-amino-5-oxo-4-(3-(4-(4-phénylthiophèn-2-yl)phényl)propanamido)pentanoïque | Ascentis Express : |
| | | | t_{R}=5,93min |
| | | | ε₂₅₉= 36526M⁻¹.cm⁻¹ |
| | | | Masse haute résolution *m*/*z* pour C₂₄H₂₄N₂NaO₄S (M+Na⁺)⁺ : calculée 459,1354 ; mesurée 459,1346 |
| **106** | 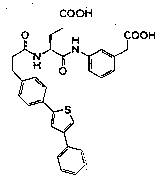 | Acide (*S*)-5-(3-(carboxyméthyl)phénylamino)-5-oxo-4-(3-(4-(4-phénylthiophèn-2-yl)phényl)propanamido)pentanoïque | Ascentis Express : |
| | | | t_{R}=6,68min |
| | | | ε₂₅₉=4790M⁻¹.cm⁻¹ |
| | | | Masse haute résolution *m*/*z* pour C₃₂H₃₀N₂NaO₆S (M+Na⁺)⁺ : calculée 593,1722 ; mesurée 593,1748 |
| **107** | 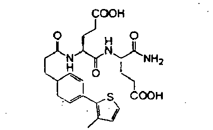 | Acide (*S*)-5-amino-4-((*S*)-4-carboxy-2-(3-(4-(3-méthylthiophèn-2-yl)phényl)propanamido)butanamido)-5-oxopentanoïque | Ascentis Express : |
| | | | t_{R}=4,53min |
| | | | ε₂₇₁= 1472M⁻¹.cm⁻¹ |
| | | | Masse haute résolution *m*/*z* pour C₂₄H₂₉N₃NaO₇S (M+Na⁺)⁺ : calculée 526,1624 ; mesurée 526,1627 |
| **95 bis** | 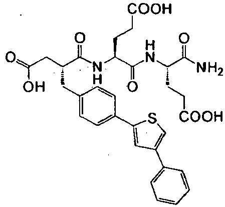 | Acide (S)-5-amino-4-((S)-4-carboxy-2-((R)-3-carboxy-2-(4-(4-phenylthiophen-2-yl)benzyl)propanamido)butanamido)-5-oxopentanoïque | Ascentis Express: |
| | | | t_{R}=5,55min |
| | | | ε₂₇₂=18230M⁻¹.cm⁻¹ |
| | | | Masse haute résolution *m*/*z* pour C₃₁H₃₄N₃O₉S⁺, (M+H⁺)⁺ : calculée 624.2010 ; mesurée 624.1999. |

### Synthèse du composé (95bis) porteur d'un groupement carboxyméthyle -CH₂COOH en R₄ :

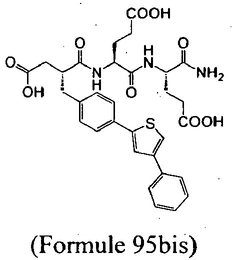

Le composé (95bis) porteur d'un groupement carboxyméthyle en R₄ est synthétisé selon le même protocole que les composés (3) à (107) décrits ci-dessus, seule la nature du bloc malonique incorporé sur le support solide ayant été modifié. En effet, il s'agit dans ce cas d'incorporer un bloc malonique bi-fonctionnalisé, ce dernier étant obtenu en quatre étapes selon le schéma général de synthèse suivant : 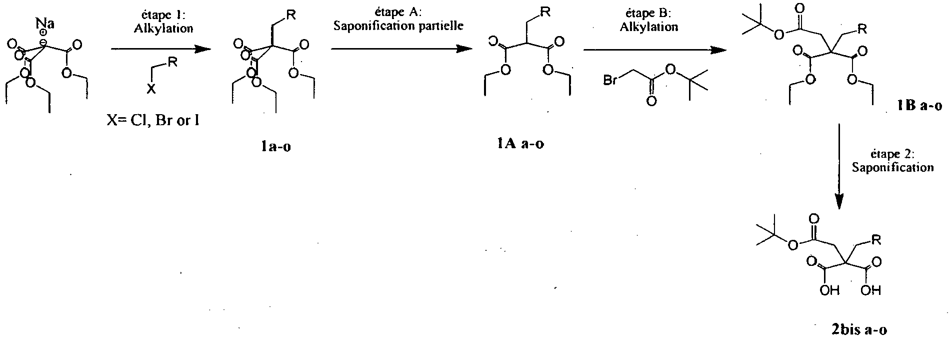 R ayant la même signification que précédemment.

L'étape 1 d'alkylation est réalisée selon le mode opératoire décrit précédemment pour la synthèse des blocs maloniques mono-fonctionnalisés.

### Etape A : étape de saponification partielle

Le triester **1** (3,93 mmol) a été solubilisé dans du tétrahydrofurane (10 mL), et de l'éthanolate de sodium (4,71 mmol, 1,2 eq) a été ajouté goutte à goutte à température ambiante. La complétion de la réaction a été contrôlée par chromatographie sur couche mince (CCM) avec un mélange d'éluant (cyclohexane CHX/acétate d'éthyle EtOAc: 9/1).

Le mélange réactionnel a ensuite été versé dans une solution d'acétate d'éthyle EtOAc /HCl 1M eau (1/1 : 10 mL/10 mL). La phase aqueuse a été extraite avec de l'acétate d'éthyle EtOAc (2 x 10mL). Les phases organiques ont été réunies puis lavées avec une solution saturée en chlorure de sodium NaCl (20 mL) et enfin séchées sur sulfate de magnésium (MgSO₄) anhydre. Le solvant a ensuite été concentré sous vide et le produit brut a été purifié par chromatographie flash (CHX/EtOAc) pour donner les diesters **1A.**

### Diethyl 2-(4-iodobenzyl)malonate 1Ab

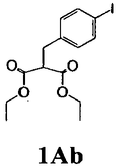

Préparé à partir du triester **1b** selon le protocole de saponification partielle pour donner le composé titre sous la forme d'une huile incolore (rendement 82%).

RMN ¹H (CDCl₃) : δ 1,21 (t, 6H, *J*=6,75Hz) ; 3,15 (d, 2H, *J*=8Hz) ; 3,59 (t, 1H, *J*=8Hz) 4,16 (q, 4H, *J*=6,75Hz) ; 6.96 (d, 2H, *J*=8,25Hz) ; 7,59 (d, 2H, *J*=8,25Hz).

RMN ¹³C (CDCl₃) : δ 14.16 ; 34,25 ; 53,69 ; 61.76 ; 92,25 ; 131.07 ; 137.68; 137.70; 168.76.

### Etape B: étape d'alkylation

Le diester **1B** (3,93 mmol) a été solubilisé dans du tétrahydrofurane anhydre (10 mL) sous atmosphère inerte. Le mélange réactionnel a ensuite été refroidi à 0°C et de l'hydrure de sodium (4,32 mmol, 1,1 eq) a été ajouté. Après 10 minutes d'agitation à 0°C, du 2-bromoactétate de tertio-butyle (5,89 mmol, 1,5 eq) a été ajouté et le mélange réactionnel a été agité à température ambiante. La complétion de la réaction a été contrôlée par chromatographie sur couche mince (CCM) avec un mélange d'éluant (cyclohexane CHX/acétate d'éthyle EtOAc : 9/1). Le mélange réactionnel a été repris, puis versé dans de l'eau/EtOAc (1/1 : 10 mL/10 mL). La phase aqueuse a été extraite avec de l'acétate d'éthyle EtOAc (2 x 10 mL). Les phases organiques ont été regroupées, lavées avec une solution saturée en NaCl (20 mL) et séchées sur MgSO₄ anhydre. Après évaporation, le solide brut a été trituré dans DCM (1 mL) puis filtré pour donner les dérivés **1B.**

### 1-tert-butyl 2,2-diethyl 3-(4-iodophényl)propane-1,2,2-tricarboxylate 1Bb

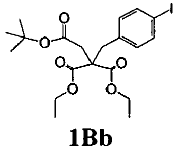

Préparé à partir du diester **lAb** selon le protocole d'alkylation (étape B) pour donner le composé titre sous la forme d'une huile incolore (rendement 95%).

RMN ¹H (CDCl₃) : δ 1,21 (t, 6H, *J =* 6,75Hz) ; 1,46 (s, 9H), 2.75 (s, 2H) ; 3.31 (s, 2H) 4,19 (q, 4H, *J* = 6,75Hz) ; 6.85 (d, 2H, *J* = 8,25Hz) ; 7,58 (d, *2H, J =* 8,25Hz).

RMN ¹³C (CDCl₃) : δ 13.97 ; 27.98; 37,62; 37.81, 56,42; 61.70 ; 81.39 ; 92,70 ; 132.09 ; 135.60 ; 137.41 ; 169.63 ; 169.76.

### Acide 2-(2-(tert-butoxy)-2-oxoéthyl)-2-(4-iodobenzyl)malonique 2b bis

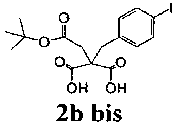

Préparé à partir du composé **1Bb** selon le protocole de saponification (étape 2) pour donner le composé titre sous la forme d'un solide blanc cassé (rendement 65%).

RMN ¹H (MeOH d₄) : δ 1.38 (s, 9H), 2.94 (s, 2H) ; 3.12 (s, 2H). 6.85 (d, 2H, *J =* 8Hz) ; 7,56 (d, 2H, *J =* 8Hz).

Masse haute résolution m/z pour C₁₆H₂₀INO₆ (M+H⁺)⁺, calculée 435.0299 ; mesurée 435.0310.

L'étape 2 de saponification est réalisée selon le mode opératoire décrit précédemment pour la synthèse des blocs maloniques mono-fonctionnalisés.

Les blocs maloniques bi-fonctionnalisés ainsi synthétisés sont ensuite incorporés sur un support solide, et les pseudo-peptides obtenus sont modifiés via des réactions de cyclo-addition 1,3 dipolaire ou des couplages au palladium (réaction de Suzuki ou de Sonogashira) comme décrit précédemment.

Les données analytiques concernant le composé (95bis) figurent au tableau III ci-dessus.

Le composé (95bis) a ensuite été évalué sur des MMP humaines, et comparé au composé (95). Les résultats figurent dans le Tableau IV suivant :

**Tableau IV**

| | **MMP-1h** | **MMP-2h** | **MMP-3h** | **MMP-7h** | **MMP-8h** | **MMP-9h** | **MMP-10h** | **MMP-12h** | **MMP-13h** | **MMP-14h** |
|---|---|---|---|---|---|---|---|---|---|---|
| **Ki (nM) Composé (95)** | >10000 | >1000 | >1000 | >1000 | 410 | >10000 | 872 | 1,92 | 684 | >2500 |
| 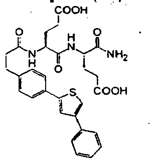 | | | | | | | | | | |
| **Facteur de sélectivité/MMP12** | 5208 | 520 | 520 | 520 | 213 | 5200 | 454 | 1 | 356 | 1300 |
| | | | | | | | | | | |
| **Ki (nM) Composé (95bis)** | >1000 | 401 | 579 | >1000 | 520 | >1000 | 80 | 0,3 | 198 | 118 |
| 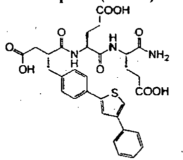 | | | | | | | | | | |
| **Facteur de sélectivité/MMP12** | 3333 | 1340 | 1930 | 3333 | 1730 | 3333 | 270 | 1 | 660 | 395 |

On observe que lorsque R₄ est un groupement carboxyméthyle - CH₂COOH, l'affinité du composé résultant (Formule 95 bis) vis-à-vis de la MMP-12, par rapport au composé de Formule 95, est améliorée d'un facteur 6 (de 1,92 nM à 0,3 nM). En ce qui concerne les facteurs de sélectivité des autres membres de la famille MMP, ces derniers ont été soient maintenus, soient améliorés. Seule la sélectivité vis-à-vis de la MMP-14 est légèrement diminuée par rapport au composé (95).

### Evaluation des pseudo-peptides sur les MMP :

Les tests d'inhibition et l'évaluation des constantes d'inhibition (Ki) sur les différentes MMP ont été réalisés comme décrit par Devel *et al.* (Devel et al. 2006 J. Biol. Chem. (7))

Les résultats obtenus sont reportés aux tableaux I, II et IV.

### Evaluation de la stabilité dans le sang et de la concentration plasmatique des composés de formule (1), chez la souris :

Des expériences sur les composés de formules (40) et (91) ont permis d'évaluer la stabilité dans le sang de ces composés, ainsi que leur concentration plasmatique chez la souris après une perfusion sur 30 minutes.

### Test de stabilité :

Au bout de 24 heures dans du sang de souris, et après analyse LC-MS (*Liguid Chromatography-Mass Spectrometry*) et confirmation de l'identité par fragmentation par spectrométrie de masse MS/MS, 50 fmol (solution à 5 nM pour une solution initiale à 10 nM à t = 0) du composé (40) sont détectés sous forme intacte. Aucun produit secondaire issu du composé (40) n'a été détecté. La perte de 50% du matériel de départ peut être attribuée à un phénomène d'association aspécifique du composé à la paroi de l'eppendorf. Il est à noter que cette perte de 50% du composé a également été observée au bout de 24 heures pour une solution à 10 nM dans du tampon PBS ou dans une solution à 1 µM de BSA (Serum Albumine Bovine).

### Détermination de la concentration plasmique :

Après perfusion sur cinq souris d'une solution du composé (91) à 10 mg/kg (soit 0,2 mg/50 L, soit à 8 mM dans une solution tampon PBS) sur 30 minutes, et prélèvement de sang après 5 minutes, le sang est extrait et une concentration plasmatique moyenne pour le composé (91) de 1 µM a été déterminée à l'aide d'un test d'inhibition sur la MMP-8. L'identité stricte du composé (91) a par ailleurs été confirmée par analyse LC-MS et fragmentation MS/MS.

### Synthèse des composés de formule (2) porteurs d'un marqueur TAG :

Les composés marqués de formule (2) peuvent être synthétisés selon le Schéma 3 suivant :

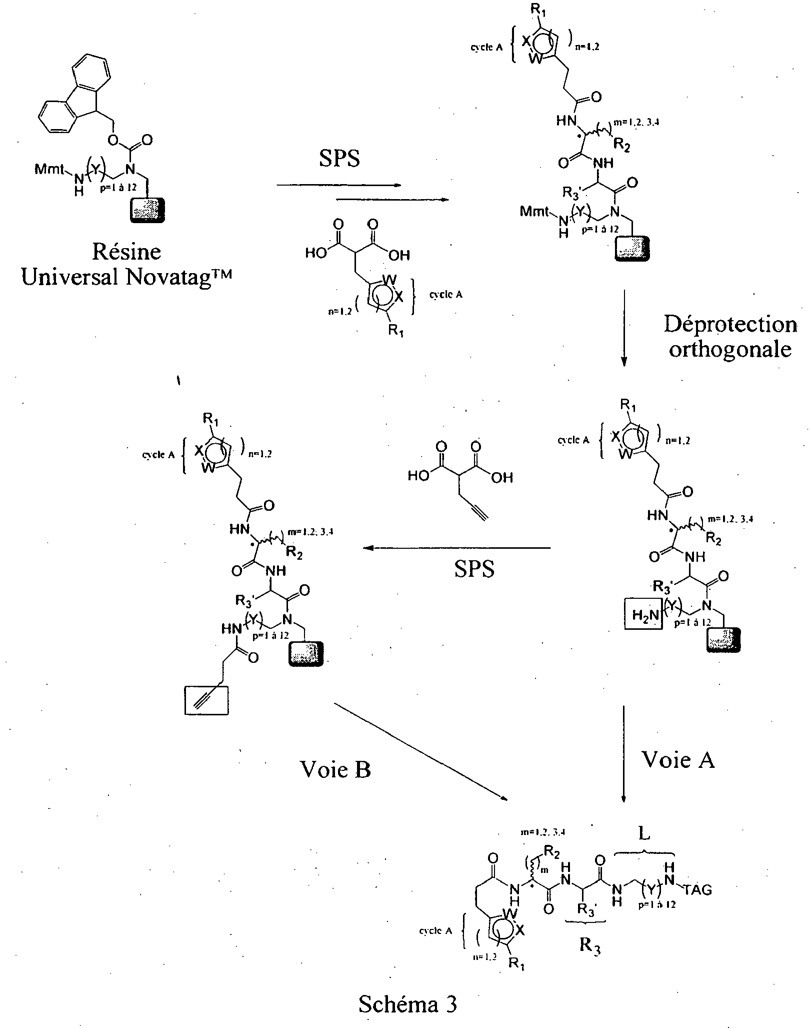

Les composés de formule générale (1) peuvent être marqués en position C-terminale selon les différentes voies de synthèses représentées ci-dessus au Schéma 3. Après construction de la séquence peptidique sur un support solide, le bloc malonique est incorporé, puis le cycle A formé par cyclo-addition 1,3 dipolaire ou fonctionnalisé par couplage au palladium, comme décrit précédemment (réaction de Suziki ou réaction de Sonogashira). Une déprotection orthogonale sans clivage du support solide peut ensuite être réalisée. L'amine ainsi libérée peut : a) soit réagir avec un ester activé (voie A), b) soit être préalablement modifiée et transformée en une nouvelle fonction chimique permettant l'introduction du TAG selon une voie différente (voie B).

Les différentes voies de synthèse et techniques d'imagerie utilisées en fonction de la nature du marqueur TAG sont résumées dans la Tableau V ci-dessous :

**Tableau V**

| Structure du TAG | Voie de synthèse | Technique d'imagerie | Références |
|---|---|---|---|
| 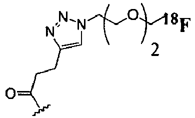 | Voie B | PET | (11) |
| 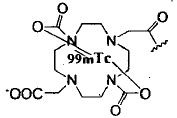 | Voie A | SPECT | (12) |
| 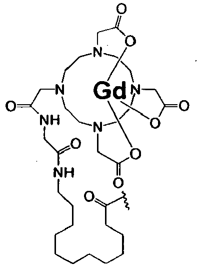 | Voie A | MRI | (13) |
| 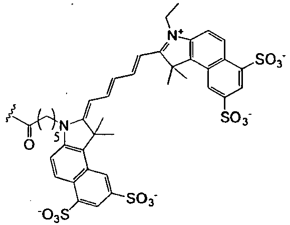 | Voie A | NIRF | (14) |
| 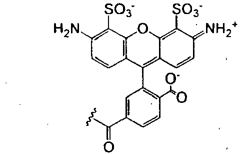 | Voie A | NIRF | (14) |
| Marqueur peptidique XₐX₁X₂X₃X₄X₅X_{b}X_{c} ou sa forme retro-inversée | Voie A | SPECT | WO 2010/076654 |

Un composé de formule (2) porteur d'un marqueur TAG fluorescent de type Alexa fluor^{®} (Formule (108)) a été synthétisé selon le Schéma 3 ci-dessus.

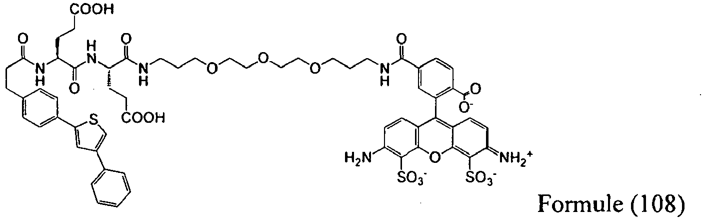

Après construction de la séquence peptidique, introduction sur support solide du bloc malonique 2b et couplage au palladium dans les conditions décrites ci-dessus, l'amine primaire est déprotégée en présence d'une solution de HOBt dans le DCM/TFE : 1/1 (0,6 M, 2 x 30 min). La résine est ensuite lavée deux fois au DMF (pendant 5 minutes) et deux fois au DCM (pendant 5 minutes). Une solution d'acide carboxylique d'Alexa Fluor^{®} 488 activé sous forme d'ester de succinimidyle (1,1 éq, Invitrogen, réf : A20100) dans du DMF anhydre est ajoutée, et le mélange réactionnel est ensuite agité une nuit à température ambiante à l'abri de la lumière. La résine est ensuite lavée deux fois au DMF (pendant 5 minutes) et deux fois au DCM (pendant 5 minutes). Le pseudo peptide est alors clivé de son support puis purifié comme décrit précédemment. Les données analytiques concernant le composé de formule (108) sont résumées dans le tableau VI ci-dessous.

**Tableau VI**

| | | | |
|---|---|---|---|
| **108** | 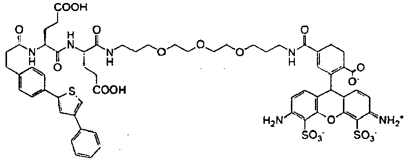 | 2-(6-amino-3-iminio-4,5-disutfonato-3H-xanthen-9-yl)-4-(((5S,8S)-5,8-bis(2-carboxyethyl)-3,6,9-trioxo-1-(4-(4-phenylthiophen-2-yl)phenyl)-14,17,20-trioxa-4,7,10-triazatricosan-23-yl)carbamoyl)benzoate | Ascentis Express : t_{R}=5.93min ε₅₀₀= 808510M⁻¹. cm⁻¹ |
| | | | Masse *m*/*z* pour C₆₀H₆₂N₆O₂₀S₃²⁻ (M+H⁺)⁺ =1285,4 |

Le composé (108) a ensuite été évalué sur des MMP humaines, et comparé au composé (95). Les résultats figurent dans le Tableau VII suivant :

**Tableau VII**

| | **MMP-1h** | **MMP-2h** | **MMP-3h** | **MMP-7h** | **MMP-8h** | **MMP-9h** | **MMP-10h** | **MMP-12h** | **MMP-13h** | **MMP-14h** |
|---|---|---|---|---|---|---|---|---|---|---|
| **Ki(nM) Composé (95)** | >10000 | >1000 | >1000 | >1000 | 410 | >10000 | 872 | 1,92 | 684 | >2500 |
| 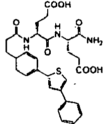 | | | | | | | | | | |
| **Facteur de sélectivité/MMP12** | 5208 | 520 | 520 | 520 | 213 | 5200 | 454 | 1 | 356 | 1300 |
| | | | | | | | | | | |
| **Ki (nM) Composé (108)** | >10000 | >2500 | >10000 | >10000 | >2500 | >5000 | 833 | 15 | >1000 | >10000 |
| 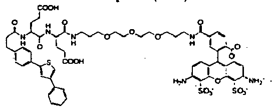 | | | | | | | | | | |
| **Facteur de sélectivité/MMP12** | 670 | 170 | 670 | 670 | 170 | 333 | 55 | 1 | 66 | 670 |

L'introduction d'un espaceur et d'un groupement fluorescent modifie assez peu l'affinité du composé de formule (108) vis-à-vis de la MMP-12, comparativement au composé de formule (95) (Ki = 15 nM vs Ki = 1,92 nM). D'autre part, le composé de formule (108) s'avère être assez sélectif vis-à-vis de la MMP-12.

### Références bibliographiques

(1) Brinckerhoff CE, Matrisian LM. Matrix metalloproteinases: a tail of a frog that became a prince. Nat Rev Mol Cell Biol. 2002 Mar;3(3):207-14.
(2) Page-McCaw A, Ewald AJ, Werb Z. Matrix metalloproteinases and the regulation of tissue remodelling. Nat Rev Mol Cell Biol. 2007 Mar;8(3):221-33.
(3) Egeblad M, Werb Z. New functions for the matrix metalloproteinases in cancer progression. Nat Rev Cancer. 2002 Mar;2(3):161-74.
(4) Fingleton B. Matrix metalloproteinases as valid clinical targets.Fingleton B. Curr Pharm Des. 2007;13(3):333-46.
(5) Hu J, Van den Steen PE, Sang QX, Opdenakker G. Matrix metalloproteinase inhibitors as therapy for inflammatory and vascular diseases. Nat Rev Drug Discov. 2007 Jun;6(6):480-98.
(6) Overall CM, López-Otín C. Strategies for MMP inhibition in cancer: innovations for the post-trial era. Nat Rev Cancer. 2002 Sep;2(9):657-72.
(7) Devel L, Rogakos V, David A, Makaritis A, Beau F, Cuniasse P, Yiotakis A, Dive V. Development of selective inhibitors and substrate of matrix metalloproteinase-12. J Biol Chem. 2006 Apr 21;281(16):11152-60.
(8) Engel CK, Pirard B, Schimanski S, Kirsch R, Habermann J, Klingler O, Schlotte V, Weithmann KU, Wendt KU. Structural basis for the highly selective inhibition of MMP-13. Chem Biol. 2005 Feb;12(2):181-9.
(9) Makaritis A, Georgiadis D, Dive V, Yiotakis A. Diastereoselective solution and multipin-based combinatorial array synthesis of a novel class of potent phosphinic metalloprotease inhibitors. Chemistry. 2003 May 9;9(9):2079-94.
(10) F.A. Jaffer, P. Libby, R. Weissleder, Optical and multimodality molecular imaging: insights into atherosclerosis, Arterioscler Thromb Vasc Biol. 29 2009 1017-1024.
(11) M. Nahrendorf, E. Keliher, B. Marinelli, P. Waterman, P.F. Feruglio, L. Fexon, M. Pivovarov, F.K. Swirski, M.J. Pittet, C. Vinegoni, R. Weissleder, Hybrid PET-optical imaging using targeted probes, Proc. Natl. Acad. Sci. U S A 107 2010 7910-7915.
(12) H. Su, F.G. Spinale, L.W. Dobrucki, J. Song, J. Hua, S. Sweterlitsch, D.P. Dione, P. Cavaliere, C. Chow; B.N. Bourke, X.Y. Hu, M. Azure, P. Yalamanchili, R. Liu, E.H. Cheesman, S. Robinson, D.S. Edwards, A.J. Sinusas, Noninvasive targeted imaging of matrix metalloproteinase activation in a murine model of postinfarction remodeling, Circulation. 112 2005 3157-3167.
(13) B. Jastrzebska, R. Lebel, H. Therriault, J.O. McIntyre, E. Escher, B. Guerin, B. Paquette, W.A. Neugebauer, M. Lepage, New enzyme-activated solubility-switchable contrast agent for magnetic resonance imaging: from synthesis to in vivo imaging, J. Med. Chem. 52 2009 1576-1581.
(14) A. Faust, B. Waschkau, J. Waldeck, C. Holtke, H.J. Breyholz, S. Wagner, K. Kopka, O. Schober, W. Heindel, M. Schafers, C. Bremer, Synthesis and evaluation of a novel hydroxamate based fluorescent photoprobe for imaging of matrix metalloproteinases, Bioconjug. Chem. 20 2009 904-912.

## Revendications

1. Composés de formule (1) suivante : 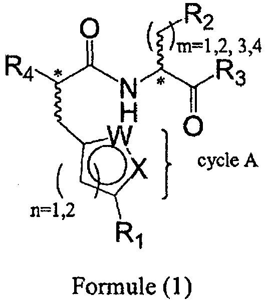 dans laquelle :
- n vaut 1 ou 2,
- lorsque n = 1 : W et X, indépendamment l'un de l'autre, sont O, N ou C,
- lorsque n = 2 : W et X sont C,
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène, 3a,7a-dihydrobenzo[*d*]thiazole, 3-aminophényle, 3-hydroxyphényle, 3-nitrophényle, 3-carboxyphényle, 3'-chlorophényle, 3,5-dichlorophényle, 3-méthoxyphényle, 3-hydroxyméthylphényle, ou un cycle thiophène substitué en positions, indépendamment l'une de l'autre, 2 et/ou 3 et/ou 4 et/ou 5 par un groupe choisi parmi un groupe méthyle, phényle, 3a,7a-dihydrobenzo[*d*]thiazole ou un atome d'hydrogène,
- m est un entier compris entre 1 et 4 inclus,
- lorsque m = 4, R₂ est un groupe amine,
- lorsque m = 1 ou 2 ou 3, R₂ est une groupe acide carboxylique,
- lorsque m = 1 ou 2, R₂ est un groupe carboxamide,
- lorsque m = 1, R₂ est un groupe 4-hydroxyphényle ou un groupe *1H-*imidazole ou un groupe hydroxyle ou un groupe isopropyle ou un groupe méthyle,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1) via une fonction amino, et
- R₄ est H ou un groupe carboxyméthyle -CH₂COOH,
et les diastéréoisomères et énantiomères de ceux-ci.

2. Composés selon la revendication 1 **caractérisés en ce que** dans la formule (I) :
- W est O, X est N, et n = 1, formant un cycle A qui est un cycle isoxazole, et **en ce qu'**ils ont la formule (1-A) suivante : 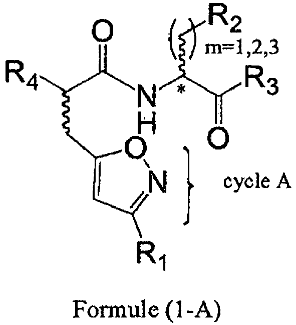 dans laquelle :
- R₁ est un groupe phényle, biphényle ou 3'-chlorobiphényle,
- m est un entier compris entre 1 et 3 inclus,
- lorsque m vaut 1 ou 3, R₂ est un groupe acide carboxylique, et lorsque m vaut 2, R₂ est un groupe acide carboxylique ou un groupe carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-A) via une fonction amino, et
- R₄ est H ou un groupe carboxyméthyle -CH₂COOH, et les diastéréoisomères et énantiomères de ceux-ci.

3. Composés selon la revendication 1 ou 2 **caractérisés en ce qu'**ils sont choisis parmi les composés de formules (3) à (23) suivantes :

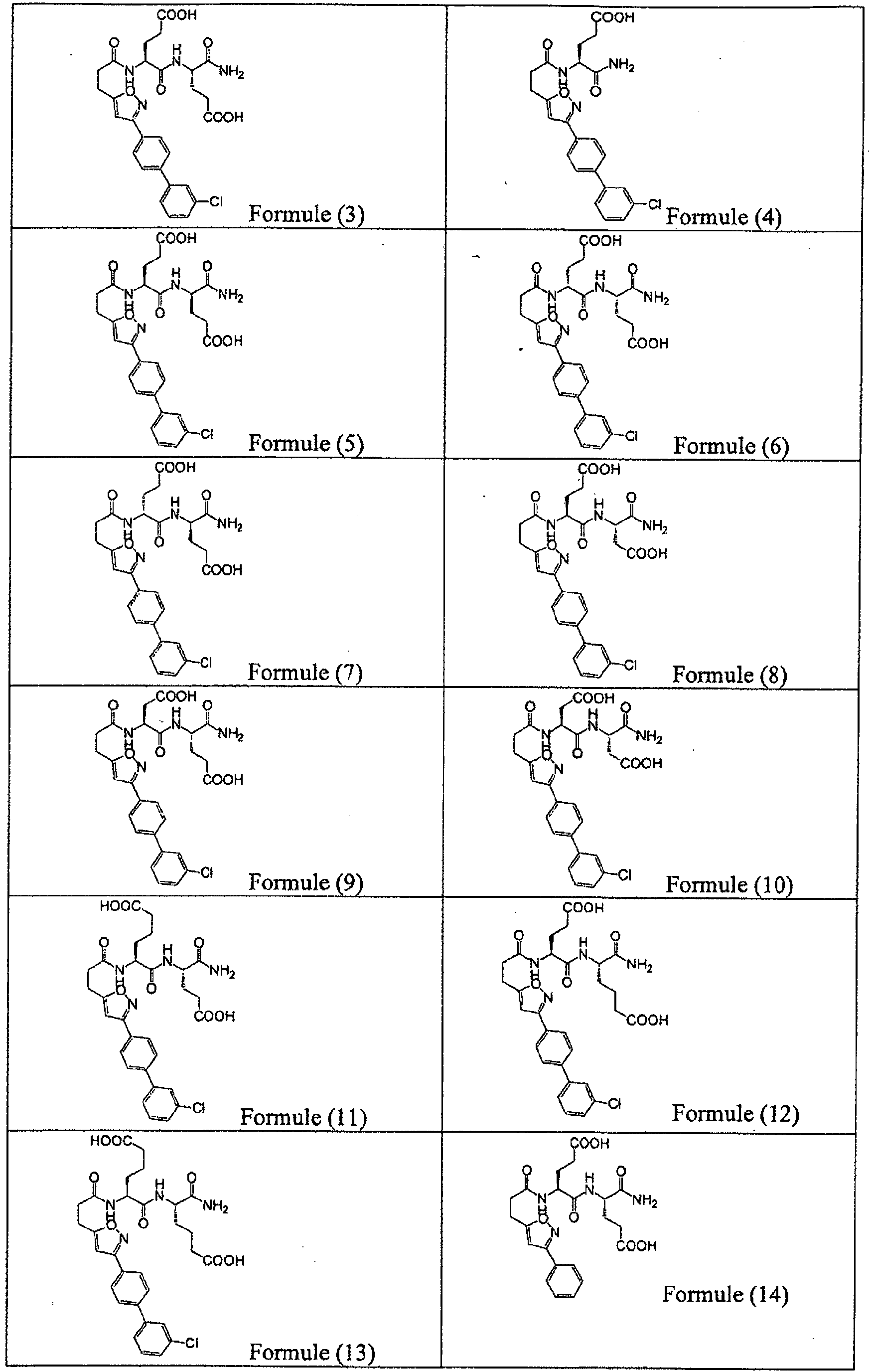

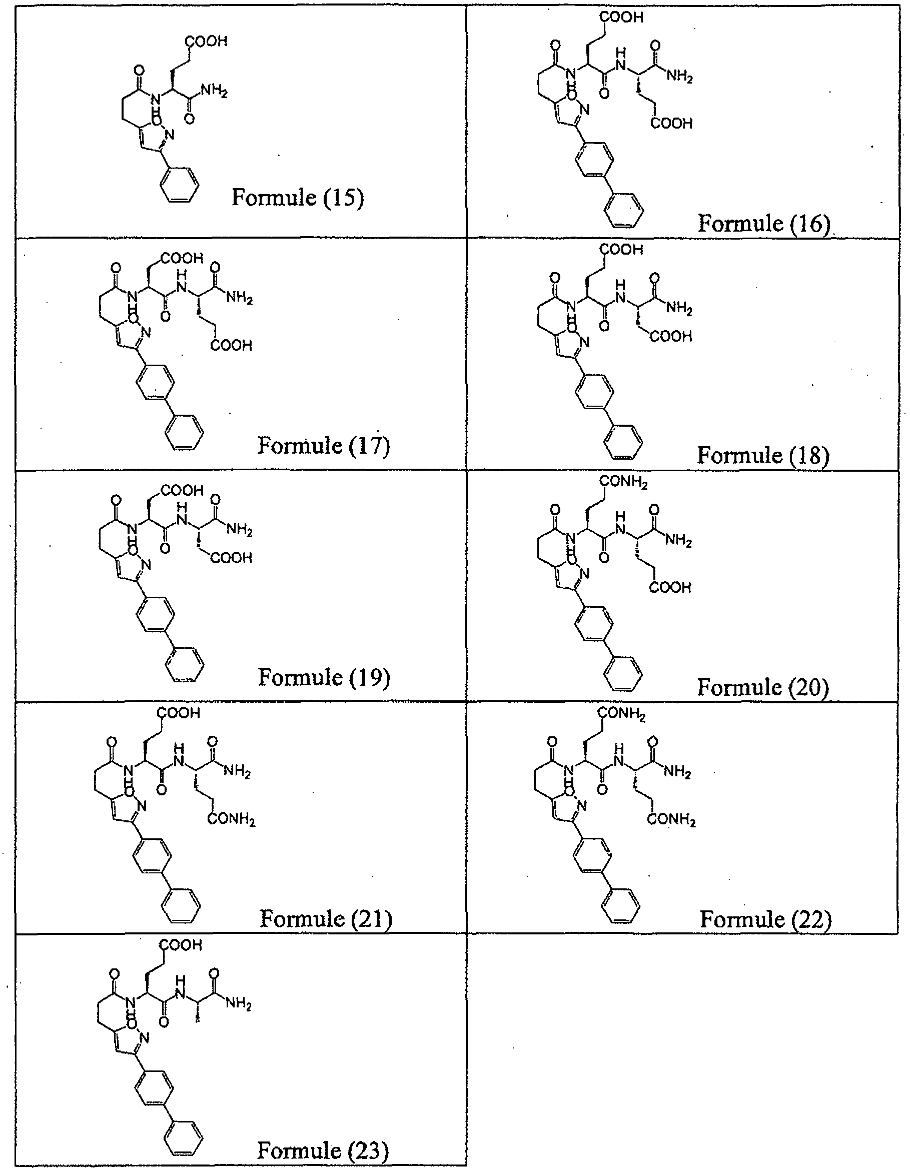

4. Composés selon la revendication 1, **caractérisés en ce que** dans la formule (1) :
- n = 1,
- W est N,
- X est O,
- R₁ est un groupe phényle, biphényle ou 3'-chlorobiphényle
- m est un entier compris entre 1 et 3 inclus,
- lorsque m vaut 1 ou 3, R₂ est un groupe acide carboxylique, et lorsque m vaut 2, R₂ est un groupe acide carboxylique ou un groupe carboxamide,
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la fonnule (1) via une fonction amino, et
- R₄ est H ou un groupe carboxyméthyle -CH₂COOH,
et **en ce qu'**ils ont la formule (1-B) suivante : 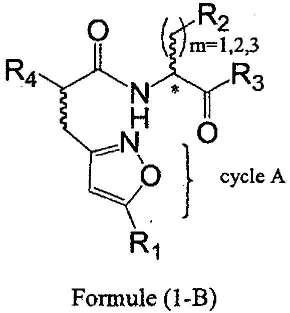 et les diastéréoisomères et énantiomères de ceux-ci,

5. Composés selon la revendication 1, **caractérisés en ce que** dans la formule 1 :
- W et X sont C et n = 2, formant ainsi un cycle A qui est un cycle benzène,
et **en ce qu'**ils ont la formule (1-C) suivante : 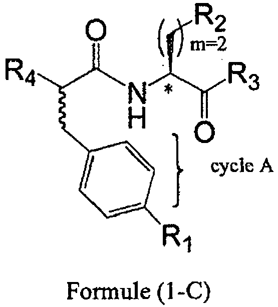 dans laquelle :
- R₁ est choisi parmi un atome d'iode ou un groupe phényle, biphényle, 3'-chlorobiphényle, phénoxy, phénoxyméthyle, phényléthynyle, pyrimidine, 1-méthyl-1*H-*pyrazole, 5-méthyl-1,2,4-oxadiazole, 1,2,3-thiadiazole, 1*H*-pyrrole, thiazole, thiophène et 3a,7a-dihydrobenzo[*d*]thiazole,
- m = 2,
- R₂ est un groupe acide carboxylique, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-C) via une fonction amino, et
- R₄ est H ou un groupe carboxyméthyle -CH₂COOH,
et les diastéréoisomères de ceux-ci.

6. Composés selon la revendication 1, **caractérisés en ce que** dans la formule (1) :
- W et X sont C, et n = 2 formant ainsi un cycle A qui est un cycle benzène,
et **en ce qu'**ils ont la formule (1-D) suivante : 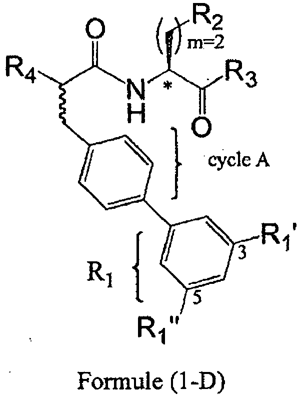 dans laquelle :
- R₁ est :
- soit un groupe phényle non substitué (R_{1'} = H et R_{1"} = H),
- soit un groupe phényle monosubstitué en position 3 par un groupe amino (R_{1'} = NH₂, R_{1"} = H) ou par un groupe hydroxyle (R_{1'} = OH, R_{1"} = H) ou par un groupe nitro (R_{1'} = NO₂, R_{1"} = H) ou par un groupe carboxyle (R_{1'} = COOH, R_{1"} = H) ou par un atome de chlore (R_{1'} = Cl, R_{1"} = H) ou par un groupe méthoxy (R_{1'} = OMe, R_{1"} = H) ou par un groupe hydroxyméthyle (R_{1'} = CH₂OH, R_{1"} = H),
- soit un groupe phényle di-substitué en positions 3 et 5 par un atome de chlore (R_{1'} = CI et R_{1"} = Cl),
- m=2,
- R₂ est un groupe acide carboxylique, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-D) via une fonction amino, et
- R₄ est H ou un groupe carboxyméthyle -CH₂COOH,
et les diastéréoisomères de ceux-ci.

7. Composés selon la revendication 1, **caractérisés en ce que** dans la formule (1) :
- W et X sont C, et n = 2 formant ainsi un cycle A qui est un cycle benzène, et R₁ est un groupe biphényle,
et **en ce qu'**ils ont la formule (1-E) suivante : 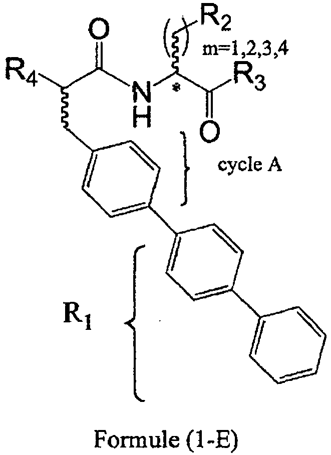 dans laquelle :
- m vaut 1, 2, 3 ou 4,
- lorsque m = 4, R₂ est un groupe amino,
- lorsque m = 1 ou 2 ou 3, R₂ est un groupe acide carboxylique,
- lorsque m = 2, R₂ est un groupe carboxamide,
- lorsque m = 1, R₂ est un groupe 4-hydroxyphényle ou un *1H*-imidazole ou hydroxyle ou isopropyle ou méthyle,
- R₃ est choisi parmi un groupe glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, ou un groupe carboxyméthyle pipéridine, un groupe carboxyméthyle 3-aminophényle ou un groupe amino, ledit groupement R₃ étant lié au groupement carbonyle de la formule (1-E) via une fonction amino, et
- R₄ est H ou un groupe carboxyméthyle -CH₂COOH,
et les diastéréoisomères et énantiomères de ceux-ci.

8. Composés selon la revendication 1, **caractérisés en ce que** dans la formule (1) :
- W et X, sont C et n = 2, formant un cycle A qui est un cycle benzène, et
- R₁ est un cycle thiophène substitué par un groupe R_{1'''} et **en ce qu'**ils ont la formule (1-F) suivante : 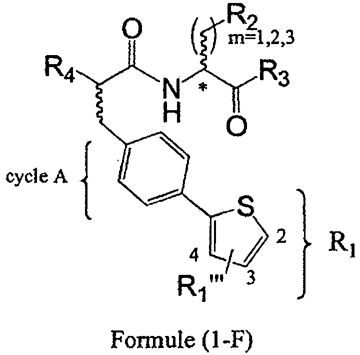 dans laquelle :
- R₁ est un cycle thiophène non substitué (R_{1'''} = H), ou monosubstitué soit en position 2 par un groupe choisi parmi un groupe méthyle (R_{1'''} = CH₃) ou phényle (R_{1'''} = Ph), ou 3a,7a-dihydrobenzo[*d*]thiazole, soit en position 3 par un groupe choisi parmi un groupe méthyle (R_{1'''} = CH₃) ou phényle (R_{1'''} = Ph), soit en position 4 par un groupe méthyle (R_{1'''} = CH₃).
- m = 1, 2 ou 3, et
- R₂ est un groupe acide carboxylique ou imidazole lorsque m = 1, ou un groupe acide carboxylique ou carboxamide lorsque m = 2, ou un groupe acide carboxylique lorsque m = 3, et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F) via une fonction amino, et
- R₄ est H ou un groupe carboxyméthyle -CH₂COOH,
et les diastéréoisomères et énantiomères de ceux-ci.

9. Composés selon la revendication 8, **caractérisés en ce qu'**ils ont la formule (1-F1) suivante : 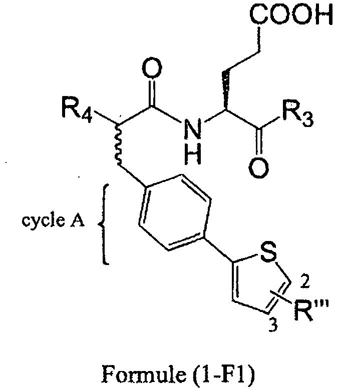 dans laquelle :
- R_{1'''} est soit en position 2 soit en position 3 du cycle thiophène et est choisi parmi un groupe méthyle (R_{1'''} = CH₃) ou phényle (R_{1'''} = Ph), et
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F1) via une fonction amino, et
- R₄ est H ou un groupe carboxyméthyle -CH₂COOH,
et les diastéréosimères de ceux-ci.

10. Composés selon la revendication 9, **caractérisés en ce qu'**ils sont choisis parmi les composés de formules (91), (92), (95), (97), (99), (101), (103), (105), (106) et (95bis) suivantes:

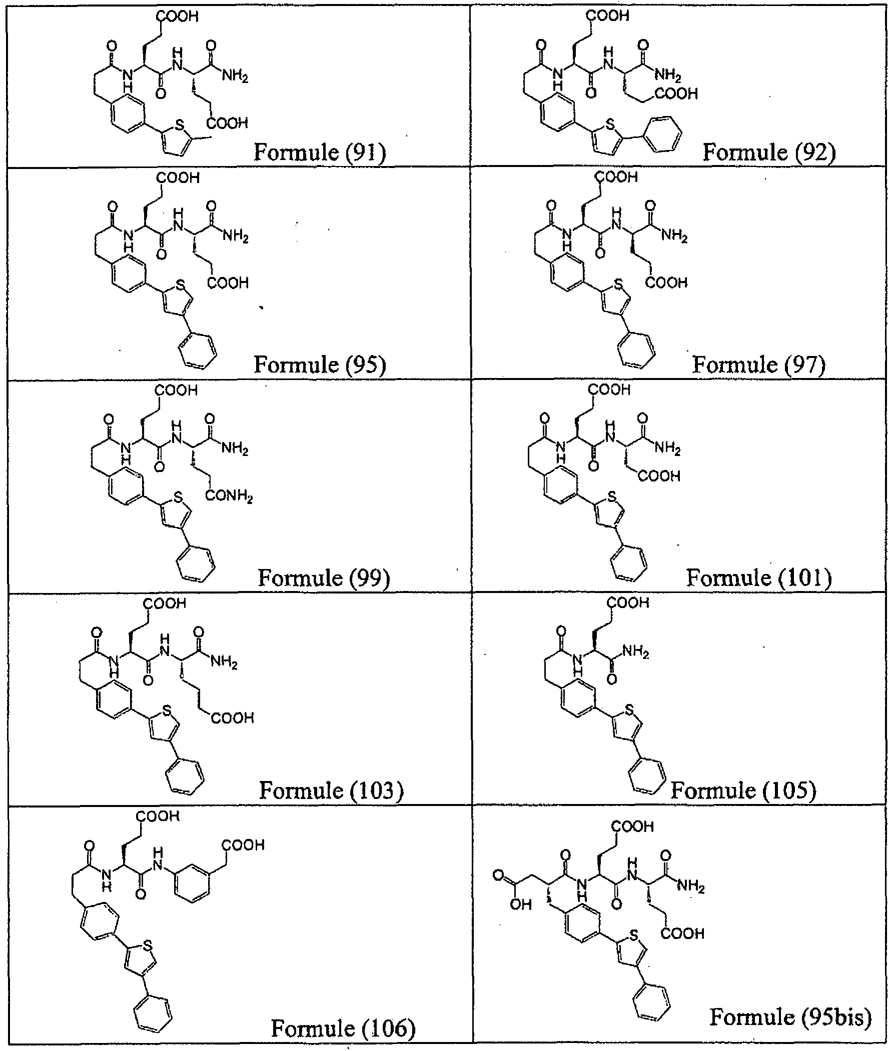

11. Composés selon la revendication 9 ou 10, **caractérisés en ce que** dans la formule (1-F1) :
- R_{1'''} est en position 3 du cycle thiophène et est un groupe phényle (R_{1'''} = Ph), et **en ce qu'**ils ont la formule (1-F2) suivante : 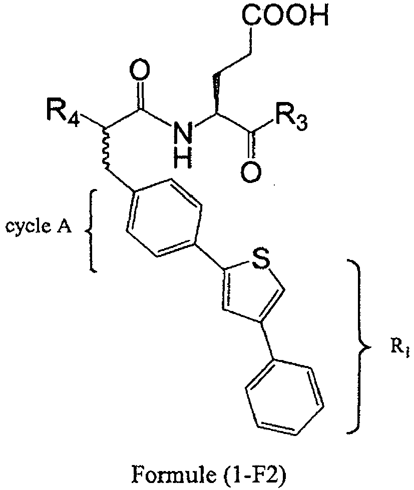 dans laquelle :
- R₃ est choisi parmi un groupe amino ; un groupe carboxyméthyle pipéridine ; un groupe carboxyméthyle 3-aminophényle ; un résidu glutamate de configuration L ou D, homoglutamate, aspartate, glutamine, alanine, lysine, tyrosine, histidine, sérine, leucine, les fonctions carboxyliques terminales desdits acides aminés pouvant être des fonctions carboxamides -C(=O)NH₂, et ledit groupement R₃ étant lié au groupement carbonyle de la formule (1-F2) via une fonction amino, et
- R₄ est H ou un groupe carboxyméthyle -CH₂COOH.

12. Composés selon l'une quelconque des revendications précédentes, **caractérisé en ce que** R₄ est H.

13. Composés selon la revendication 1 **caractérisés en ce qu'**ils sont choisis parmi les composés de formules (25), (28) à (40), (42) à (107) suivantes :

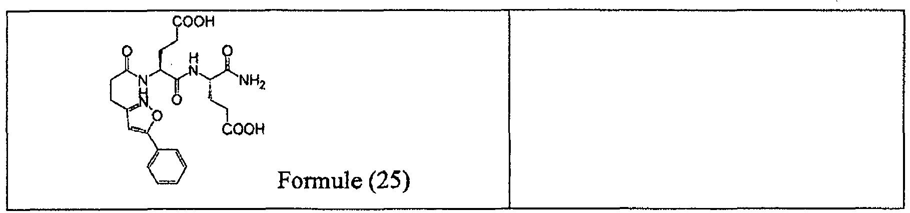

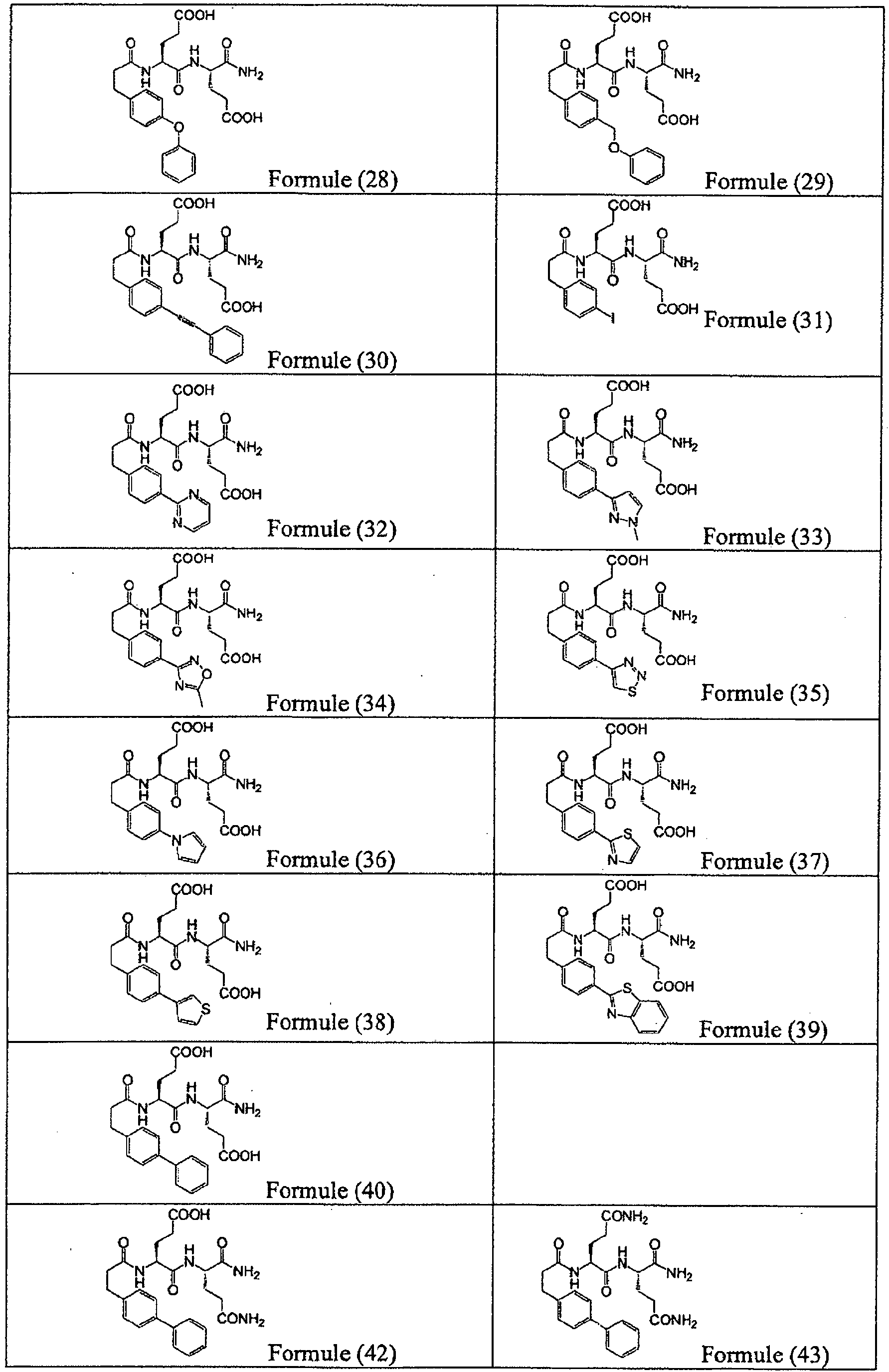

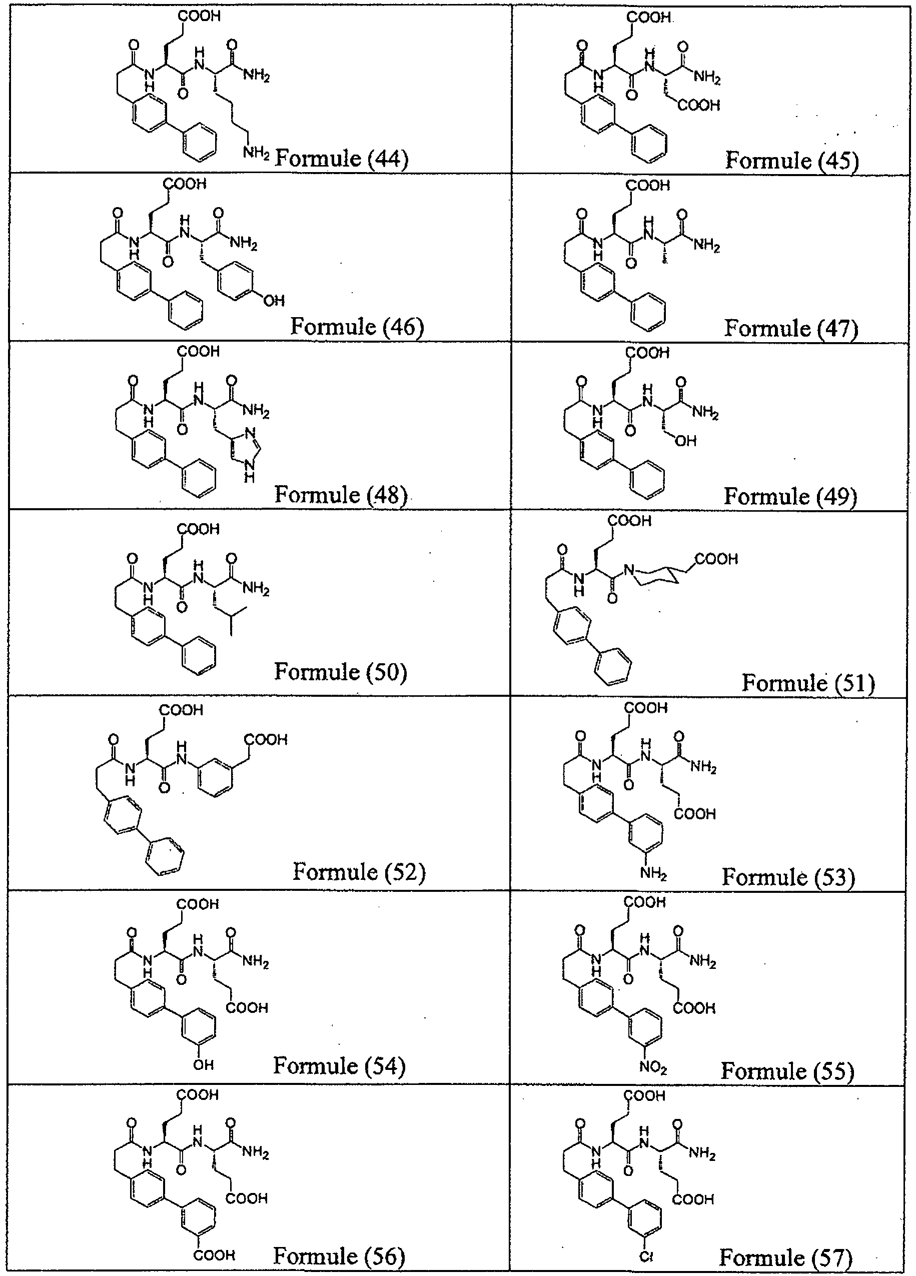

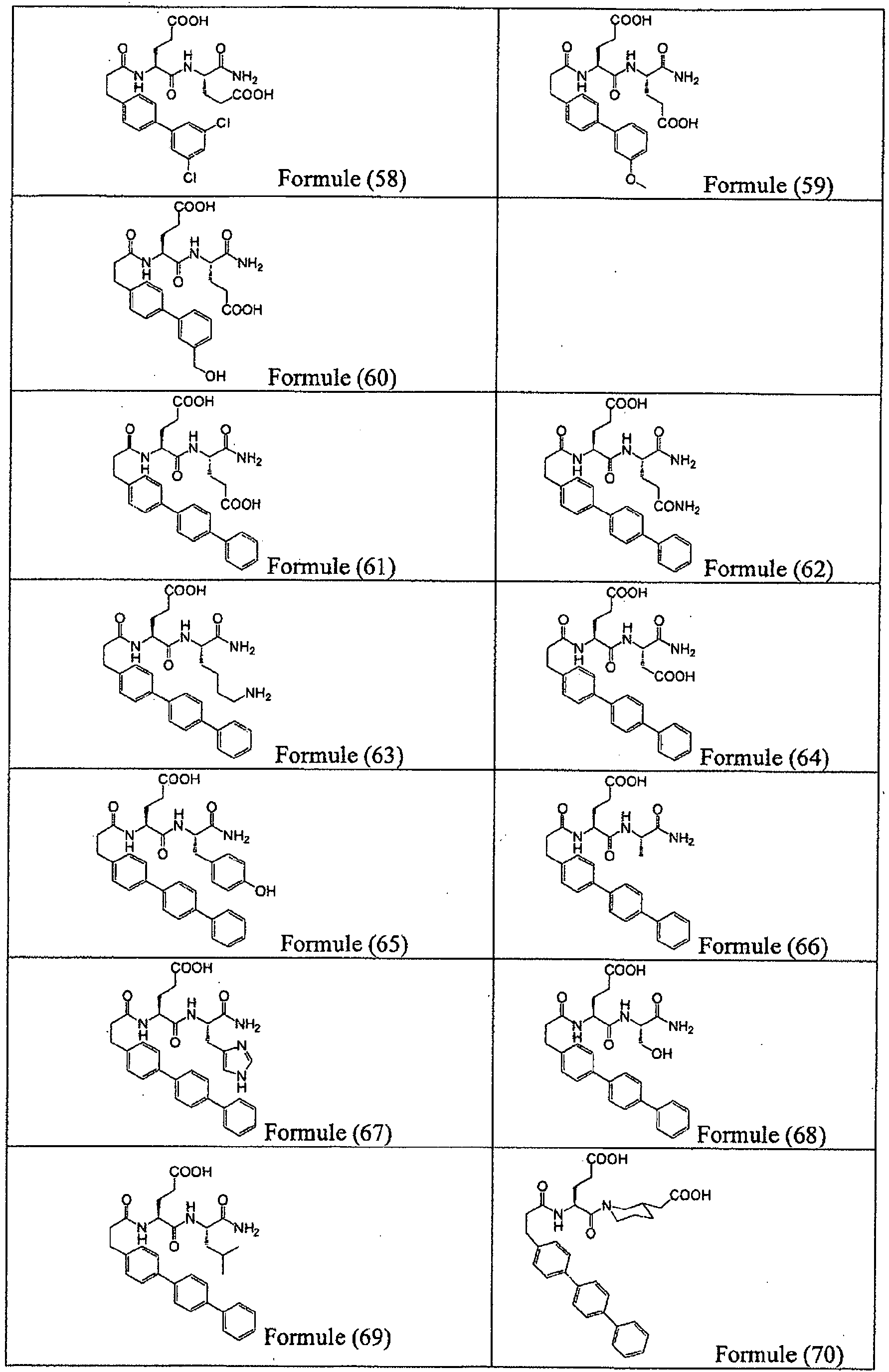

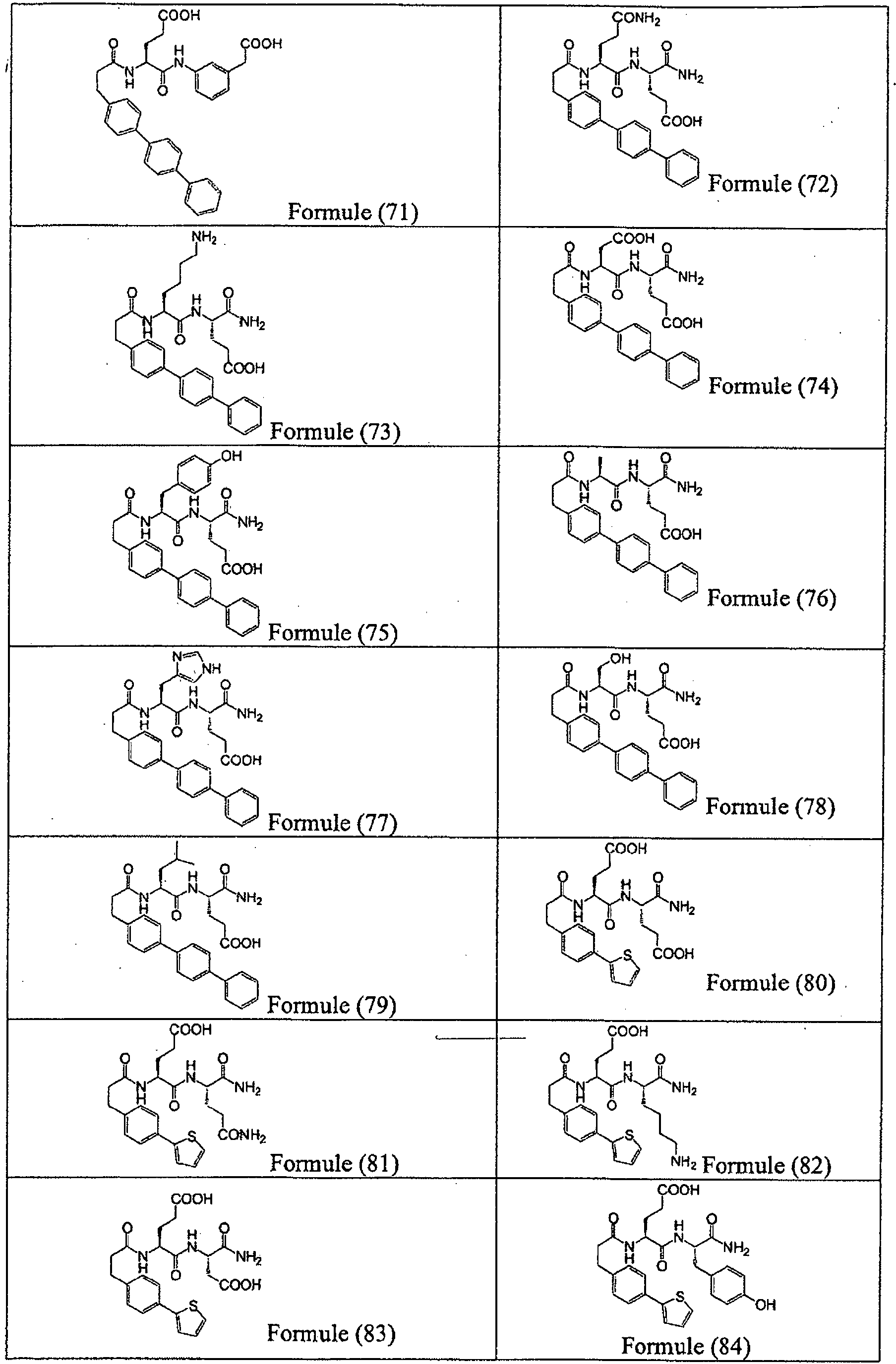

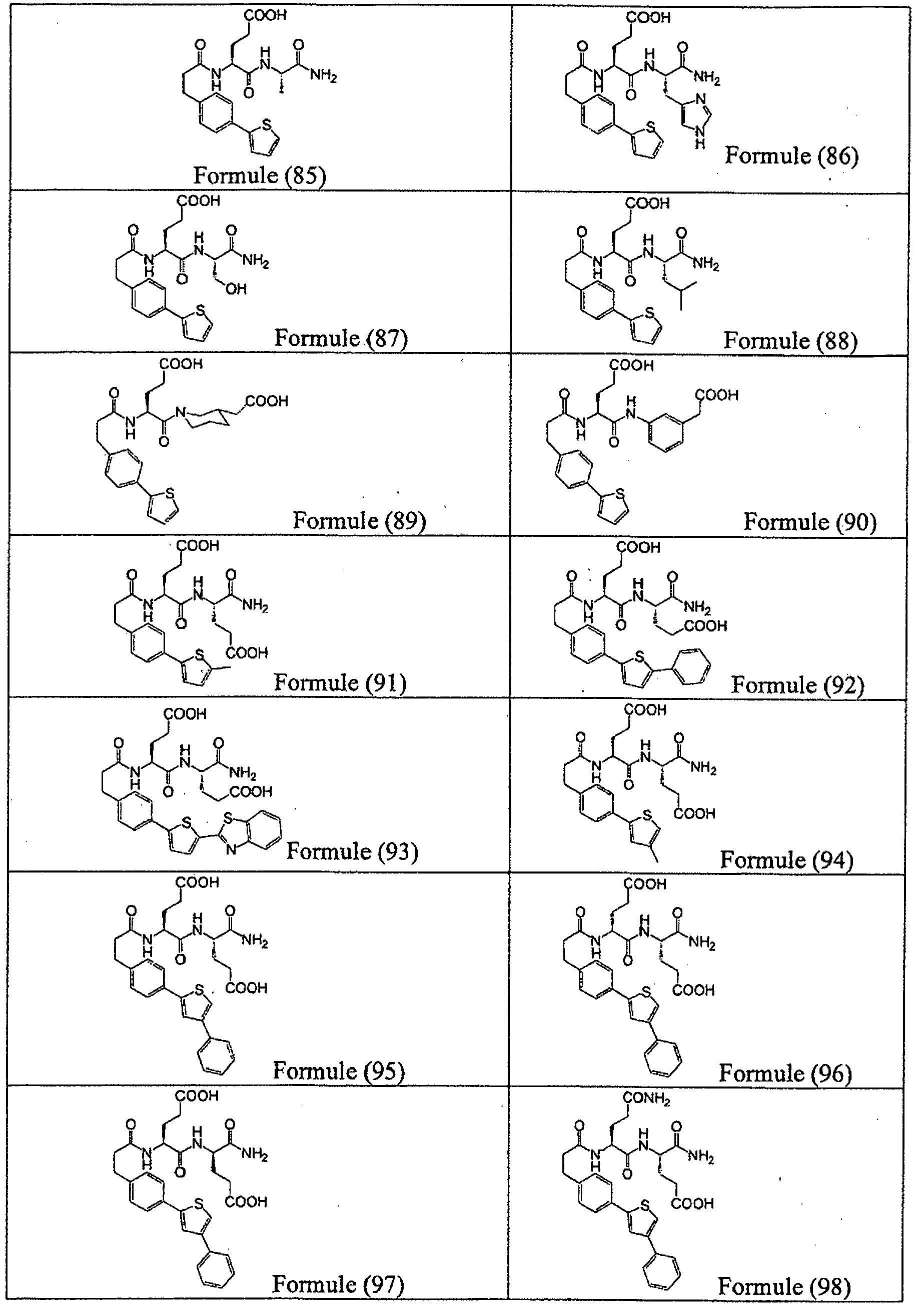

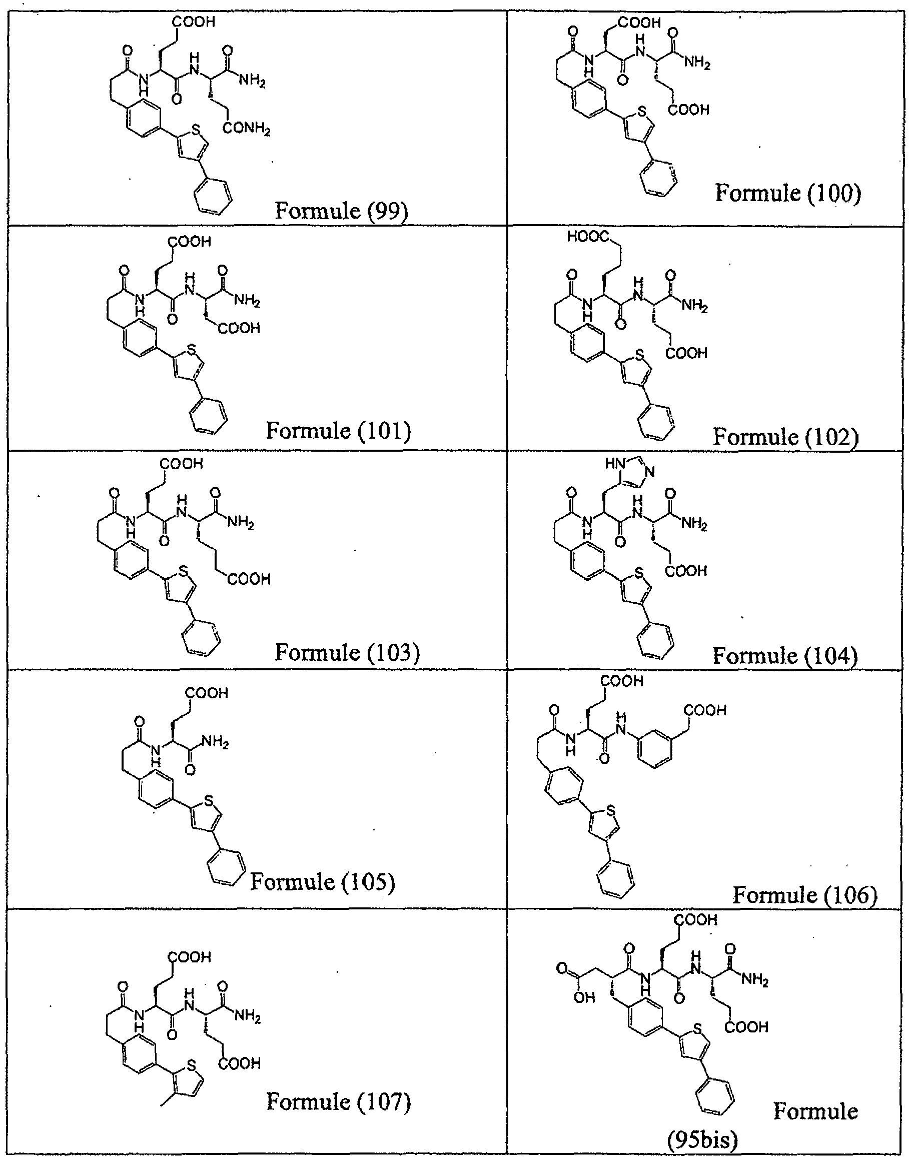

14. Composés selon l'une quelconque des revendications précédentes pour une utilisation à titre de médicament.

15. Composés selon l'une quelconque des revendications 1 à 13 pour une utilisation en tant qu'inhibiteurs des métallo protéinases de la matrice extracellulaire.

16. Composés selon l'une quelconque des revendications 10 à 13 pour une utilisation en tant qu'inhibiteurs de la métallo protéinase de la matrice extracellulaire 12, MMP-12.

17. Composition pharmaceutique comprenant au moins un composé selon l'une quelconque des revendications 1 à 13 et un excipient pharmaceutiquement acceptable.

18. Composés selon l'une quelconque des revendications 1 à 13 pour une utilisation à titre de médicament pour le traitement du cancer, des maladies inflammatoires, de la maladie pulmonaire obstructive chronique (COPD), de l'arthrite, de l'arthrite rhumatoïde, de l'athérosclérose et de la rupture d'anévrisme.

19. Composés de formule (2) suivante : 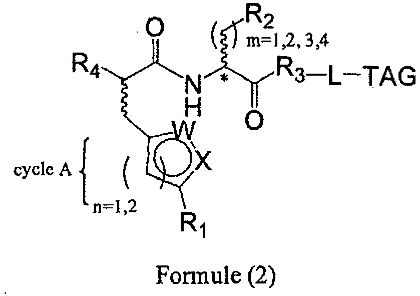 dans laquelle :
- R₁, R₂, R₃ et R₄ sont tels que définis à la revendication 1,
- L est un bras espaceur choisi parmi les chaînes alkyles en C₁-C₁₂, et les éthers de glycol dans lesquels la chaîne carbonée comporte de 2 à 12 atomes de carbone, et
- TAG est un marqueur,
le groupement R₃ étant lié au bras espaceur L via une fonction carboxamide -C(=O)NH₂ terminale.

20. Composés selon la revendication 19 pour une utilisation en tant qu'agents de contraste pour la détection des métallo protéinases de la matrice extracellulaire.

## Patentansprüche

1. Verbindungen der folgenden Formel (1) : 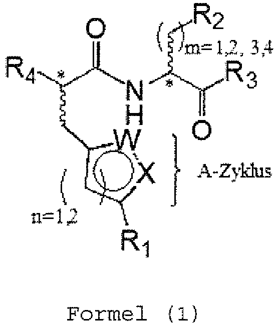 in der:
- n gleich 1 oder 2 ist,
- wenn n = 1 : sind W und X unabhängig voneinander O, N oder C,
- wenn n = 2: sind W und X C,
- R₁ ausgewählt ist aus einem Jodatom oder einer Phenyl-, Biphenyl-, 3'-Chlorobiphenyl-, Phenoxy-, Phenoxymethyl-, Phenylethynyl-, Pyrimidin-, 1-Methyl-1*H*-Pyrazol-, 5-Methyl-1, 2, 4-Oxadiazol-, 1, 2, 3-Thiadiazol-, 1*H*-Pyrrol-, Thiazol-, Thiophen-, 3a,7a-Dihydrobenzo[*d*]Thiazol-, 3-Aminophenyl-, 3-Hydroxyphenyl-, 3-Nitrophenyl-, 3-Carboxyphenyl-, 3'-Chlorophenyl-, 3,5-Dichlorophenyl-, 3-Methoxyphenyl-, 3-Hydroxymethylphenylgruppe oder einem Thiophenzyklus, der bei den, voneinander unabhängigen, Positionen 2 und/oder 3 und/oder 4 und/oder 5 durch eine aus einer Methyl-, Phenyl-, 3a,7a-Dihydrobenzo[*d*]Thiazolgruppe oder aus einem Wasserstoffatom ausgewählte Gruppe substituiert ist,
- m eine Ganzzahl zwischen 1 und einschließlich 4 ist,
- wenn m = 4, R₂ eine Aminogruppe ist,
- wenn m = 1 oder 2 oder 3, R₂ eine Carbonsäuregruppe ist,
- wenn m = 1 oder 2, R₂ eine Carboxamidgruppe ist,
- wenn m = 1, R₂ eine 4-Hydroxyphenylgruppe oder eine *1H-*Imidazolgruppe oder eine Hydroxylgruppe oder eine Isopropylgruppe oder eine Methylgruppe ist,
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1) über eine Aminofunktion verbunden ist, und
- R₄ H oder eine Carboxymethylgruppe -CH₂COOH ist,
und deren Diastereoisomere und Enantiomere.

2. Verbindungen nach Anspruch 1, **dadurch gekennzeichnet, dass** in der Formel (I):
- W O ist, X N ist und n = 1, die einen A-Zyklus bilden, der ein Isoxazolzyklus ist, und dadurch, dass sie die folgende Formel (1-A) aufweisen: 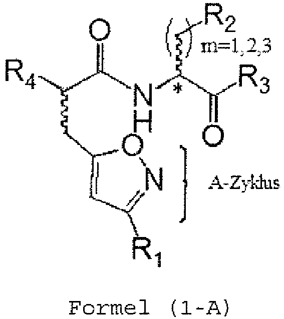 in der:
- R₁ eine Phenyl-, Biphenyl- oder 3'-Chlorobiphenylgruppe ist,
- m eine Ganzzahl zwischen 1 und einschließlich 3 ist,
- wenn m gleich 1 oder 3 ist, R₂ eine Carbonsäuregruppe ist, und wenn m gleich 2 ist, R₂ eine Carbonsäuregruppe oder eine Carboxamidgruppe ist,
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-A) über eine Aminofunktion verbunden ist, und
- R₄ H oder eine Carboxymethylgruppe -CH₂COOH ist,
und deren Diastereoisomere und Enantiomere.

3. Verbindungen nach Anspruch 1 oder 2, **dadurch gekennzeichnet, dass** sie aus den Verbindungen der folgenden Formeln (3) bis (23) ausgewählt sind:

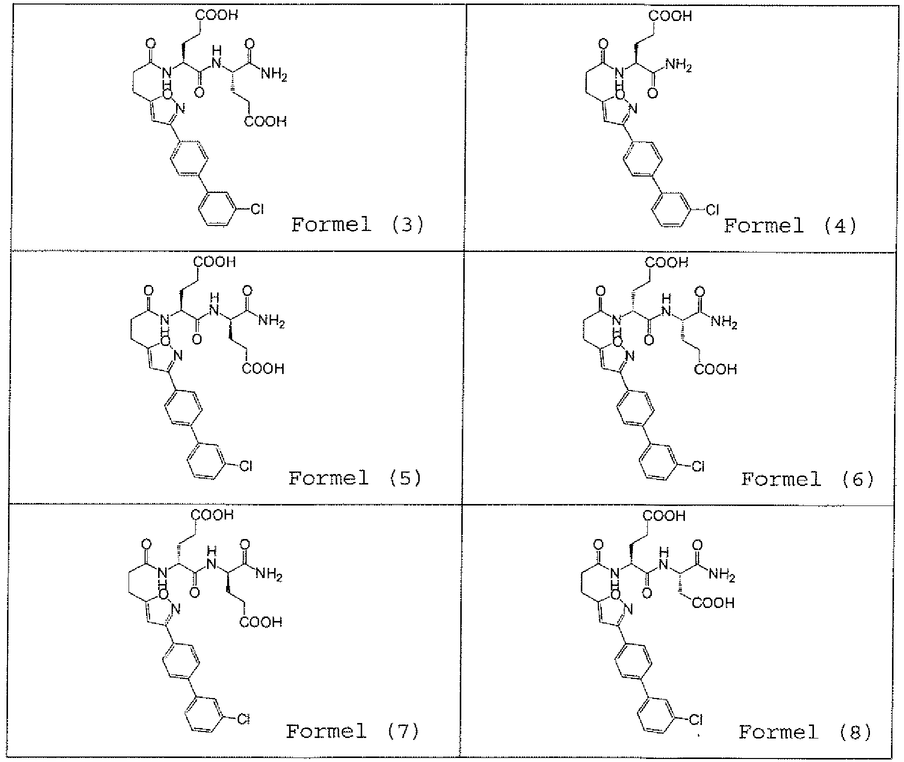

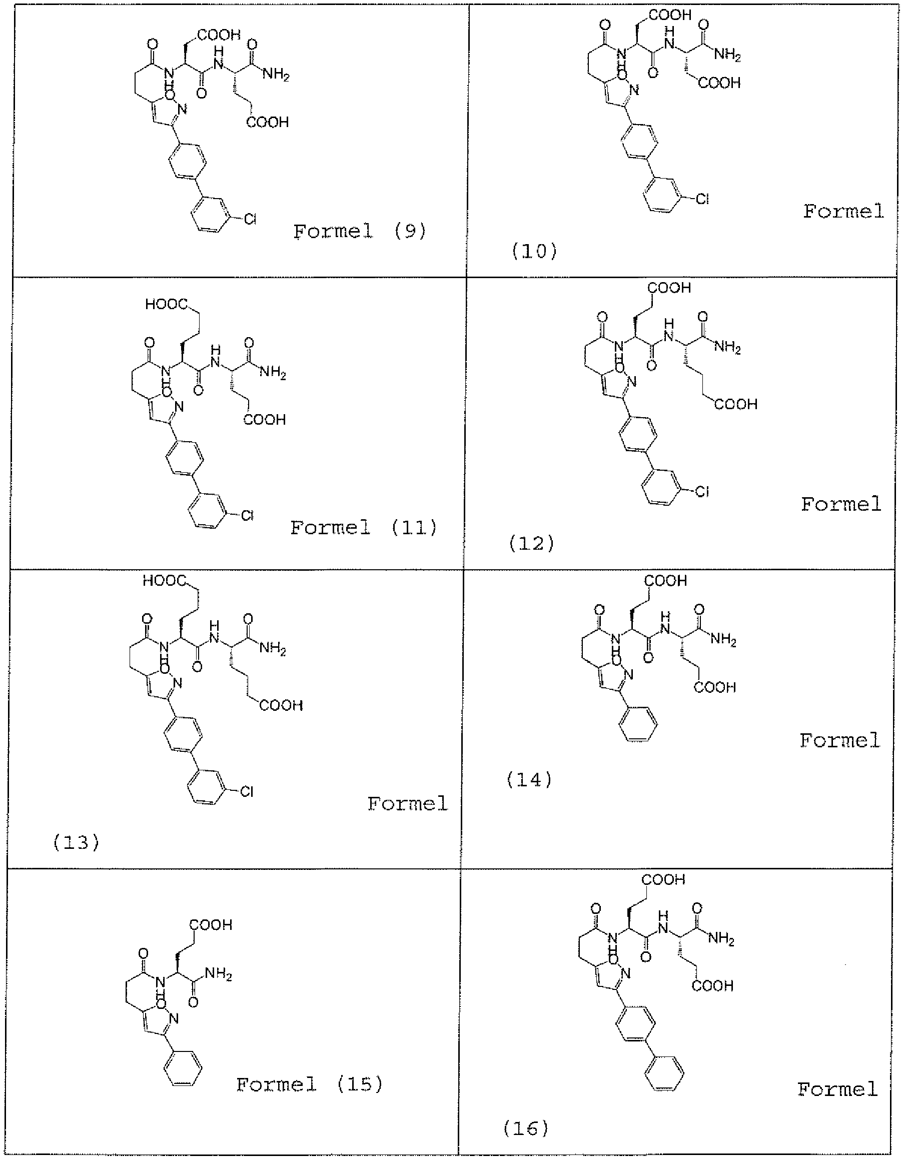

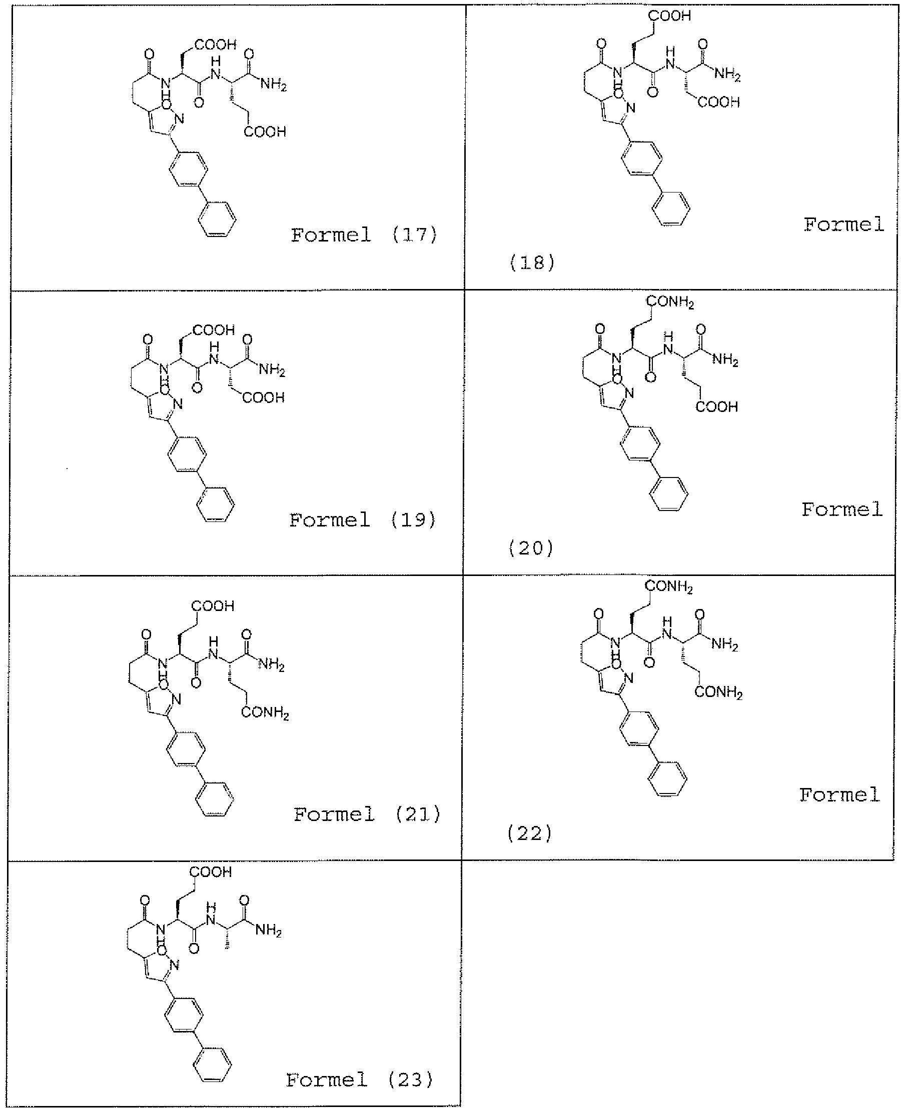

4. Verbindungen nach Anspruch 1, **dadurch gekennzeichnet, dass** in der Formel (1):
- n = 1,
- W N ist,
- X O ist,
- R₁ eine Phenyl-, Biphenyl- oder 3'-Chlorobiphenylgruppe ist
- m eine Ganzzahl zwischen 1 und einschließlich 3 ist,
- wenn m gleich 1 oder 3 ist, R₂ eine Carbonsäuregruppe ist, und wenn m gleich 2 ist, R₂ eine Carbonsäuregruppe oder eine Carboxamidgruppe ist,
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1) über eine Aminofunktion verbunden ist, und
- R₄ H oder eine Carboxymethylgruppe -CH₂COOH ist,
und dadurch, dass sie die folgende Formel (1-B) aufweisen: 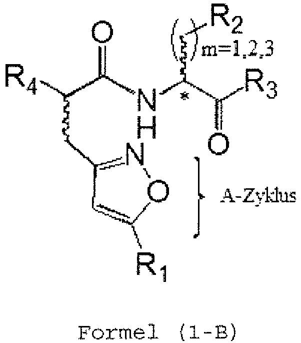 und deren Diastereoisomere und Enantiomere.

5. Verbindungen nach Anspruch 1, **dadurch gekennzeichnet, dass** in der Formel 1:
- W und X C sind und n = 2, die auf diese Weise einen A-Zyklus bilden, der ein Benzolzyklus ist,
und dadurch, dass sie die folgende Formel (1-C) aufweisen: 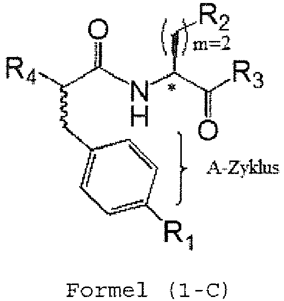 in der:
- R₁ ausgewählt ist aus einem Jodatom oder einer Phenyl-, Biphenyl-, 3'-Chlorobiphenyl-, Phenoxy-, Phenoxymethyl-, Phenylethynyl-, Pyrimidin-, 1-Methyl-1*H*-Pyrazol-, 5-Methyl-1,2,4-Oxadiazol-, 1,2,3-Thiadiazol-, 1*H*-Pyrrol-, Thiazol-, Thiophen- und 3a,7a-Dihydrobenzo[*d*]Thiazolgruppe,
- m = 2,
- R₂ eine Carbonsäuregruppe ist und
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-C) über eine Aminofunktion verbunden ist, und
- R₄ H oder eine Carboxymethylgruppe -CH₂COOH ist,
und deren Diastereoisomere.

6. Verbindungen nach Anspruch 1, **dadurch gekennzeichnet, dass** in der Formel (1):
- W und X C sind und n = 2, die auf diese Weise einen A-zyklus bilden, der ein Benzolzyklus ist,
und dadurch, dass sie die folgende Formel (1-D) aufweisen: 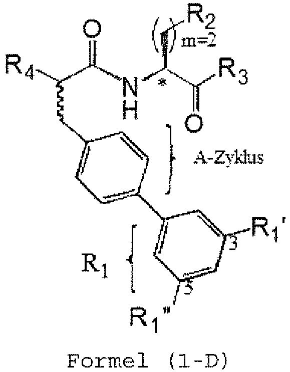 in der:
- R₁ ist:
- entweder eine nicht substituierte Phenylgruppe (R₁' = H et R₁" = H),
- oder eine Phenylgruppe, die bei Position 3 monosubstituiert wird durch eine Aminogruppe (R₁' = NH₂, R₁" = H) oder eine Hydroxylgruppe (R₁' = OH, R₁" = H) oder durch eine Nitrogruppe (R₁' = NO₂, R₁" = H) oder durch eine Carboxylgruppe (R₁' = COOH, R₁" = H) oder durch ein Chloratom (R₁' = Cl, R₁", = H) oder durch eine Methoxygruppe (R₁' = OMe, R₁" = H) oder durch eine Hydroxymethylgruppe (R₁' = CH₂OH, R₁" = H),
- oder eine Phenylgruppe, die bei den Positionen 3 und 5 disubstituiert wird durch ein Chloratom (R₁' = Cl et R₁" = Cl),
- m = 2,
- R₂ eine Carbonsäuregruppe ist und
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-D) über eine Aminofunktion verbunden ist, und
- R₄ H oder eine Carboxymethylgruppe -CH₂COOH ist,
und deren Diastereoisomere.

7. Verbindungen nach Anspruch 1, **dadurch gekennzeichnet, dass** in der Formel (1):
- W und X C sind und n = 2, die auf diese Weise einen A-Zyklus bilden, der ein Benzolzyklus ist, und R₁ eine Biphenylgruppe ist, und dadurch, dass sie die folgende Formel (1-E) aufweisen: 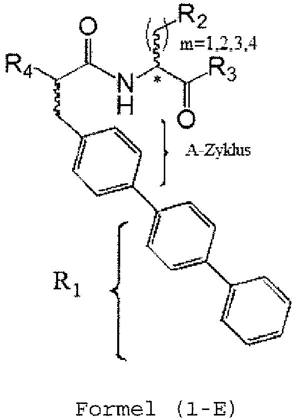 in der:
- m gleich 1, 2, 3 oder 4 ist,
- wenn m = 4, R₂ eine Aminogruppe ist,
- wenn m = 1 oder 2 oder 3, R₂ eine Carbonsäuregruppe ist,
- wenn m = 2, R₂ eine Carboxamidgruppe ist,
- wenn m = 1, R₂ eine 4-Hydroxyphenyl- oder eine *1H*-Imidazol- oder eine Hydroxyl- oder eine Isopropyl- oder eine Methylgruppe ist,
- R₃ ausgewählt ist aus einer Glutamatgruppe der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ oder eine Carboxymethylpiperidingruppe, eine Carboxymethyl-3-Aminaphenylgruppe oder eine Aminogruppe sein können, wobei die Gruppierung R3 mit der Carbonylgruppierung der Formel (1-E) über eine Aminofunktion verbunden ist, und
- R₄ H oder eine Carboxymethylgruppe -CH₂COOH ist,
und deren Diastereoisomere und Enantiomere.

8. Verbindungen nach Anspruch 1, **dadurch gekennzeichnet, dass** in der Formel (1):
- W und X C sind und n = 2, die einen A-Zyklus bilden, der ein Benzolzyklus ist, und
- R₁ ein durch eine Gruppe R₁''' substituierter Thiophenzyklus ist und daduch, dass sie die folgende Formel(1-F) aufweisen: 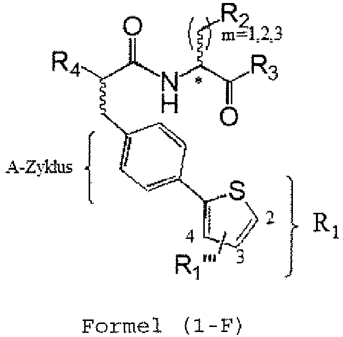 in der:
- R₁ ein Thiophenzyklus ist, der nicht substituiert ist (R₁''' = H) oder der entweder bei Position 2 monosubstituiert ist durch eine aus einer Methyl- (R₁''' = CH₃) oder Phenyl-e (R₁''' = Ph) oder 3a,7a-Dihydrobenzo[*d*]Thiazolgruppe ausgewählte Gruppe oder bei Position 3 durch eine aus einer Methyl- (R₁''' = CH₃) oder Phenylgruppe (R₁''' = Ph) ausgewählte Gruppe oder bei Position 4 durch eine Methylgruppe (R₁''' = CH₃).
- m = 1, 2 der 3 und
- R₂ eine Carbonsäure- oder Imidazolgruppe ist, wenn m = 1, oder eine Carbonsäure- oder Carboxamidgruppe ist, wenn m = 2, oder eine Carbonsäuregruppe ist, wenn m = 3, und
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-F) über eine Aminofunktion verbunden ist, und
- R₄ H oder eine Carboxymethylgruppe -CH₂COOH ist,
und deren Diastereoisomere und Enantiomere.

9. Verbindungen nach Anspruch 8, **dadurch gekennzeichnet, dass** in der folgenden Formel (1-F1): 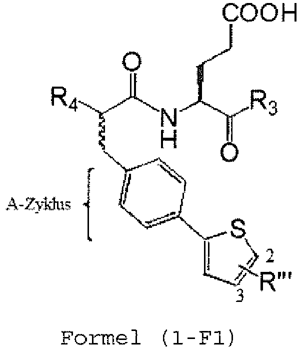 in der:
- R₁''' entweder bei Position 2 oder bei Position 3 des Thiophenzyklus ist und ausgewählt ist aus einer Methyl- (R₁''' = CH₃) oder Phenylgruppe (R₁''' = Ph), und
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-F1) über eine Aminofunktion verbunden ist, und
- R₄ H oder eine Carboxymethylgruppe -CH₂COOH ist,
und deren Diastereoisomere.

10. Verbindungen nach Anspruch 9, **dadurch gekennzeichnet, dass** sie aus den Verbindungen der folgenden Formeln (91), (92), (95), (97), (99), (101), (103), (105), (106) und (95 a) ausgewählt sind:

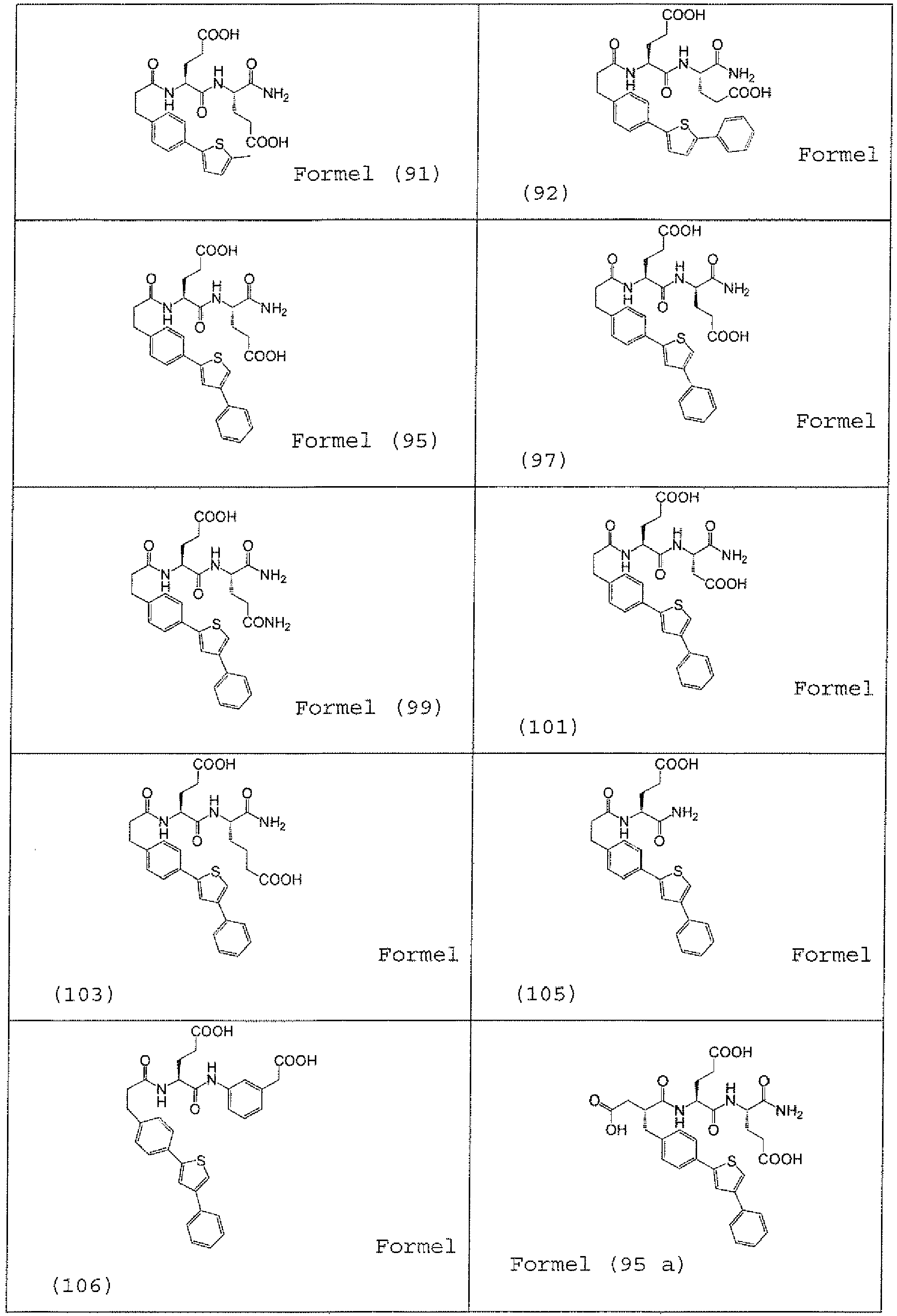

11. Verbindungen nach Anspruch 9 oder 10, **dadurch gekennzeichnet, dass** in der Formel (1-F1):
- R₁''' bei Position 3 des Thiophenzyklus ist und eine Phenylgruppe (R₁''' = Ph) ist und dadurch, dass sie die folgende Formel (1-F2) aufweisen: 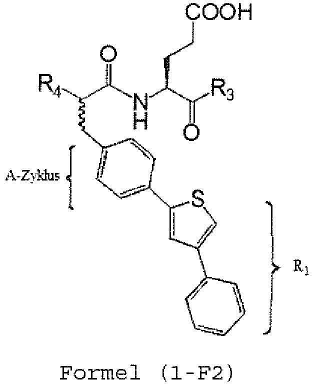 in der:
- R₃ ausgewählt ist aus einer Aminogruppe; einer Carboxymethylpiperidingruppe; einer Carboxymethyl-3-Aminophenylgruppe; einem Glutamatrest der Konfiguration L oder D, Homoglutamat, Aspartat, Glutamin, Alanin, Lysin, Tyrosin, Histidin, Serin, Leucin, wobei die Carboxylendfunktionen der Aminosäuren Carboxamidfunktionen -C(=O)NH₂ sein können und wobei die Gruppierung R₃ mit der Carbonylgruppierung der Formel (1-F2) über eine Aminofunktion verbunden ist, und
- R₄ H oder eine Carboxymethylgruppe -CH₂COOH ist.

12. Verbindungen nach einem der vorstehenden Ansprüche, **dadurch gekennzeichnet, dass** R₄ H ist.

13. Verbindungen nach Anspruch 1, **dadurch gekennzeichnet, dass** sie aus den Verbindungen der folgenden Formeln (25), (28) bis (40), (42) bis (107) ausgewählt sind:

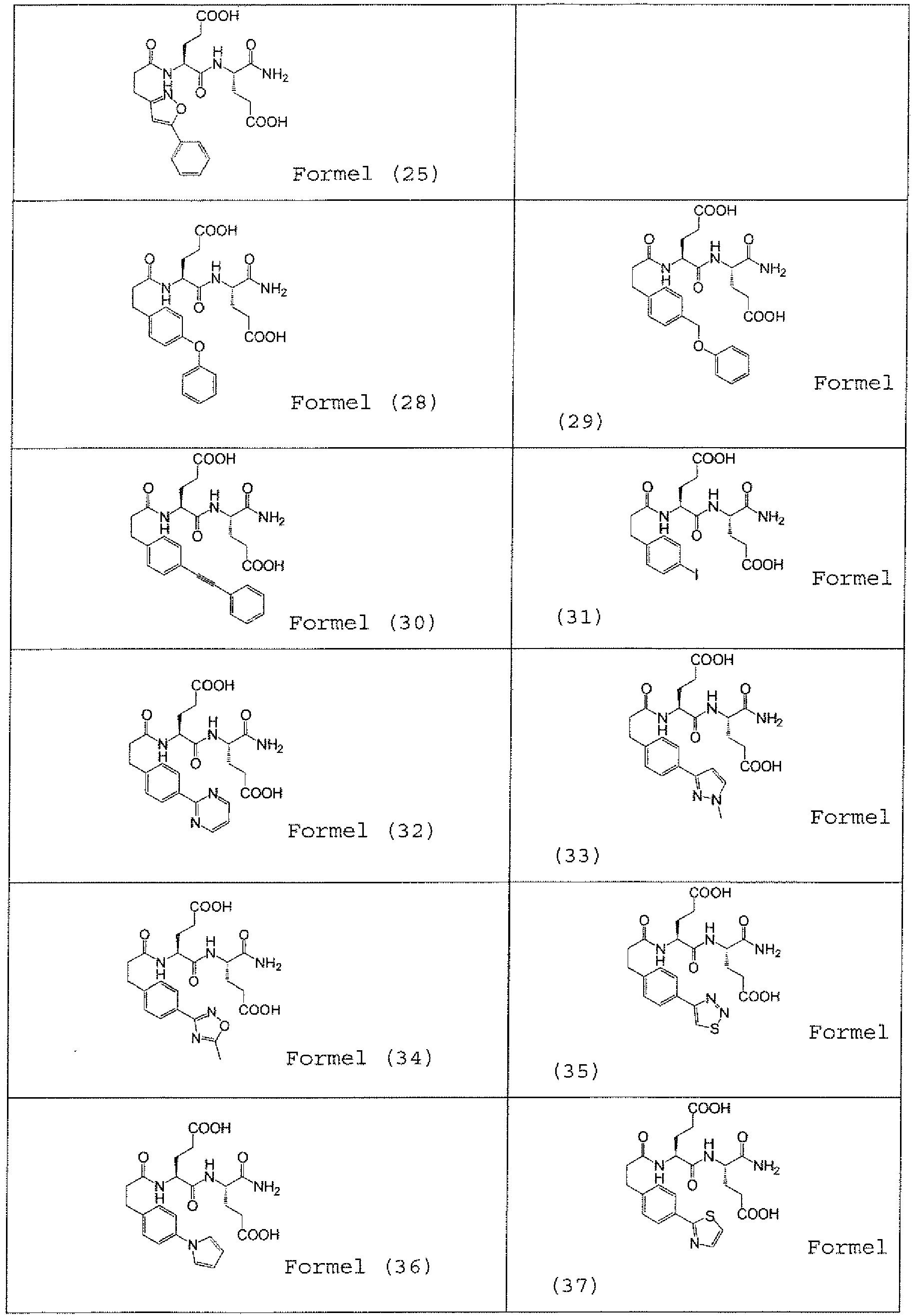

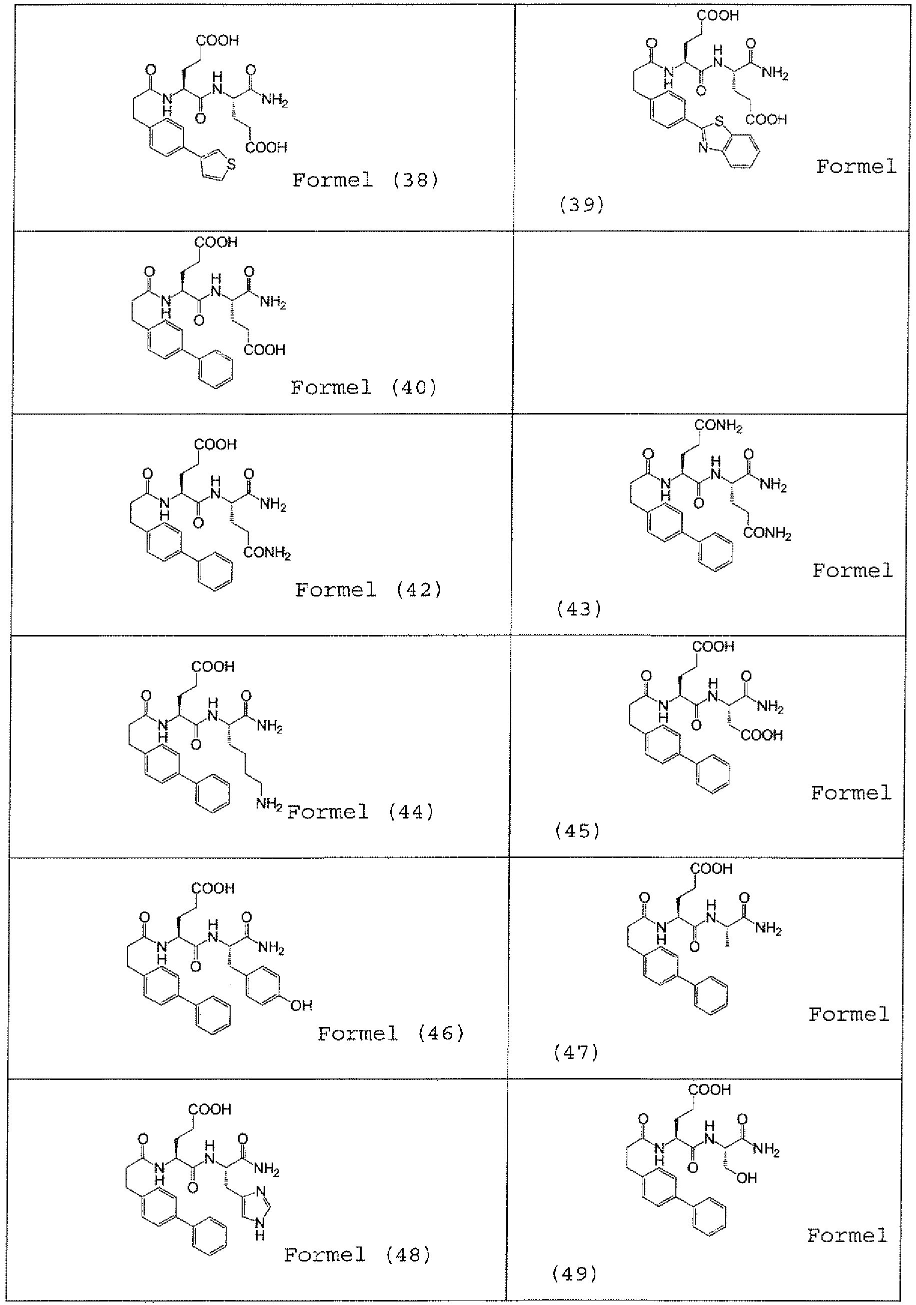

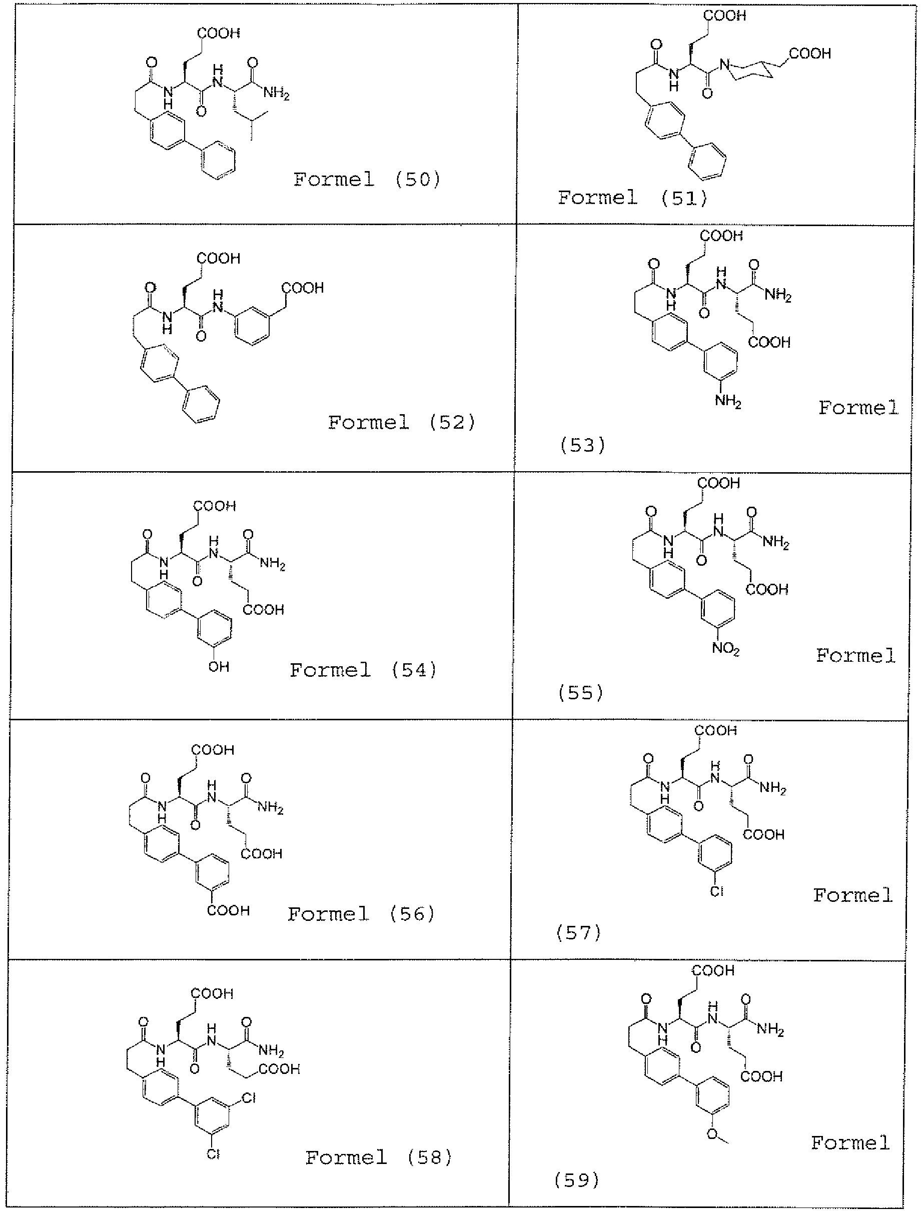

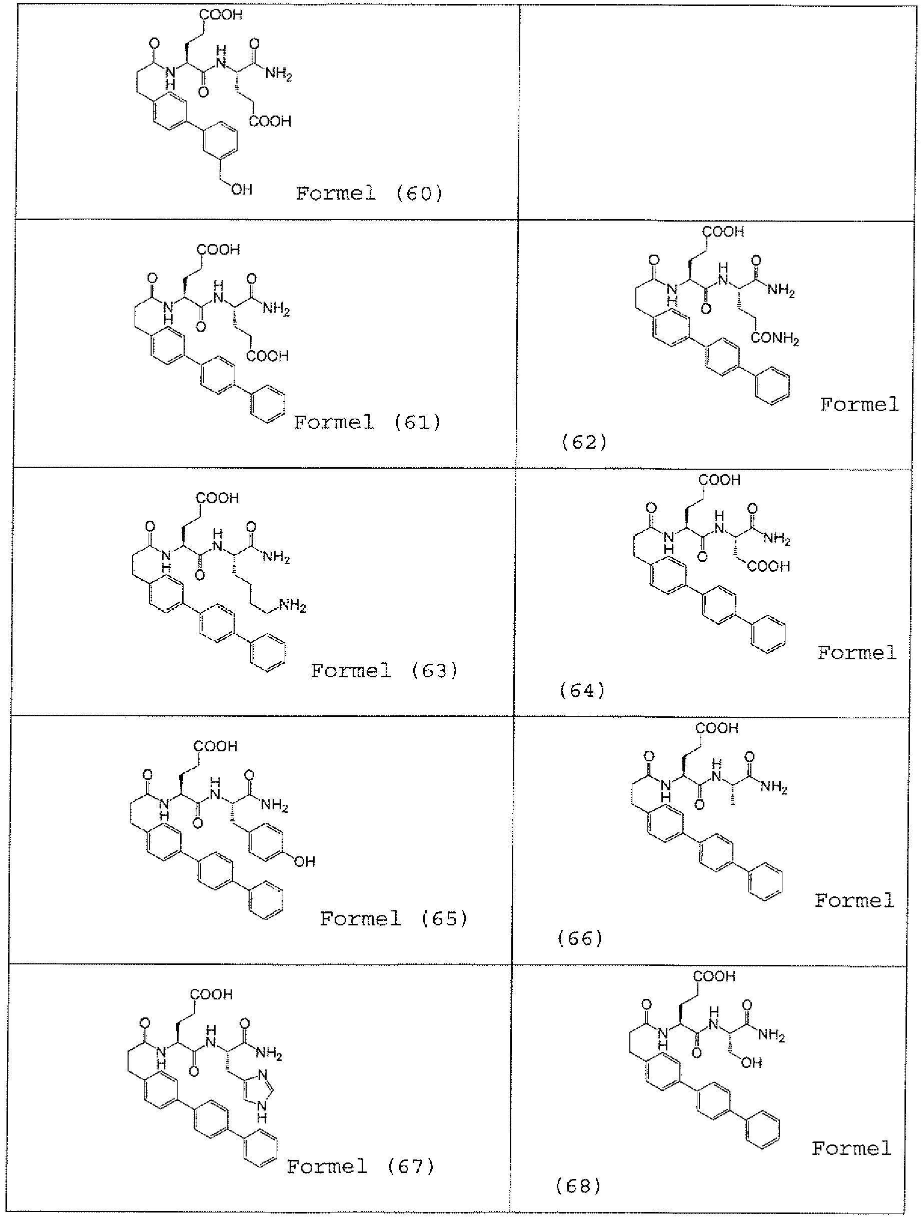

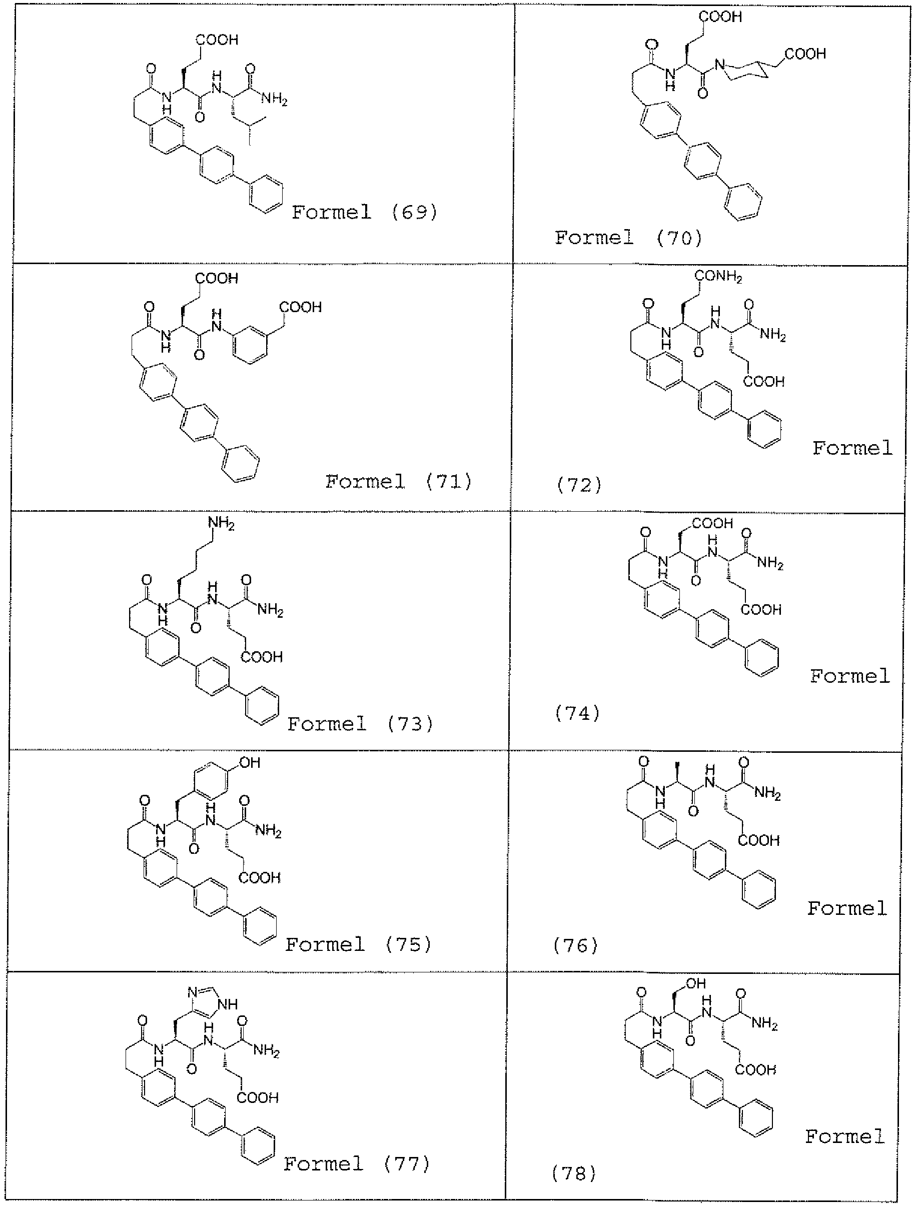

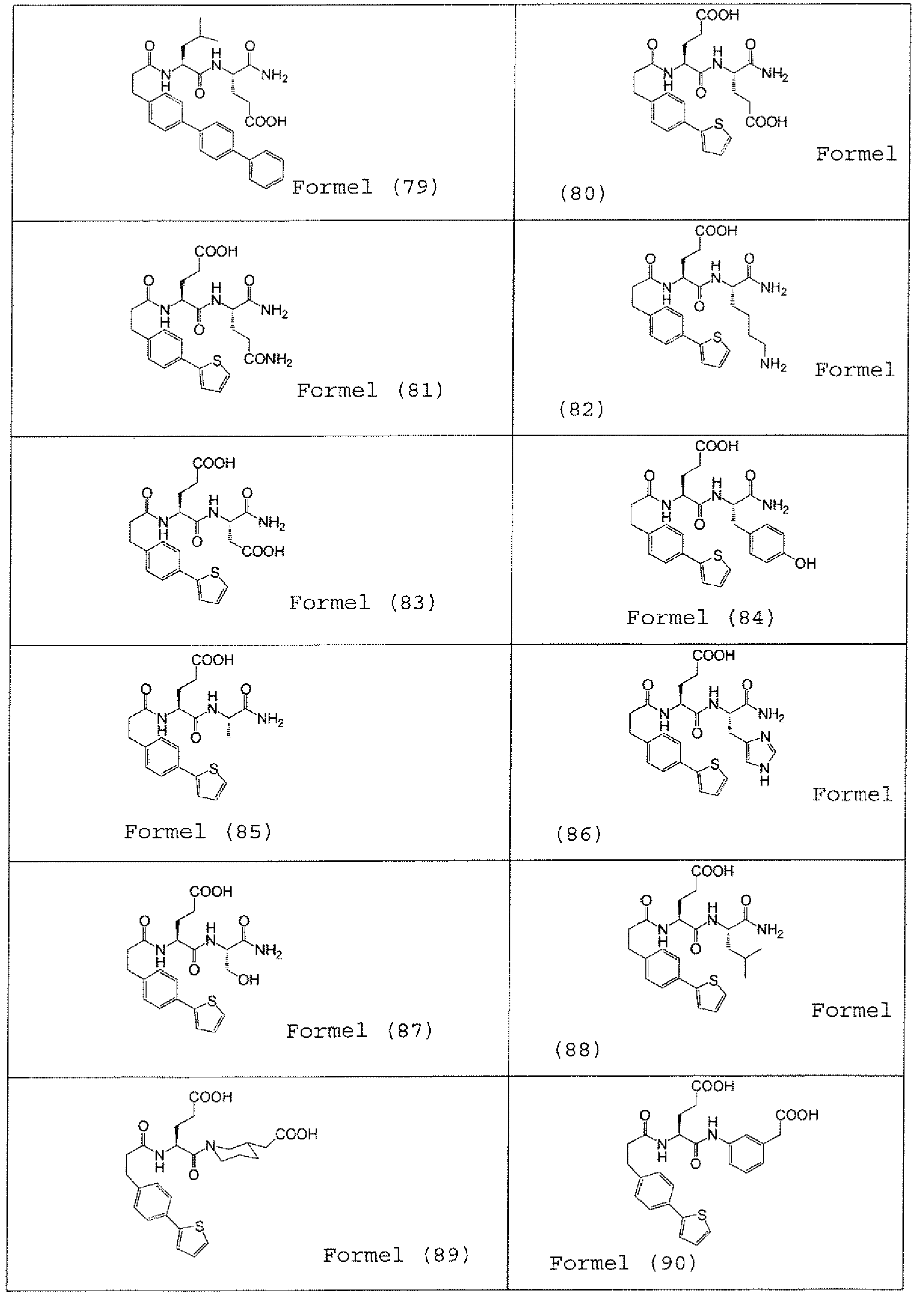

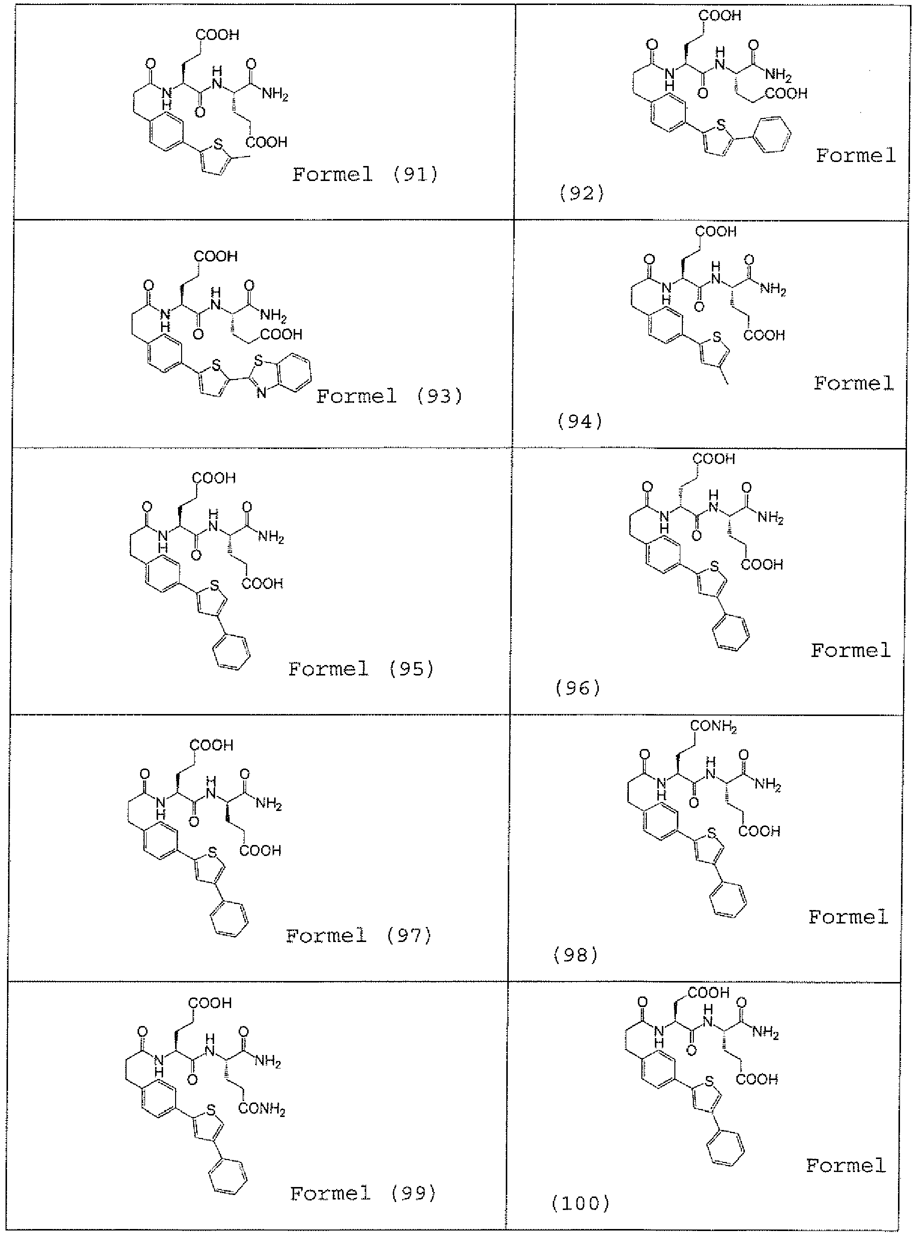

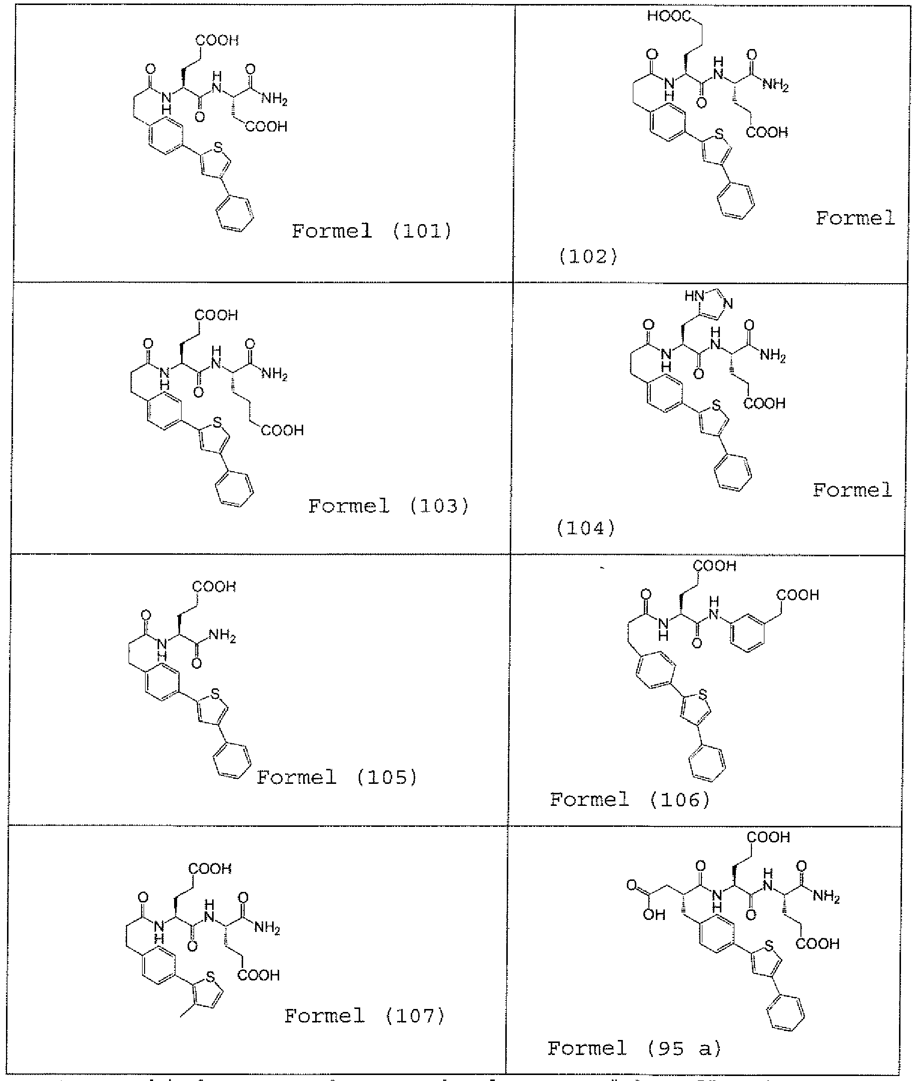

14. Verbindungen nach vorstehenden Ansprüchen für eine Verwendung als Arzneimittel.

15. Verbindungen nach Ansprüchen 1 bis 13 für eine Verwendung als Inhibitoren der Metallproteinasen der extrazellulären Matrix.

16. Verbindungen nach Ansprüchen 10 bis 13 für eine Verwendung als Inhibitoren der Metallproteinase der extrazellulären Matrix 12, MMP-12.

17. Pharmazeutische Verbindung mit mindestens einer Verbindung von Ansprüchen 1 bis 13 und einem pharmazeutisch akzeptablen Trägerstoff.

18. Verbindungen nach Ansprüchen 1 bis 13 für eine Verwendung als Arzneimittel zur Behandlung von Krebs, entzündlichen Erkrankungen, der chronisch-obstruktiven Lungenkrankheit (COPD), der Arthritis, der rheumatoiden Arthritis, der Atherosklerose und der Aneurysma-Ruptur.

19. Verbindungen der folgenden Formel (2): 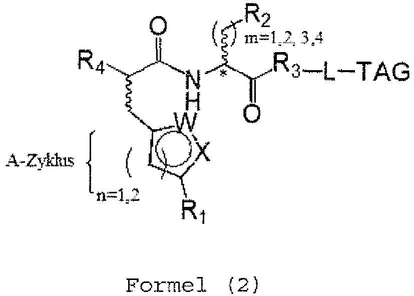 in der:
- R₁, R₂, R₃ und R₄ entsprechend ihrer Definition in Anspruch 1 sind,
- L ein aus den Alkylketten bei C₁-C₁₂ und den Glykolethern, in denen die Kohlenstoffkette 2 bis 12 Kohlenstoffatome umfasst, ausgewählter Spacerarm ist, und
- TAG ein Marker ist,
wobei die Gruppierung R₃ mit dem Spacerarm L über eine Carboxamidendfunktion -C(=O)NH₂ verbunden ist.

20. Verbindungen nach Anspruch 19 für eine Verwendung als Kontrastmittel zur Erkennung der Metallproteinasen der extrazellulären Matrix.

## Claims

1. Compounds with the following formula (1): 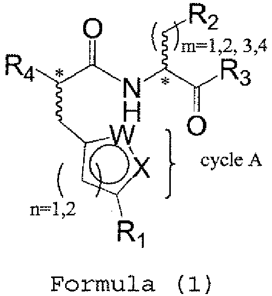 in which:
- n is equal to 1 or 2,
- when n = 1: W and X, independently of each other, are O, N or C,
- when n = 2: W and X are C,
- R₁ is chosen from among an iodine atom or a phenyl, biphenyl, 3'-chlorobiphenyl, phenoxy, phenoxymethyl, phenylethynyl, pyrimidine, 1-methyl-1*H*-pyrazole, 5-methyl-1,2,4-oxadiazole, 1,2,3-thiadiazole, 1*H*-pyrrole, thiazole, thiophene, 3a,7a-dihydrobenzo[*d*]thiazole, 3-aminophenyl, 3-hydroxyphenyl, 3-nitrophenyl, 3-carboxyphenyl, 3'-chlorophenyl, 3,5-dichlorophenyl, 3-methoxyphenyl, 3-hydroxymethylphenyl group, or a thiophene cycle substituted in positions, independently of each other, 2 and/or 3 and/or 4 and/or 5 by a group chosen from among a methyl, phenyl, 3a,7a-dihydrobenzo[*d*]thiazole or a hydrogen atom,
- m is an integer between 1 and 4 inclusive,
- when m = 4, R₂ is an amine group,
- when m = 1 or 2 or 3, R₂ is a carboxylic acid group,
- when m = 1 or 2, R₂ is an carboxamide group,
- when m = 1, R₂ is a 4-hydroxyphenyl group or a *1H*-imidazole or a hydroxyl group or an isopropyl group or a methyl group,
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1) through an amino function, and
- R₄ is H or a carboxymethyl group -CH₂COOH,
and diastereoisomers and enantiomers of them.

2. Compounds according to claim 1, **characterised in that** in formula (I):
- W is O, X is N, and n = 1, forming a cycle A that is an isoxazole cycle, and **in that** their chemical formula (1-A) is as follows: 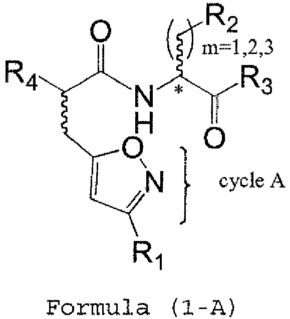 in which:
- R₁ is a phenyl, biphenyl or 3'-chlorobiphenyl group,
- m is an integer between 1 and 3 inclusive,
- when m is equal to 1 or 3, R₂ is a carboxylic acid group, and when m is equal to 2, R₂ is a carboxylic acid group or a carboxamide group,
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-A) through an amino function, and
- R₄ is H or a carboxymethyl group -CH₂COOH,
and diastereoisomers and enantiomers of them.

3. Compounds according to claim 1 or 2, **characterised in that** they are chosen from among compounds with the following formulas (3) to (23) :

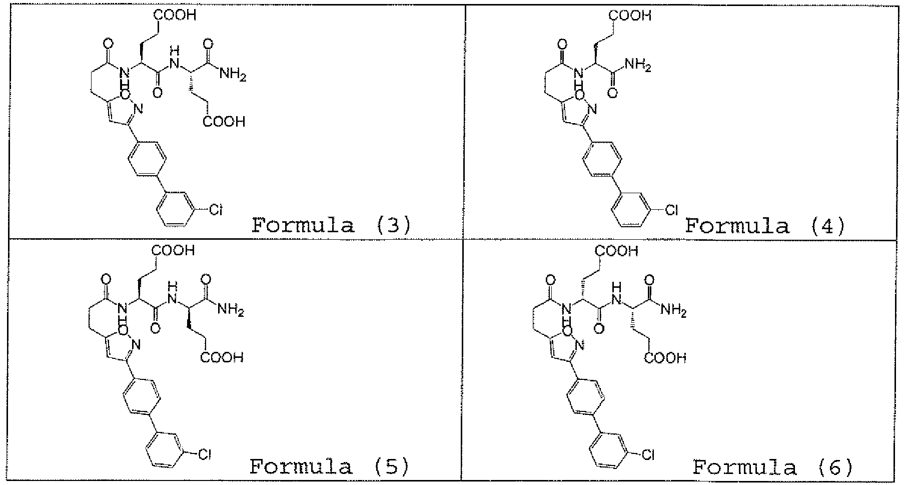

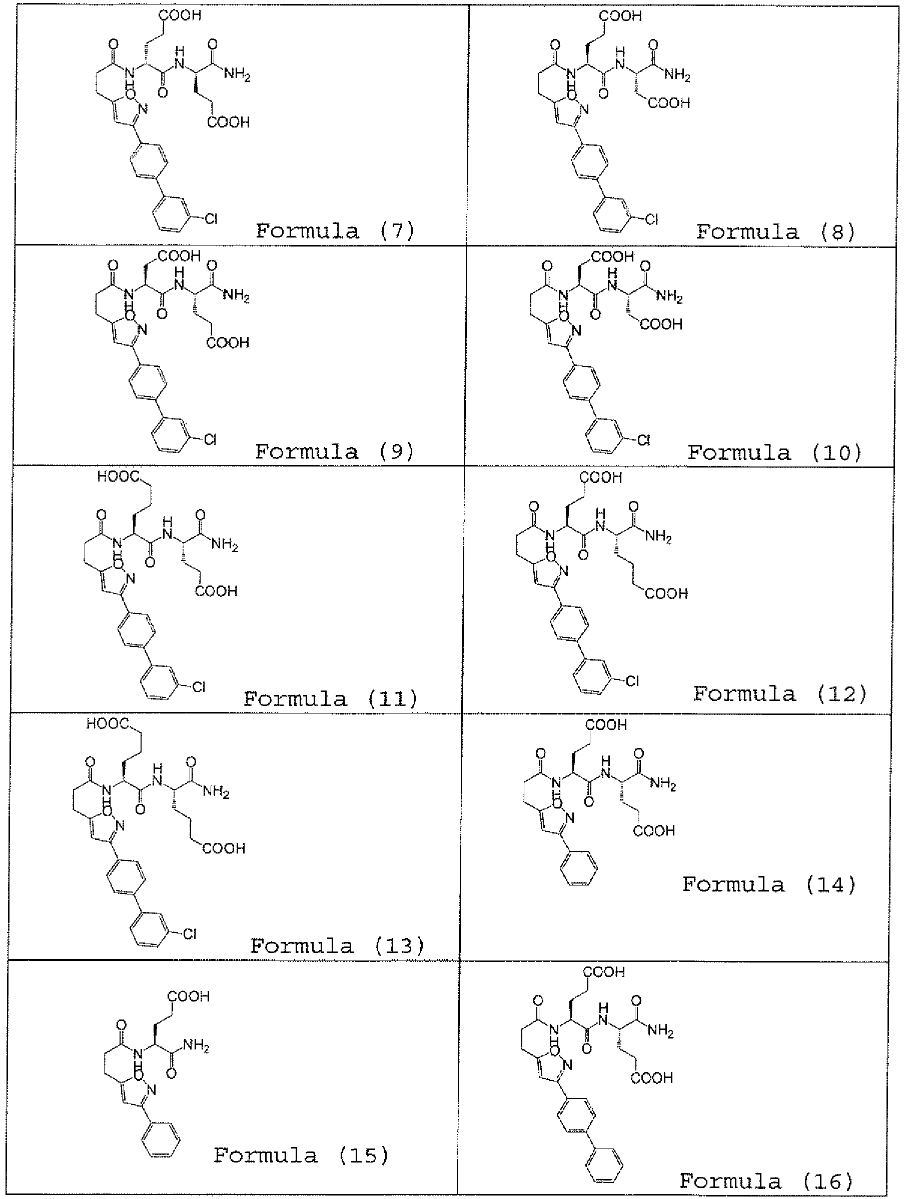

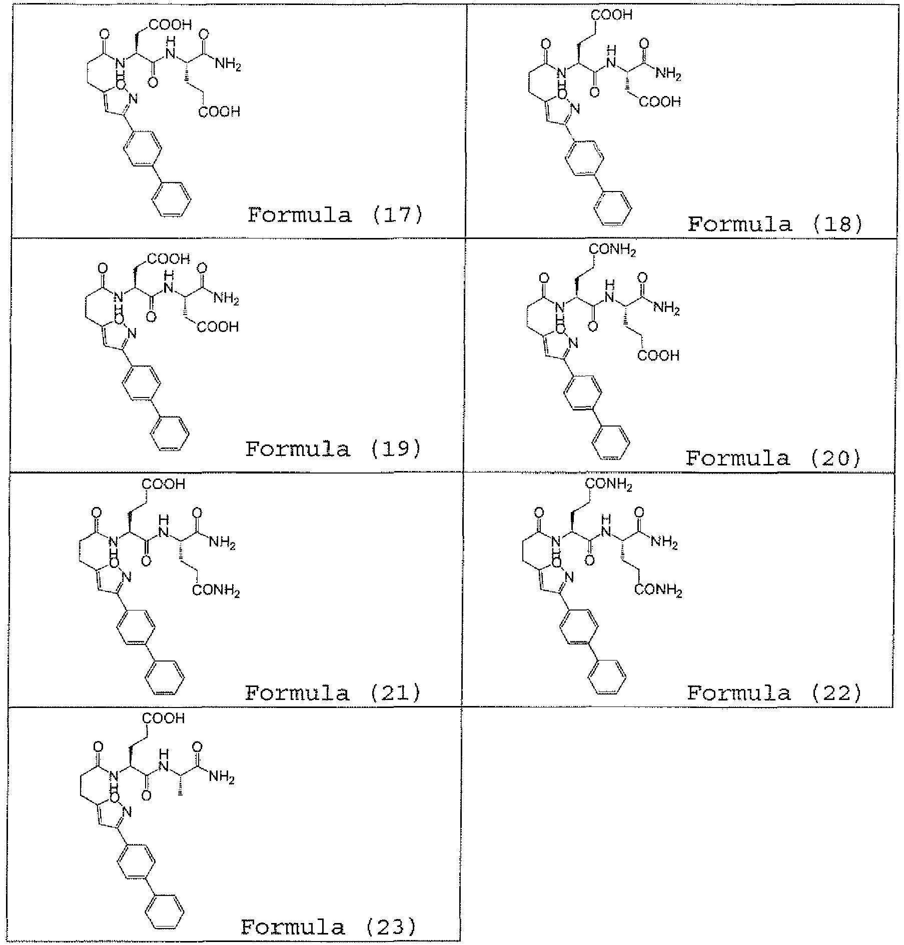

4. Compounds according to claim 1, **characterised in that** in formula (1) :
- n = 1,
- W is N,
- X is O,
- R₁ is a phenyl, biphenyl or 3'-chlorobiphenyl group,
- m is an integer between 1 and 3 inclusive,
- when m is equal to 1 or 3, R₂ is a carboxylic acid group, and when m is equal to 2, R₂ is a carboxylic acid group or a carboxamide group,
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1) through an amino function, and
- R₄ is H or a carboxymethyl group -CH₂COOH,
and **in that** their formula is (1-B) below: 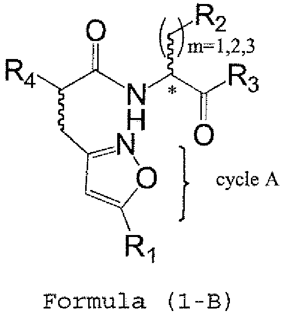 and diastereoisomers and enantiomers of them,

5. Compounds according to claim 1, **characterised in that** in formula 1:
- W and X are C and n = 2, thus forming a cycle A that is a benzene cycle,
and **in that** their formula is (1-C) below: 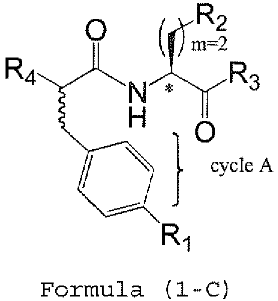 in which:
- R₁ is chosen from among an iodine atom or a phenyl, biphenyl'-chlorobiphenyl, phenoxy, phenoxymethyl, phenylethynyl, pyrimidine, 1-methyl-1*H*-pyrazole, 5-methyl-1,2,4-oxadiazole, 1,2,3-thiadiazole, 1*H*-pyrrole, thiazole, thiophene and 3a,7a-dihydrobenzo[*d*]thiazole group,
- m = 2,
- R₂ is a carboxylic acid group, and
- R₃ is chosen from among an amino group; a carboxymethyl piperidine group; a carboxymethyl 3-aminophenyl group; an L or D configuration glutamate, residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-C) through an amino function, and
- R₄ is H or a carboxymethyl group -CH₂COOH,
and diastereoisomers and enantiomers of them.

6. Compounds according to claim 1, **characterised in that** in formula (1):
- W and X are C and n = 2, thus forming a cycle A that is a benzene cycle,
and **in that** their formula is (1-D) below: 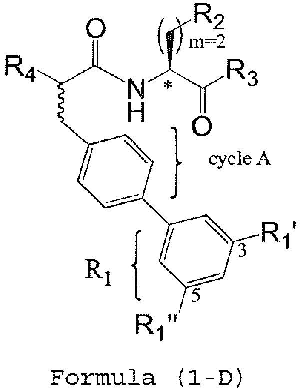 in which:
- R₁ is
- either an unsubstituted phenyl group (R_{1'} = H and R_{1"} = H),
- or a phenyl group monosubstituted in position 3 by an amino group (R_{1'} = NH₂, R_{1"} = H) or by a hydroxyl group (R_{1'} = OH, R_{1"} = H) or by a nitro group (R_{1'} = NO₂, R_{1"} = H) or by a carboxyl group (R_{1'} = COOH, R_{1"} = H) or by a chlorine atom (R_{1'} = Cl, R_{1"} = H) or by a methoxy group (R_{1'} = OMe, R_{1"} = H) or by a hydroxymethyl group (R_{1'} = CH₂OH, R_{1"} = H),
- or a phenyl group di-substituted in positions 3 and 5 by a chlorine atom (R_{1'} = Cl and R_{1"} = Cl),
- m = 2,
- R₂ is a carboxylic acid group, and
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-D) through an amino function, and
- R₄ is H or a carboxymethyl group -CH₂COOH,
and diastereoisomers and enantiomers of them.

7. Compounds according to claim 1, **characterised in that** in formula (1):
- W and X are C and n = 2, thus forming a cycle A that is a benzene cycle, and R₁ is a biphenyl group,
and **in that** their formula is (1-E) below: 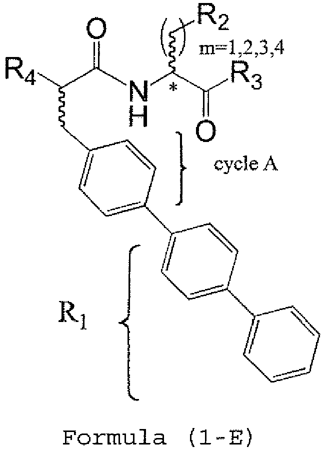 in which:
- m is equal to 1, 2, 3 or 4,
- when m = 4, R₂ is an amino group,
- when m = 1 or 2 or 3, R₂ is a carboxylic acid group,
- when m = 2, R₂ is a carboxamide group,
- when m = 1, R₂ is a 4-hydroxyphenyl group or a *1H-*imidazole or a hydroxyl or an isopropyl or methyl,
- R₃ is chosen from among an L or D configuration glutamate group, homoglutamate, aspartate, glutamine, alanine, lysine, tyrosine, histidine, serine, leucine, terminal carboxylic functions of said amino acids being possibly carboxamide -C(=O)NH₂ functions, or a carboxymethyl piperidine group; a carboxymethyl 3-aminophenyl group or an amino group, said R₃ group being linked to the carbonyl group with formula (1-E) through an amino function, and
- R₄ is H or a carboxymethyl group -CH₂COOH,
and diastereoisomers and enantiomers of them.

8. Compounds according to claim 1, **characterised in that** in formula (1):
- W and X are C and n = 2, forming a cycle A that is a benzene cycle, and
- R₁ is a thiophene cycle substituted by an R_{1'''} group and **in that** they have the following formula (1-F): 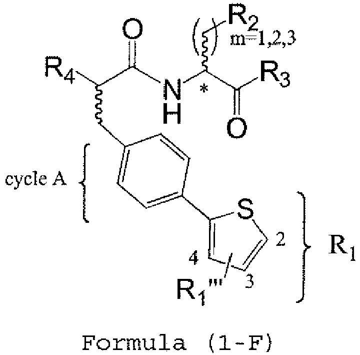 in which:
- R₁ is an unsubstituted thiophene cycle (R_{1'''} = H), or monosubstituted either in position 2 by a group chosen from among a methyl (R_{1'''} = CH₃) or phenyl (R_{1'''} = Ph) group, or 3a,7a-dihydrobenzo[*d*]thiazole, or in position 3 par by a group chosen from among a methyl (R_{1'''} = CH₃) or phenyl (R_{1'''} = Ph) group, or in position 4 by a methyl group (R_{1'''} = CH₃).
- m is equal to 1, 2 or 3, and
- R₂ is a carboxylic acid group or imidazole when m = 1, or a carboxylic acid group or carboxamide when m = 2, or a carboxylic acid group when m = 3, and
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-F) through an amino function, and
- R₄ is H or a carboxymethyl group -CH₂COOH,
and diastereoisomers and enantiomers of them.

9. Compounds according to claim 8, **characterised in that** they have formula (1-F1) given below: 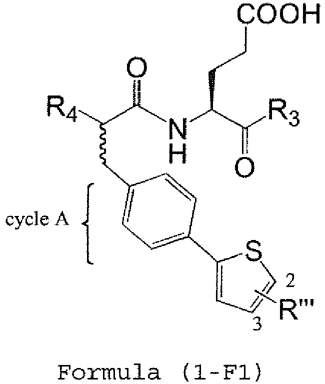 in which:
- R_{1'''} is either in position 2 or in position 3 in the thiophene cycle and is chosen from among a methyl (R_{1'''} = CH₃) or phenyl group or (R_{1'''} = Ph), and
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-F1) through an amino function, and
- R₄ is H or a carboxymethyl group -CH₂COOH,
and diastereoisomers of them.

10. Compounds according to claim 9, **characterised in that** they are chosen from among compounds with the following formulas (91), (92), (95), (97), (99), (101), (103), (105), (106) and (95bis):

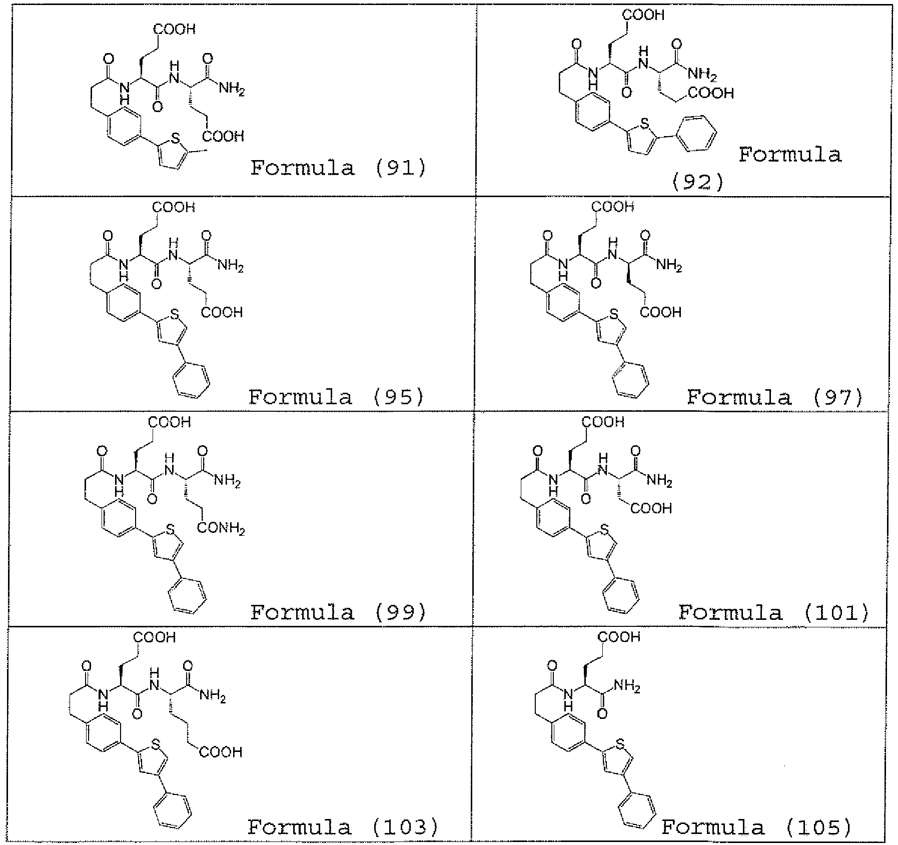

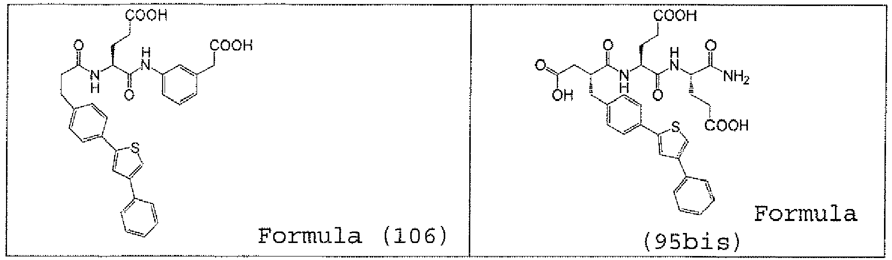

11. Compounds according to claim 9 or 10, **characterised in that** in formula (1-F1):
- R_{1'''} is in position 3 of the thiophene cycle and is a phenyl group (R_{1'''} = Ph), and **in that** they have the following formula (1-F2): 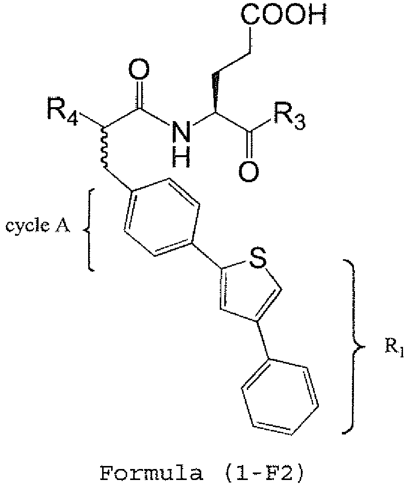 in which:
- R₃ is chosen from among an amino group; a carboxymethyl piperidine group; a carboxymethyl 3-aminophenyl group; an L or D configuration glutamate residue, homoglutamate, aspartate, glutamine, alanine, lysine, tyrosine, histidine, serine, leucine, terminal carboxylic functions of said amino acids being possibly carboxamide -C(=O)NH₂ functions, and said R₃ group being linked to the carbonyl group with formula (1-F2) through an amino function, and
- R₄ is H or a carboxymethyl group -CH₂COOH,

12. Compounds according to any one of the previous claims, **characterised in that** R₄ is H.

13. Compounds according to claim 1, **characterised in that** they are chosen from among compounds with the following formulas (25), (28) to (40), (42) to (107):

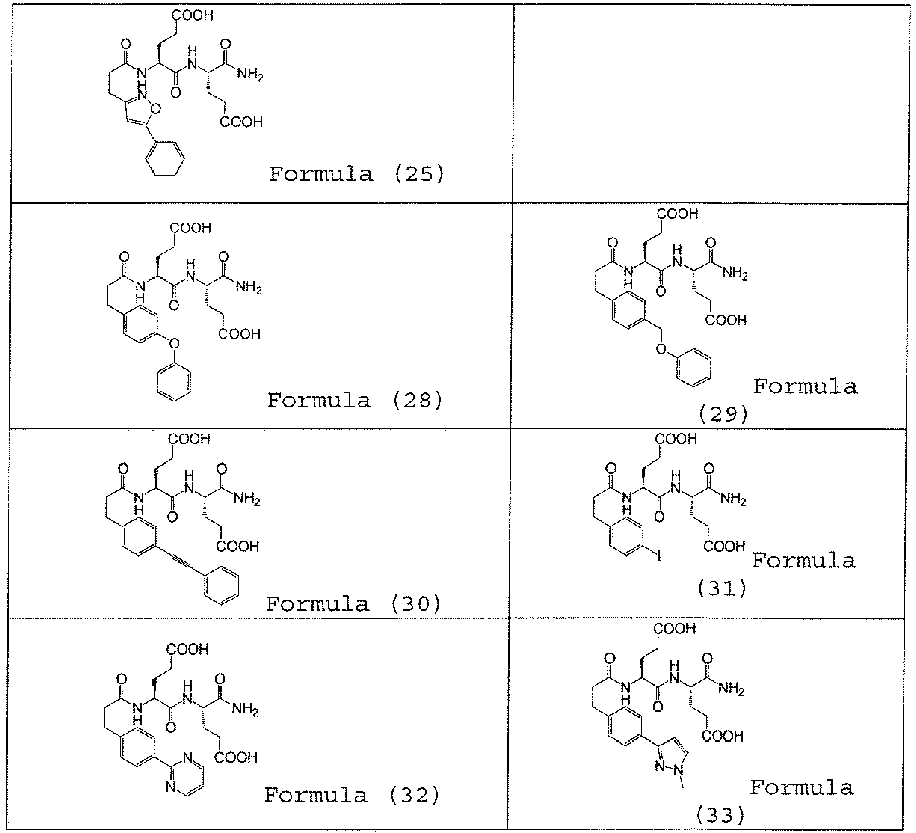

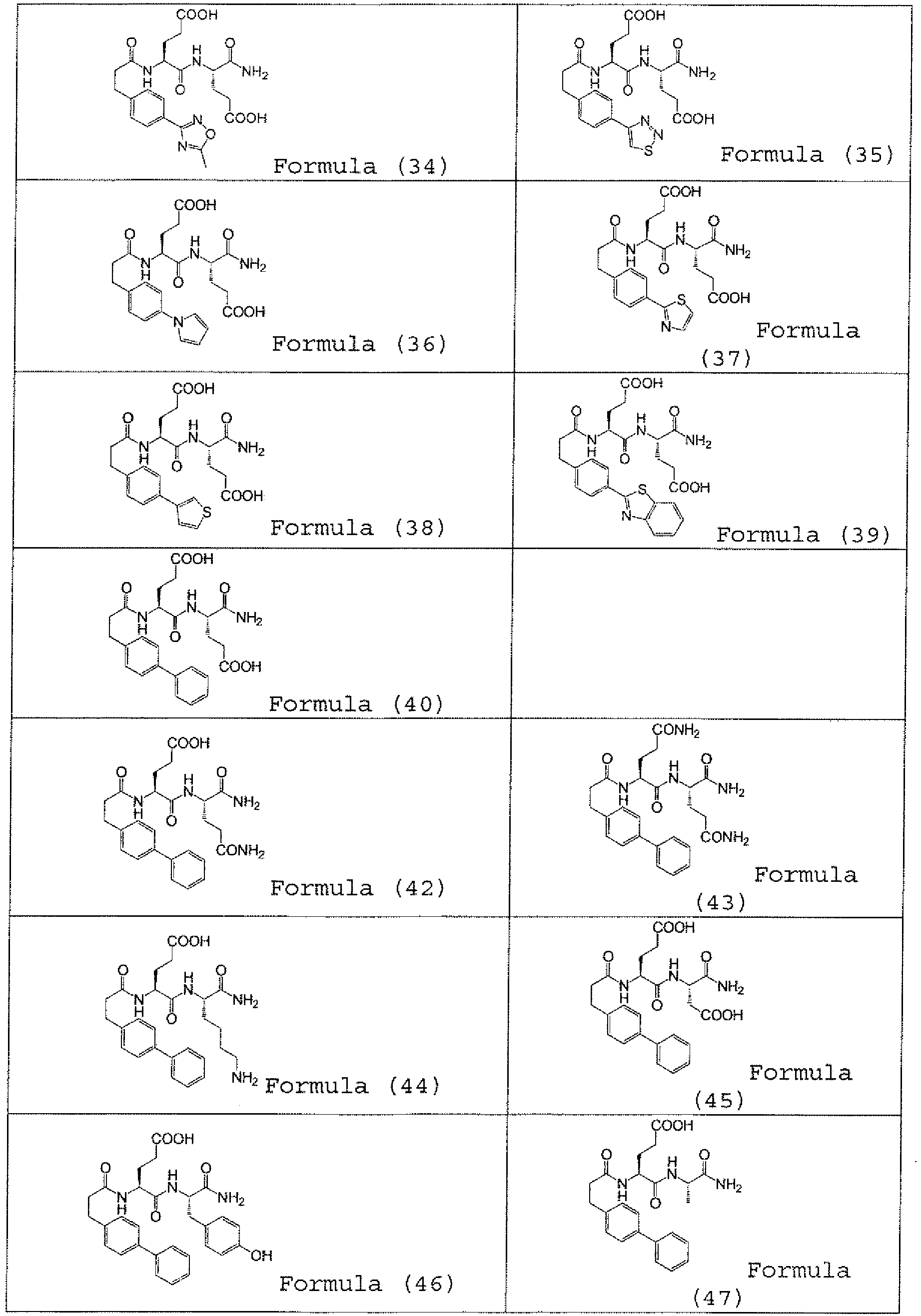

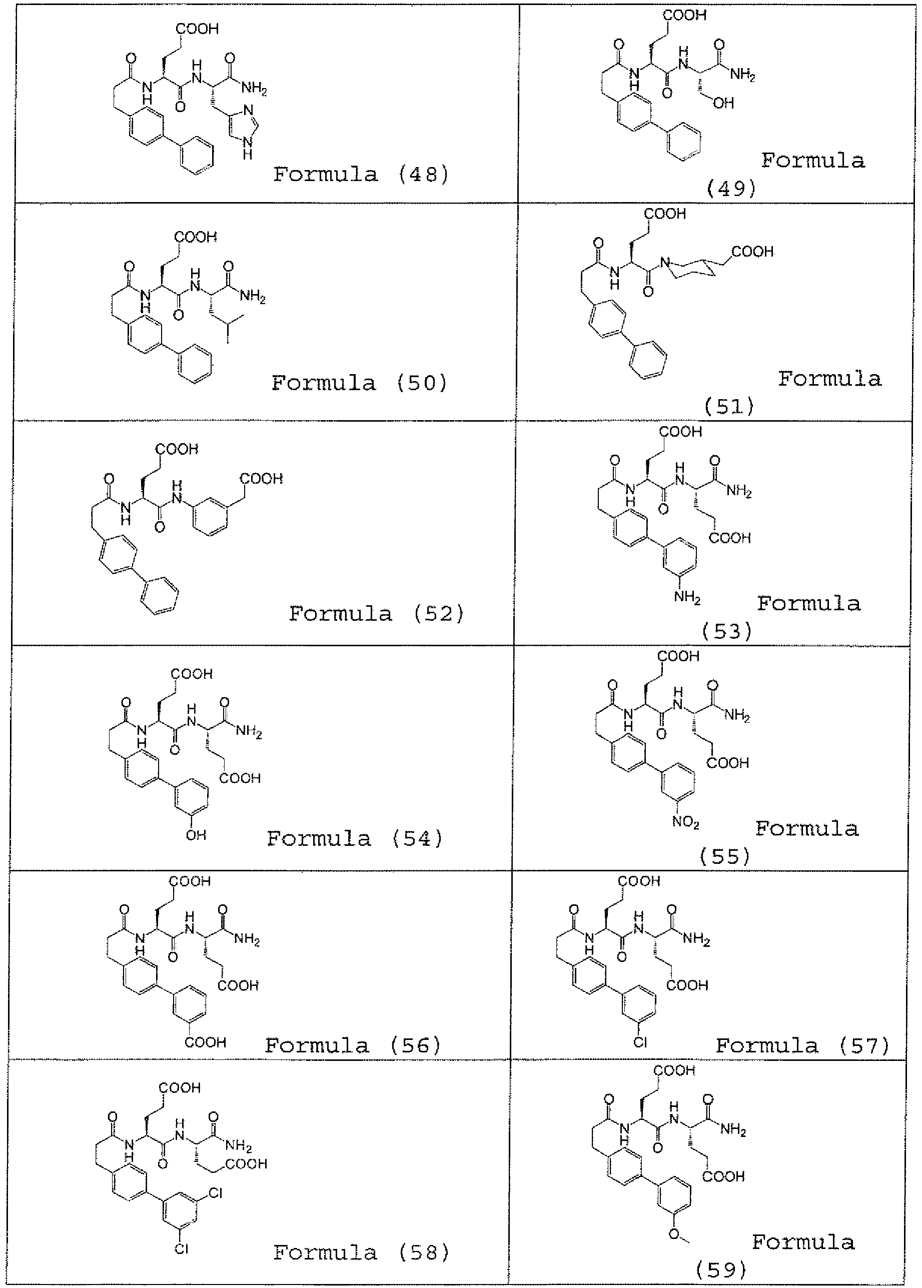

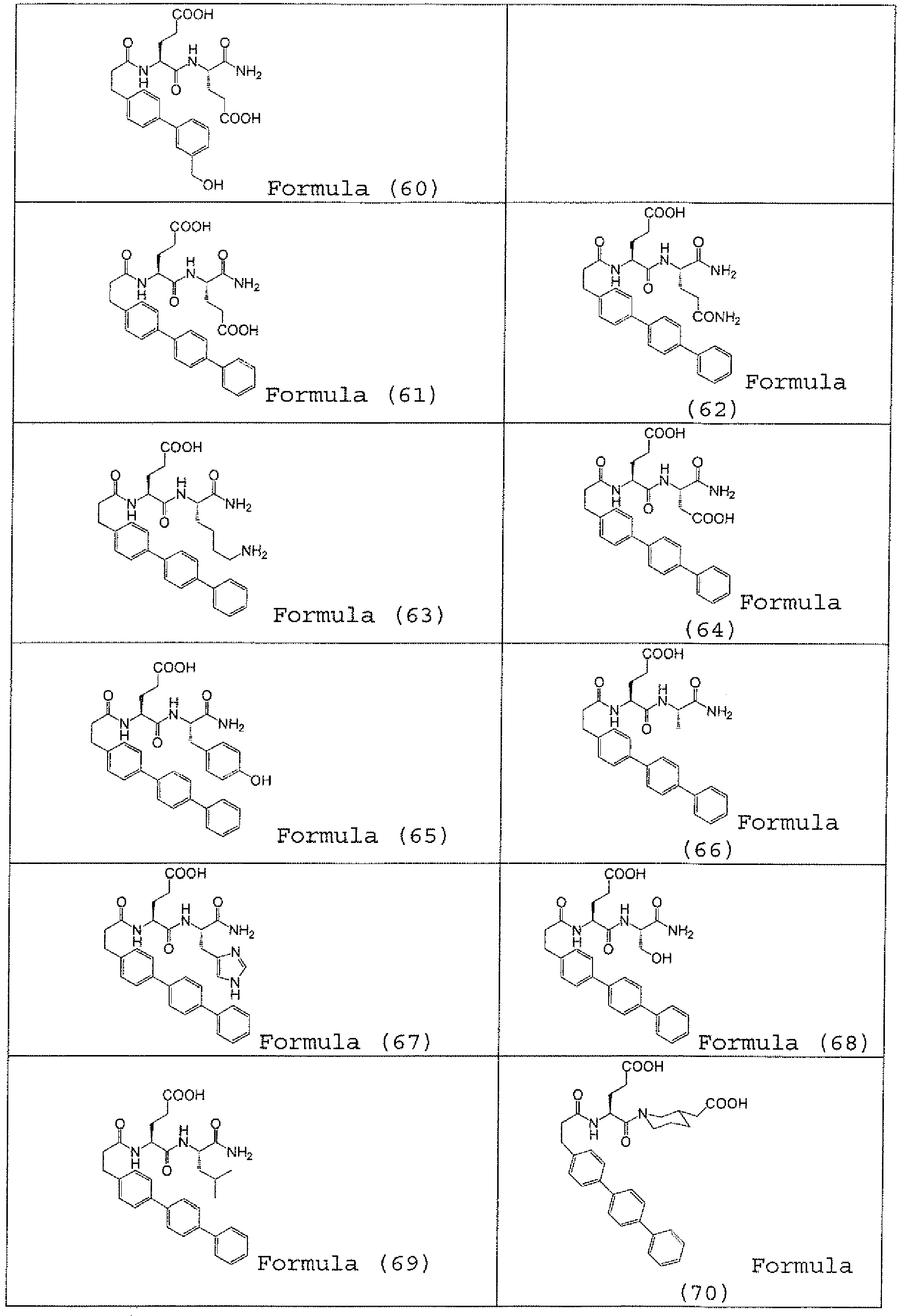

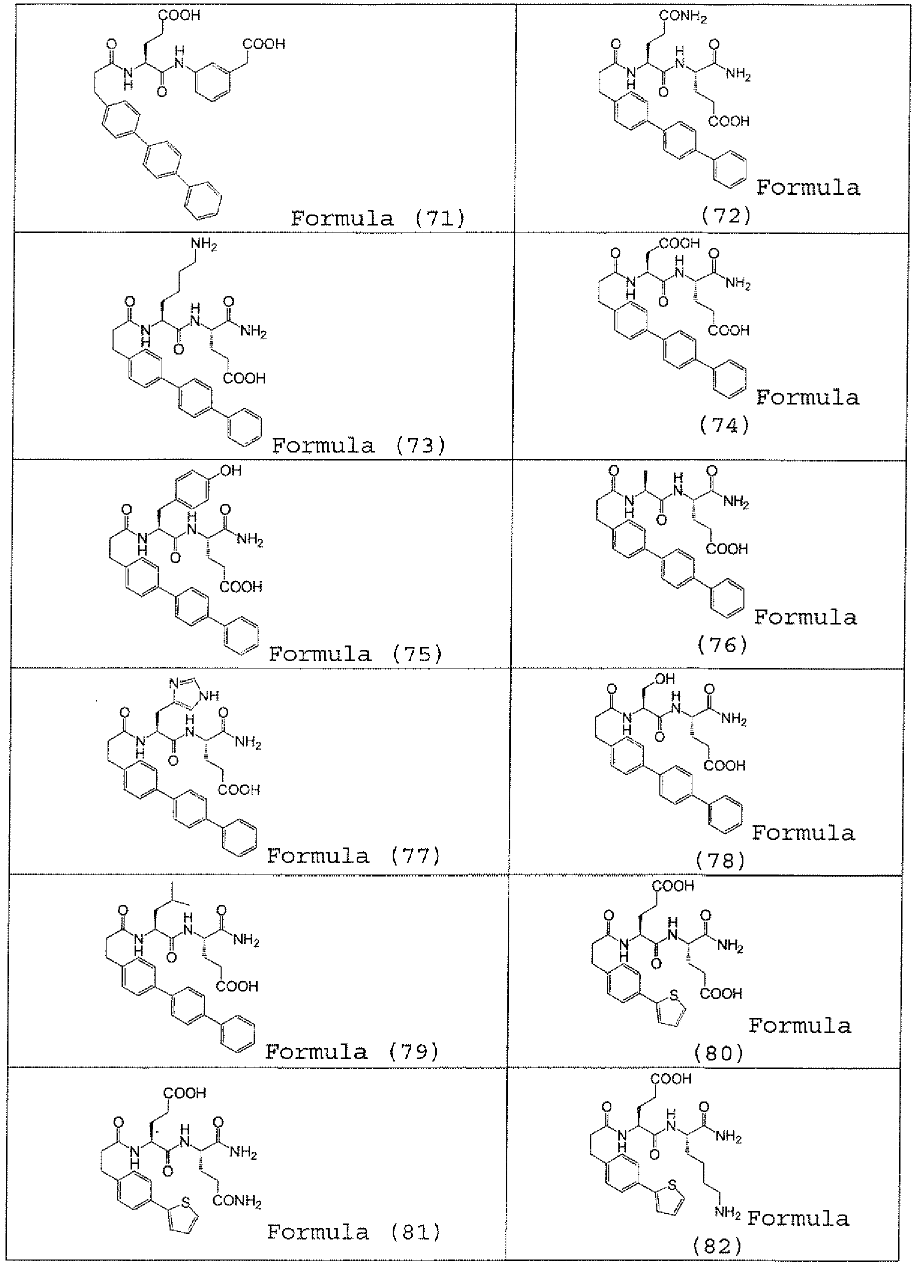

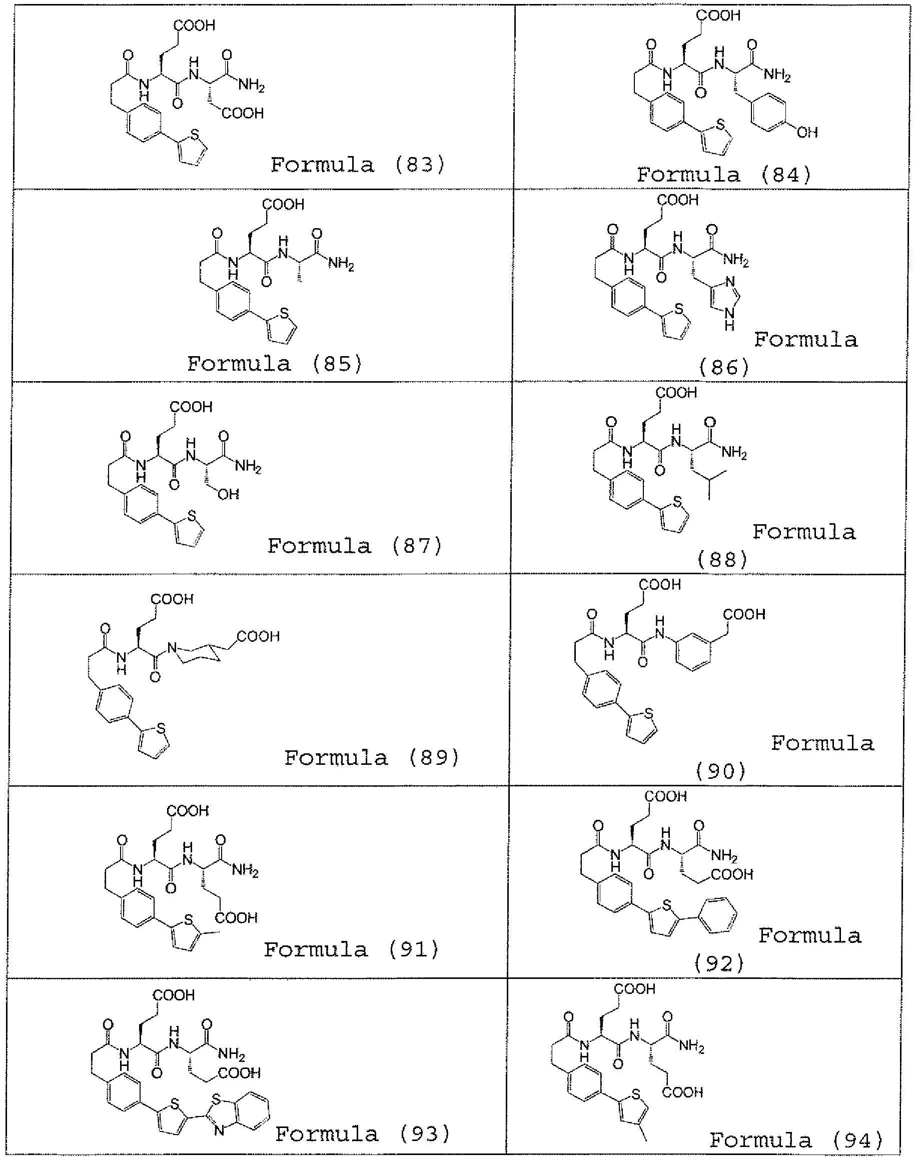

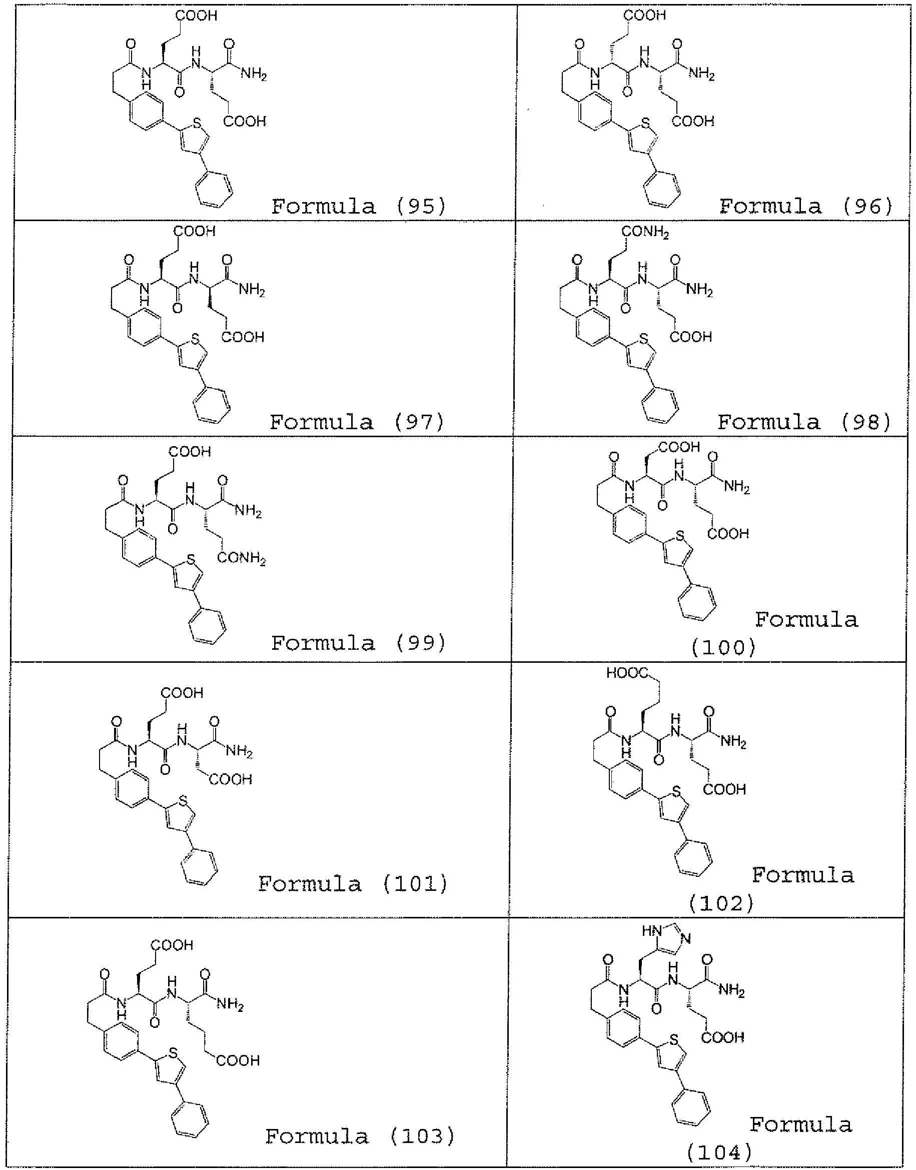

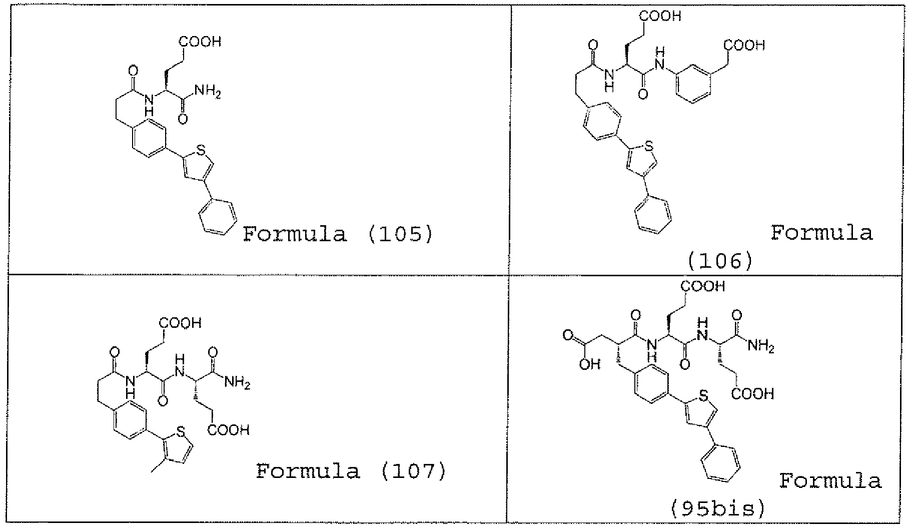

14. Compounds according to any one of the previous claims for use as a medicine.

15. Compounds according to any one of claims 1 to 13 for use as inhibitors of metallo proteinases of the extracellular matrix.

16. Compounds according to any one of claims 10 to 13 for use as inhibitors of metallo proteinase of the extracellular matrix 12, MMP-12.

17. Pharmaceutical composition comprising at least one compound according to any one of claims 1 to 13 and a pharmaceutically acceptable excipient.

18. Compounds according to any one of claims 1 to 13 for use as a medicine for the treatment of cancer, inflammatory diseases; chronic obstructive pulmonary disease (COPD), arthritis, rhumatoid arthritis, atherosclerosis and aneurysm rupture.

19. Compounds with formula (2) below: 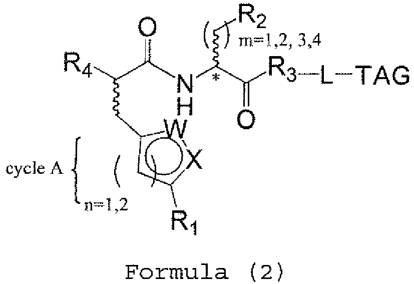 in which:
- R₁, R₂, R₃ and R₄ are as defined in claim 1,
- L is a spacer arm chosen from among the alkyl chains inC₁-C₁₂, and glycol ethers in which the carbonated chain comprises 2 to 12 carbon atoms, and
- TAG is a marker,
the R₃ group being linked to the spacer arm L through a terminal carboxamide function -C(=O)NH₂.

20. Compounds according to claim 19 for use as contrast agents for the detection of metallo proteinases in the extracellular matrix.
